# EUROPEAN PATENT APPLICATION

(11) **EP 1 707 560 A1**
(43) Date of publication of application: **04.10.2006**
(21) Application number: 04807867.9
(22) Date of filing: 27.12.2004
(51) Int. Cl.: C07C 259/06, C07D 209/22, C07D 209/24, C07D 211/58, C07D 213/40, C07D 235/16, C07D 295/18, C07D 401/04, C07D 405/04, A23L 1/30, A61K 8/18, A61K 31/164, A61K 31/165, A61K 31/167, A61K 31/17, A61K 31/18, A61K 31/275, A61K 31/405, A61K 31/4184, A61K 31/4402, A61K 31/4453

(54) **HYDROXAMIC ACID DERIVATIVE AND AGE GENERATION INHIBITOR CONTAINING THE DERIVATIVE**

(30) Priority: 25.12.2003 JP 2003428901
(71) Applicant: Kureha Corporation, Tokyo 103-8552 (JP)
(72) Inventor: KAKUCHI, Junji, 1820001 (JP); YAMAZAKI, Toru, 1240022 (JP); OBARA, Kazumi, 2700127 (JP); YAMATO, Hideyuki, 1210813 (JP)
(74) Representative: HOFFMANN EITLE
(86) International application number: PCT/JP2004/019512
(87) International publication number: WO 2005/073180

(57) **Abstract**

To provide a novel compound which inhibits the generation of AGE and an AGE generation inhibitor containing the compound. A compound represented by the following formula or a pharmaceutically acceptable salt thereof, a medicinal composition containing the compound or such a salt thereof, and an additive composition containing the compound.

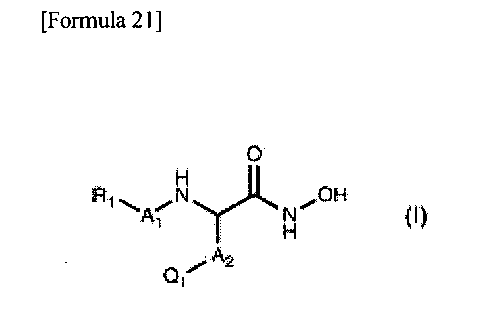

## Description

### Technical Field

The present invention relates to a compound which inhibits the generation of AGE and an AGE generation inhibitor. More specifically, the present invention relates to an AGE generation inhibitor, a medicinal composition used for treating and preventing diseases associated with AGE generation, and an additive for use in foods and cosmetics, comprising the compound as active ingredient, and a cosmetic comprising the compound.

### Background Art

A protein glycosylation reaction, Maillard reaction, is a reaction which was found from the fact that amino acid was heated together with reducing sugar to produce a brown dye (Non-Patent Document 1), and has been considered to be important from a long time ago as a browning reaction in the context of the hue, fragrance, freshness, and quality of food in the field of food chemistry.
The importance thereof in biological reactions has been more recently pointed out: it has been demonstrated that the protein glycosylation reaction occurs in a normal state as well as in a state where hyperglycemia persists, such as diabetes and the reaction product comes to build up in a living body with aging. The buildup of the protein glycosylation reaction product has been further shown to be closely related to the onset and progress of various diseases, which has made the physiological significance thereof extremely important.

The protein glycosylation reaction is divided into the first half reaction and the second half reaction. In the first half reaction, the aldehyde group of a reducing sugar such as glucose is reacted with the N-terminal amino group and side-chain amino group of a protein to form a relatively unstable Schiff base (almidine), which is soon enolized to cause Amadori rearrangement for stabilization to provide a ketoamine, known as an "Amadori compound".
In the subsequent second half reaction, the Amadori compound is subjected to complex reactions such as dehydration, rearrangement, and condensation to produce, via carbonyl compounds such as deoxyglucosone, glyoxal, and methylglyoxal as intermediates, various structures, part of which then form bridges in a protein molecule or between protein molecules. These final products of the protein glycosylation reaction are designated as AGE (Advanced Glycosylation Endproducts) (Non-Patent Document 2).

As the process of formation of AGE becomes more evident, it has been revealed that the formation of the AGE is important not only in the glycosylation reaction but also in the process of oxidation (Non-Patent Document 3). Thus, saccharides, lipids, and amino acids present in a living body provide targets of reactive oxygen species (ROSs) generated in the oxidative stress condition of the living body to generate various highly reactive carbonyl compounds such as glyoxal, arabinose, glycolaldehyde, dehydroascorbic acid, malondialdehyde, hydroxynonenal, and acrolein. These substances react nonenzymatically with the amino group of a protein to produce, in addition to AGE, ALE (Advanced Lipoxidation Endproducts) which proteins modified by lipid-derived carbonyl compounds provide (Non-Patent Documents 4, 5 and 6). Although many carbonyl compounds are thus produced in an oxidative pathway, there are also present carbonyl compounds nonoxidatively generated like 3-deoxyglucosone.

The following structures have been previously reported as AGE or ALE, for example. Pentosidine (Non-Patent Document 7), crossline (Non-Patent Document 8), pyrropyridine (Non-Patent Document 9), pyrraline (Non-Patent Document 10), imidazolone compounds (Non-Patent Documents 11 and 12), glyoxal-derived lysine dimmer (GOLD) (Non-Patent Document 13), methylglyoxal-derived lysine (MOLD) (Non-Patent Document 14), N^{ε} -(carboxymethyl)lysine (CML) (Non-Patent Document 15), Nε-(carbonyethyl)lysine (CEL) (Non-Patent Document 16), X1, fluorolink, argpyrimidine (Non-Patent Document 17), tetrahydropyrimidine (THP) (Non-Patent Document 18), MRX (Non-Patent Document 19), vesperlysine A, acrolein-lysine (Non-Patent Document 20), MDA-lysine (Non-Patent Document 21), and 4-HNE-lysine (Non-Patent Document 22).

These AGE or ALE proteins are known to react with AGE receptors expressed e.g. on vascular endothelial cells, vascular smooth muscle cells, and macrophages to induce various physiological responses. The reported AGE receptors include RAGE (receptor for AGE) (Non-Patent Document 23), SR-A (scavenger receptor class A) (Non-Patent Document 24), scavenger receptor class B (CD36 and SR-BI), galectin-3/OST-48/80K-H (Non-Patent Documents 25 and 26), and LOX-1. The AGE proteins are incorporated by macrophages through the SR-A receptor to induce the bubbling of the cells (Non-Patent Document 27).

The process of recognizing the AGE protein as a ligand or incorporating it in cells before decomposition in lysosomes has been reported to provide a signal, which induces various phenomena in macrophages, vascular endothelial cells and the like. In macrophages, for example, it triggers the secretion of cytokines such as TNF, IL-1, IL-6, IGF-1, and GM-CSF and the proliferation and migration of the cells (Non-Patent Document 28). In vascular endothelial cells, it induces the inhibition ofthrombomodulin activity and, in parallel, the activation of tissue factor. It has been also suggested that it facilitates the subendothelial migration of monocytes and participates in the formation of foam cells associated with early arteriosclerotic lesions.

Further, the AGE protein has been observed to induce the expression of RAGE e.g. on alveolar epithelial cells, blood vessel wall cells such as pericytes, renal mesangial cells, and red blood cells in addition to vascular endothelial cells, smooth muscle cells, and macrophages (Non-Patent Document 29). It has been thought that the RAGE, via AGE protein, induces biological reactions including the migration of smooth muscle cells through TGF-β secretion therefrom and the expression of VEGF on vascular endothelial cells and promotes arteriosclerosis and angiogenesis (Non-Patent Document 30).

It has been recently reported that the administration of the extracellular domain of RAGE (sRAGE) significantly inhibited the onset of arteriosclerosis in apoE knockout mice in which diabetes had been induced (Non-Patent Document 31). Further, an increase in the AGE protein in the blood and a reduction in renal function accompanied by nephropathy were observed in transgenic mice specifically overexpressing RAGE in blood vessels and iNOS in pancreatic β cells, showing nephropathy due to the AGE protein through RAGE (Non-Patent Document 32). It is also thought that the stimulation of AGE receptors on renal mesangial cells by AGE increases the production of extracellular matrixes (Non-Patent Document 33) and enhances the production of PDGF, TGF-β, and the like (Non-Patent Document 34), which, in turn, promotes nephropathy.

In addition, it is thought that AGE and ALE provide important factors in the onset and progression of many other diseases because associations thereof with various pathophysiologies have been indicated: for example, AGE and ALE have been observed to build up in the lens of eye under aging or hyperglycemia, and the buildup and an increase in cataract (Non-Patent Document 35) have been noted to be correlated with each other; an association with neuropathy has been suggested because AGE receptors are also present in nerve cells and βAP or PHF forming fiber is changing into AGE in Alzheimer's disease; an association with the progression of rheumatism or arthritis has been suggested because buildup in arthrodial cartilage inhibits the collagen metabolism of cartilage cells (Non-Patent Documents 36 and 37); an association with reduced dialysis efficiency or with sclerosing peritonitis has been suggested in peritoneal dialysis patients because the peritoneum is changed into AGE by dialysates; and β2M changed into AGE has been demonstrated to be present in the site of amyloid deposition efficiently developed in long-term dialysis patients including hemodialysis (Non-Patent Document 38).

Reported diseases in which AGE or ALE is thus involved include diabetic complications (diabetic neurosis, diabetic retinopathy, and Diabetec Nephrophathy), atherosclerosis, cataract, Alzheimer's disease, rheumatic arthritis, osteoarthritis, chronic renal failure, dialysis amyloidosis, and peritoneal sclerosis, and means resulting in the inhibition of AGE or ALE production are thought to be effective in preventing the onset of these disease and in suppressing the progression thereof.
For the purpose of treating the above-described diseases, many compounds inhibiting the production of AGE or ALE have been reported (Patent Documents 1 to 12), but none of such compounds is in the actual use as AGE generation inhibitor.
In addition, Patent Document 13 discloses that compositions containing compounds having an active nitrogen-containing group (a guanidino group or an amino group bonded to a guanidino group) capable of reacting with an active carbolic group in an Amadori rearrangement product inhibit the production of secondary glycosylated final products and specific examples of such compounds are aminoguanidine, α-hydrazinohistidine, and lysine. However, these compounds were also not in the actual use as AGE generation inhibitors as this case was filed; their side effects and effectiveness are also uncertain.

Patent Document 1: Japanese Patent Laid-Open No. 09-40626
Patent Document 2: Japanese Patent Laid-Open No. 09-59233
Patent Document 3: Japanese Patent Laid-Open No. 09-124471
Patent Document 4: Japanese Patent Laid-Open No. 09-221473
Patent Document 5: Japanese Patent Laid-Open No. 10-158244
Patent Document 6: Japanese Patent Laid-Open No. 10-17954

Patent Document 7: Japanese Patent Laid-Open No. 10-167965
Patent Document 8: Japanese Patent Laid-Open No. 11-124379
Patent Document 9: Japanese Patent Laid-Open No. 2002-256259
Patent Document 10: Japanese Patent Laid-Open No. 2002-255813
Patent Document 11: Japanese Patent Laid-Open No. 2002-302472
Patent Document 12: Japanese Patent No. 3267698
Patent Document 13: Japanese Patent Laid-Open No. 62-142114

Non-Patent Document 1: Maillard L C, C R Acad Sci 154: 66, 1912
Non-Patent Document 2: Brownlee M, N End J Med 318:1315-1321, 1988
Non-Patent Document 3: Miyata T, Nephrol Dial Transplant 12: 255-258, 1997
Non-Patent Document 4: Miyata T, Kidney Int 55:389-399, 1999
Non-Patent Document 5: Esterbauer H, Free Radic Bio Med 11: 81-128, 1991
Non-Patent Document 6: Uchida K, Proc. Natl Acad Sci 95: 4882-4887, 1998
Non-Patent Document 7: Sell DR, J Biol Chem 264: 21597-21602, 1989

Non-Patent Document 8: Nakamura K, J Chem Soc Chem Commun 992-994, 1992
Non-Patent Document 9: Hayase F, J Biol Chem 264: 3758-3764, 1989
Non-Patent Document 10: Njoroge FG, Carbohydr Res 167: 211-220, 1987
Non-Patent Document 11: Konishi Y, Biosci Biotech Biochem 58: 1953-1955, 1994
Non-Patent Document 12: Uchida K FEBS Lett 410: 313-318, 1997
Non-Patent Document 13: Well-Knecht KJ, J Org Chem 60: 6246-6247, 1995
Non-Patent Document 14: Nagaraji RH, J Biol Chem 271: 19338-19345, 1996
Non-Patent Document 15: Ahmed MU, J Biol Chem 261: 4889-4894, 1986

Non-Patent Document 16: Ahmed MU, Biochem J 324: 565-570, 1997
Non-Patent Document 17: Shipanova IN, Arch Biochem Biophys 344: 29-36, 1997
Non-Patent Document 18: Oya T, J Biol Chem 274: 18492-18502, 2000
Non-Patent Document 19: Oya T, Biochem Biophys Res Commum 246: 267-271, 1998
Non-Patent Document 20: Uchida K, J Biol Chem 273: 165058-16066, 1998
Non-Patent Document 21: Requena JR, Biochem J 322: 317-325, 1997
Non-Patent Document 22: Requena JR, Biochem J 322: 317-325, 1997
Non-Patent Document 23: Neeper M, J Biol Chem 267: 14998-15004, 1992

Non-Patent Document 24: Takata K, J Biol Chem 263: 14819-14825, 1988
Non-Patent Document 25: Vlassara H, Molecular Med 6: 634-646, 1995
Non-Patent Document 26: Li Y, Proc Natl Acad Sci USA 93: 11047-11052,1996
Non-Patent Document 27: Jinnouchi Y, J Biochem (Tokyo) 123: 1208-1217, 1998
Non-Patent Document 28: Fu MX, Diabetes 43: 676-683, 1994
Non-Patent Document 29: Schmidt AM, J Biol Chem 267: 14987-14997, 1992
Non-Patent Document 30: Higashi T, Diabetes 46: 463-472, 1997
Non-Patent Document 31: Park L, Nat Med 4: 1025-1031, 1998

Non-Patent Document 32: Yamamoto Y, J Clin Invest 108: 261-268, 2001
Non-Patent Document 33: Doi T, Proc Natl Acad Sci USA 89: 2873-2877, 1992
Non-Patent Document 34: Throckmorton DC, Kidney Int 48: 111-117, 1995
Non-Patent Document 35: Monnier VM, N Engl J Med 314: 403, 1986
Non-Patent Document 36: Bank RA, Biochem J 330: 345-351, 1998
Non-Patent Document 37: Takahashi M, Bri J Rheum 36: 637642, 1997
Non-Patent Document 38: Miyata T, J Clin Invest 92: 1243-1252, 1993

### Disclosure of the Invention

### Problems to be Solved by the Invention

An object of the present invention is to provide a novel compound having an activity of inhibiting AGE generation, particularly a novel compound having an activity of inhibiting AGE generation which can prevent the onset of and suppress the progression of various diseases believed to be attributable to the above-described AGE or ALE, an AGE generation inhibitor containing the compound as active ingredient, and a medicinal composition used for the treatment or prevention of diseases associated with AGE generation.
A further object of the invention is to provide an AGE generation-inhibiting additive for use in cosmetics and foods which contains the compound having an activity of inhibiting AGE generation, and a cosmetic and processed food hardly causing deterioration due to AGE generation.

### Means for Solving the Problems

As the result of synthesizing various compounds in order to find a compound useful for preventing or treating the above-described diseases and studying the AGE generation-inhibiting activities thereof, the present inventors have discovered that a compound represented by general the following formula (I) or a pharmaceutically acceptable salt thereof has an excellent AGE generation-inhibiting activity.
Thus, the present invention provides a hydroxamic acid derivative which is a compound represented by the following formula (I) and a pharmaceutically acceptable salt thereof:

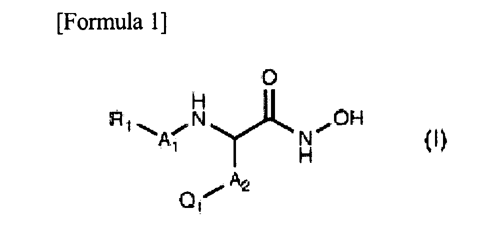

wherein R, represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
Q₁ represents -Y₁-A₃-R₂, an aromatic ring compound group Q₂, a heteroaromatic ring compound group Q₃, or a saturated cyclic compound group Q₄;
Y₁ represents -O-, -S-, -NR₃-, -CONR₃-, -NR₃CO-, -NR₃COO-, -NR₃CONR₄-, - NR₃SO₂-, or -NR₃SO₂NR₄-;
A₃ represents a single bond or an alkylene group having 1 to 6 carbon atoms;
R₂ represents an alkyl group having 1 to 10 carbon atoms, an alkenyl group having 2 to 10 carbon atoms, an aryl group, a heteroaryl group, or a saturated heterocyclic group;
R₃ and R₄ each independently represent a hydrogen atom, an alkyl group, an alkenyl group, an aryl group, a heteroaryl group, a saturated heterocyclic group, or a group selected from the group consisting of an aryl or heteroaryl group and an alkylene group having 1 to 3 carbon atoms; R₂ and R₃ optionally bond together to form a ring;
Q₂ is a group of the following formula (II-a):

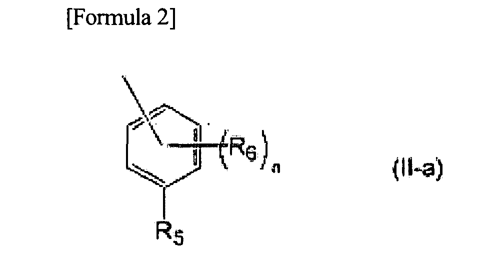

wherein R₅ represents a nitro group, a cyano group, or -Y₂-A₃-R₂;
n represents an integer of 0 to 4;
Y₂ represents a single bond, -O-, -S-, -NR₃-, -CONR₃-, -NR₃CO-, -NR₃COO-, - NR₃CONR₄-, -NR₃SO₂-, or -NR₃SO₂NR₄-;
R₆s are each independently optionally substituted for at any carbon atom on the ring, each independently represent a halogen atom, an alkyl group, a nitro group, a cyano group, -OR₇, -COOR₇, or -CONR₇R₈, and optionally form a ring; and
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;
Q₃ represents a group selected from groups of the following formula (II-b):

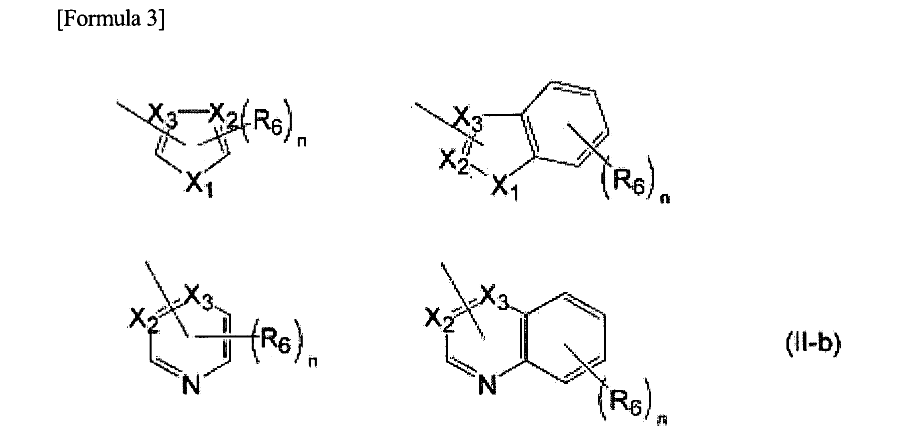

wherein X, represents -O- or -N(-Y₃-A₃-R₂)-; X₂ and X₃ each represent N or CH; Y₃ represents a single bond -CO- or -SO₂-; and
Q₄ represents a 3 to 10- membered hydrocarbon optionally substituted in any position or a cyclic compound containing 1 to 3 nitrogen, oxygen, or sulfur atoms;
provided that the following cases are excluded in which: R₁ is a hydrogen atom, A, is a single bond, A₂ is methylene or ethylene, Q₁ is Q₂, R₅ is Y₂-A₃-R₂, Y₂ is an oxygen atom, A₃ is methylene, and R₂ is phenyl;
R₁ is a hydrogen atom, A₁ is a single bond, A₂ is methylene, Q₁ is Y₁-A₃-R₂, Y₁ is S, and A₃ is ethylene;
R₁ is a hydrogen atom, A₁ is a single bond, A₂ is ethylene, Q₁ is Y₁-A₃-R₂, Y₁ is S, and A₃ is a single bond, and R₂ is ethyl; and
A₂ is methylene, Q₁ is -Y₁-A₃-R₂, Y₁ is NR₃CO, A₃ is ethylene, and R₂ is phenyl.
In addition, the present invention provides a compound of the following formula (III) or a pharmaceutically acceptable salt thereof.

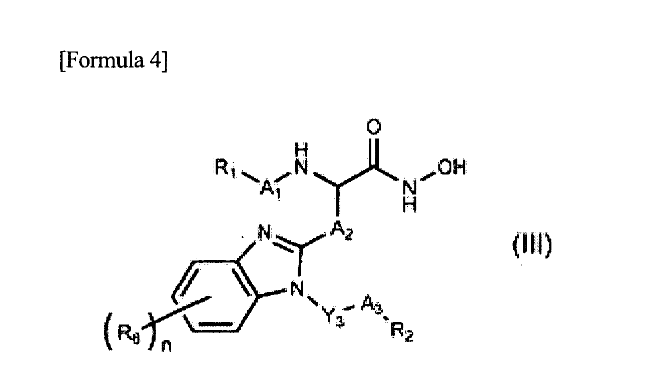

wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₆s are each independently optionally substituted for at any carbon atom on the ring, each independently represent a halogen atom, an alkyl group, a nitro group, a cyano group, -OR₇, -COOR₇, or -CONR₇R₈, and optionally form a ring;
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;

n represents an integer of 0 to 4;
Y₃ represents a single bond, -CO- or -SO₂-;
A₃ represents a single bond or an alkylene group having 1 to 6 carbon atoms; and
R₂ represents an alkyl group having 1 to 10 carbon atoms, an alkenyl group having 2 to 10 carbon atoms, an aryl group, a heteroaryl group, or a saturated heterocyclic group.
The compound of the formula (III) or a pharmaceutically acceptable salt thereof is preferably that in which A₂ represents methylene or ethylene; R₁ represents a hydrogen atom, an alkyl group, or an aryl group; A₁ represents a single bond, methylene, or ethylene; Y₃ represents a single bond; and R₆ represents a hydrogen atom, a halogen atom, or an alkyl group.

The compound of the formula (III) or a pharmaceutically acceptable salt thereof is particularly preferably that in which A₂ represents ethylene; A₁ represents a single bond; and R₁ represents a hydrogen atom.
Further, the invention provides the above-described compound of the formula (III) or a pharmaceutically acceptable salt thereof, wherein the compound has the following groups.
Thus, in the formula (III), R₁ is a hydrogen atom, A₁ is a single bond, A₂ is ethylene, n is 0, Y₃ and A₃ are each a single bond, and R₂ is a propyl group;
R₁ is a hydrogen atom, A₁ is a single bond, A₂ is ethylene, n is 0, Y₃ is a single bond, A₃ is ethylene, and R₂ is a phenyl group;
R₁ is a hydrogen atom, A₁ is a single bond, A₂ is ethylene, n is 0, Y₃ is a single bond, A₃ is methylene, and R₂ is a 4-nitrophenyl group;

R₁ is a hydrogen atom, A₁ is a single bond, A₂ is ethylene, n is 0, Y₃ and A₃ are each a single bond, and R₂ is a pentyl group;
R₁ is a hydrogen atom, A₁ is a single bond, A₂ is ethylene, n is 0, Y₃ is a single bond, A₃ is methylene, and R₂ is a 2-methoxyphenyl group;
R₁ is a hydrogen atom, A₁ is a single bond, A₂ is ethylene, n is 0, Y₃ is a single bond, A₃ is methylene, and R₂ is a pyridin-2-yl group;
R₁ is a hydrogen atom, A₁ is a single bond, A₂ is ethylene, n is 0, Y₃ and A₃ are each a single bond, and R₂ is a butyl group;
R₁ is a hydrogen atom, A₁ is a single bond, A₂ is ethylene, n is 0, Y₃ and A₃ are each a single bond, and R₂ is an octyl group;

R₁ is a hydrogen atom, A₁ is a single bond, A₂ is ethylene, n is 0, Y₃ is a single bond, A₃ is methylene, and R₂ is a cyclohexyl group;
R₁ is a hydrogen atom, A₁ is a single bond, A₂ is ethylene, n is 0, Y₃ and A₃ are each a single bond, and R₂ is a 2,2-dimethylpropyl group;
R₁ is a hydrogen atom, A₁ is a single bond, A₂ is ethylene, n is 0, Y₃ and A₃ are each a single bond, and R₂ is an isobutyl group;
R₁ is a hydrogen atom, A₁ is a single bond, A₂ is ethylene, n is 0, Y₃ is a single bond, A₃ is methylene, and R₂ is a 4-fluorophenyl group;

R₁ is a hydrogen atom, A₁ is a single bond, A₂ is ethylene, n is 0, Y₃ and A₃ are each a single bond, and R₂ is a t-butyl group;
R₁ is a hydrogen atom, A₁ is a single bond, A₂ is ethylene, n is 0, Y₃ and A₃ are each a single bond, and R₂ is a cyclohexyl group;
R₁ is a hydrogen atom, A₁ is a single bond, A₂ is ethylene, n is 0, Y₃ and A₃ are each a single bond, and R₂ is a tetrahydropyran-4-yl group;
R₁ is a hydrogen atom, A₁ is a single bond, A₂ is ethylene, n is 0, Y₃ and A₃ are each a single bond, and R₂ is a 1-methylpiperidin-4-yl group;
R₁ is a hydrogen atom, A₁ is a single bond, A₂ is ethylene, n is 0, Y₃ and A₃ are each a single bond, and R₂ is a 2-methylbutyl group;
R₁ is a hydrogen atom, A₁ is a single bond, A₂ is ethylene, n is 0, Y₃ and A₃ are each a single bond, and R₂ is a hexyl group;
R₁ is a hydrogen atom, A₁ is a single bond, A₂ is ethylene, n is 0, Y₃ and A₃ are each a single bond, and R₂ is a heptyl group;

R₁ is a hydrogen atom, A₁ is a single bond, A₂ is ethylene, n is 0, Y₃ is a single bond, A₃ is 2-methylpropylen-2-yl, and R₂ is a 4-t-butylphenyl group;
R₁ is a hydrogen atom, A₁ is a single bond, A₂ is ethylene, n is 0, Y₃ is a single bond, A₃ is methylene, and R₂ is a naphthalen-1-yl group;
R₁ is a hydrogen atom, A₁ is a single bond, A₂ is ethylene, n is 0, Y₃ is a single bond, A₃ is methylene, and R₂ is a 5-chlorothiophen-2-yl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a cyclohexyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-t-butylphenyl group;

R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-fluorophenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-methoxybenzyl group;
R₁ is a hydrogen atom, A₁ is a single bond, A₂ is ethylene, n is 0, Y₃ and A₃ are each a single bond, and R₂ is a methyl group;
R₁ is a propyl group, A₁ is a single bond, A₂ is ethylene, n is 0, Y₃ and A₃ are each a single bond, and R₂ is a propyl group;

R₁ is a cyclohexyl group, A₁ is methylene, A₂ is ethylene, n is 0, Y₃ and A₃ are each a single bond, and R₂ is a methyl group;
R₁ is a propyl group, A₁ is methylene, A₂ is ethylene, n is 0, Y₃ and A₃ are each a single bond, and R₂ is a methyl group;
R₁ is an octyl group, A₁ is methylene, A₂ is ethylene, n is 0, Y₃ and A₃ are each a single bond, and R₂ is a methyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a propyl group;

R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a propyl group;
R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a pentyl group;
R₁ is a cyclohexyl group, A₁ is methylene, A₂ is ethylene, n is 0, Y₃ and A₃ are each a single bond, and R₂ is a propyl group;
R₁ is a hydrogen atom, A₁ is a single bond, A₂ is ethylene, n is 0, Y₃ is a single bond, A₃ is methylene, and R₂ is a 2,3,5,6-tetrafluoro-4-methoxyphenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a cyclohexyl group;

R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 3,4-difluorophenyl group;
R₁ is a hydrogen atom, A₁ is a single bond, A₂ is ethylene, n is 0, Y₃ and A₃ are each a single bond, and R₂ is a phenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a phenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 4-methylphenyl group;

R₁ is a hydrogen atom, A₁ is a single bond, A₂ is ethylene, n is 2, R₆s are methyl groups present at the 5-and 6-positions, Y₃ and A₃ are each a single bond, and R₂ is a 4-methoxyphenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 4-fluorophenyl group;
R₁ is a hydrogen atom, A₁ is a single bond, A₂ is ethylene, n is 0, Y₃ and A₃ are each a single bond, and R₂ is a 4-methylphenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 3-fluorophenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 3-nitrophenyl group;

R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-trifluoromethylphenyl group;
R₁ is a hydrogen atom, A₁ is a single bond, A₂ is ethylene, n is 2, R₆s are chlorine atoms present at the 5-and 6-positions, Y₃ and A₃ are each a single bond, and R₂ is a phenyl group;
R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a phenyl group;
R₁ is a hydrogen atom, A₁ is a single bond, A₂ is methylene, n is 0, Y₃ and A₃ are each a single bond, and R₂ is a pentyl group;
R₁ is a hydrogen atom, A₁ is a single bond, A₂ is methylene, n is 0, Y₃ and A₃ are each a single bond, and R₂ is a 4-methylphenyl group;

R₁ is a hydrogen atom, A₁ is a single bond, A₂ is methylene, n is 0, Y₃ is a single bond, A₃ is methylene, and R₂ is a 4-t-butylphenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a pentyl group; or
R₁ is a hydrogen atom, A₁ is a single bond, A₂ is ethylene, n is 2, R₆s are methyl groups present at the 5-and 6-positions, Y₃ and A₃ are each a single bond, and R₂ is a 2,2-dimethylpropyl group.
Further, the invention provides a compound of the following formula (IV) or a pharmaceutically acceptable salt thereof

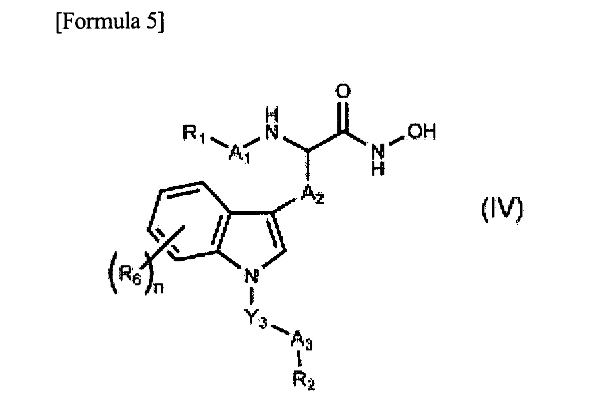

wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₆s are each independently optionally substituted for at any carbon atom on the ring, each independently represent a halogen atom, an alkyl group, a nitro group, a cyano group, -OR₇, -COOR₇, or -CONR₇R₈, and optionally form a ring;
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;

n represents an integer of 0 to 4; Y₃ represents -CO- or -SO₂-; and
A₃ represents a single bond or an alkylene group having 1 to 6 carbon atoms; and R₂ represents an alkyl group having 1 to 10 carbon atoms, an alkenyl group having 2 to 10 carbon atoms, an aryl group, a heteroaryl group, or a saturated heterocyclic group.
The compound of the formula (TV) or a pharmaceutically acceptable salt thereof is preferably that in which R₆ represents a hydrogen atom. In addition, the compound of the formula (IV) or such a salt thereof is preferably that in which R₁ represents a hydrogen atom and A₁ represents a single bond. Further, the compound of the formula (IV) or such a salt thereof is preferably that in which Y₃ represents a single bond.
Further, the invention provides a compound of the formula (IV) or a pharmaceutically acceptable salt thereof, wherein the compound has the following groups.

Thus, in the formula (IV), R₁ is a hydrogen atom, A₁ is a single bond, A₂ is methylene, n is 0, Y₃ is a single bond, A₃ is methylene, and R₂ is a 2,4-difluorophenyl group;
R₁ is a hydrogen atom, A₁ is a single bond, A₂ is methylene, n is 0, Y₃ is a single bond, A₃ is methylene, and R₂ is a 4-trifluorophenyl group;
R₁ is a hydrogen atom, A₁ is a single bond, A₂ is methylene, n is 0, Y₃ is a single bond, A₃ is methylene, and R₂ is a 4-nitrophenyl group;
R₁ is a hydrogen atom, A₁ is a single bond, A₂ is methylene, n is 0, Y₃ is a single bond, A₃ is ethylene, and R₂ is a phenyl group;
R₁ is a hydrogen atom, A₁ is a single bond, A₂ is methylene, n is 0, Y₃ is a single bond, A₃ is methylene, and R₂ is a 2,3,4,5,6-pentafluorophenyl group;
R₁ is a hydrogen atom, A₁ is a single bond, A₂ is methylene, n is 0, Y₃ and A₃ are each a single bond, and R₂ is a pentyl group;
R₁ is a hydrogen atom, A₁ is a single bond, A₂ is methylene, n is 0, Y₃ is a single bond, A₃ is methylene, and R₂ is a 4-t-butylphenyl group;
R₁ is a hydrogen atom, A₁ is a single bond, A₂ is methylene, n is 0, Y₃ is a single bond, A₃ is methylene, and R₂ is a 4-cyanophenyl group;
R₁ is a hydrogen atom, A₁ is a single bond, A₂ is methylene, n is 0, Y₃ is a single bond, A₃ is methylene, and R₂ is a 3,4-difluorophenyl group;
R₁ is a hydrogen atom, A₁ is a single bond, A₂ is methylene, n is 0, Y₃ and A₃ are each a single bond, and R₂ is a methyl group;
R₁ is a hydrogen atom, A₁ is a single bond, A₂ is methylene, n is 0, Y₃ and A₃ are each a single bond, and R₂ is an ethyl group;
R₁ is a hydrogen atom, A₁ is a single bond, A₂ is methylene, n is 0, Y₃ and A₃ are each a single bond, and R₂ is a propyl group;
R₁ is a hydrogen atom, A₁ is a single bond, A₂ is methylene, n is 0, Y₃ and A₃ are each a single bond, and R₂ is a butyl group;
R₁ is a hydrogen atom, A₁ is a single bond, A₂ is methylene, n is 0, Y₃ and A₃ are each a single bond, and R₂ is a hexyl group; or
R₁ is a hydrogen atom, A₁ is a single bond, A₂ is methylene, n is 0, Y₃ and A₃ are each a single bond, and R₂ is a 3-methylbutyl group.
Further, the invention provides a compound of the following formula (V) or a pharmaceutically acceptable salt thereof.

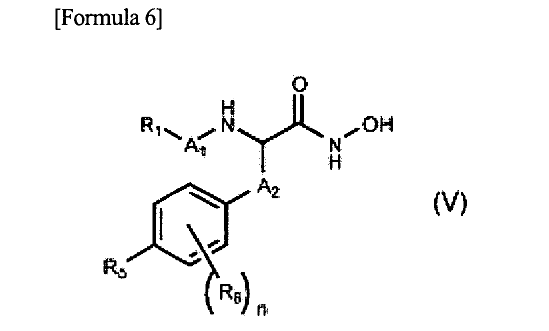

wherein R₁ represents a hydrogen atom, an alkyl group having 1 to 10 carbon atoms, an alkenyl group having 2 to 10 carbon atoms, an aryl group, a heteroaryl group, or a saturated heterocyclic group; A₁ and A₂ each independently represent a single bond or an alkylene group having 1 to 6 carbon atoms; R₅ represents a nitro group, a cyano group, or - Y₂-A₃-R₂; n represents an integer of 0 to 4; Y₂ represents a single bond, -O-, -S-, -NR₃-, - CONR₃-, -NR₃CO-, -NR₃COO-, -NR₃CONR₄-, -NR₃SO₂-, or -NR₃SO₂NR₄-;
R₆s are each independently optionally substituted for at any carbon atom on the ring, each independently represent a halogen atom, an alkyl group, a nitro group, a cyano group, -OR₇, -COOR₇, or -CONR₇R₈, and optionally form a ring; and

R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms.
Further, the invention provides the above-described compound of the formula (V) or a pharmaceutically acceptable salt thereof, wherein A₂ represents methylene and R₆ represents a hydrogen atom.
Further, the invention provides the above-described compound of the formula (V) or a pharmaceutically acceptable salt thereof, wherein the compound has the following groups.

Thus, in the formula (V), R₁ represents a hydrogen atom, A₁ represents a single bond, R₅ represents a nitro group, -NR₉R₁₀, or -OR₉, R₉ and R₁₀ each represent -Y₄-R₁₁, Y₄ represents a single bond, -CO-, -COO-, -CONR₁₂-, or SO₂-, and R₁₁ and R₁₂ each independently represent an alkyl group having 1 to 10 carbon atoms, an aryl group, a heteroaryl group, a saturated heterocyclic group, or a group selected from the group consisting of an aryl or heteroaryl group and an alkylene group having 1 to 3 carbon atoms provided that the following cases are excluded in which: R₅ represents -OR₉, R₉ represents -Y₄R₁₁, and Y₄ represents 0; or R₁₁ represents a benzyl group.
Further, the invention provides the above-described compound of the formula (V) or a pharmaceutically acceptable salt thereof, wherein R₅ is a nitro group.
Further, the invention provides the above-described compound of the formula (V) or a pharmaceutically acceptable salt thereof, wherein the compound has the following groups.

Thus, in the formula (V), R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a methyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a 4-methylphenyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a butyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a t-butyl group;

R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is methylene, and R₂ is a 4-fluorophenyl group;
R₁ is a hydrogen atom, A₁ is a single bond, A₂ is methylene, n is 0, R₅ is -Y₂-A₃-R₂, Y₂ is -NR₃SO₂-, R₃ is a hydrogen atom, A₃ is a single bond, and R₂ is a 4-methylphenyl group;
R₁ is a hydrogen atom, A₁ is a single bond, A₂ is methylene, n is 0, R₅ is -Y₂-A₃-R₂, Y₂ is -NR₃CONR₄-, R₃ and R₄ are each a hydrogen atom, A₃ is a single bond, and R₂ is a 4-trifluoromethylphenyl group;
R₁ is a hydrogen atom, A₁ is a single bond, A₂ is methylene, n is 0, R₅ is -Y₂-A₃-R₂, Y₂ is -NR₃COO-, R₃ is a hydrogen atom, A₃ is a single bond, and R₂ is a methyl group;

R₁ is a hydrogen atom, A₁ is a single bond, A₂ is methylene, n is 0, R₅ is -Y₂-A₃-R₂, Y₂ is -NR₃CONR₄-, R₃ is a hydrogen atom, A₃ is a single bond, and R₂ and R₄ are each a methyl group;
R₁ is a hydrogen atom, A₁ is a single bond, A₂ is methylene, n is 0, R₅ is -Y₂-A₃-R₂, Y₂ is -NR₃-, A₃ is a single bond, and R₂ and R₃ are each a propyl group;
R₁ is a hydrogen atom, A₁ is a single bond, A₂ is methylene, n is 0, R₅ is -Y₂-A₃-R₂, Y₂ is -NR₃-, A₃ is methylene, R₂ is a hydrogen atom, and R₃ is a 4-methylphenyl group;
R₁ is a hydrogen atom, A₁ is a single bond, A₂ is methylene, n is 0, and R₅ is a nitro group;

R₁ is a phenyl group, A₁ is methylene, A₂ is methylene, n is 0, and R₅ is a nitro group;
R₁ is a pyridin-2-yl group, A₁ is methylene, A₂ is methylene, n is 0, and R₅ is a nitro group;
R₁ is a propyl group, A₁ is a single bond, A₂ is methylene, n is 0, and R₅ is a nitro group;
R₁ is a cyclohexyl group, A₁ is methylene, A₂ is methylene, n is 0, and R₅ is a nitro group;

R₁ is a 2-propyl group, A₁ is a single bond, A₂ is methylene, n is 0, and R₅ is a nitro group;
R₁ is a cyclohexyl group, A₁ is a single bond, A₂ is methylene, n is 0, and R₅ is a nitro group;
R₁ is a 1-methylpiperazin-4-yl group, A₁ is a single bond, A₂ is methylene, n is 0, and R₅ is a nitro group;
R₁ is a hydrogen atom, A₁ is a single bond, A₂ is methylene, n is 0, and R₅ is -Y₂₋A₃-R₂, Y₂ is -O-, A₃ is ethylene, and R₂ is a 4-phenyl group;

R₁ is a hydrogen atom, A₁ is a single bond, A₂ is methylene, n is 0, and R₅ is -Y₂₋A₃-R₂, Y₂ is -O-, A₃ is a single bond, and R₂ is a propyl group;
R₁ is a hydrogen atom, A₁ is a single bond, A₂ is methylene, n is 0, and R₅ is -Y₂₋A₃-R₂, Y₂ is -O-, A₃ is ethylene, and R₅ is a morpholin-4-yl group;
R₁ is a hydrogen atom, A₁ is a single bond, A₂ is methylene, n is 0, and R₅ is -Y₂₋A₃-R₂, Y₂ is -O-, A₃ is a single bond, and R₂ is a butyl group;
R₁ is a hydrogen atom, A₁ is a single bond, A₂ is methylene, n is 0, and R₅ is -Y₂₋A₃-R₂, Y₂ is -O-, A₃ is a single bond, and R₂ is a pentyl group;

R₁ is a hydrogen atom, A₁ is a single bond, A₂ is methylene, n is 0, and R₅ is -Y₂₋A₃-R₂, Y₂ is -O-, A₃ is methylene, and R₂ is a 4-fluorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a naphthalen-2-yl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2-chlorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-trifluoromethylphenyl group;

R₁ is a hydrogen atom, A₁ is a single bond, A₂ is methylene, n is 0, and R₅ is -Y₂₋A₃-R₂, Y₂ is -O-, A₃ is methylene, and R₂ is a 2,3,4,5,6-pentafluorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-t-butylphenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2-biphenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-nitrophenyl group;

R₁ is a hydrogen atom, A₁ is a single bond, A₂ is methylene, n is 0, and R₅ is -Y₂₋A₃-R₂, Y₂ is -O-, A₃ is methylene, and R₂ is a 2,4-difluorophenyl group; or
R₁ is a hydrogen atom, A₁ is a single bond, A₂ is methylene, n is 0, and R₅ is -Y₂₋A₃-R₂, Y₂ is -O-, A₃ is methylene, and R₂ is a 4-cyanophenyl group.
Further, the invention provides a compound of the following formula (VI) or a pharmaceutically acceptable salt thereof.

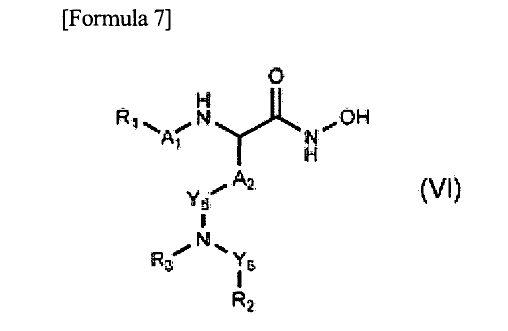

wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₂ represents an alkyl group having 1 to 10 carbon atoms, an alkenyl group having 2 to 10 carbon atoms, an aryl group, a heteroaryl group, or a saturated heterocyclic group;
Y₅ represents a single bond or a carbonyl group; Y₆ represents a single bond, -CO-, -COO-, -CONR₄-, or -SO₂-; and
R₃ and R₄ each represent a hydrogen atom, an alkyl group, an alkenyl group, an aryl group, a heteroaryl group, a saturated heterocyclic group, or a group selected from the group consisting of an aryl or heteroaryl group and an alkylene group having 1 to 3 carbon atoms.
Further, the invention provides the above-described compound of the formula (VI) or a pharmaceutically acceptable salt thereof, wherein A₂ represents ethylene or propylene. Further, the invention provides the above-described compound of the formula (VI) or a pharmaceutically acceptable salt thereof, wherein R₁ represents a hydrogen atom and A₁ represents a single bond.

Further, the invention provides the above-described compound of the formula (VI) or a pharmaceutically acceptable salt thereof, wherein the compound has the following groups.
Thus, in the formula (VI), R₁ is a hydrogen atom, A₁ is a single bond, A₂ is ethylene, Y₅ is a carbonyl group, Y₆ is a single bond, R₃ is a hydrogen atom, and R₂ is a naphthalen-1-ylmethyl group;
R₁ is a hydrogen atom, A₁ is a single bond, A₂ is ethylene, Y₅ is a carbonyl group, Y₆ is a single bond, R₃ is a hydrogen atom, and R₂ is a 4-t-butylphenyl group;
R₁ is a hydrogen atom, A₁ is a single bond, A₂ is ethylene, Y₅ is a carbonyl group, Y₆ is a single bond, R₃ is a hydrogen atom, and R₂ is a 4-fluorobenzyl group;

R₁ is a hydrogen atom, A₁ is a single bond, A₂ is ethylene, Y₅ is a carbonyl group, Y₆ is a single bond, R₃ is a hydrogen atom, and R₂ is a 4-t-butylbenzyl group;
R₁ is a hydrogen atom, A₁ is a single bond, A₂ is ethylene, Y₅ is a carbonyl group, Y₆ is a single bond, R₃ is a hydrogen atom, and R₂ is a cyclohexyl group;
R₁ is a hydrogen atom, A₁ is a single bond, A₂ is ethylene, Y₅ is a carbonyl group, Y₆ is a single bond, R₃ is a hydrogen atom, and R₂ is a 2,2-diphenylethyl group;
R₁ is a hydrogen atom, A₁ is a single bond, A₂ is ethylene, Y₅ is a carbonyl group, Y₆ is a single bond, R₃ is a hydrogen atom, and R₂ is a pyridin-2-ylmethyl group;

R₁ is a hydrogen atom, A₁ is a single bond, A₂ is ethylene, Y₅ is a carbonyl group, Y₆ is a single bond, R₃ is a methyl group, and R₂ is a benzyl group;
R₁ is a hydrogen atom, A₁ is a single bond, A₂ is ethylene, Y₅ is a carbonyl group, Y₆ is a single bond, R₃ is an ethyl group, and R₂ is an ethyl group;
R₁ is a hydrogen atom, A₁ is a single bond, A₂ is ethylene, Y₅ is a carbonyl group, Y₆ is a single bond, and R₂ and R₃ are each a pentylene group and form a ring;
R₁ is a hydrogen atom, A₁ is a single bond, A₂ is ethylene, Y₅ is a carbonyl group, Y₆ is a single bond, R₃ is a hydrogen atom, and R₂ is an adamantan-1-yl group;

R₁ is a hydrogen atom, A₁ is a single bond, A₂ is ethylene, Y₅ is a carbonyl group, Y₆ is a single bond, R₃ is a hydrogen atom, and R₂ is a benzyl group;
R₁ is a hydrogen atom, A₁ is a single bond, A₂ is propylene, Y₅ is a single bond, Y₆ is -CO-, R₃ is a hydrogen atom, and R₂ is a methyl group;
R₁ is a hydrogen atom, A₁ is a single bond, A₂ is propylene, Y₅ is a single bond, Y₆ is -CO-, R₃ is a hydrogen atom, and R₂ is a phenyl group;
R₁ is a hydrogen atom, A₁ is a single bond, A₂ is propylene, Y₅ is a single bond, Y₆ is -CO-, R₃ is a hydrogen atom, and R₂ is a 4-nitrophenyl group;

R₁ is a hydrogen atom, A₁ is a single bond, A₂ is propylene, Y₅ is a single bond, Y₆ is -CO-, R₃ is a hydrogen atom, and R₂ is a 4-nitrobenzyl group;
R₁ is a hydrogen atom, A₁ is a single bond, A₂ is propylene, Y₅ is a single bond, Y₆ is -CO-, R₃ is a hydrogen atom, and R₂ is a naphthalen-1-yl group;
R₁ is a hydrogen atom, A₁ is a single bond, A₂ is propylene, Y₅ is a single bond, Y₆ is -CO-, R₃ is a hydrogen atom, and R₂ is a naphthalen-2-yl group;
R₁ is a hydrogen atom, A₁ is a single bond, A₂ is propylene, Y₅ is a single bond, Y₆ is -CO-, R₃ is a hydrogen atom, and R₂ is a cyclohexylmethyl group;

R₁ is a hydrogen atom, A₁ is a single bond, A₂ is propylene, Y₅ is a single bond, Y₆ is -CO-, R₃ is a hydrogen atom, and R₂ is a 4-chlorophenyl group;
R₁ is a hydrogen atom, A₁ is a single bond, A₂ is propylene, Y₅ is a single bond, Y₆ is -CO-, R₃ is a hydrogen atom, and R₂ is a benzhydryl group;
R₁ is a hydrogen atom, A₁ is a single bond, A₂ is propylene, Y₅ is a single bond, Y₆ is -CO-, R₃ is a hydrogen atom, and R₂ is a 2-phenylbenzyl group;
R₁ is a hydrogen atom, A₁ is a single bond, A₂ is propylene, Y₅ is a single bond, Y₆ is -CO-, R₃ is a hydrogen atom, and R₂ is a 3,5-di-t-butylbenzyl group;

R₁ is a hydrogen atom, A₁ is a single bond, A₂ is propylene, Y₅ is a single bond, Y₆ is -CO-, R₃ is a hydrogen atom, and R₂ is a t-butyl group;
R₁ is a hydrogen atom, A₁ is a single bond, A₂ is propylene, Y₅ is a single bond, Y₆ is a single bond, R₃ is a propyl group, and R₂ is a propyl group;
R₁ is a hydrogen atom, A₁ is a single bond, A₂ is propylene, Y₅ is a single bond, Y₆ is a single bond, R₃ is a pentyl group, and R₂ is a pentyl group;
R₁ is a hydrogen atom, A₁ is a single bond, A₂ is propylene, Y₅ is a single bond, Y₆ is a single bond, R₃ is a hydrogen atom, and R₂ is a cyclohexyl group; or
R₁ is a hydrogen atom, A₁ is a single bond, A₂ is propylene, Y₅ is a single bond, Y₆ is a single bond, R₃ is a 4-methylbenzyl group, and R₂ is a 4-methylbenzyl group.
Further, the invention provides an AGE generation inhibitor comprising a compound of the following formula (VII) or a pharmaceutically acceptable salt thereof:

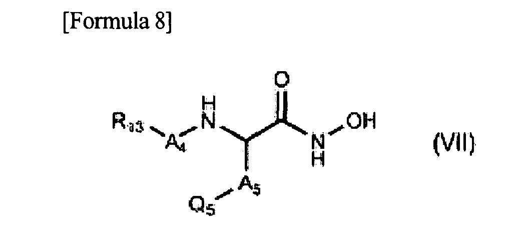

wherein R₁₃ represents an alkyl group having 1 to 10 carbon atoms, an aryl group, a heteroaryl group, or a saturated heterocyclic group;
A₄ and A₅ each independently represent a single bond or an alkylene group having 1 to 6 carbon atoms;
Q₅ represents -Y₇-A₆-R₁₄, an aromatic ring compound group Q₆, a heteroaromatic ring compound group Q₇, or a saturated cyclic compound group Q₈;
Y₇ represents a single bond, -O-, -S-, -NR₁₅-, -CONR₁₅-, -NR₁₅CO-, -NR₁₅COO-, - NR₁₅CONR₁₆-, -NR₁₅SO₂-, or -NR₁₅SO₂NR₁₆-;

A₆ represents a single bond or an alkylene group having 1 to 6 carbon atoms;
R₁₄ represents a hydrogen atom, an alkyl group having 1 to 10 carbon atoms, an aryl group, a heteroaryl group, or a saturated heterocyclic group;
R₁₅ and R₁₆ each represent an alkyl group having 1 to 6 carbon atoms, an aryl group, a heteroaryl group, a saturated heterocyclic group, or a group selected from the group consisting of an aryl or heteroaryl group and an alkylene group having 1 to 3 carbon atoms; R₁₄ and R₁₅ optionally bond together to form a ring;
Q₆ is a group of the following formula (VIII-a):

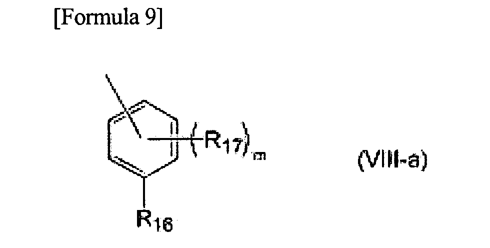

wherein R₁₆ represents a hydrogen atom, a halogen atom, a nitro group, a cyano group, or - Y₇-A₆-R₁₄;
m represents an integer of 0 to 4;
R₁₇ may be each independently substituted for at any atom on the ring, each independently represents a halogen atom, an alkyl group, a nitro group, a cyano group, - OR₁₈, -COOR₁₈, or -CONR₁₈R₁₉, and optionally form a ring; and
R₁₈ and R₁₉ each represent an alkyl group having 1 to 6 carbon atoms, an aryl group, a heteroaryl group, a saturated heterocyclic group;
Q₇ is any of groups of the following formula (VIII-b):

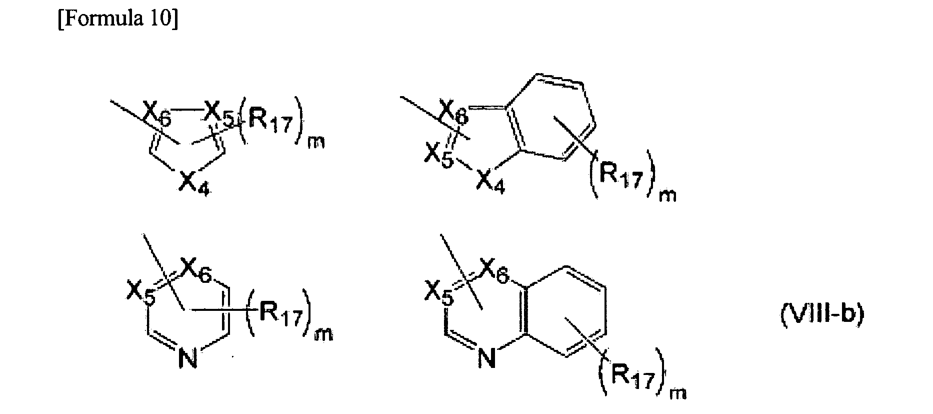

wherein X₄ represents -O-, -S-, or -N(-Y₇-A₆-R₁₄)-; X₅ and X₆ each represent N or CH; and
Q₈ represents a 3 to 10-membered hydrocarbon optionally substituted in any position or a cyclic compound group which can contain 1 to 3 nitrogen, oxygen, and/or sulfur atoms.
Further, the invention provides a medicinal composition, food additive composition, and cosmetic additive composition comprising the above-described AGE generation inhibitor, and a cosmetic and processed food containing the AGE generation inhibitor.
In this regard, the AGE generation inhibitor of the invention may be also used as a Maillard reaction inhibitor or protein modification product inhibitor, and the medicinal composition of the invention may be also used as a therapeutic or preventive agent against diabetic complications and further as a therapeutic or preventive agent against Alzheimer's disease and dialysis amyloidosis.
When an asymmetric carbon is present in the hydroxamic acid derivative molecule of the invention, the compound of the invention or a salt thereof may be the S or R enantiomer thereof or a mixture of both enantiomers mixed at an optional ratio.

As used herein, the term "AGE generation inhibition" refers to suppressing the progress of the protein glycosylation reaction to inhibit the formation of AGE and ALE.
The functional groups as used herein are then described.
For the purpose of this specification, the alkyl group is a generic term applied to functional groups consisting of linear, branched, and cyclic hydrocarbons, and refers to a 1 to 6 carbon group unless otherwise noted. Examples thereof include methyl, ethyl, propyl, isopropyl, butyl, isobutyl, sec-butyl, t-butyl, pentyl, 2-methylbutyl, 2,2-dimethylpropyl, hexyl, cyclopropyl, cyclobutyl, cyclopentyl, cyclohexyl, cyclopropylmethyl, cyclobutylmethyl, cyclopentylmethyl, and cyclohexylmethyl.

The alkylene group is a generic term applied to functional groups consisting of linear, branched, and cyclic hydrocarbons, in each of which connection occurs by the substitution of the two points of the terminal and prefix indication position. Examples thereof include methylene, 1-ethylene, 2-ethylene, 1-propylene, 2-propylene, 3-propylene, 1-butylene, 2-butylene, 3-butylene, and 4-butylene. The aryl group is a generic term applied to aromatic hydrocarbon groups. Examples thereof include benzene, naphthalene, indene, anthracene, and phenanthrene. The heteroaryl group refers to an aromatic cyclic compound having one or more atoms of nitrogen, oxygen, sulfur, or the like. Examples thereof include pyrrole, furan, thiophene, imidazole, thiazole, oxazole, triazole, pyridine, pyrimidine, pyridazine, pyrazine, quinoline, isoquinoline, indole, benzofuran, and benzothiophene.
The aralkyl group is a generic term applied to functional groups consisting of the above-described aryl and alkylene groups. Examples thereof include benzyl, phenethyl, phenylpropyl, 1-naphthylmethyl, and 2-naphthylmethyl. The haloalkyl group is a generic term applied to alkyl groups substituted by an arbitrary number of halogen atoms. Examples thereof include trifluoromethyl, trifluoroethyl, pentafluoroethyl, trifluoropropyl, and heptafluoropropyl. The saturated heterocyclic group is a generic term applied to 3-to 7-membered cyclic alkyl groups containing one to two atoms of oxygen, nitrogen, or sulfur. Examples thereof include oxirane, thiirane, aziridine, tetrahydrofuran, tetrahydropyran, pyrrolidine, piperidine, and piperazine.

The compounds described herein have asymmetric centers derived from the main backbone or side chain thereof; they are distinguished by absolute configuration, which is indicated using the prefix (R)-, (S)-, or (RS)-.
As used herein, the protective group represented by Pₙ, is a conventional protective group employed typically e.g. in peptide chemistry. Examples thereof include a benzyloxycarbonyl group, a t-butoxycarbonyl group, a 9-fluorenyloxymethylcarbonyl group, a benzyl group, a formyl group, and a trityl group for an amino group; a methyl group, an ethyl group, and a benzyl group for a carboxyl group; a formyl group, a trityl group, a benzyl group, and a t-butoxycarbonyl group for a nitrogen atom on an indole ring; and a benzyl group, a trityl group, and an acetyl group for the protective group of an oxygen atom in a hydroxylamine.

### Advantages of the Invention

The compound of the invention can be used to provide an effective AGE generation inhibitor, and a medicinal composition used for treating or preventing diseases associated with AGE generation. The compound of the invention is also useful for use in cosmetics and foods because the deterioration of protein or amino acid due to AGE generation also occurs in cosmetics and foods containing the protein or amino acid. Thus, the invention can provide an AGE generation-inhibiting additive for use in cosmetics and foods, having an effective AGE generation-inhibiting activity, and a cosmetic and processed food hardly causing deterioration due to AGE generation.

### Brief Description of the Drawings

Figure 1 is an illustrative picture of reaction showing Production Example 1 representing a process for producing a compound of the formula (VI) (a straight-chain hydroxamic acid derivative);
Figure 2 is an illustrative picture of reaction showing Production Example 2 representing a process for producing a compound of the formula (VI) (a straight-chain hydroxamic acid derivative);
Figure 3 is an illustrative picture of reaction showing Production Example 3 representing a process for producing a compound of the formula (V) (a phenylalanine type hydroxamic acid derivative);
Figure 4 is an illustrative picture of reaction showing Production Example 4 representing a process for producing a compound of the formula (V) (a phenylalanine type hydroxamic acid derivative);
Figure 5 is an illustrative picture of reaction showing Production Example 5 representing a process for producing a compound of the formula (III) (a benzimidazole type hydroxamic acid derivative);
Figure 6 is an illustrative picture of reaction showing Production Example 6 representing a process for producing a compound of the formula (IV) (a tryptophan type hydroxamic acid derivative);
Figure 7 is an illustrative picture of reaction showing Production Example 7 representing a process for producing a compound of the formula (III) (a benzimidazole type hydroxamic acid derivative);
Figure 8 is an illustrative picture of reaction showing Production Example 8 representing a process for producing a compound of the formula (III) (a benzimidazole type hydroxamic acid derivative);
Figure 9 is an illustrative picture of reaction showing Production Example 9 representing a process for producing a compound of the formula (IV) (a tryptophan type hydroxamic acid derivative);
Figure 10 is an illustrative picture of reaction showing Production Example 10 representing a process for producing a compound of the formula (V) (a phenylalanine type hydroxamic acid derivative);
Figure 11 is an illustrative picture of reaction showing Production Example 11 representing a process for producing a compound of the formula (V) (a phenylalanine type hydroxamic acid derivative);
Figure 12 is an illustrative picture of reaction showing Production Example 12 representing a process for producing a compound of the formula (V) (a phenylalanine type hydroxamic acid derivative); and
Figure 13 is an illustrative picture of reaction showing Production Example 13 representing a process for producing a compound of the formula (V) (a phenylalanine type hydroxamic acid derivative).

### Best Mode for Carrying Out the Invention

Preferable examples of the compounds of the present invention include the followings.
(S)-2-amino-5-[(naphthalen-1-ylmethyl)-aminocarbonyl]-pentanoic acid hydroxyamide
(S)-2-amino-5-(4-t-butylphenylaminocarbonyl)-pentanoic acid hydroxyamide
(S)-2-amino-5-(4-fluorobenzylaminocarbonyl)-pentanoic acid hydroxyamide
(S)-2-amino-5-(4-t-butylbenzylaminocarbonyl)-pentanoic acid hydroxyamide
(S)-2-amino-5-(cyclohexylaminocarbonyl)-pentanoic acid hydroxyamide
(S)-2-amino-5-(2,2-diphenylethylaminocarbonyl)-pentanoic acid hydroxyamide
(S)-2-amino-5-(pyridin-2-ylmethylaminocarbonyl)-pentanoic acid hydroxyamide
(S)-2-amino-5-(N-benzyl-N-methylaminocarbonyl)-pentanoic acid hydroxyamide

(S)-2-amino-5-(diethylaminocarbonyl)-pentanoic acid hydroxyamide
(S)-2-amino-5-(piperidin-1-ylcarbonyl)-pentanoic acid hydroxyamide
(S)-2-amino-5-(adamantan-1-ylaminocarbonyl)-pentanoic acid hydroxyamide
(S)-2-amino-5-(benzylaminocarbonyl)-pentanoic acid hydroxyamide
(S)-2-amino-5-acetylamino-pentanoic acid hydroxyamide
(S)-2-amino-5-benzoylamino-pentanoic acid hydroxyamide
(S)-2-amino-5-(4-nitrobenzoylamino)-pentanoic acid hydroxyamide
(S)-2-amino-5-(4-nitrophenylacetylamino)-pentanoic acid hydroxyamide
(S)-2-amino-5-(naphthalen-1-yl-carbonylamino)-pentanoic acid hydroxyamide
(S)-2-amino-5-(naphthalen-2-yl-carbonylamino)-pentanoic acid hydroxyamide
(S)-2-amino-5-(cyclohexylacetylamino)-pentanoic acid hydroxyamide
(S)-2-amino-5-(4-chlorobenzoylamino)-pentanoic acid hydroxyamide
(S)-2-amino-5-diphenylacetylamino-pentanoic acid hydroxyamide

(S)-2-amino-5-(2-biphenyl-4-ylacetylamino)-pentanoic acid hydroxyamide
(S)-2-amino-5-(3,5-di-t-butylphenylacetylamino)-pentanoic acid hydroxyamide
(S)-2-amino-5-pivaloylaminopentanoic acid hydroxyamide
(S)-2-amino-5-dipropylamino-pentanoic acid hydroxyamide
(S)-2-amino-5-dipentylamino-pentanoic acid hydroxyamide
(S)-2-amino-5-cyclohexylamino-pentanoic acid hydroxyamide
(S)-2-amino-5-[bis-(4-methyl-benzyl)-amino]-pentanoic acid hydroxyamide
(S)-2-amino-3-(4-acetylamino-phenyl)-N-hydroxy-propionamide
(S)-2-amino-3-[4-(4-methylbenzoyl)amino-phenyl]-N-hydroxy-propionamide
(S)-2-amino-3-(4-valeroylamino-phenyl)-N-hydroxy-propionamide
(S)-2-amino-3-(4-pivaloylaminophenyl)-N-hydroxy-propionamide
(S)-2-ammo-3-{4-[2-(4-fluoro-phenyl)-acetylamino]-phenyl}-N-hydroxy-propionamide

(S)-2-amino-3-[4-(toluene-4-ylsulfonylamino)-phenyl]-N-hydroxypropionamide
(S)-2-amino-3-[4-(4-trifluoromethylureido)-phenyl]-N-hydroxy-propionamide
(S)-2-amino-3-(4-methoxycarbonylamino-phenyl)-N-hydroxy-propionamide
(S)-2-amino-3-(4-dimethylaminocarbamoyl-phenyl)-N-hydroxy-propionamide
(S)-2-amino-3-(4-dipropylamino-1-phenyl)-N-hydroxy-propionamide
(S)-2-amino-3-[4-(4-methylbenzyl)amino-phenyl]-N-hydroxy-propionamide
(S)-2-benzylamino-3-(4-nitro-phenyl)-N-hydroxy-propionamide
(S)-2-(pyridin-2-ylmethyl)amino-3-(4-nitro-phenyl)-N-hydroxy-propionamide
(S)-2-propylamino-3-(4-nitro-phenyl)-N-hydroxy-propionamide
(S)-2-cyclohexylmethylamino-3-(4-nitro-phenyl)-N-hydroxy-propionamide
(S)-2-isopropylamino-3-(4-nitro-phenyl)-N-hydroxy-propionamide
(S)-2-cyclohexylamino-3-(4-nitro-phenyl)-N-hydroxy-propionamide

(S)-2-(1-methylpiperidine-4-yl)amino-3-(4-nitro-phenyl)-N-hydroxy-propionamide
(S)-2-amino-N-hydroxy-3-(4-phenethyloxy-phenyl)-propionamide
(S)-2-amino-N-hydroxy-3-(4-propyloxy-phenyl)-propionamide
(S)-2-amino-N-hydroxy-3-{4-[2-(morpholin-4-yl)ethoxy]-phenyl}-propionamide
(S)-2-amino-N-hydroxy-3-(4-butyloxy-phenyl)-propionamide
(S)-2-amino-N-hydroxy-3-(4-pentyloxy-phenyl)-propionamide
(S)-2-amino-N-hydroxy-3-[4-(4-fluorobenzyloxy)-phenyl]-propionamide
(S)-2-amino-N-hydroxy-3-[4-(naphthalen-2-yloxy)-phenyl]-propionamide
(S)-2-amino-N-hydroxy-3-[4-(2-chlorobenzyloxy)-phenyl]-propionamide
(S)-2-amino-N-hydroxy-3-[4-(4-trifluoromethylbenzyloxy)-phenyl]-propionamide
(S)-2-amino-N-hydroxy-3-[4-(2,3,4,5,6-pentafluorobenzyloxy)-phenyl]-propionamide

(S)-2-amino-3-[1-(2,4-difluoro-benzyl)-1H-indol-3-yl]-N-hydroxy-propionamide
(S)-2-amino-3-[1-(4-trifluoromethylbenzyl)-1H-indol-3-yl]-N-hydroxy-propionamide
(S)-2-amino-3-[1-(3-methyl-4-nitrobenzyl)-1H-indol-3-yl]-N-hydroxy-propionamide
(S)-2-amino-3-[1-(2-phenylethyl)-1H-indol-3-yl]-N-hydroxy-propionamide
(S)-2-amino-N-hydroxy-4-(1-propyl-1H-benzimidazol-2-yl)-butyramide
(S)-2-amino-N-hydroxy-4-(1-phenethyl-1H-benzimidazol-2-yl)-butyramide
(S)-2-amino-N-hydroxy-4-[1-(4-nitrobenzyl)-1H-benzimidazol-2-yl]-butyramide
(S)-2-amino-N-hydroxy-4-(1-pentyl-1H-benzimidazol-2-yl)-butyramide
(S)-2-amino-N-hydroxy-4-[1-(2-methoxybenzyl)-1H-benzimidazol-2-yl]-butyramide
(S)-2-amino-N-hydroxy-4-[1-(pyridin-2-ylmethyl)-1H-benzimidazol-2-yl]-butyramide

(S)-2-amino-N-hydroxy-4-(1-butyl-1H-benzimidazol-2-yl)-butyramide
(S)-2-amino-N-hydroxy-4-(1-octyl-1H-benzimidazol-2-yl)-butyramide
(S)-2-amino-N-hydroxy-4-(1-cyclohexylmethyl-1H-benzimidazol-2-yl)-butyramide
(S)-2-amino-N-hydroxy-4-[1-(2,2-dimethylpropyl)-1H-benzimidazol-2-yl]-butyramide
(S)-2-amino-N-hydroxy-4-(1-isobutyl-1H-benzimidazol-2-yl)-butyramide
(S)-2-amino-N-hydroxy-4-[1-(4-fluorobenzyl)-1H-benzimidazol-2-yl]-butyramide
(S)-2-amino-N-hydroxy-4-[1-(4-t-butylbenzyl)-1H-benzimidazol-2-yl]-butyramide
(S)-2-amino-N-hydroxy-4-(1-cyclohexyl-1H-benzimidazol-2-yl)-butyramide
(S)-2-amino-N-hydroxy-4-[1-(tetrahydropyrane-4-yl)-1H-benzimidazol-2-yl]-butyramide
(S)-2-amino-N-hydroxy-4-[1-(1-methyl-piperidine-4-yl)-1H-benzimidazol-2-yl]-butyramide
(S)-2-amino-N-hydroxy-4-[1-(2-methylbutyl)-1H-benzimidazol-2-yl]-butyramide
(S)-2-amino-N-hydroxy-4-(1-hexyl-1H-benzimidazol-2-yl)-butyramide

(S)-2-amino-N-hydroxy-4-(1-heptyl-1H-benzimidazol-2-yl)-butyramide
(S)-2-amino-4-{1-[2-(4-t-butyl-phenyl)-2-methyl-propyl]-1H-benzimidazol-2-yl}-N-hydroxy-butyramide
(S)-2-amino-N-hydroxy-4-[1-(naphthalen-1-ylmethyl)-1H-benzimidazol-2-yl]-butyramide
(S)-2-amino-N-hydroxy-4-[1-(5-chlorothiophene-2-yl)-1H-benzimidazol-2-yl]-butyramide
(S)-2-amino-4-(1-cyclohexyl-5,6-dimethyl-1H-benzimidazol-2-yl)-N-hydroxy-butyramide
(S)-2-amino-4-[1-(4-t-butylbenzyl)-5,6-dimethyl-1H-benzimidazol-2-yl]-N-hydroxy-butyramide
(S)-2-amino-4-[1-(4-fluorobenzyl)-5,6-dimethyl-1H-benzimidazol-2-yl]-N-hydroxy-butyramide
(S)-2-amino-4-[1-(4-methoxybenzyl)-5,6-dimethyl-1H-benzimidazol-2-yl]-N-hydroxy-butyramide
(S)-2-amino-3-(4-amino-phenyl)-N-hydroxy-propionamide
(S)-2-(cyclohexylmethyl-amino)-N-hydroxy-3-(4-nitro-phenyl)-propionamide

(S)-2-amino-4-(1-methyl-1H-benzimidazol-2-yl)-N-hydroxy-butyramide
(S)-2-propylamino-4-(1-propyl-1H-benzimidazol-2-yl)-N-hydroxy-butyramide
(S)-2-(cyclohexylmethyl-amino)-4-(1-methyl-1H-benzimidazol-2-yl)-N-hydroxy-butyramide
(S)-2-propylamino-4-(1-methyl-1H-benzimidazol-2-yl)-N-hydroxy-butyramide
(S)-2-octylamino-4-(1-methyl-1H-benzimidazol-2-yl)-N-hydroxy-butyramide
(S)-2-amino-4-(1-propyl-1H-benzimidazol-2-yl)-N-hydroxy-butyramide
(S)-2-amino-4-(1-pentyl-1H-benzimidazol-2-yl)-N-hydroxy-butyramide
(S)-2-amino-4-(1-octyl-1H-benzimidazol-2-yl)-N-hydroxy-butyramide
(S)-2-amino-4-(1-cyclohexylmethyl-1H-benzimidazol-2-yl)-N-hydroxy-butyramide
(S)-2-amino-4-[1-(4-t-butylbenzyl)-1H-benzimidazol-2-yl]-N-hydroxy-butyramide
(S)-2-amino-4-(5,6-dimethyl-1-propyl-1H-benzimidazol-2-yl)-N-hydroxy-butyramide

(S)-2-amino-4-[1-(4-t-butylbenzyl)-5,6-dimethyl-1H-benzimidazol-2-yl]-N-hydroxy-butyramide
(S)-2-amino-4-(5,6-dichloro-1-propyl-1H-benzimidazol-2-yl)-N-hydroxy-butyramide
(S)-2-amino-4-(5,6-dichloro-1-pentyl-1H-benzimidazol-2-yl)-N-hydroxy-butyramide
(S)-2-(cyclohexylmethyl-amino)-4-(1-propyl-1H-benzimidazol-2-yl)-N-hydroxy-butyramide
(S)-2-amino-4-[1-(2,3,5,6-tetrafluoro-4-methoxy-benzyl)-1H-benzimidazol-2-yl]-N-hydroxy-butyramide
(S)-2-amino-3-(4-propoxy-phenyl)-N-hydroxy-propionamide
(S)-2-amino-3-[4-(4-t-butylbenzyloxy)-phenyl]-N-hydroxy-propionamide
(S)-2-amino-3-[4-(2-phenyl-benzyloxy)-phenyl]-N-hydroxy-propionamide
(S)-2-amino-3-[4-(4-nitro-benzyloxy)-phenyl]-N-hydroxy-propionamide
(S)-2-amino-3-[4-(2,4-difluoro-benzyloxy)-phenyl]-N-hydroxy-propionamide

(S)-2-amino-3-[4-(4-cyano-benzyloxy)-phenyl]-N-hydroxy-propionamide
(S)-2-amino-3-(1-pentafluorophenylmethyl-1H-indol-3-yl)-N-hydroxy-propionamide
(RS)-2-amino-3-(1-pentyl-1H-indol-3-yl)-N-hydroxy-propionamide
(RS)-2-amino-3-[1-(4-t-butyl-benzyl)-1H-indol-3-yl]-N-hydroxy-propionamide (RS)-2-amino-3-[1-(4-cyano-benzyl)-1H-indol-3-yl]-N-hydroxy-propionamide
(RS)-2-amino-3-[1-(3,4-difluoro-benzyl)-1H-indol-3-yl]-N-hydroxy-propionamide
(S)-2-amino-3-(1-pentyl-1H-indol-3-yl)-N-hydroxy-propionamide
(R)-2-amino-3-(1-pentyl-1H-indol-3-yl)-N-hydroxy-propionamide
(R)-2-amino-3-[1-(3,4-difluorobenzyl)-1H-indol-3-yl]-N-hydroxypropionamide

(R)-2-amino-3-(1-methyl-1H-indol-3-yl)-N-hydroxypropionamide
(R)-2-amino-4-(1-cydohexylmethyl-1H-benzimidazol-2-yl)-N-hydroxybutyramide
(R)-2-amino-4-(1-cyclohexylmethyl-5,6-dimethyl-1H-benzimidazol-2-yl)-N-hydroxybutyramide
(S)-2-amino-4-[1-(3,4-difluorobenzyt)-5,6-dimethyl-1H-benzimidazol-2-yl]-N-hydroxybutyramide
(S)-2-amino-4-(1-phenyl-1H-benzimidazol-2-yl)-N-hydroxybutyramide
(R)-2-amino-4-(1-phenyl-5,6-dimethyl-1H-benzimidazol-2-yl)-N-hydroxybutyramide
(R)-2-amino-4-(1-4-methylphenyl-5,6-dimethyl-1H-benzimidazol-2-yl)-N-hydroxybutyramide
(S)-2-amino-4-(1-phenyl-5,6-dimethyl-1H-benzimidazol-2-yl)-N-hydroxybutyramide
(S)-2-amino-4-[1-(4-methylphenyl)-5,6-dimethyl-1H-benzimidazol-2-yl]-N-hydroxybutyramide

(S)-2-amino-4-[1-(4-methoxyphenyl)-5,6-dimethyl-1H-benzimidazol-2-yl]-N-hydroxybutyramide
(S)-2-amino-4-[1-(4-fluorophenyl)-5,6-dimethyl-1H-benzimidazol-2-yl]-N-hydroxybutyramide
(R)-2-amino-4-[1-(4-methylphenyl)-1H-benzimidazol-2-yl]-N-hydroxybutyramide
(S)-2-amino-4-[1-(3-fluorobenzyl)-5,6-dimethyl-1H-benzimidazol-2-yl]-N-hydroxybutyramide
(S)-2-amino-4-[1-(3-nitrophenyl)-5,6-dimethyl-1H-benzimidazol-2-yl]-N-hydroxybutyramide
(S)-2-amino-4-[1-(4-trifluoromethylbenzyl)-5,6-dimethyl-1H-benzimidazol-2-yl]-N-hydroxybutyramide
(S)-2-amino-4-(1-phenyl-5,6-dichloro-1H-benzimidazol-2-yl)-N-hydroxybutyramide

(S)-2-amino-4-(1-benzyl-5,6-dichloro-1H-benzimidazol-2-yl)-N-hydroxybutyramide
(S)-2-amino-3-(1-pentyl-1H-benzimidazol-2-yl)-N-hydroxypropionamide
(S)-2-amino-3-[1-(4-methylphenyl)-1H-benzimidazol-2-yl]-N-hydroxypropionamide
(S)-2-amino-3-[1-(4-t-butylbenzyl)-1H-benzimidazol-2-yl]-N-hydroxypropionamide
(R)-2-amino-3-(1-ethyl-1H-indol-3-yl)-N-hydroxypropionamide
(R)-2-amino-3-(1-propyl-1H-indol-3-yl)-N-hydroxypropionamide
(R)-2-amino-3-(1-butyl-1H-indol-3-yl)-N-hydroxypropionamide
(S)-2-amino-3-(1-ethyl-1H-indot-3-yl)-N-hydroxypropionamide
(S)-2-amino-3-(1-propyl-1H-indol-3-yl)-N-hydroxypropionamide
(S)-2-amino-3-(1-butyl-1H-indol-3-yl)-N-hydroxypropionamide
(S)-2-amino-4-(1-pentyl-5,6-dimethyl-1H-benzimidazol-2-yl)-N-hydroxybutyramide
(S)-2-amino-4-[1-(2,2-dimethylpropyl)-5,6-dimethyl-1H-benzimidazol-2-yl]-N-hydroxybutyramide
(S)-2-amino-3-(1-hexyl-1H-indol-3-yl)-N-hydroxypropionamide
(S)-2-amino-3-[1-(3-methylbutyl)-1H-indol-3-yl]-N-hydroxypropionamide

According to the invention, the above-described compounds can be used in the form of pharmaceutically acceptable salts. The salt is an addition salt with an organic or inorganic acid. Examples of the addition salt with an inorganic acid include hydrofluorides, hydrochlorides, hydrobromides, nitrates, sulfates, hydrogensulfates, phosphates, monohydrogenphosphates, dihydrogenphosphates, and acetates. In this regard, the salt can, for example, also be formed using a base compound such as sodium hydroxide, potassium hydroxide, calcium hydroxide, sodium carbonate, or potassium bicarbonate.
Examples of the addition salt with an organic acid include salts derived from nontoxic organic acids such as monocarboxylic acids of fatty acids, dicarboxylic acids, hydroxyalkanioic acids, hydroxyalkanedioic acids, and amino acids or such as aromatic acids, fatty acids, and aromatic sulfonic acids. Examples thereof include methanesulfonates, sulfamates, tartrates, fumarate, glycolates, citrates, maleates, malates, succinates, acetates, benzoates, ascorbates, p-toluenesulfonates, benzenesulfonates, naphthalenesulfonates, propionates, lactates, pyruvates, oxalates, stearates, cinnamates, aspartates, salicylates, and gluconates.
Such acid addition salts are generally excellent in dispersibility, absorbability, and the like, and have characteristics advantageous for formulation and therapeutics. The acid addition salts are well-known in the art, and can be easily prepared by contacting with a suitable acidic or basic compound.

Then, a process for producing the compound according to the invention is comprehensively exemplified. However, the process for producing the compound of the invention is not intended to be limited to the following Production Examples.

### (Production Example 1)

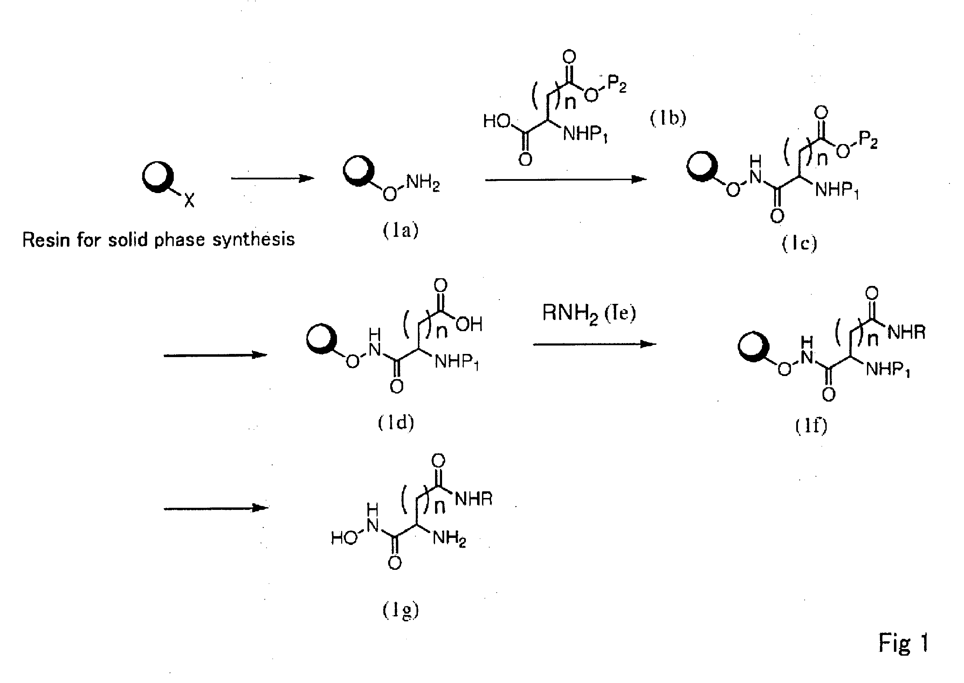

Production Example 1 represents a process for producing a compound of the formula (VI) (a straight-chain hydroxamic acid derivative). The production process will be described based on the illustrative picture of reaction in Figure 1.
Step 1-1
   A commercial leaving group-substituted resin, which is substituted by chlorine groups or the like, (e.g., trityl chloride resin or Merrifield resin) is suspended in an organic solvent such as dimethylformamide (hereinafter referred to as DMF) or tetrahydrofuran (hereinafter referred to as THF), to which a base such as N-hydroxyphthalimide or triethylamine is then added before reaction under shaking at room temperature for 3 to 72 hours, followed by suspending in a DMF solution of hydrazine hydrate, a methanol solution of methylamine, or the like for reaction at room temperature for 3 to 72 hours to provide a desired compound (1a).

Step 1-2
   The compound (1a) obtained in step 1-1 is suspended in an organic solvent such as chloroform or methylene chloride, to which a protective amino acid represented by (1b) (where n is an integer of 1 to 3 and P₁ and P₂ each represent a suitable protective group), a condensation agent such as diisopropylcabodiimide (hereinafter referred to as DIC) or dimethylaminopropylethylcarbodiimide (hereinafter referred to as WSCI) hydrochloride, and an assistant such as 1-hydroxybenzotriazole (hereinafter referred to as HOBt) are then added, followed by shaking at room temperature for 2 to 72 hours to provide a desired compound (1c).
Step 1-3
   In the compound (1c) obtained in step 1-2, the protective group P₂ is removed under appropriate conditions. For example, when P₂ is a methyl group or a benzyl group, a basic aqueous solution of sodium hydroxide, lithium hydroxide, potassium carbonate, or the like is added to the compound in an organic solvent such as methanol or THF, followed by reaction at room temperature for 3 to 72 hours for removing P₂ to provide a compound (1d).
Step 1-4
   The compound (1d) obtained in step 1-3 is suspended in an organic solvent such as DMF, chloroform, or methylene chloride, to which an amine represented by (1e), a condensation agent such as DIC or WSCI hydrochloride, and an assistant such as HOBt are then added, followed by stirring at room temperature for 2 to 72 hours to provide a desired compound (1f).

Step 1-5
   The removal of the protective group P₁ from the compound (1f) obtained in step 1-4 and the cutting-out of the compound from the supporting solid phase are simultaneously or each independently carried out to provide a desired compound (1g). For example, when the protective group is t-butoxycarbonyl group (hereinafter referred to as Boc), the deprotection and cutting-out can be simultaneously accomplished by exposing to a strong acid such as trifluoroacetic acid.

### (Production Example 2)

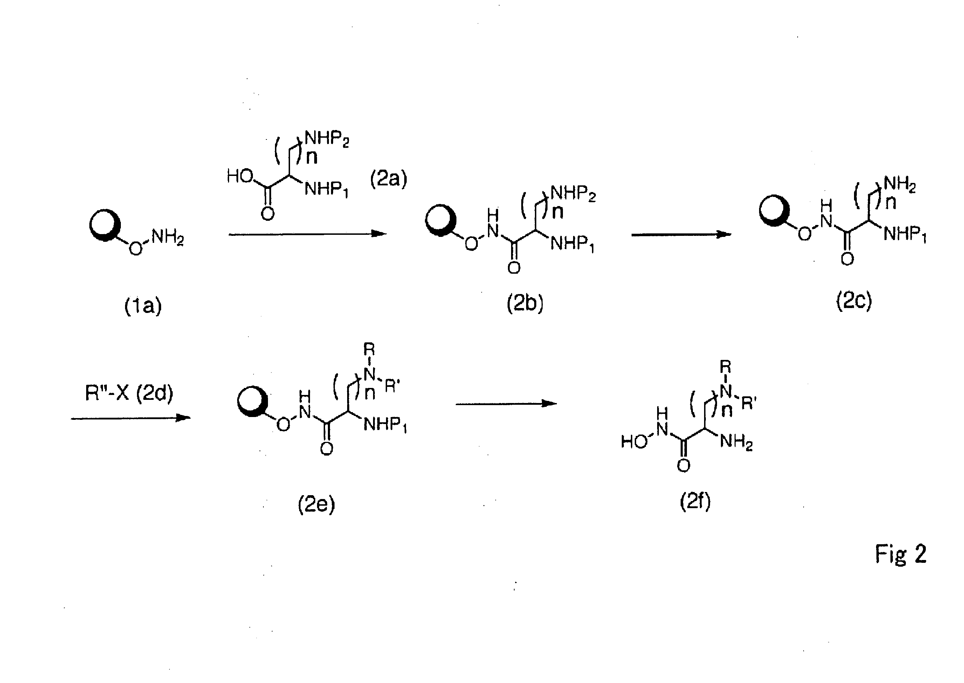

Production Example 2 represents another process for producing a compound of the formula (VI) (a straight-chain hydroxamic acid derivative). The production process will be described based on the illustrative picture of reaction in Figure 2.
Step 2-1
   The compound (1a) obtained in step 1-1 is suspended in an organic solvent such as DMF, chloroform, or methylene chloride, to which a protective amino acid represented by (2a) (where n is an integer of 1 to 4 and P₁ and P₂ each represent a suitable protective group), a condensation agent such as DIC or WSCI hydrochloride, and an assistant such as HOBt are then added, followed by stirring at room temperature for 2 to 72 hours to provide a desired compound (2b).

Step 2-2
   In the compound (2b) obtained in step 2-1, the protective group P₂ is removed under appropriate conditions. For example, when P₂ is a 9-fluorenylmethoxycarbonyl group (hereinafter referred to as Fmoc), 5 to 25% of an organic base such as piperazine, morpholine, or diethylamine is added to the compound in an organic solvent such as DMF, THF or methylene chloride, followed by reaction at room temperature for 1 to 24 hours for removing P₂ to provide a compound (2c).
Step 2-3
   The compound (2c) obtained in step 2-2 is acylated or alkylated. For example, when the compound (2c) is acylated, it is suspended in an organic solvent such as DMF, chloroform, or methylene chloride, to which an acid represented by (2d) (where R" represents alkyl, aryl, or the like and X represents a carboxyl group), a condensation agent such as DIC or WSCI hydrochloride, and an assistant such as HOBt are then added, followed by stirring at room temperature for 2 to 72 hours to provide a desired compound. In the case of alkylation, an aldehyde represented by (2d) (where R represents an alkyl group, an aryl group, or the like and X represents an aldehyde group) and a reducing agent such as sodium borohydride or sodium cyanoborohydride are allowed to react therewith to provide a desired compound (2e).
Step 2-4
   The removal of the protective group P₁ from the compound (2e) obtained in step 2-3 and the cutting-out of the compound from the supporting solid phase are simultaneously or each independently carried out to provide a desired compound (2f). For example, when the protective group is t-butoxycarbonyl group, the deprotection and cutting-out can be simultaneously accomplished by exposing to a strong acid such as trifluoroacetic acid.

### (Production Example 3)

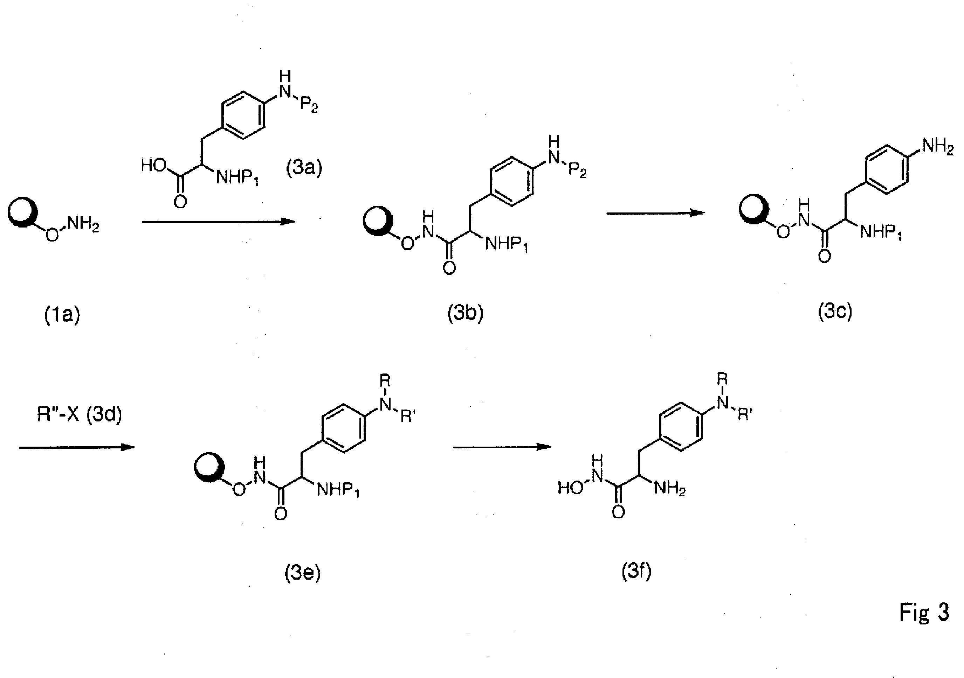

Production Example 3 represents a process for producing a compound of the formula (V) (a phenylalanine type hydroxamic acid derivative). The production process will be described based on the illustrative picture of reaction in Figure 3.
Step 3-1
   The compound (1a) obtained in step 1-1 is suspended in an organic solvent such as DMF, chloroform, or methylene chloride, to which a protective amino acid represented by (3a), a condensation agent such as DIC or WSCI hydrochloride, and an assistant such as HOBt are then added, followed by stirring at room temperature for 2 to 72 hours to provide a desired compound (3b).
Step 3-2
   In the compound (3b) obtained in step 3-1, the protective group P₂ is removed under appropriate conditions. For example, when P₂ is a Fmoc group, 5 to 25% of an organic base such as piperazine, morpholine, or diethylamine is added to the compound in an organic solvent such as DMF, THF or methylene chloride, followed by reaction at room temperature for 1 to 24 hours for removing P₂ to provide a compound (3c).

Step 3-3
   The compound obtained in step 3-2 is acylated or alkylated. In the case of acylation, for example, the compound (3c) can be suspended in an organic solvent such as DMF, chloroform, or methylene chloride, to which an acid represented by (3d) (where R" represents alkyl, aryl, or the like and X represents a carboxyl group), a condensation agent such as DIC or WSCI hydrochloride, and an assistant such as HOBt are added, followed by stirring at room temperature for 2 to 72 hours, or, when (3d) is an acid chloride or an equivalent thereof (where X represents -COCl, -SO₂Cl, or -NCO), can be allowed to react with a base such as triethylamine or dimethylaminopyridine in an organic solvent such as DMF, to provide a desired compound (3e). In the case of alkylation, an aldehyde represented by (3d) (where R represents an alkyl group, an aryl group, or the like and X represents an aldehyde group) and a reducing agent such as sodium borohydride or sodium cyanoborohydride are allowed to react to provide a desired compound (3e).
Step 3-4
   The removal of the protective group P₁ from the compound obtained in step 3-3 and the cutting-out of the compound from the supporting solid phase are simultaneously or each independently carried out to provide a desired compound (3f). For example, when the protective group is t-butoxycarbonyl group, the deprotection and cutting-out can be simultaneously accomplished by exposing to a strong acid such as trifluoroacetic acid.

### (Production Example 4)

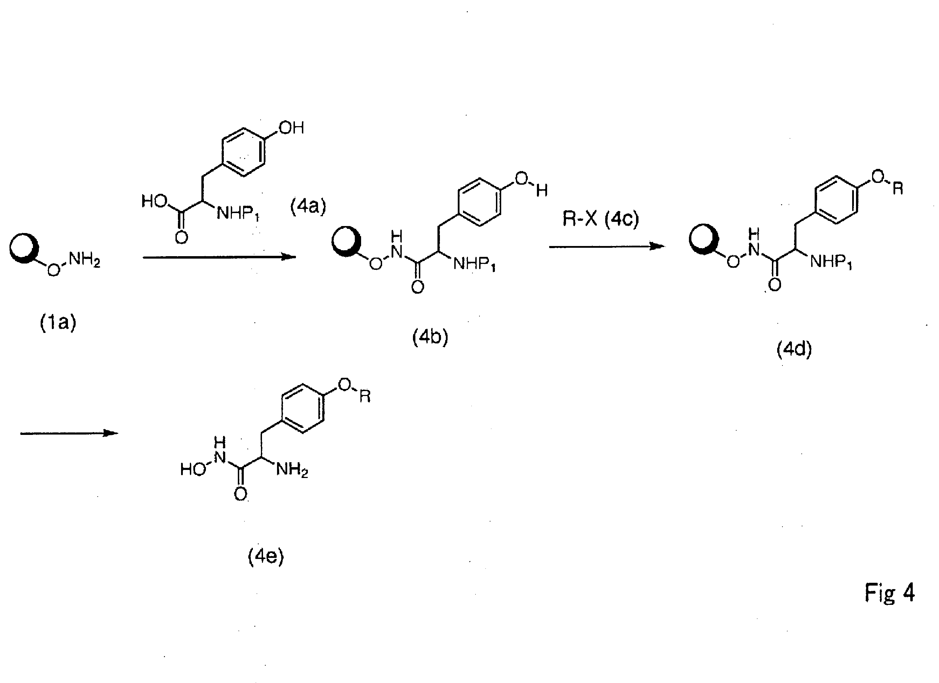

Production Example 4 represents another process for producing a compound of the formula (V) (a phenylalanine type hydroxamic acid derivative). The production process will be described based on the illustrative picture of reaction in Figure 4.
Step 4-1
   The compound (1a) obtained in step 1-1 is suspended in an organic solvent such as DMF, chloroform, or methylene chloride, to which a protective amino acid represented by (4a), a condensation agent such as DIC or WSCI hydrochloride, and an assistant such as HOBt are then added, followed by stirring at room temperature for 2 to 72 hours and further washing under basic conditions to provide a desired compound (4b).

Step 4-2
   The compound (4b) obtained in step 4-1 is acylated or alkylated. For example, the compound (4b) is suspended in an organic solvent such as DMF, chloroform, or methylene chloride, to which a reagent having a leaving group represented by (4c) (where R represents an alkyl group, an aryl group, or the like and X represents a halogen atom, a tosyloxy group, or the like) and a base such as sodium hydride or cesium carbonate are then added, followed by stirring at room temperature for 2 to 72 hours to provide a desired compound (4d).
Step 4-3
   The removal of the protective group P₁ from the compound (4d) obtained in step 4-2 and the cutting-out of the compound from the supporting solid phase are simultaneously or each independently carried out to provide a desired compound (4e). For example, when the protective group is t-butoxycarbonyl group, the deprotection and cutting-out can be simultaneously accomplished by exposing to a strong acid such as trifluoroacetic acid.

### (Production Example 5)

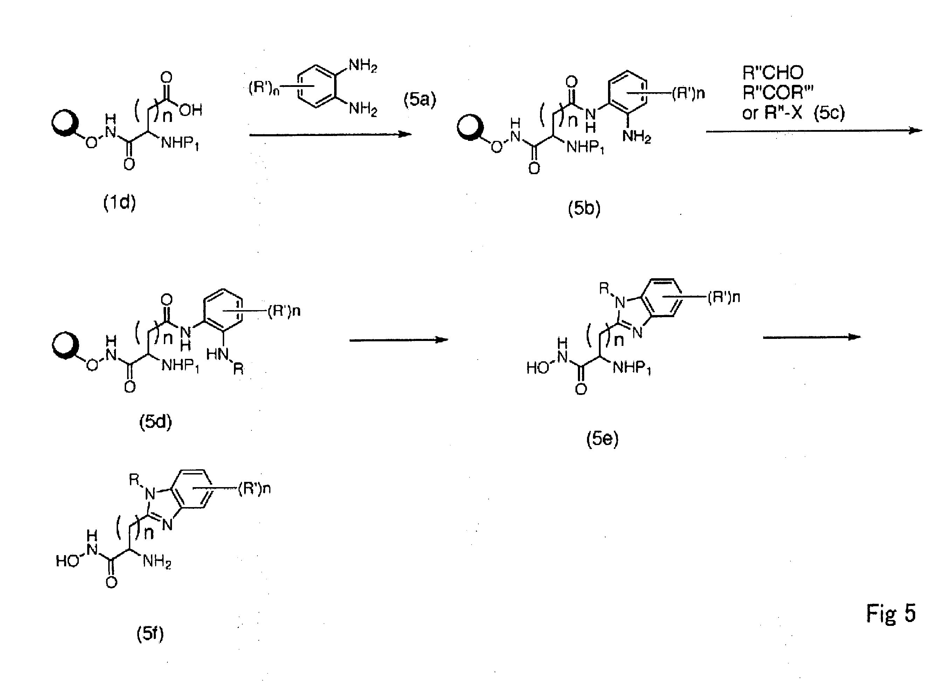

Production Example 5 represents a process for producing a compound of the formula (III) (a benzimidazole type hydroxamic acid derivative). The production process will be described based on the illustrative picture of reaction in Figure 5.
Step 5-1
   The compound (1d) obtained in step 1-3 is suspended in an organic solvent such as DMF, chloroform, or methylene chloride, to which a 1,2-phenylenediamine represented by (5a) (where R' represents an alkyl group, a halogen atom, a carboxyl group, or the like and n represents the number of 1 to 4 groups substituted for in arbitrary positions), a condensation agent such as DIC or WSCI hydrochloride, and an assistant such as HOBt are then added, followed by stirring at room temperature for 2 to 72 hours to provide a desired compound (5b).

Step 5-2
   The compound (5b) obtained in step 5-1 is suspended in an organic solvent such as DMF, chloroform, or methylene chloride, and alkylated using a combination of an aldehyde or ketone represented by (5c) (where R" represents an alkyl or aryl group and, for the ketone, R''' represents a group identical to R" or having the same meaning as a different R") and a reducing agent such as sodium borohydride or sodium cyanoborohydride, or a combination of a reagent having a leaving group represented by (5c) (where R" represents an alkyl group or an aryl group and X represents a halogen atom or a tosyloxy group) and a base such as sodium hydride or cesium carbonate to provide a desired compound (5d).
Step 5-3
   The compound (5d) obtained in step 5-2 is suspended in an organic acid such as acetic acid, followed by stirring at room temperature to 100°C for 1 to 72 hours to provide a desired compound (5e).
Step 5-4
   The protective group P₁ is removed from the compound (5e) obtained in step 5-3 to provide a desired compound. For example, when P₁ is a t-butoxycarbonyl group, a strong acid such as trifluoroacetic acid is allowed to react to provide a desired compound (5f).

### (Production Example 6)

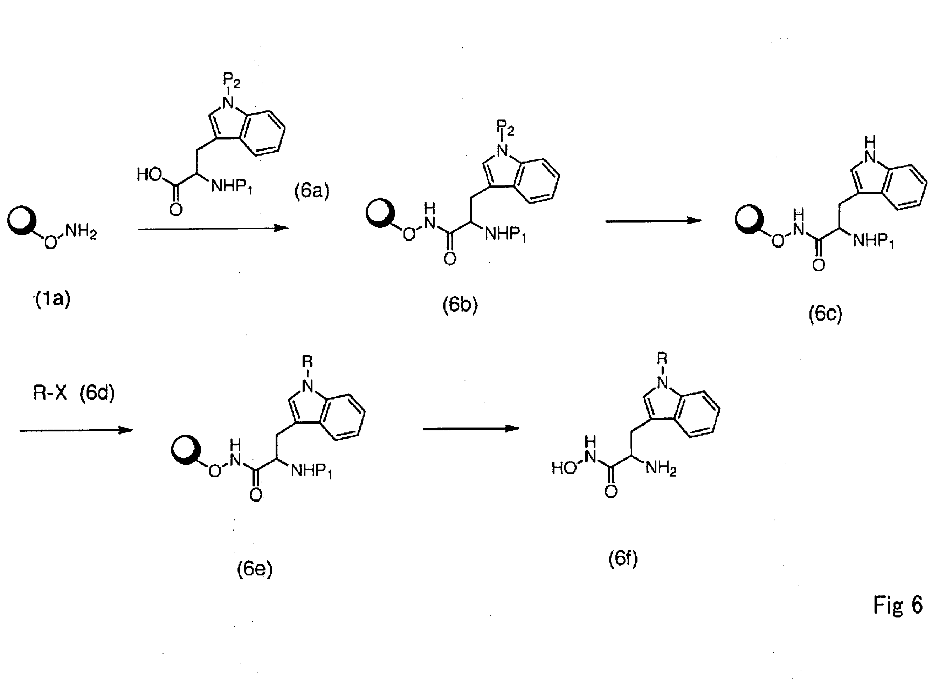

Production Example 6 represents a process for producing a compound of the formula (IV) (a triptophan type hydroxamic acid derivative). The production process will be described based on the illustrative picture of reaction in Figure 6.
Step 6-1
   The compound (1a) obtained in step 1-1 is suspended in an organic solvent such as DMF, chloroform, or methylene chloride, to which a protective amino acid represented by (6a), a condensation agent such as DIC or WSCI hydrochloride, and an assistant such as HOBt are then added, followed by stirring at room temperature for 2 to 72 hours and further washing under basic conditions to provide a desired compound (6b).
Step 6-2
   In the compound (6b) obtained in step 6-1, the protective group P₂ is removed under appropriate conditions. For example, when P₂ is a formyl group, a base such as sodium hydroxide or potassium carbonate is added to the compound in an organic solvent such as DMF, THF or methylene chloride, followed by reaction at room temperature for 1 to 24 hours for removing P₂ to provide a compound (6c).

Step 6-3
   The compound obtained in step 6-2 is alkylated using a combination of a reagent having a leaving group represented by (6d) (where R represents an alkyl group or an aryl group and X represents a halogen atom or a tosyloxy group) and a base such as sodium hydride or cesium carbonate, or a combination of an alcohol represented by (6d) (where R represents an alkyl group, an aralkyl group, an aryl group, or the like and X represents a hydroxyl group), an azodicarboxylate, a triphenylphosphine, and the like to provide a compound (6e).
Step 6-4
   The removal of the protective group P₁ from the compound (6e) obtained in step 6-3 and the cutting-out of the compound from the supporting solid phase are simultaneously or each independently carried out to provide a desired compound (6f). For example, when the protective group is t-butoxycarbonyl group, the deprotection and cutting-out can be simultaneously accomplished by exposing to a strong acid such as trifluoroacetic acid.

### (Production Example 7)

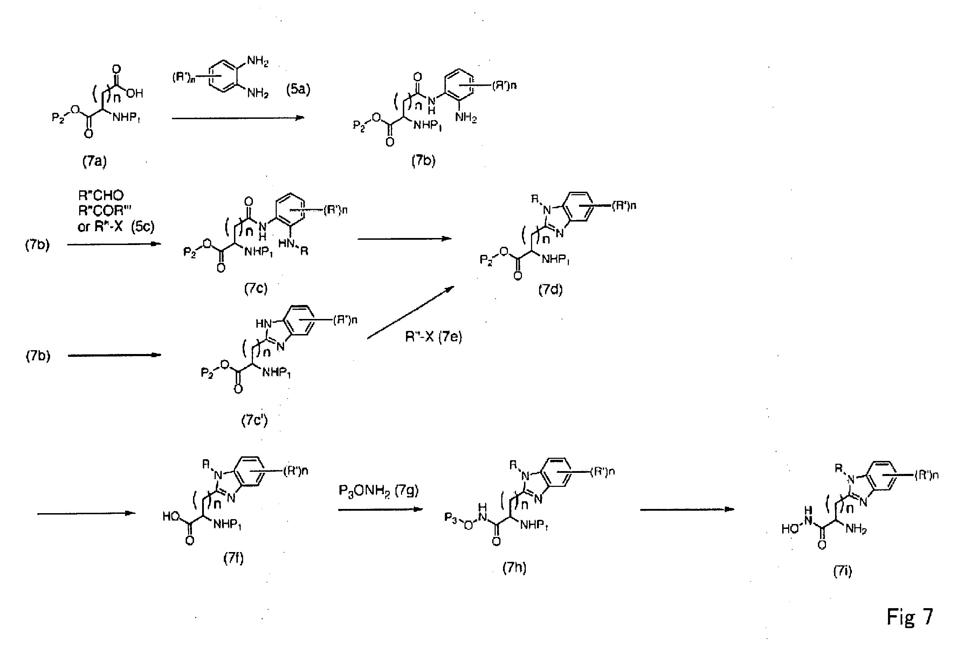

Production Example 7 represents a process for producing a compound of the formula (III) (a benzimidazole type hydroxamic acid derivative). The production process will be described based on the illustrative picture of reaction in Figure 7.
Step 7-1
   A commercial compound represented by (7a) (where P₁ and P₂ each represent a suitable protective group and n represents an integer of 1 to 3) is dissolved in an organic solvent such as chloroform or DMF and mixed with WSCI hydrochloride and HOBt, and the mixture is then added dropwise to a DMF solution of a phenylenediamine derivative represented by (5a) (where each of the substituents is as described above) and stirred at room temperature to provide a desired compound (7b).
Step 7-2a
   The compound (7b) obtained in step 7-1 is suspended in an organic solvent such as DMF, chloroform, or methylene chloride, and alkylated using a combination of an aldehyde or ketone represented by (5c) (where each of the substituents is as described above) and a reducing agent such as sodium borohydride or sodium cyanoborohydride, or a combination of a reagent having a leaving group represented by (5c) (where each of the substituents is as described above) and a base such as sodium hydride or cesium carbonate to provide a desired compound (7c).

Step 7-3a
   The compound (7c) obtained in step 7-2a is dissolved in an organic acid such as acetic acid, followed by stirring at room temperature to 100°C for 1 to 72 hours to provide a desired compound (7d).
Step 7-2b
   The compound obtained in step 7-1 is dissolved in an organic acid such as acetic acid, followed by stirring at room temperature to 100°C for 1 to 72 hours to provide a desired compound (7c').
Step 7-3b
   The compound (7c') obtained in step 7-2b is dissolved in DMF, THF, or the like, to which a reagent having a leaving group represented by (7e) (where R represents an alkyl group, an aryl group, or the like and X represents a halogen atom, a tosyloxy group, or the like) and a base such as sodium hydride are then added, followed by stirring at 0°C to 100°C for 1 to 96 hours to provide a desired compound (7d).
   Alternatively, the compound (7c') obtained in step 7-2b is dissolved in DMF, THF, or the like, to which a reagent having a leaving group represented by (7e) (where R represents an aryl group, an heteroaryl group, or the like and X represents boronic acid or the like) and a catalyst such as pyridine or copper acetate are then added, followed by stirring at 0°C to 100°C for 1 to 168 hours to provide a desired compound (7d).
Step 7-4
   The protective group P₂ is removed from the compound (7d) obtained in step 7-3a or 7-3b to provide a desired compound (7f). For example, when P₂ is an alkyl group or an aralkyl group, it can be removed using a sodium hydroxide aqueous solution or the like in an alcohol; when P₂ is a benzyl group, it can be removed using a palladium catalyst under an atmosphere of hydrogen.

Step 7-5
   The compound (7f) obtained in step 7-4 is suspended in an organic solvent such as DMF, chloroform, or methylene chloride, to which a protective hydroxylamine represented by (7g) (where P₃ represents a suitable protective group such as benzyl or trityl), a condensation agent such as DIC or WSCI hydrochloride, and an assistant such as HOBt are then added, followed by stirring at room temperature for 2 to 72 hours to provide a desired compound (7h).
Step 7-6
   The protective groups P₁ and P₃ of the compound (7h) obtained in step 7-5 are simultaneously or each independently removed to provide a desired compound (7i). For example, when P₁ is Boc and P₃ is a trityl group, the simultaneous removal can be accomplished by exposing to a strong acid such as hydrochloric acid; when P₁ is Boc and P₃ is a benzyl group, P₃ can be removed using a palladium catalyst in a hydrogenated atmosphere, followed by removing P₁ employing a strong acid.

### (Production Example 8)

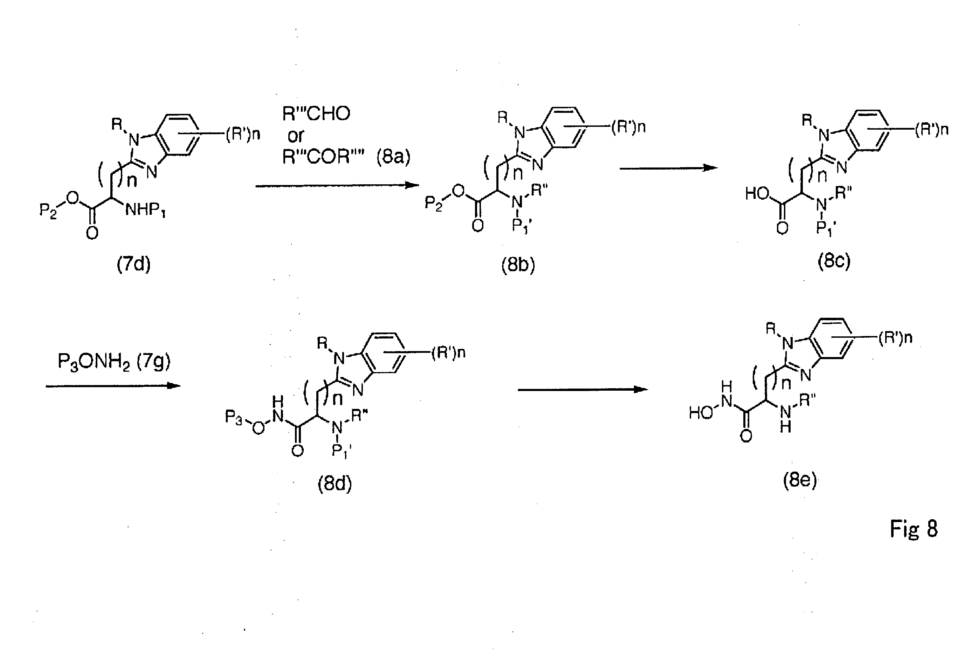

Production Example 8 represents a process for producing a compound of the formula (III) (a benzimidazole type hydroxamic acid derivative). The production process will be described based on the illustrative picture of reaction in Figure 8.
Step 8-1
   P₁ is removed from the compound (7d) obtained in step 7-3a or -3b, into which R" is then introduced using an aldehyde or ketone represented by (8a) (where R''' represents an alkyl or aryl group and, for the ketone, R''' represents a group identical to R" or having the same meaning as a different R") and a reducing agent such as sodium borohydride or sodium cyanoborohydride, followed by further introducing a protective group, P₁, to provide a desired compound (8b).
Step 8-2
   The protective group P₃ is removed from the compound (8b) obtained in step 8-1 to provide a desired compound (8c). For example, when P₃ is an alkyl group or an aralkyl group, it can be removed using a sodium hydroxide aqueous solution or the like in an alcohol; when P₃ is a benzyl group, it can be removed using a palladium catalyst under an atmosphere of hydrogen.

Step 8-3
   The compound (8c) obtained in step 8-2 is suspended in an organic solvent such as DMF, chloroform, or methylene chloride, to which a protective hydroxylamine represented by (7g) (where P₃ is as described above), a condensation agent such as DIC or WSCI hydrochloride, and an assistant such as HOBt are then added, followed by stirring at room temperature for 2 to 72 hours and further washing under basic conditions to provide a desired compound (8d).
Step 8-4
   The protective groups P_{1'} and P₃ of the compound (8d) obtained in step 8-3 are simultaneously or each independently removed to provide a desired compound. For example, when P_{1'} is Boc and P₃ is a trityl group, the simultaneous removal can be accomplished by exposing to a strong acid such as hydrochloric acid; when P_{1'} is Boc and P₃ is a benzyl group, P₃ can be removed using a palladium catalyst in a hydrogenated atmosphere, followed by removing P_{1'} employing a strong acid.

### (Production Example 9)

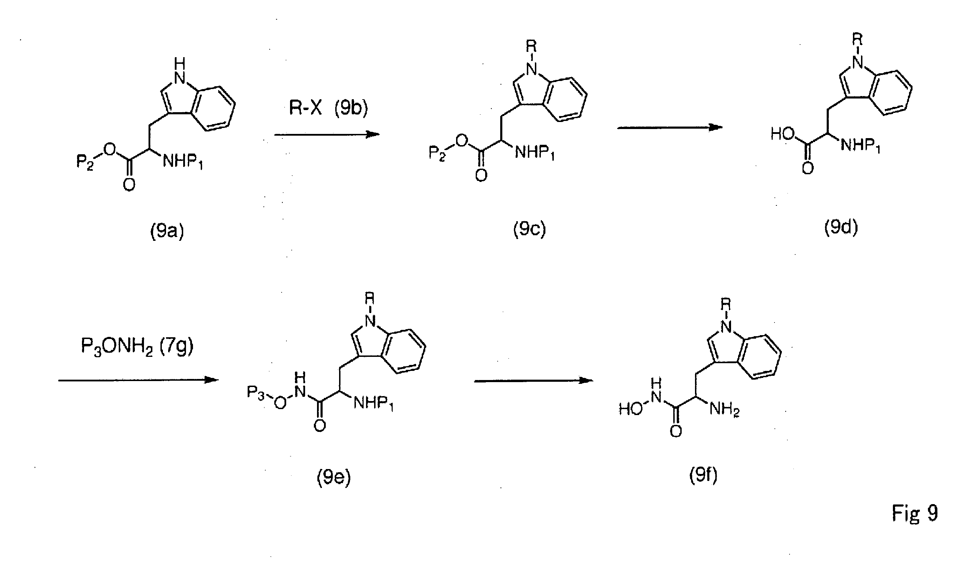

Production Example 9 represents a process for producing a compound of the formula (IV) (a tryptophan type hydroxamic acid derivative). The production process will be described based on the illustrative picture of reaction in Figure 9.
Step 9-1
   A commercial compound represented by (9a) is dissolved in an organic solvent such as DMF or THF, and reacted with a reagent having a leaving group represented by (9b) (where R represents an alkyl group, an aryl group, or the like and X represents a halogen atom, a tosyloxy group, or the like) and a base such as sodium hydride or cesium carbonate at-10°C to 100°C for 0.5 to 72 hours to provide a desired compound (9c).
Step 9-2
   The protective group P₂ is removed from the compound (9c) obtained in step 9-1 to provide a desired compound (9d). For example, when P₂ is an alkyl group or an aralkyl group, it can be removed using a sodium hydroxide aqueous solution or the like in an alcohol; when P₂ is a benzyl group, it can be removed using a palladium catalyst under an atmosphere of hydrogen.

Step 9-3
   The compound (9d) obtained in step 9-2 is suspended in an organic solvent such as DMF, chloroform, or methylene chloride, to which a protective hydroxylamine represented by (7g) (where P₃ is as described above), a condensation agent such as DIC or WSCI hydrochloride, and an assistant such as HOBt are then added, followed by stirring at room temperature for 2 to 72 hours and further washing under basic conditions to provide a desired compound (9e).
Step 9-4
   The protective groups P₁ and P₃ of the compound (9e) obtained in step 9-3 are simultaneously or each independently removed to provide a desired compound (9f). For example, when P₁ is Boc and P₃ is a trityl group, the simultaneous removal can be accomplished by exposing to a strong acid such as hydrochloric acid; when P₁ is Boc and P₃ is a benzyl group, P₃ can be removed using a palladium catalyst in a hydrogenated atmosphere, followed by removing P₁ employing a strong acid.

### (Production Example 10)

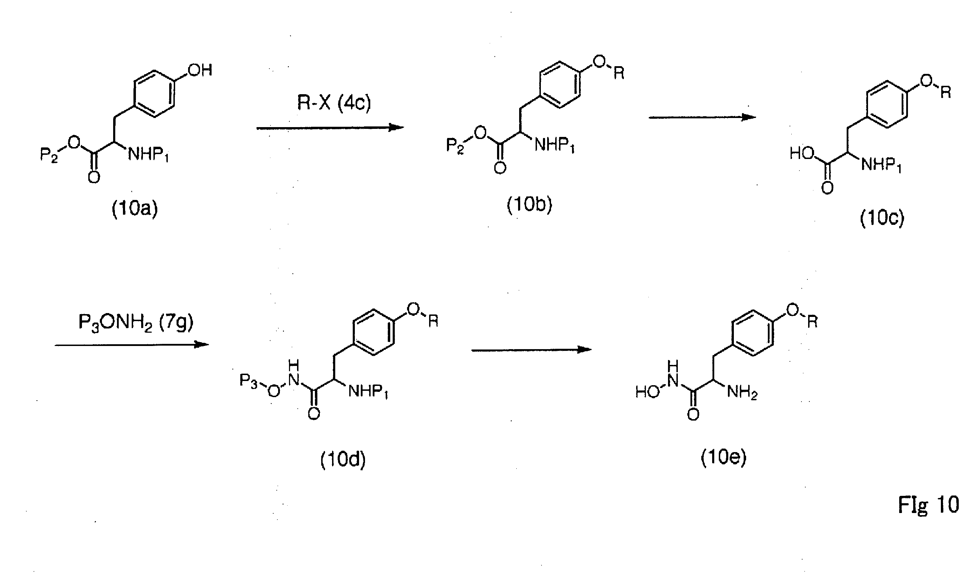

Production Example 10 represents a process for producing a compound of the formula (V) (a phenylalanine type hydroxamic acid derivative). The production process will be described based on the illustrative picture of reaction in Figure 10.
Step 10-1
   A commercial compound represented by (10a) is dissolved in an organic solvent such as DMF or THF, and reacted with a reagent having a leaving group represented by (4c) (where each of the substituents is as described above) and a base such as sodium hydride or cesium carbonate at-10°C to 100°C for 0.5 to 72 hours to provide a desired compound (10b).
Step 10-2
   The protective group P₂ is removed from the compound (10b) obtained in step 10-1 to provide a desired compound (10c). For example, when P₂ is an alkyl group or an aralkyl group, it can be removed using a sodium hydroxide aqueous solution or the like in an alcohol; when P₂ is a benzyl group, it can be removed using a palladium catalyst under an atmosphere of hydrogen.

Step 10-3
   The compound (10c) obtained in step 10-2 is suspended in an organic solvent such as DMF, chloroform, or methylene chloride, to which a protective hydroxylamine represented by (7g), a condensation agent such as DIC or WSCI hydrochloride, and an assistant such as HOBt are then added, followed by stirring at room temperature for 2 to 72 hours and further washing under basic conditions to provide a desired compound (10d).
Step 10-4
   The protective groups P₁ and P₃ of the compound (10d) obtained in step 10-3 are simultaneously or each independently removed to provide a desired compound (10e). For example, when P₁ is Boc and P₃ is a trityl group, the simultaneous removal can be accomplished by exposing to a strong acid such as hydrochloric acid; when P₁ is Boc and P₃ is a benzyl group, P₃ can be removed using a palladium catalyst in a hydrogenated atmosphere, followed by removing P₁ employing a strong acid.

### (Production Example 11)

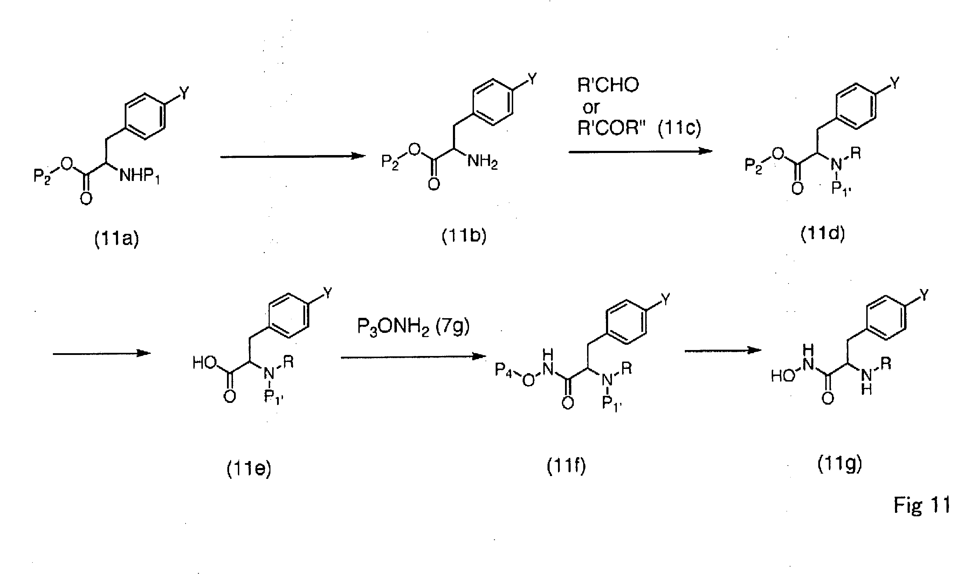

Production Example 11 represents a process for producing a compound of the formula (V) (a phenylalanine type hydroxamic acid derivative). The production process will be described based on the illustrative picture of reaction in Figure 11.
Step 11-1
   A commercial compound (11a) (where P₁ and P₂ each represent a suitable protective group and Y represents a nitro group, a cyano group, an alkoxy group, an alkylamino group, an acylamino group, or the like) is dissolved in an organic solvent, followed by removing the protective group P₁ to provide a desired compound (11b). For example, it is obtained by exposing to a strong acid when P₁ is Boc, and by exposing to an organic base such as piperidine when P₁ is Fmoc.
Step 11-2
   The compound (11b) obtained in step 11-1 is dissolved in an organic solvent such as alcohol or acetonitrile before introducing R using an aldehyde or ketone represented by (11c) (where R' represents an alkyl or aryl group and, for the ketone, R" represents a group identical to R' or having the same meaning as a different R') and a reducing agent such as sodium borohydride or sodium cyanoborohydride, followed by further introducing a protective group, P_{1'} to provide a desired compound (11d).

Step 11-3
   The protective group P₂ is removed from the compound (11d) obtained in step 11-2 to provide a desired compound (11e). For example, when P₂ is an alkyl group or an aralkyl group, it can be removed using a sodium hydroxide aqueous solution or the like in an alcohol.
Step 11-4
   The compound (11e) obtained in step 11-3 is suspended in an organic solvent such as DMF, chloroform, or methylene chloride, to which a protective hydroxylamine represented by (7g), a condensation agent such as DIC or WSCI hydrochloride, and an assistant such as HOBt are then added, followed by stirring at room temperature for 2 to 72 hours to provide a desired compound (11f).
Step 11-5
   The protective groups P_{1'} and P₃ of the compound (11e) obtained in step 11-4 are simultaneously or each independently removed to provide a desired compound (11f). For example, when P_{1'} is Boc and P₃ is a trityl group, the simultaneous removal can be accomplished by exposing to a strong acid such as hydrochloric acid; when P_{1'} is Boc and P₃ is a benzyl group, P₃ can be removed using a palladium catalyst in a hydrogenated atmosphere, followed by removing P_{1'} employing a strong acid.

### (Production Example 12)

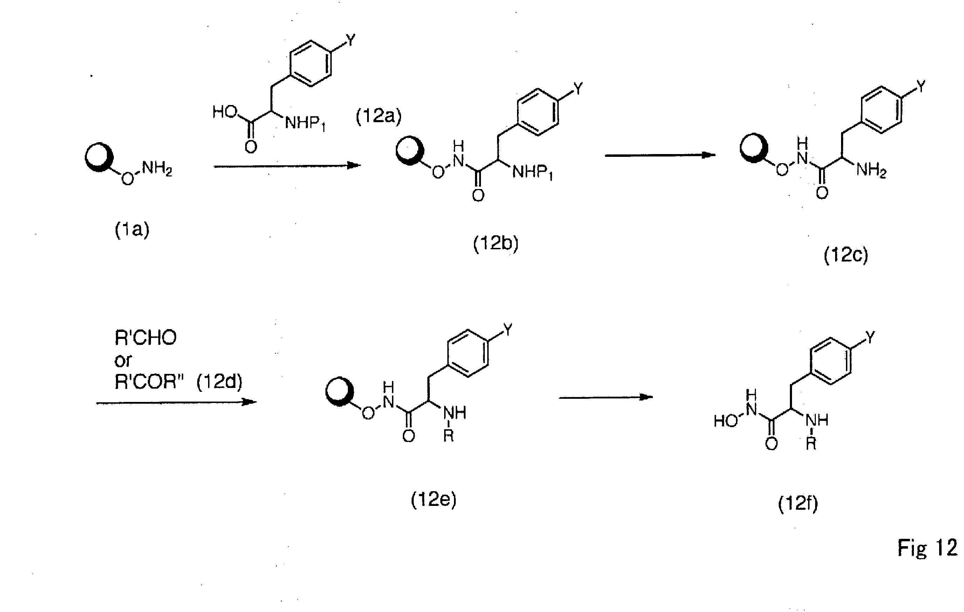

Production Example 12 represents a process for producing a compound of the formula (V) (a phenylalanine type hydroxamic acid derivative). The production process will be described based on the illustrative picture of reaction in Figure 12.
Step 12-1
   The compound obtained in step 1-1 is suspended in an organic solvent such as DMF, chloroform, or methylene chloride, to which a protective amino acid represented by (12a), a condensation agent such as DIC or WSCI hydrochloride, and an assistant such as HOBt are then added, followed by stirring at room temperature for 2 to 72 hours and further washing under basic conditions to provide a desired compound (12b).
Step 12-2
   The protective group P₁ is removed from the compound (12b) obtained in step 12-1 to provide a desired compound (12c). For example, when P₁ is Fmoc, 5 to 25% of an organic base such as piperazine, morpholine, or diethylamine can be added to the compound in an organic solvent such as DMF, THF or methylene chloride, followed by reaction at room temperature for 1 to 24 hours for the removal.

Step 12-3
   The compound (12c) obtained in step 12-2 is dissolved in an organic solvent such as THF or methanol, followed by introducing R using an aldehyde or ketone represented by (12d) (where R' represents an alkyl or aryl group and, for the ketone, R" represents a group identical to R' or having the same meaning as a different R') and a reducing agent such as sodium borohydride or sodium cyanoborohydride to provide a desired compound (12e).
Step 12-4
   The compound (12e) obtained in step 12-3 is detached from a supporting solid phase to provide a desired compound (12f). For example, the desired compound is obtained using 10 to 30% trifluoroacetic acid when the solid phase is of a trityl type, or by exposing e.g., to trifluoroacetic acid/trimethylsilyltriflate/thioanisole when the phase is of a phenylmethylene type.

### (Production Example 13)

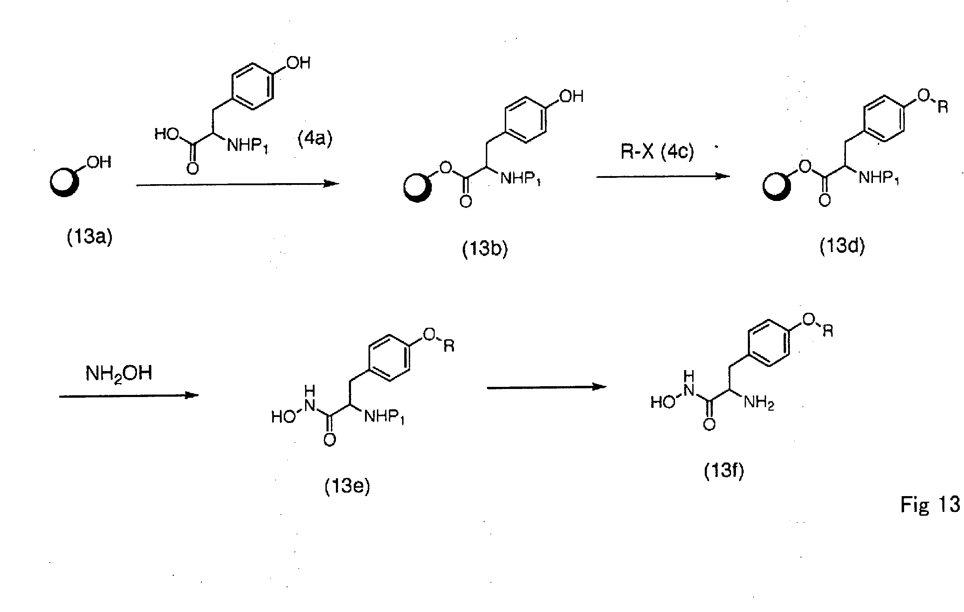

Production Example 13 represents a process for producing a compound of the formula (V) (a phenylalanine type hydroxamic acid derivative). The production process will be described based on the illustrative picture of reaction in Figure 13.
Step 13-1
   A commercial hydroxy type resin represented by (13a) (e.g., Wang resin or oxime resin) is suspended in an organic solvent such as DMF, to which a commercial protective amino acid derivative represented by (4a) (where P1 represents a suitable protective group), a condensation agent such as DIC or WSCI hydrochloride, and an assistant such as HOBt are then added, followed by stirring at room temperature for 2 to 72 hours to provide a desired compound (13b).
Step 13-2
   The compound (13b) obtained in step 13-1 is acylated or alkylated. For example, the compound (13b) is suspended in an organic solvent such as DMF, chloroform, or methylene chloride, to which a reagent having a leaving group represented by (4c) (where each of the substituents is as described above) and a base such as sodium hydride or cesium carbonate are then added, followed by stirring at room temperature for 2 to 72 hours to provide a desired compound (13d).

Step 13-3
   The compound (13d) obtained in step 13-2 is exposed to an organic solvent solution such as a 1 to 30% methanol or THF solution of hydroxylamine to provide a desired compound (13e).
Step 13-4
   The protective group P1 is removed from the compound (13e) obtained in step 13-3 to provide a desired compound. For example, when P₁ is a t-butoxycarbonyl group, a strong acid such as trifluoroacetic acid can be allowed to act to provide a desired compound (13f).

Then, a composition containing the compound of the invention is described. The novel compound of the invention can prevent the onset of and suppress the progression of various diseases believed to be attributable particularly to AGE or ALE. Thus, there can be provided an AGE generation inhibitor containing the compound as active ingredient, a medicinal composition used for the treatment or prevention of diseases associated with AGE generation, an AGE generation-inhibiting additive for use in cosmetics and foods, and a cosmetic and processed food hardly causing deterioration due to AGE generation.
When the compound of the invention or a salt thereof is used as active ingredient in a medicinal composition, the composition is useful as a preventive or therapeutic agent for diseases associated with AGE generation: for example, diabetic complications including coronary artery cardiac disease, peripheral circulatory disturbance, cerebrovascular disease, diabetic neurosis, nephropathy, arteriosclerosis, arthrosclerosis, cataract, retinopathy, coagulopathy and diabetic osteopenia; diseases thought to be caused by aging, including atherosclerosis, glomerular nephritis, cataract, osteoarthropathy, periarticular rigidity, arthrosclerosis, senile osteoporosis, and Alzheimer's disease; dialysis amyloidosis as one of dialysis complications; peritoneal sclerosis in peritoneal dialysis patients; and diseases for which one of the chief causes is active oxygen because active oxygen species is produced through the second half reaction of the Maillard reaction, including arteriosclerosis, coronary artery disease, cerebrovascular disease, liver failure, kidney failure, cataract, retinopathy, and autoimmune disease.

Among the above-described diabetic complications are coronary artery cardiac disease, peripheral circulatory disturbance, cerebrovascular disease, diabetic neurosis, diabetic retinopathy, diabetic arteriosclerosis, diabetic arthrosclerosis, cataract, diabetic retinopathy, diabetic coagulopathy, and diabetic osteopenia.
The composition of the invention is then described based on the medicinal composition. The medicinal composition of the invention may be orally or parenterally administered. For oral administration, it may be prepared in the form of hard capsules, soft capsules, tablets, granules, powders, subtle granules, pills, troches, active ingredient sustained-release preparations, elixirs, emulsions, syrups, solutions, suspensions, or the like. Examples of the parentaral administration include iniections such as drip infusion and intravenous, subcutaneous, and intramuscular injections, percutaneous administration using ointments or transdermal preparations, rectal administration using oil and fat suppositories, water-soluble suppositories, or suppositories, and administration using external preparations or ophthalmic solutions. The preparation can be easily carried out by a conventional method using a common carrier known in the pharmaceutical field.
When the medicinal composition of the invention is prepared in the form of oral administration, there may be employed widely used components for formulation such as a carrier, including, for example, a filler, an extender, a binder, a disintegrator, a disintegration inhibitor, a buffer, an isotonizing agent, an emulsifier, a dispersant, a stabilizer, a coating agent, a surfactant, an absorption promoter, a humectant, a wetting agent, an absorbent, a lubricant, and an excipient. In addition, additives such as a coloring agent, a preservative, a flavor, a seasoning, and a sweetening agent may be optionally added.

Specific examples thereof include: excipients such as lactose, sucrose, sodium chloride, dextrose, urea, starch, calcium carbonate, kaolin, crystalline cellulose, and silicic acid; binders such as water, ethanol, simple syrup, dextrose in water, starch in water, gelatin solution, carboxymethyl cellulose, methyl cellulose, potassium phosphate, and polyvinylpyrrolidone; disintegrators such as dry starch, sodium alginate, powdered agar, powdered laminaran, sodium bicarbonate, calcium carbonate, polyoxyethylene sorbitan fatty acid ester, sodium lauryl sulfate, monoglyceride stearate, starch, and lactose; disintegration inhibitors such as sucrose, stearic acid, cocoa butter, and hydrogenated oil; absorption promoters such as quarternary ammonium salts and sodium lauryl sulfate; humectants such as glycerin and starch; adsorbents such as starch, lactose, kaolin, bentonite, and colloidal silicic acid; and lubricants such as purified talc and stearates. Further, each of the above-described dosage forms can be changed into a sustained release type, a topical application type (troches, buccals, sublingual tablets, or the like), a drug controlled release type, an enteric-soluble type, or a gastric-soluble type using a well-known technique for drug delivery system.
In the medicinal composition containing the compound of the invention, the content of the compound varies depending on the dosage form thereof, but is typically on the order of 0.1 to 100 wt%, preferably 0.3 to 30 wt% based on the whole composition. The dosage of the compound is properly determined according to the sex, age, and body weight of patients of interest, disease difference, the degree of pathology, therapeutic effect, the method of administration, and the like; however, it is given once daily or in several portions daily in the range of 0.1 to 5,000 mg/adult, preferably 1 to 1,000 mg/adult for oral administration or in the range of 0.1 to 1,000 mg/adult, preferably 0.3 to 300 mg/adult for parenteral administration. Of course the dosage varies depending on various conditions; therefore, in some cases, the dosage is sufficient in an amount down from the above-described range, or required to exceed the range.

When used in the form of an ophthalmic solution, the compound of the invention is formulated in the range of 0.05 w/v% to 5 w/v% for preparation by the law of the art. The administration frequency thereof can be properly determined according to the degree of patient pathology and the like.
When an additive composition is prepared using the compound of the invention, components according to the application of the composition are mixed with the compound. The content of the compound in the additive composition is typically 1 to 100 parts by weight.
When used in an external preparation or a cosmetic, the compound of the invention may be formulated so as to provide a content of 0.05 to 10 parts by weight based on the whole composition for preparation by the law of the art employing a widely used base. In addition, the compound of the invention may be prepared for use in the form of a food by the law of the art, and also employed by adding to foods.
The invention will be described based on Examples, Reference Examples, and the like. However, they are not intended to limit the scope of the protection of the invention.

### Examples

### (Example 1) Synthesis of hydroxylamine O-supporting 2-chlorotrityl resin

In a reaction vessel equipped with a filter was taken 1.00 g of commercial polystyrene-supporting 2-chlorotrityl resin (100 to 200 mesh, 1.25 mmol/g), to which a reaction liquid consisting of 1.02 g of N-hydroxyphthalimide and 8.88 ml of triethylamine dissolved in 20 ml of dimethylformamide was then added. After shaking at room temperature for 27 hours, the solution was removed, and the resultant resin was washed sequentially with DMF, distilled water, methanol, and ether and then dried. Subsequently, a 40% methylamine/methanol solution was added thereto, followed by shaking at room temperature for 24 hours. After the end of reaction, the solution was removed, followed by washing sequentially with DMF, 10% aqueous ammonia, distilled water, THF, methanol, and ether before drying to provide the title resin.

### (Example 2) Synthesis of N-Boc-glutamic acid hydroxamate O-supporting 2-chlorotrityl resin

In a reaction vessel was placed 1.006 g (equivalent to 1.25 mmol) of the resin synthesized in Example 1, to which 0.926 ml (6.25 mmol) of DIC, 0.844 g (6.25 mmol) of HOBt, and 2.108 g (6.25 mmol) of commercial N^{α}-Boc-δ-benzyl-L-glutamic acid were then added, followed by shaking at room temperature for 3 days. After the end of reaction, the solvent was removed and the resultant resin was washed with chloroform/methanol, DMF, methanol, THF, chloroform, and ether in that order. To 150 mg (0.13 mmol) of this resin were added 1 ml of methanol, 1 ml of THF, and 1 ml of 1 mol/1 sodium hydroxide aqueous solution, followed by shaking at room temperature for 20 hours. After the end of reaction, the resultant resin was washed with acetic acid/THF, acetic acid/methanol, acetic acid/DMF, DMF, methanol, chloroform, and ether in that order to provide the title resin.

### (Example 3) Synthesis of (S)-2-amino-4-[(naphthalen-1-ylmethyl)aminocarbonyl]-N-hydroxybutyramide (compound No. 1)

To the resin obtained in Example 2 was added 2 ml of a DMF solution of 124 mg (0.65 mmol) of DIC and 87.5 mg (0.65 mmol) of HOBt before suspension, to which 102 mg (0.65 mmol) of 1-naphtylmethylamine was then added, followed by shaking at room temperature for 19 hours. After the end of reaction, the resultant resin was washed with DMF, methanol, THF, chloroform, and ether in that order. To this resin was added 2 ml of 30% trifluoroacetic acid/chloroform solution, followed by shaking at room temperature for 2 hours. The solution obtained by filtration and the solution with which the resin had been washed were combined, from which the solvent was then distilled off. The residue was purified by a reverse phase system solid-phase extraction column to provide 24.2 mg of the trifluoroacetate of the title compound as a white solid.
MS (Fab, Pos.): m/z = 302 [M+H]⁺

### (Example 4) Synthesis of (S)-2-amino-4-(4-t-butylphenylaminocarbonyl)-N-hydroxybutyramide (compound No. 2)

An operation similar to that in Example 3 was carried out using 97.0 mg (0.65 mmol) of 4-t-butylaniline in place of 1-naphtylmethylamine to provide 32.0 mg of the trifluoroacetate of the title compound as a white solid.
MS (Fab, Pos.): m/z = 294 [M+H]⁺

### (Example 5) Synthesis of (S)-2-amino-4-(4-fluorobenzylaminocarbonyl)-N-hydroxybutyramide (compound No. 3)

An operation similar to that in Example 3 was carried out using 81.3 mg (0.65 mmol) of 4-fluorobenzylamine in place of 1-naphtylmethylamine to provide 9.2 mg of the trifluoroacetate of the title compound as a white solid.
MS (Fab, Pos.): m/z = 270 [M+H]⁺

### (Example 6) Synthesis of(S)-2-amino-4-(4-t-butylbenzylaminocarbonyl)-N-hydroxybutyramide (compound No. 4)

An operation similar to that in Example 3 was carried out using 106.1 mg (0.65 mmol) of 4-butylbenzylamine in place of 1-naphtylmethylamine to provide 16.0 mg of the trifluoroacetate of the title compound as a white solid.
MS (Fab, Pos.): m/z = 308 [M+H]⁺

### (Example 7) Synthesis of (S)-2-amino-4-(cyclohexylaminocarbonyl)-N-hydroxybutyramide (compound No. 5)

An operation similar to that in Example 3 was carried out using 64.5 mg (0.65 mmol) of cyclohexylamine in place of 1-naphtylmethylamine to provide 12.4 mg of the trifluoroacetate of the title compound as a white solid.
MS (Fab, Pos.): m/z = 244 [M+H]⁺

### (Example 8) Synthesis of (S)-2-amino-4-(2,2-diphenylethylaminocarbonyl)-N-hydroxybutyramide (compound No. 6)

An operation similar to that in Example 3 was carried out using 119.1 mg (0.65 mmol) of 2,2-diphenylethylamine in place of 1-naphtylmethylamine to provide 27.2 mg of the trifluoroacetate of the title compound as a white solid.
MS (Fab, Pos.): m/z = 342 [M+H]⁺

### (Example 9) Synthesis of (S)-2-amino-4-(pyridin-2-ylmethylaminocarbonyl)-N-hydroxybutyramide (compound No. 7)

An operation similar to that in Example 3 was carried out using 70.3 mg (0.65 mmol) of 2-picolylamine in place of 1-naphtylmethylamine to provide 18.9 mg of the trifluoroacetate of the title compound as a white solid.
MS (Fab, Pos.): m/z = 253 [M+H]⁺

### (Example 10) Synthesis of (S)-2-amino-4-(N-benzyl-N-methylaminocarbonyl)-N-hydroxybutyramide (compound No. 8)

An operation similar to that in Example 3 was carried out using 78.8 mg (0.65 mmol) of N-benzyl-N-methylamine in place of 1-naphtylmethylamine to provide 27.1 mg of the trifluoroacetate of the title compound as a white solid.
MS (Fab, Pos.): m/z = 266 [M+H]⁺

### (Example 11) Synthesis of (S)-2-amino-4-(diethylaminocarbonyl)-N-hydroxybutyramide (compound No. 9)

An operation similar to that in Example 3 was carried out using 47.5 mg (0.65 mmol) of diethylamine in place of 1-naphtylinethylamine to provide 31.0 mg of the trifluoroacetate of the title compound as a white solid.
MS (Fab, Pos.): m/z = 218 [M+H]⁺

### (Example 12) Synthesis of (S)-2-amino-4-(piperidin-1-ylcarbonyl)-N-hydroxybutyramide (compound No. 10)

An operation similar to that in Example 3 was carried out using 55.3 mg (0.65 mmol) of piperidine in place of 1-naphtylmethylamine to provide 22 mg of the trifluoroacetate of the title compound as a white solid.
MS (Fab, Pos.): m/z = 230 [M+H]⁺

### (Example 13) Synthesis of (S)-2-amino-4-(adamantan-1-ylaminocarbonyl)-N-hydroxybutyramide (compound No. 11)

An operation similar to that in Example 3 was carried out using 98.3 mg (0.65 mmol) of 1-adamantane in place of 1-naphtylmethylamine to provide 9.5 mg of the trifluoroacetate of the title compound as a white solid.
MS (Fab, Pos.): m/z = 296 [M+H]⁺

### (Example 14) Synthesis of (S)-2-amino-4-(benzylaminocarbonyl)-N-hydroxybutyramide (compound No. 12)

An operation similar to that in Example 3 was carried out using 69.6 mg (0.65 mmol) of benzylamine in place of 1-naphtylmethylamine to provide 13.4 mg of the trifluoroacetate of the title compound as a white solid.
MS (Fab, Pos.): m/z = 252 [M+H]⁺

### (Example 15) Synthesis of N^{α}-Boc-ornithine hydroxamate O-supporting 2-chlorotrityl resin

In a reaction vessel was placed 1.98 g (equivalent to 2.50 mmol) of the hydroxamate-supporting resin obtained in Example 1, to which 1.197 g (6.25 mmol) of WSCI hydrochloride, 0.676 g (5.00 mmol) of HOBt, and 2.270 g (0.625 mmol) of commercial N^{α}-Boc-N^{δ}-Fmoc-omithine were then added, followed by shaking at room temperature for 24 hours. After the end of reaction, the solvent was removed and the resultant resin was washed with DMF, a 1 mol/l hydrochloric acid aqueous solution, distilled water, THF, methanol, chloroform, and ether in that order to provide the title resin. (Example 16) Synthesis of (S)-2-amino-5-acetylaminopentanoic acid hydroxyamide (compound No. 13)
Into a reaction vessel was dispensed 116 mg (equivalent to 0.10 mmol) of the resin obtained in Example 17, to which 2 ml of 20% DMF solution of piperidine was then added, followed by shaking for 3 hours. After the end of reaction, the solvent was removed and the resultant resin was washed with DMF, methanol, THF, chloroform, and ether in that order. To 2 ml of a DMF solution of the resin, 57.5 mg (0.3 mmol) of WSCI hydrochloride, and 27.0 mg (0.2 mmol) of HOBt was added 12.1 mg (0.2 mmol) of acetic acid, followed by shaking at room temperature for 7 hours. After the end of reaction, the solvent was removed and the resultant resin was washed with DMF, methanol, THF, chloroform, and ether in that order. To this resin was added 2 ml of 30% trifluoroacetic acid/chloroform solution, followed by stirring at room temperature for 2 hours. After the end of reaction, the solution obtained by filtration and the solution with which the resin had been washed were combined, from which the solvent was then distilled off. The residue was purified by a reverse phase system solid-phase extraction column to provide 24.9 mg of the trifluoroacetate of the title compound as a white solid.
MS (Fab, Pos.): m/z = 190 [M+H]⁺

### (Example 17) Synthesis of (S)-2-amino-5-benzoylaminopentanoic acid hydroxyamide (compound No. 14)

An operation similar to that in Example 16 was carried out using 24.4 mg (0.20 mmol) of benzoic acid in place of acetic acid to provide 15.2 mg of the trifluoroacetate of the title compound as a white solid.
MS (Fab, Pos.): m/z = 252 [M+H]⁺

### (Example 18) Synthesis of (S)-2-amino-5-(4-nitrobenzoylamino)pentanoic acid hydroxyamide (compound No. 15)

An operation similar to that in Example 16 was carried out using 33.4 mg (0.20 mmol) of 4-nitrobenzoic acid in place of acetic acid to provide 16.9 mg of the trifluoroacetate of the title compound as a white solid.
MS (Fab, Pos.): m/z = 297 [M+H]⁺

### (Example 19) Synthesis of (S)-2-amino-5-(4-nitrophenylacetylamino)pentanoic acid hydroxyamide (compound No. 16)

An operation similar to that in Example 16 was carried out using 36.2 mg (0.20 mmol) of 4-nitrophenylacetic acid in place of acetic acid to provide 8.7 mg of the trifluoroacetate of the title compound as a white solid.
MS (Fab, Pos.): m/z = 311 [M+H]⁺

### (Example 20) Synthesis of (S)-2-amino-5-(naphthalen-1-yl-carbonylamino)pentanoic acid hydroxyamide (compound No. 17)

An operation similar to that in Example 16 was carried out using 34.4 mg (0.20 mmol) of 1-naphthaleneacetic acid in place of acetic acid to provide 14.8 mg of the trifluoroacetate of the title compound as a white solid.
MS (Fab, Pos.): m/z = 302 [M+H]⁺

### (Example 21) Synthesis of (S)-2-amino-5-(naphthalen-2-yl-carbonylamino)pentanoic acid hydroxyamide (compound No. 18)

An operation similar to that in Example 16 was carried out using 34.4 mg (0.20 mmol) of 2-naphthaleneacetic acid in place of acetic acid to provide 14.5 mg of the trifluoroacetate of the title compound as a white solid.
MS (Fab, Pos.): m/z = 302 [M+H]⁺

### (Example 22) Synthesis of (S)-2-amino-5-(cyclohexylacetylamino)pentanoic acid hydroxyamide (compound No. 19)

An operation similar to that in Example 16 was carried out using 28.4 mg (0.20 mmol) of cyclohexylacetic acid in place of acetic acid to provide 16.1 mg of the trifluoroacetate of the title compound as a white solid.
MS (Fab, Pos.): m/z = 272 [M+H]⁺

### (Example 23) Synthesis of (S)-2-amino-5-(4-chlorobenzoylamino)pentanoic acid hydroxyamide (compound No. 20)

An operation similar to that in Example 16 was carried out using 31.2 mg (0.20 mmol) of 4-chlorobenzoic acid in place of acetic acid to provide 15.6 mg of the trifluoroacetate of the title compound as a white solid.
MS (Fab, Pos.): m/z = 286, 288 [M+H]⁺

### (Example 24) Synthesis of (S)-2-amino-5-diphenylacetylaminopentanoic acid hydroxyamide (compound No. 21)

An operation similar to that in Example 16 was carried out using 42.4 mg (0.20 mmol) of diphenylacetic acid in place of acetic acid to provide 13.5 mg of the trifluoroacetate of the title compound as a white solid.
MS (Fab, Pos.): m/z = 342 [M+H]⁺

### (Example 25) Synthesis of (S)-2-amino-5-(2-biphenyl-4-ylacetylamino)pentanoic acid hydroxyamide (compound No. 22)

An operation similar to that in Example 16 was carried out using 39.6 mg (0.20 mmol) of 4-phenylbenzoic acid in place of acetic acid to provide 16.7 mg of the trifluoroacetate of the title compound as a white solid.
MS (Fab, Pos.): m/z = 342 [M+H]⁺

### (Example 26) Synthesis of (S)-2-amino-5-(3,5-di-t-butylphenylacetylamino)pentanoic acid hydroxyamide (compound No. 23)

An operation similar to that in Example 16 was carried out using 46.9 mg (0.20 mmol) of 3,5-di-t-butylbenzoic acid in place of acetic acid to provide 9.0 mg of the trifluoroacetate of the title compound as a white solid.
MS (Fab, Pos.): m/z = 364 [M+H]⁺

### (Example 27) Synthesis of (S)-2-amino-5-pivaloylaminopentanoic acid hydroxyamide (compound No. 24)

An operation similar to that in Example 16 was carried out using 20.4 mg (0.20 mmol) of pivalic acid in place of acetic acid to provide 17.2 mg of the trifluoroacetate of the title compound as a white solid.
MS (Fab, Pos.): m/z = 232 [M+H]⁺

### (Example 28) Synthesis of (S)-2-amino-5-dipropylaminopentanoic acid hydroxyamide (compound No. 25)

In a reaction vessel was dispensed 150 mg (equivalent to 0.13 mmol) of the resin obtained in Example 15, to which 2 ml of 20% DMF solution of piperidine was then added, followed by shaking for 3 hours. After the end of reaction, the solvent was removed and the resultant resin was washed with DMF, methanol, THF, chloroform, and ether in that order. This resin was suspended in 1 ml of THF, to which 37.8 mg (0.65 mmol) of propionaldehyde and then 1 ml of methanol solution of 40.8 mg (0.65 mmol) of sodium cyanoborohydride and 0.05 ml of acetic acid were then added, followed by shaking at room temperature for 22 hours. After the end of reaction, the solvent was removed and the resultant resin was washed with DMF, methanol, THF, chloroform, and ether in that order. To this resin was added 2 ml of 30% trifluoroacetic acid/chloroform solution, followed by stirring at room temperature for 2 hours. After the end of reaction, the solution obtained by filtration and the solution with which the resin had been washed were combined, from which the solvent was then distilled off. The residue was purified by a reverse phase system solid-phase extraction column to provide 7.2 mg of the trifluoroacetate of the title compound as a white solid.
MS (Fab, Pos.): m/z = 232 [M+H]⁺

### (Example 29) Synthesis of (S)-2-amino-5-dipentylaminopentanoic acid hydroxyamide (compound No. 26)

An operation similar to that in Example 28 was carried out using 56.0 mg (0.65 mmol) of valeraldehyde in place of propionaldehyde to provide 4.2 mg of the trifluoroacetate of the title compound as a white solid.
MS (Fab, Pos.): m/z = 288 [M+H]⁺

### (Example 30) Synthesis of (S)-2-amino-5-cyclohexylaminopentanoic acid hydroxyamide (compound No. 27)

An operation similar to that in Example 28 was carried out using 63.8 mg (0.65 mmol) of cyclohexanone in place of propionaldehyde to provide 8.4 mg of the trifluoroacetate of the title compound as a white solid.
MS (Fab, Pos.): m/z = 230 [M+H]⁺

### (Example 31) Synthesis of (S)-2-amino-5-[bis-(4-methyl-benzyl)amino]pentanoic acid hydroxyamide (compound No. 28)

An operation similar to that in Example 28 was carried out using 78.1 mg (0.65 mmol) of p-tolualdehyde in place of propionaldehyde to provide 3.6 mg of the trifluoroacetate of the title compound as a white solid.
MS (Fab, Pos.): m/z = 356 [M+H]⁺

### (Example 32) Synthesis of N^{α}-Boc-4-aminophenylalanine hydroxamate O-supporting 2-chlorotrityl resin

In a reaction vessel was placed 1.31 g (equivalent to 1.63 mmol) of the resin obtained in Example 1, to which 1.25 g (6.53 mmol) of WSCI hydrochloride, 0.869 g (6.43 mmol) of HOBt, and 2.27 g (6.52 mmol) of commercial N^{α}-Boc-(4-N-Fmocamino)phenylalanine were then added, followed by shaking at room temperature for 72 hours. After the end of reaction, the reaction liquid was removed and the resultant resin was washed with DMF, a 0.2 mol/l hydrochloric acid aqueous solution, a saturated sodium bicarbonate aqueous solution, distilled water, methanol, THF, chloroform, and ether. This resin was dried before adding a 20% DMF solution of piperidine, followed by shaking at room temperature for 3 hours. After the end of reaction, the reaction liquid was removed and the resultant resin was washed with DMF, methanol, THF, chloroform, and ether to provide the title compound.

### (Example 33) Synthesis of (S)-2-amino-3-(4-acetylaminophenyl)-N-hydroxypropionamide (compound No. 29)

To 130 mg (equivalent to 0.12 mmol) of the resin obtained in Example 32 were added a mixed solution of 1 ml of pyridine, 1 ml of chloroform, and 73.3 mg (0.60 mmol) of dimethylaminopyridine and 61.3 mg (0.60 mmol) of acetic anhydride, followed by shaking at room temperature for 2 days. After the end of reaction, the solvent was removed and the resultant resin was washed with DMF, methanol, THF, chloroform, chloroform/methanol mixed solution, and ether in that order. To this resin was added a 30% trifluoroacetic acid/chloroform solution, followed by shaking at room temperature for 2 hours. After the end of reaction, the solution obtained by filtration and the solution with which the resin had been washed were combined, from which the solvent was then distilled off. The residue was purified by a reverse phase system solid-phase extraction column to provide 11.3 mg of the trifluoroacetate of the title compound as a white solid.
MS (Fab, Pos.): m/z = 238 [M+H]⁺

### (Example 34) Synthesis of (S)-2-amino-3-[4-(4-methylbenzoyl)amino-phenyl]-N-hydroxypropionamide (compound No. 30)

An operation similar to that in Example 33 was carried out using 92.8 mg (0.60 mmol) of p-toluyl chloride in place of acetic anhydride to provide 3.3 mg of the trifluoroacetate of the title compound as a white solid.
MS (Fab, Pos.): m/z = 314 [M+H]⁺

### (Example 35) Synthesis of (S)-2-amino-3-(4-valeroylaminophenyl)-N-hydroxypropionamide (compound No. 31)

An operation similar to that in Example 33 was carried out using 72.3 mg (0.60 mmol) of valeric acid chloride in place of acetic anhydride to provide 18.7 mg of the trifluoroacetate of the title compound as a white solid.
MS (Fab, Pos.): m/z = 280 [M+H]⁺

### (Example 36) Synthesis of (S)-2-amino-3-(4-pivaloylaminophenyl)-N-hydroxypropionamide (compound No. 32)

An operation similar to that in Example 33 was carried out using 72.3 mg (0.60 mmol) of pivalic acid chloride in place of acetic anhydride to provide 17.8 mg of the trifluoroacetate of the title compound as a white solid.
MS (Fab, Pos.): m/z = 280 [M+H]⁺

### (Example 37) Synthesis of (S)-2-amino-3-{4-[2-(4-fluorophenyl)acetylamino]-phenyl}-N-hydroxypropionamide (compound No. 33)

An operation similar to that in Example 33 was carried out using 95.1 mg (0.60 mmol) of 4-fluorobenzoic acid chloride in place of acetic anhydride to provide 18.6 mg of the trifluoroacetate of the title compound as a white solid.
MS (Fab, Pos.): m/z = 332 [M+H]⁺

### (Example 38) Synthesis of (S)-2-amino-3-[4-(toluen-4-ylsulfonylamino)phenyl]-N-hydroxypropionamide (compound No. 34)

An operation similar to that in Example 33 was carried out using 114.4 mg (0.60 mmol) of 4-toluenesulfonic acid chloride in place of acetic anhydride to provide 26.4 mg of the trifluoroacetate of the title compound as a white solid.
MS (Fab, Pos.): m/z = 350 [M+H]⁺

### (Example 39) Synthesis of (S)-2-amino-3-[4-(4-trifluoromethylphenylureide)phenyl]-N-hydroxypropionamide (compound No. 35)

An operation similar to that in Example 33 was carried out using 121.6 mg (0.60 mmol) of 4-trifluoromethylphenyl isocyanate in place of acetic anhydride to provide 20.5 mg of the trifluoroacetate of the title compound as a white solid.
MS (Fab, Pos.): m/z = 383 [M+H]⁺

### (Example 40) Synthesis of (S)-2-amino-3-(4-methoxycarbonylaminophenyl)-N-hydroxypropionamide (compound No. 36)

An operation similar to that in Example 33 was carried out using 56.7 mg (0.60 mmol) of methyl chloroformate in place of acetic anhydride to provide 16.0 mg of the trifluoroacetate of the title compound as a white solid.
MS (Fab, Pos.): m/z = 254 [M+H]⁺

### (Example 41) Synthesis of (S)-2-amino-3-(4-dimethylaminocarbamoyl-phenyl)-N-hydroxypropionamide (compound No. 37)

An operation similar to that in Example 33 was carried out using 64.5 mg (0.60 mmol) of dimetylcarbamoyl chloride in place of acetic anhydride to provide 7.0 mg of the trifluoroacetate of the title compound as a white solid.
MS (Fab, Pos.): m/z = 267 [M+H]⁺

### (Example 42) Synthesis of (S)-2-amino-3-(4-dipropylamino-1-phenyl)-N-hydroxypropionamide (compound No. 38)

In 1 ml of THF was suspended 140 mg (equivalent to 0.13 mmol) of the resin obtained in Example 32, to which 37.8 (0.65 mmol) of propionaldehyde and then 1 ml of methanol solution of 40.8 mg (0.65 mmol) of sodium cyanoborohydride and 0.05 ml of acetic acid were then added, followed by shaking at room temperature for 2 days. After the end of reaction, the solution was removed and the resultant resin was washed with DMF, methanol, THF, chloroform, and ether in that order. To this resin was added 2 ml of 30% trifluoroacetic acid/chloroform solution, followed by stirring at room temperature for 2 hours. After the end of reaction, the solution obtained by filtration and the solution with which the resin had been washed were combined, from which the solvent was then distilled off. The residue was purified by a reverse phase system solid-phase extraction column to provide 11.8 mg of the trifluoroacetate of the title compound as a white solid.
MS (Fab, Pos.): m/z = 280 [M+H]⁺

### (Example 43) Synthesis of (S)-2-amino-3-[4-(4-methylbenzyl)aminophenyl]-N-hydroxypropionamide (compound No. 39)

An operation similar to that in Example 42 was carried out using 78.1 mg (0.65 mmol) ofp-toluic aldehyde in place of propionaldehyde to provide 2.1 mg of the trifluoroacetate of the title compound as a white solid.
MS (Fab, Pos.): m/z = 300 [M+H]⁺

### (Example 44) Synthesis of (S)-2-amino-3-(4-nitrophenyl)-N-hydroxypropionamide (compound No. 40)

To 105 mg (equivalent to 0.13 mmol) of the resin obtained in Example 1 was added a reaction liquid consisting of 201.7 mg (0.65 mmol) of commercial Boc-4-nitrophenylalanine, 87.8 mg (0.65 mmol) of HOBt, and 124.6 mg (0.65 mmol) of WSCI hydrochloride dissolved in 1.5 ml of DMF, followed by shaking at room temperature for 21 hours. After the end of reaction, the reaction liquid was removed and the resultant resin was washed with DMF, distilled water, methanol, and ether. After drying this resin, a 10% trifluoroacetic acid/chloroform solution was added, followed by shaking at room temperature for 2 hours. After the end of reaction, the filtrate obtained by filtration and the chloroform with which the resin had been washed were combined, from which the solvent was then distilled off, followed by purification using a reverse phase system solid-phase extraction column to provide 11.7 mg of the trifluoroacetate of the title compound as a pale yellow solid.
MS (Fab, Pos.): m/z = 226 [M+H]⁺
¹H-NMR(500MHz, DMSO-d₆): δ = 3.11 (1H, dd, J = 13.4,6.8 Hz), 3.15 (1H, dd, J = 13.4, 6.4 Hz), 3.83 (1H, m), 7.50 (2H, d, J = 8.0 Hz), 8.22 (2H, d, J = 8.0 Hz), 8.36 (2H, brs), 9.32 (1H, s), 10.99 (1H,s).

### (Example 45) Synthesis of 4-nitrophenylalanine hydroxamate O-supporting 2-chlorotrityl resin

In a reaction vessel was placed 1.08 g (equivalent to 1.3 mmol) of the resin obtained in Example 1, to which 0.397 ml (2.3 mmol) of DIC, 0.262 g (1.94 mmol) of HOBt, and 1.02 g (2.3 mmol) of commercial N^{α}-Fmoc-4-nitrophenylalanine were then added, followed by shaking at room temperature for 18 hours using a shaker. After the end of reaction, the solvent was removed, followed by washing with DMF, THF, methanol, and chloroform. To this resin was added 10 ml of 25% piperidine/DMF, followed by shaking for 1 hour. After the end of reaction, the solvent was removed and the resultant resin was washed with DMF, methanol, THF, methanol, chloroform, methanol, chloroform, and ether in that order to provide the title resin.

### (Example 46) Synthesis of (S)-2-benzylamino-3-(4-nitrophenyl)-N-hydroxypropionamide (compound No. 41)

In 2 ml of chloroform was suspended 150 mg (equivalent to 0.15 mmol) of the resin obtained in Example 45, to which 79.6 mg (0.75 mmol) of benzaldehyde and a proper amount of molecular sieves were then added, followed by shaking for 4.5 hours. The reaction liquid was extracted and the resultant resin was washed with dry chloroform, followed by adding 1 ml of dry chloroform and 1 ml of dry methanol and then 17.0 mg (0.45 mmol) of sodium borohydride before stirring at room temperature for 2 days. After the end of reaction, the solvent was removed and the resultant resin was washed with DMF/water, DMF, methanol, THF, chloroform, and ether in that order. To this resin was added a 10% trifluoroacetic acid/chloroform solution, followed by stirring at room temperature for 2 hours. After the end of reaction, the solution obtained by filtration and the solution with which the resin had been washed were combined, from which the solvent was then distilled off. The residue was purified by a reverse phase system solid-phase extraction column to provide 3.7 mg of the trifluoroacetate of the title compound as a pale yellow solid.
MS (Fab, Pos.): m/z = 316 [M+H]⁺

### (Example 47) Synthesis of (S)-2-(pyridin-2-ylmethyl)amino-3-(4-nitrophenyl)-N-hydroxypropionamide (compound No. 42)

An operation similar to that in Example 46 was carried out using 80.3 mg (0.75 mmol) of 2-pyridinecarbaldehyde in place of benzaldehyde to provide 9.3 mg of the trifluoroacetate of the title compound as a pale yellow solid.
MS (Fab, Pos.): m/z = 317 [M+H]⁺

### (Example 48) Synthesis of (S)-2-propylamino-3-(4-nitrophenyl)-N-hydroxypropionamide (compound No. 43)

An operation similar to that in Example 46 was carried out using 44.3 mg (0.75 mmol) of propionaldehyde in place of benzaldehyde to provide 8.0 mg of the trifluoroacetate of the title compound as a pale yellow solid.
MS (Fab, Pos.): m/z = 268 [M+H]⁺

### (Example 49) Synthesis of (S)-2-cyclohexylmethylamino-3-(4-nitrophenyl)-N-hydroxypropionamide (compound No. 44)

An operation similar to that in Example 46 was carried out using 84.1 mg (0.75 mmol) of cyclohexanecarbaldehyde in place of benzaldehyde to provide 4.8 mg of the trifluoroacetate of the title compound as a pale yellow solid.
MS (Fab, Pos.): m/z = 322 [M+H]⁺

### (Example 50) Synthesis of (S)-2-isopropylamino-3-(4-nitrophenyl)-N-hydroxypropionamide (compound No. 45)

In 1 ml of dry chloroform and 1 ml of dry methanol was suspended 150 mg (equivalent to 0.15 mmol) of the resin obtained in Example 48, to which 43.6 mg (0.75 mmol) of acetone and 47.1 mg (0.75 mmol) of sodium cyanoborohydride were then added before adjusting the pH to 4 using acetic acid, followed by shaking at room temperature for 2 days. To this was added 2 ml of 20% trifluoroacetic acid/chloroform solution, followed by shaking at room temperature for 2 hours. The solution obtained by filtration and the solution with which the resin had been washed were combined, from which the solvent was then distilled off. The residue was purified by a reverse phase system solid-phase extraction column to provide 36.9 mg of the trifluoroacetate of the title compound as a pale yellow solid.
MS (Fab, Pos.): m/z = 268 [M+H]⁺

### (Example 51) Synthesis of (S)-2-cyclohexylamino-3-(4-nitrophenyl)-N-hydroxypropionamide (compound No. 46)

An operation similar to that in Example 50 was carried out using 73.6 mg (0.75 mmol) of cyclohexanone in place of acetone to provide 51.7 mg of the trifluoroacetate of the title compound as a pale yellow solid.
MS (Fab, Pos.): m/z = 308 [M+H]⁺

### (Example 52) Synthesis of(S)-2-(1-methylpiperidin-4-yl)amino-3-(4-nitrophenyl)-N-hydroxypropionamide (compound No. 47)

An operation similar to that in Example 50 was carried out using 84.9 mg (0.75 mmol) of 1-methylpiperidin-4-one in place of acetone to provide 78.2 mg of the trifluoroacetate of the title compound as a pale yellow solid.
MS (Fab, Pos.): m/z = 323 [M+H]⁺

### (Example 53) Synthesis of N^{α}-Boc-tyrosine-supporting hydroxybenzyl resin

In 10 ml of DMF was suspended 2.01 g (equivalent to 2.20 mmol) of commercial Wang resin (polystyrene-supporting, 100 to 200 mesh, DVB 1%), to which 1.253 g (4.40 mmol) of commercial N^{α}-Boc-tyrosine, 594.7 mg (4.40 mmol) ofHOBt, and 41 mg (0.33 mmol) of DMAP were then added. Thereto was added 510 ml (4.40 mmol) of DIC, followed by shaking at room temperature for one day. After the end of reaction, the reaction liquid was filtered off before washing with chloroform, DMF, and ether, followed by again conducting the reaction under the same conditions as the above. After the end of reaction, the reaction liquid was filtered off, followed by washing with chloroform, an aqueous ammonia-methanol solution, an acetic acid-methanol solution, DMF, a chloroform/methanol solution, chloroform, and ether before drying. Thereto was added a 20% piperidine/DMF solution, followed by shaking at room temperature for 30 minutes. After the end of reaction, the reaction liquid was filtered off, followed by washing with a 5% acetic acid-chloroform solution, a chloroform/methanol solution, DMF, chloroform, and ethanol before drying to provide the title resin.

### (Example 54) Synthesis of (S)-2-amino-N-hydroxy-3-(4-phenethyloxyphenyl)propionamide (compound No. 48)

In 2 ml of THF/chloroform (= 1/1) solution of 126 mg (0.48 mmol) of triphenylphosphine was suspended 200 mg (equivalent to 0.16 mmol) of the resin obtained in Example 53, to which 58.6 mg (0.48 mmol) of phenethyl alcohol and 0.22 ml (0.48 mmol) of 40% toluene solution of diethylazodicarboxylate (hereinafter referred to as DEAD) were then added, followed by stirring at room temperature for 28 hours. After the end of reaction, the reaction liquid was filtered off, followed by washing with DMF, THF, methanol, chloroform, a chloroform/methanol solution, and ethanol before drying. Thereto was added a 12.5% THF/methanol/water (= 2/1/1) solution of hydroxylamine, which was then shaken at room temperature for 2 days before filtering off the solution, and the resultant resin was washed with 2 ml of chloroform/methanol (= 1/1) solution, followed by combining with the above filtrate. This solution was concentrated and purified by a normal phase system solid-phase extraction column. A 10% trifluoroacetic acid/chloroform solution was added to this intermediate before stirring at room temperature for 4 hours, followed by concentration and drying to provide 3.0 mg of the trifluoroacetate of the title compound as a pale yellow solid.
MS (Fab, Pos.): m/z = 301 [M+H]⁺

### (Example 55) Synthesis of (S)-2-amino-N-hydroxy-3-(4-propyloxyphenyl)propionamide (compound No. 49)

An operation similar to that in Example 54 was carried out using 28.8 mg (0.48 mmol) of 1-propanol in place of phenethyl alcohol to provide 4.5 mg of the trifluoroacetate of the title compound as a pale yellow solid.
MS (Fab, Pos.): m/z = 239 [M+H]⁺

### (Example 56) Synthesis of (S)-2-amino-N-hydroxy-3-{4-[2-(morpholin-4-yl)ethoxy]phenyl}propionamide (compound No. 50)

An operation similar to that in Example 54 was carried out using 63.0 mg (0.48 mmol) of 2-morpholinethanol in place of phenethyl alcohol to provide 6.9 mg of the trifluoroacetate of the title compound as a pale yellow solid.
MS (Fab, Pos.): m/z = 310 [M+H]⁺

### (Example 57) Synthesis of N^{α}-Boc-tyrosine hydroxamate O-supporting 2-chlorotrityl resin

In a solution consisting of 1.360 g (3.86 mmol) of commercial N^{α}-Boc-tyrosine and 652 mg (4.82 mmol) of HOBt dissolved in 10 ml of DMF was suspended 1.91 g (equivalent to 1.93 mmol) of the resin obtained in Example 1, to which 0.74 ml (5.79 mmol) of DIC was then added, followed by shaking at room temperature for 3 days. After the end of reaction, the reaction liquid was filtered off, followed by washing with DMF, methanol, THF, chloroform, a chloroform/methanol solution, and diethyl ether before drying. Thereto were added 5 ml of methanol, 5 ml of THF, and 5 ml of 1 mol/l sodium hydroxide aqueous solution, followed by shaking at room temperature for 24 hours. After the end of reaction, the resultant resin was washed with acetic acid/methanol, water/DMF, DMF, methanol, chloroform, and ether to provide the title resin.

### (Example 58) Synthesis of (S)-2-amino-N-hydroxy-3-(4-butyloxyphenyl)propionamide (compound No. 51)

To 200 mg (equivalent to 0.19 mmol) of the resin obtained in Example 57 were added 2 ml of DMF and 15 mg (0.38 mmol) of sodium hydride (60%), followed by shaking at room temperature for 40 minutes. Thereto was added 69.9 mg (0.38 mmol) of butane iodide, followed by stirring at room temperature for 23 hours. After the end of reaction, the solvent was removed and the resultant resin was washed with DMF/water, water/methanol, chloroform/methanol, DMF, methanol, THF, chloroform, and ether. Thereto was added a 25% trifluoroacetic acid/chloroform solution, followed by shaking at room temperature for one hour. The solution obtained by filtration and the solution with which the resin had been washed were combined, from which the solvent was then distilled off. The residue was purified by a reverse phase system solid-phase extraction column to provide 3.9 mg of the trifluoroacetate of the title compound as a pale yellow solid.
MS (Fab, Pos.): m/z = 253 [M+H]⁺

### (Example 59) Synthesis of (S)-2-amino-N-hydroxy-3-(4-pentyloxyphenyl)propionamide (compound No. 52)

An operation similar to that in Example 58 was carried out using 75.3 mg (0.38 mmol) of pentyl iodide in place of butane iodide to provide 1.8 mg of the trifluoroacetate of the title compound as a pale yellow solid.
MS (Fab, Pos.): m/z = 267 [M+H]⁺

### (Example 60) Synthesis of (S)-2-amino-N-hydroxy-3-[4-(4-fluorobenzyloxy)phenyl]propionamide (compound No. 53)

An operation similar to that in Example 58 was carried out using 54.9 mg (0.38 mmol) of 4-fluorobenzyl chloride in place of butane iodide to provide 2.8 mg of the trifluoroacetate of the title compound as a pale yellow solid.
MS (Fab, Pos.): m/z = 305 [M+H]⁺

### (Example 61) Synthesis of (S)-2-amino-N-hydroxy-3-[4-(naphthalen-2-ylmethyloxy)phenyl]propionamide (compound No. 54)

An operation similar to that in Example 58 was carried out using 67.1 mg (0.38 mmol) of 2-naphthalenemethyl chloride in place of butane iodide to provide 2.0 mg of the trifluoroacetate of the title compound as a pale yellow solid.
MS (Fab, Pos.): m/z = 337 [M+H]⁺

### (Example 62) Synthesis of (S)-2-amino-N-hydroxy-3-[4-(2-chlorobenzyloxy)phenyl]propionamide (compound No. 55)

An operation similar to that in Example 58 was carried out using 61.2 mg (0.38 mmol) of 2-chlorobenzyl chloride in place of butane iodide to provide 4.1 mg of the trifluoroacetate of the title compound as a pale yellow solid.
MS (Fab, Pos.): m/z = 321, 323 [M+H]⁺

### (Example 63) Synthesis of (S)-2-amino-N-hydroxy-3-[4-(4-trifluoromethylbenzyloxy)phenyl]propionamide (compound No. 56)

An operation similar to that in Example 58 was carried out using 73.9 mg (0.38 mmol) of trifluoromethylbenzyl chloride in place of butane iodide to provide 6.4 mg of the trifluoroacetate of the title compound as a pale yellow solid.
MS (Fab, Pos.): m/z = 355 [M+H]⁺

### (Example 64) Synthesis of (S)-2-amino-N-hydroxy-3-[4-(2,3,4,5,6-pentafluorobenzyloxy)phenyl]propionamide (compound No. 57)

An operation similar to that in Example 58 was carried out using 82.3 mg (0.38 mmol) of 2,3,4,5,6-pentafluorobenzyl chloride in place of butane iodide to provide 8.0 mg of the trifluoroacetate of the title compound as a pale yellow solid.
MS (Fab, Pos.): m/z = 377 [M+H]⁺

### (Example 65) Synthesis of N-Boc-tryptophan hydroxamate O-supporting 2-chlorotrityl resin

In a reaction vessel was placed 961.5 mg (equivalent to 1.20 mmol) of the resin obtained Example 1, to which 0.577 ml (4.8 mmol) of DIC, 0.844 g (6.2 mmol) of HOBt, and 1.262 g (3.61 mmol) of commercial N^{α}-Boc-Nⁱⁿ-formyltryptophan were then added, followed by shaking at room temperature for 20 hours. After the end of reaction, the solvent was removed, followed by washing with DMF, THF, methanol, and chloroform before drying. Thereto were added 10 ml of DMF and 0.5 ml of hydrazine monohydrate, followed by shaking for 4 hours. After the end of reaction, the solvent was removed and the resultant resin was washed with DMF, methanol, THF, methanol, chloroform, methanol, chloroform, and ether to provide the title resin.

### (Example 66) Synthesis of (S)-2-amino-3-[1-(2,4-difluorobenzyl)-1H-indol-3-yl]-N-hydroxypropionamide (compound No. 58)

In 3 ml of DMF was suspended 150 mg (equivalent to 0.13 mmol) of the resin obtained in Example 65, to which 84.7 mg (0.26 mmol) of cesium carbonate and 53.8 mg (0.26 mmol) of 2,4-difluorobenzyl bromide were then added, followed by stirring at room temperature for 2 days. After the end of reaction, the solvent was removed, followed by washing with DMF, chloroform/methanol, THF, chloroform, and ether. Thereto was added 2 ml of 30% trifluoroacetic acid/chloroform solution, followed by shaking at room temperature for 2 hours. The solution obtained by filtration and the solution with which the resin had been washed were combined, from which the solvent was then distilled off. The residue was purified by a reverse phase system solid-phase extraction column to provide 4.7 mg of the trifluoroacetate of the title compound as a pale yellow solid.
MS (Fab, Pos.): m/z = 346 [M+H]⁺

### (Example 67) Synthesis of (S)-2-amino-3-[1-(4-trifluoromethylbenzyl)-1H-indol-3-yl]-N-hydroxypropionamide (compound No. 59)

An operation similar to that in Example 66 was carried out using 62.1 mg (0.26 mmol) of 4-trifluoromethylbenzyl bromide in place of 2,4-difluorobenzyl bromide to provide 14.9 mg of the trifluoroacetate of the title compound as a pale yellow solid.
MS (Fab, Pos.): m/z = 378 [M+H]⁺

### (Example 68) Synthesis of (S)-2-amino-3-[1-(3-methyl-4-nitrobenzyl)-1H-indol-3-yl]-N-hydroxypropionamide (compound No. 60)

In 3 ml of THF was suspended 150 mg (equivalent to 0.13 mmol) of the resin obtained in Example 65, to which 48.3 mg (0.26 mmol) of 3-methyl-4-nitrobenzyl alcohol, 68.2 mg (0.26 mmol) of triphenylphosphine, and 0.118 ml (0.26 mmol) of 40% toluene solution of DEAD were then added, followed by stirring at room temperature for 24 hours. After the end of reaction, the solvent was removed, followed by washing with DMF, chloroform/methanol, THF, chloroform, and ether. Thereto was added 2 ml of 30% trifluoroacetic acid/chloroform solution, followed by shaking at room temperature for 2 hours. The solution obtained by filtration and the solution with which the resin had been washed were combined, from which the solvent was then distilled off. The residue was purified by a reverse phase system solid-phase extraction column to provide 8.6 mg of the trifluoroacetate of the title compound as a pale yellow solid.
MS (Fab, Pos.): m/z = 369 [M+H]⁺

### (Example 69) Synthesis of (S)-2-amino-3-[1-(2-phenylethyl)-1H-indol-3-yl]-N-hydroxypropionamide (compound No. 61)

An operation similar to that in Example 68 was carried out using 31.7 ml (0.26 mmol) of phenethyl alcohol in place of 3-methyl-4-nitrobenzyl alcohol to provide 13.3 mg of the trifluoroacetate of the title compound as a pale yellow solid.
MS (Fab, Pos.): m/z = 324 [M+H]⁺

### (Example 70) Synthesis of 2-Boc-amino-4-(2-aminophenylcarbamoyl)-butyric acid hydroxamate O-supporting 2-chlorotrityl resin

In 15 ml of DMF was suspended 1.1867 g (equivalent to 1.07 mmol) of the resin obtained in Example 2, to which 347 mg (3.21 mmol) of o-phenylenediamine and 433.8 mg (3.21 mmol) of HOBt were then added. Thereto was added 0.37 ml (3.21 mmol) of DIC, followed by shaking at room temperature for 3 days. After the end of reaction, the reaction liquid was filtered off, followed by washing with chloroform, DMF, and ethanol before drying to provide the title resin.

### (Example 71) Synthesis of (S)-2-amino-N-hydroxy-4-(1-propyl-1H-benzimidazol-2-yl)butyramide (compound No. 62)

In 1 ml of 5% methyl ortho-acetate/THF solution was suspended 140 mg (equivalent to 0.11 mmol) of the resin obtained in Example 70, to which 31.9 mg (0.55 mmol) of propionaldehyde was then added, followed by shaking at room temperature for 4 hours. After the end of reaction, the solvent was removed by filtration, followed by adding 1 ml of THF and a methanol solution of 36 mg (0.95 mmol) of sodium borohydride before stirring at room temperature for 3 hours. After the end of reaction, the reaction liquid was filtered off, followed by washing with methanol, DMF, THF, chloroform, a chloroform/methanol solution, and ether before drying. Thereto was added 2 ml of acetic acid, followed by stirring at 60°C for 5 hours. The reaction liquid was collected by filtration, the resultant resin was washed with 2 ml of acetic acid, and the washings were added to the reaction liquid before concentration. The purification thereof was carried out using a normal phase system solid-phase extraction column to provide an intermediate. The intermediate was dissolved in a 20% trifluoroacetic acid/chloroform solution before stirring for 2 hours, followed by distilling off the solution under reduced pressure. The solution obtained by filtration and the solution with which the resin had been washed were combined, from which the solvent was then distilled off. The residue was purified by a reverse phase system solid-phase extraction column to provide 11.7 mg of the trifluoroacetate of the title compound as a white solid.
MS (Fab, Pos.): m/z = 277 [M+H]⁺

### (Example 72) Synthesis of (S)-2-amino-N-hydroxy-4-(1-phenethyl-1H-benzimidazol-2-yl)butyramide (compound No. 63)

An operation similar to that in Example 71 was carried out using 66.1 mg (0.55 mmol) of phenacetylaldehyde in place of propionaldehyde to provide 12.4 mg of the trifluoroacetate of the title compound as a white solid.
MS (Fab, Pos.): m/z = 339 [M+H]⁺

### (Example 73) Synthesis of (S)-2-amino-N-hydroxy-4-[1-(4-nitrobenzyl)-1H-benzimidazol-2-yl]butyramide (compound No. 64)

An operation similar to that in Example 71 was carried out using 83.1 mg (0.55 mmol) of 4-nitrobenzaldehyde in place of propionaldehyde to provide 12.2 mg of the trifluoroacetate of the title compound as a white solid.
MS (Fab, Pos.): m/z = 370 [M+H]⁺

### (Example 74) Synthesis of (S)-2-amino-N-hydroxy-4-(1-pentyl-1H-benzimidazol-2-yl)butyramide (compound No. 65)

An operation similar to that in Example 71 was carried out using 47.4 mg (0.55 mmol) of valeraldehyde in place of propionaldehyde to provide 17.6 mg of the trifluoroacetate of the title compound as a white solid.
MS (Fab, Pos.): m/z = 305 [M+H]⁺

### (Example 75) Synthesis of (S)-2-amino-N-hydroxy-4-[1-(2-methoxybenzyl)-1H-benzimidazol-2-yl]butyramide (compound No. 66)

To 125 mg (equivalent to 0.150 mmol) of the resin obtained in Example 70 were added 1 ml of THF and 102 mg (0.75 mmol) of 2-methoxybenzaldehyde, to which a solution consisting of 47.1 mg (0.75 mmol) of sodium cyanoborohydride and 0.1 ml of trimethyl ortho-formate dissolved in 1 ml of methanol was then added, followed by shaking at room temperature for 2 days. After the end of reaction, the reaction liquid was filtered off, followed by washing with methanol, DMF, THF, chloroform, a chloroform/methanol solution, and ether before drying. Thereto was added 2 ml of acetic acid, followed by stirring at 60°C for 5 hours. The reaction liquid was collected by filtration, the resultant resin was washed with 2 ml of acetic acid, and the washings were added to the reaction liquid before concentration. The purification thereof was carried out using a normal phase system solid-phase extraction column to provide an intermediate. The intermediate was dissolved in a 20% trifluoroacetic acid/chloroform solution before stirring for 2 hours, followed by distilling off the solution under reduced pressure. The solution obtained by filtration and the solution with which the resin had been washed were combined, from which the solvent was then distilled off. The residue was purified by a reverse phase system solid-phase extraction column to provide 4.3 mg of the trifluoroacetate of the title compound as a white solid.
MS (Fab, Pos.): m/z = 355 [M+H]⁺

### (Example 76) Synthesis of (S)-2-amino-N-hydroxy-4-[1-(pyridin-2-ylmethyl)-1H-benzimidazol-2-yl]butyramide (compound No. 67)

An operation similar to that in Example 75 was carried out using 80.3 mg (0.75 mmol) of 2-pyridinecarbaldehyde in place of 2-methoxybenzaldehyde to provide 2.9 mg of the trifluoroacetate of the title compound as a white solid.
MS (Fab, Pos.): m/z = 326 [M+H]⁺

### (Example 77) Synthesis of (S)-2-amino-N-hydroxy-4-(1-butyl-1H-benzimidazol-2-yl)butyramide (compound No. 68)

An operation similar to that in Example 75 was carried out using 54.1 mg (0.75 mmol) of butylaldehyde in place of 2-methoxybenzaldehyde to provide 6.2 mg of the trifluoroacetate of the title compound as a white solid.
MS (Fab, Pos.): m/z = 291 [M+H]⁺
¹H-NMR (500 MHz, DMSO-d₆+D₂O): δ = 0.93 (3H, t, J = 7.3 Hz), 1.36 (2H, sext, J = 7.3 Hz), 1.75 (2H, quint, J = 7.3 Hz), 2.29 (2H, m), 3.15 (2H, m), 3.82 (1H, t, J = 6.6 Hz), 4.33 (2H, t, J = 7.3 Hz), 7.48-7.54 (2H, m), 7.75 (1H, d, J = 6.8 Hz), 7.85 (1H, d, J = 6.8 Hz).

### (Example 78) Synthesis of (S)-2-amino-N-hydroxy-4-(1-octyl-1H-benzimidazol-2-yl)butyramide (compound No. 69)

An operation similar to that in Example 75 was carried out using 96.2 mg (0.75 mmol) of octanal in place of 2-methoxybenzaldehyde to provide 5.2 mg of the trifluoroacetate of the title compound as a white solid.
MS (Fab, Pos.): m/z = 348 [M+H]⁺

### (Example 79) Synthesis of (S)-2-amino-N-hydroxy-4-(1-cyclohexylmethyl-1H-benzimidazol-2-yl)butyramide (compound No. 70)

An operation similar to that in Example 75 was carried out using 84.1 mg (0.75 mmol) of cyclohexanecarbaldehyde in place of 2-methoxybenzaldehyde to provide 3.4 mg of the trifluoroacetate of the title compound as a white solid.
MS (Fab, Pos.): m/z = 331 [M+H]⁺

### (Example 80) Synthesis of (S)-2-amino-N-hydroxy-4-[1-(2,2-dimethylpropyl)-1H-benzimidazol-2-yl]butyramide (compound No. 71)

An operation similar to that in Example 75 was carried out using 64.6 mg (0.75 mmol) of pivalaldehyde in place of 2-methoxybenzaldehyde to provide 1.8 mg of the trifluoroacetate of the title compound as a white solid.
MS (Fab, Pos.): m/z = 305 [M+H]⁺

### (Example 81) Synthesis of (S)-2-amino-N-hydroxy-4-(1-isobutyl-1H-benzimidazol-2-yl)butyramide (compound No. 72)

An operation similar to that in Example 75 was carried out using 54.1 mg (0.75 mmol) of isobutylaldehyde in place of 2-methoxybenzaldehyde to provide 4.5 mg of the trifluoroacetate of the title compound as a white solid.
MS (Fab, Pos.): m/z = 291 [M+H]⁺
¹H-NMR (500 MHz, DMSO-d₆+D₂O): δ = 0.92 (6H, d, J = 6.6 Hz), 2.18 (1H, sept, J = 6.6 Hz), 2.27-2.35 (2H, m), 3.08-3.13 (2H, m), 3.81 (1H, t, J = 6.6 Hz), 4.12 (2H, d, J = 6.6 Hz), 7.40-7.51 (2H, m), 7.72 (1H, d, J = 7.8 Hz), 7.81 (1H, d, J = 7.3 Hz).

### (Example 82) Synthesis of (S)-2-amino-N-hydroxy-4-[1-(4-fluorobenzyl)-1H-benzimidazol-2-yl]butyramide (compound No. 73)

An operation similar to that in Example 75 was carried out using 93.1 mg (0.75 mmol) of 4-fluorobenzaldehyde in place of 2-methoxybenzaldehyde to provide 12.1 mg of the trifluoroacetate of the title compound as a white solid.
MS (Fab, Pos.): m/z = 343 [M+H]⁺

### (Example 83) Synthesis of (S)-2-amino-N-hydroxy-4-[1-(4-t-butylbenzyl)-1H-benzimidazol-2-yl]butyramide (compound No. 74)

An operation similar to that in Example 75 was carried out using 121.7 mg (0.75 mmol) of 4-t-butylbenzaldehyde in place of 2-methoxybenzaldehyde to provide 7.0 mg of the trifluoroacetate of the title compound as a white solid.
MS (Fab, Pos.): m/z = 381 [M+H]⁺

### (Example 84) Synthesis of (S)-2-amino-N-hydroxy-4-(1-cyclohexyl-1H-benzimidazol-2-yl)butyramide (compound No. 75)

An operation similar to that in Example 75 was carried out using 73.6 mg (0.75 mmol) of cyclohexanone in place of 2-methoxybenzaldehyde to provide 3.4 mg of the trifluoroacetate of the title compound as a white solid.
MS (Fab, Pos.): m/z = 317 [M+H]⁺

### (Example 85) Synthesis of(S)-2-amino-N-hydroxy-4-[1-(tetrahydropyran-4-yl)-1H-benzimidazol-2-yl]butyramide (compound No. 76)

An operation similar to that in Example 75 was carried out using 75.1 mg (0.75 mmol) of tetrahydropyran-4-one in place of 2-methoxybenzaldehyde to provide 4.7 mg of the trifluoroacetate of the title compound as a white solid.
MS (Fab, Pos.): m/z = 319 [M+H]⁺

### (Example 86) Synthesis of 2-amino-N-hydroxy-4-[1-(1-methyl-pyiperidin-4-yl)-1H-benzimidazol-2-yl]butyramide (compound No. 77)

An operation similar to that in Example 75 was carried out using 4.8 mg (0.75 mmol) of 1-methylpiperidin-4-one in place of 2-methoxybenzaldehyde to provide 2.9 mg. of the trifluoroacetate of the title compound as a white solid.
MS (Fab, Pos.): m/z = 331 [M+H]⁺

### (Example 87) Synthesis of (S)-2-amino-N-hydroxy-4-[1-(2-methylbutyl)-1H-benzimidazol-2-yl]butyramide (compound No. 78)

An operation similar to that in Example 71 was carried out using 64.6 mg (0.75 mmol) of isovaleraldehyde in place of 2-methoxybenzaldehyde to provide 9.9 mg of the trifluoroacetate of the title compound as a white solid.
MS (Fab, Pos.): m/z = 305 [M+H]⁺
¹H-NMR (500 MHz, DMSO-d₆+D₂O): δ = 0.98 (6H, d, J = 6.4 Hz), 1.61-1.73 (3H, m), 2.27-2.33 (2H, m), 3.09-3.13 (2H, m), 3.81 (1H, t, J = 6.6 Hz), 4.27-4.32 (2H, m), 7.43-7.50 (2H, m), 7.73 (1H, d, J = 7.6 Hz), 7.78 (1H, d, J = 7.6 Hz).

### (Example 88) Synthesis of (S)-2-amino-N-hydroxy-4-(1-hexyl-1H-benzimidazol-2-yl)butyramide (compound No. 79)

An operation similar to that in Example 71 was carried out using 75.1 mg (0.75 mmol) of hexanal in place of 2-methoxybenzaldehyde to provide 6.3 mg of the trifluoroacetate of the title compound as a white solid.
MS (Fab, Pos.): m/z = 319 [M+H]⁺

### (Example 89) Synthesis of (S)-2-amino-N-hydroxy-4-(1-heptyl-1H-benzimidazol-2-yl)butyramide (compound No. 80)

An operation similar to that in Example 71 was carried out using 85.6 mg (0.75 mmol) of heptanal in place of 2-methoxybenzaldehyde to provide 2.1 mg of the trifluoroacetate of the title compound as a white solid.
MS (Fab, Pos.): m/z = 333 [M+H]⁺

### (Example 90) Synthesis of (S)-2-amino-4- {1-[2-(4-t-butyl-phenyl)-2-methyl-propyl]-1H-benzimidazol-2-yl}-N-hydroxybutyramide (compound No. 81)

An operation similar to that in Example 71 was carried out using 153 mg (0.75 mmol) of 2-(4-t-butylphenyl)-2-methylpropionaldehyde in place of 2-methoxybenzaldehyde to provide 3.3 mg of the trifluoroacetate of the title compound as a white solid.
MS (Fab, Pos.): m/z = 423 [M+H]⁺

### (Example 91) Synthesis of (S)-2-amino-N-hydroxy-4-[1-(naphthalen-1-ylmethyl)-1H-benzimidazol-2-yl]butyramide (compound No. 82)

An operation similar to that in Example 75 was carried out using 117 mg (0.75 mmol) of 1-naphthoaldehyde in place of 2-methoxybenzaldehyde to provide 2.8 mg of the trifluoroacetate of the title compound as a white solid.
MS (Fab, Pos.): m/z = 375 [M+H]⁺

### (Example 92) Synthesis of (S)-2-amino-N-hydroxy-4-[1-(5-chlorothiophen-2-yl)-1H-benzimidazol-2-yl]butyramide (compound No. 83)

An operation similar to that in Example 75 was carried out using 110 mg (0.75 mmol) of 5-chlorothiophene-2-carbaldehyde in place of 2-methoxybenzaldehyde to provide 4.3 mg of the trifluoroacetate of the title compound as a white solid.
MS (Fab, Pos.): m/z = 365, 367 [M+H]⁺

### (Example 93) Synthesis of 2-Boc-amino-4-(2-amino-4,5-dimethyl-phenylcarbamoyl)-butyric acid hydroxamate O-supporting 2-chlorotrityl resin

In 15 ml of DMF was suspended 2.04 g (equivalent to 1.88 mmol) of the resin obtained in Example 2, to which 1.28 g (9.39 mmol) of 4,5-dimethyl-1,2-phenylenediamine and 1.27 g (9.40 mmol) of HOBt were then added. Thereto was added 1.45 ml (9.40 mmol) of DIC, followed by shaking at room temperature for 3 days. After the end of reaction, the reaction liquid was filtered off, followed by washing with chloroform, DMF, and ether before drying to provide the title resin.

### (Example 94) Synthesis of (S)-2-amino-4-(1-cyclohexyl-5,6-dimethyl-1H-benzimidazol-2-yl)-N-hydroxybutyramide (compound No. 84)

To 240 mg (equivalent to 0.46 mmol) of the resin obtained in Example 93 were added 1 ml of chloroform and 225.7 mg (2.30 mmol) of cyclohexanone, to which a solution consisting of 45 mg (0.72 mmol) of sodium cyanoborohydride dissolved in 1 ml of methanol and adjusted to a pH of 5 using acetic acid was further added, followed by stirring at room temperature for 3 days. After the end of reaction, the reaction liquid was filtered off, followed by washing with methanol, DMF, THF, chloroform, a chloroform/methanol solution, and ether before drying. Thereto was added 2 ml of acetic acid, followed by shaking at 60°C for 5 hours. The reaction liquid was collected by filtration, the resultant resin was washed with 2 ml of acetic acid, and the washings were added to the reaction liquid before concentration. The purification thereof was carried out using a normal phase solid-phase extraction column to provide an intermediate. The intermediate was dissolved in a 25% trifluoroacetic acid/chloroform solution before stirring for 2 hours, followed by distilling off the solution under reduced pressure. The solution obtained by filtration and the solution with which the resin had been washed were combined, from which the solvent was then distilled off. The residue was purified by a reverse phase system solid-phase extraction column to provide 27.6 mg of the trifluoroacetate of the title compound as a white solid.
MS (Fab, Pos.): m/z = 345 [M+H]⁺

### (Example 95) Synthesis of (S)-2-amino-4-[1-(4-t-butylbenzyl)-5,6-dimethyl-1H-benzimidazol-2-yl]-N-hydroxybutyramide (compound No. 85)

An operation similar to that in Example 94 was carried out using 373.1 mg (2.30 mmol) of 4-t-butylbenzaldehyde in place of cyclohexanone to provide 34.1 mg of the trifluoroacetate of the title compound as a white solid.
MS (Fab, Pos.): m/z = 409 [M+H]⁺
¹H-NMR (500 MHz, DMSO-d₆+D₂O): δ = 1.24 (9H, s), 2.27-2.35 (2H, m), 2.32 (3H, s), 2.35 (3H, s), 3.10-3.18 (2H, m), 3.80 (1H, t, J = 6.6 Hz), 5.51 (2H, d, J = 11.7 Hz), 7.11 (2H, d, J = 8.3 Hz), 7.38 (2H, d, J = 8.3 Hz), 7.45 (1H, s), 7.51 (1H, s).

### (Example 96) Synthesis of (S)-2-amino-4-[1-(4-fluorobenzyl)-5,6-dimethyl-1H-benzimidazol-2-yl]-N-hydroxybutyramide (compound No. 86)

An operation similar to that in Example 94 was carried out using 285.5 mg (2.30 mmol) of 4-fluorobenzaldehyde in place of cyclohexanone to provide 23.8 mg of the trifluoroacetate of the title compound as a white solid.
MS (Fab, Pos.): m/z = 371 [M+H]⁺
¹H-NMR (500 MHz, DMSO-d₆+D₂O): δ = 2.21-2.28 (2H, m), 2.32 (3H, s), 2.35 (3H, s), 3.11-3.18 (2H, m), 3.79 (1H, t, J = 6.6 Hz), 5.55 (2H, d, J = 11.2 Hz), 7.21 (2H, t, J = 8.5 Hz), 7.28 (2H, dd, J = 5.6, 8.5 Hz), 7.45 (1H, s), 7.53 (1H, s).

### (Example 97) Synthesis of (S)-2-amino-4-[1-(4-methoxybenzyl)-5,6-dimethyl-1H-benzimidazol-2-yl]-N-hydroxybutyramide (compound No. 87)

An operation similar to that in Example 94 was carried out using 313.1 mg (2.30 mmol) of 4-methoxybenzaldehyde in place of cyclohexanone to provide 13.2 mg of the trifluoroacetate of the title compound as a white solid.
MS (Fab, Pos.): m/z = 383 [M+H]⁺

### (Example 98) Synthesis of (S)-2-amino-3-(4-amino-phenyl)-N-hydroxybutyramide (compound No. 88)

In 1 ml of methanol was dissolved 20.0 mg (0.059 mmol) of the compound obtained in Example 44, to which 4 mg of 10% palladium carbon was then added, followed by stirring at room temperature for 15 minutes under an atmosphere of hydrogen. After the end of reaction, 1 ml of distilled water was added, followed by purification using a reverse phase system solid-phase extraction column to provide 14.0 mg of the trifluoroacetate of the title compound as a white solid.
MS (Fab, Pos.): m/z = 196 [M+H]⁺
¹H-NMR (500 MHz, DMSO-d₆): δ = 2.77 (1H, dd, J = 13.7,6.6 Hz), 2.84 (1H, dd, J = 13.7, 7.6 Hz), 2.56-3.63 (1H, m), 6.55 (2H, d, J = 8.3 Hz), 6.87 (2H, d, J = 8.3 Hz), 8.23 (3H, brs), 9.27 (1H, s), 10.95 (1H, s).

### (Example 99) Synthesis of (S)-2-(cyclohexylmethyl-amino)-N-hydroxy-3-(4-nitrophenyl)propionamide (compound No. 44)

### (Example 99-1) Synthesis of 2-(cyclohexylmethyl-amino)-3-(4-nitrophenyl)propionamide methyl ester

To 1.54 g (4.75 mmol) of commercial N^{α}-Boc-4-nitrophenylalanine was added 75 ml of 10% hydrogen chloride/methanol cooled at 0°C, followed by stirring at room temperature for 5 hours. After the end of reaction, the precipitated crystals were filtered. In chloroform was suspended 203.5 mg (0.780 mmol) of the crystals, which was then washed with a 1 mol/l sodium hydroxide aqueous solution and dried over anhydrous sodium sulfate, followed by distilling off the solvent. The residue was dissolved in 3 ml of methanol, to which 0.091 ml (0.780 mmol) of cyclohexylcarbaldehyde and 0.2 ml of trimethyl ortho-formate were then added, followed by stirring at room temperature for 2 hours. Thereto was added 341.4 mg (1.61 mmol) of sodium triacetoxyborohydride, followed by further stirring at room temperature for 30 minutes. After the end of reaction, the solvent was distilled off before dissolution in chloroform, followed by washing with a 1 mol/l sodium hydroxide aqueous solution and a saturated saline solution and then drying the organic layer with anhydrous sodium sulfate before distilling off the solvent to provide 308.3 mg of the title compound as a pale yellow oil.
MS (Fab, Pos.): m/z = 321 [M+H]⁺
¹H-NMR (500 MHz, DMSO-d₆): δ = 0.73-0.83 (2H, m), 1.05-1.20 (3H, m), 1.20-1.31 (1H, m), 1.54-1.69 (5H, m), 2.19 (1H, dd, J =11.2, 6.6 Hz), 2.33 (1H, dd, J =11.2, 6.8 Hz), 2.97 (2H, d, J = 7.1 Hz), 3.45 (1H, dd, J = 6.8, 6.6 Hz), 3.58 (3H, s), 7.49 (2H, d, J =8.8 Hz), 8.14 (2H, d, J = 8.8 Hz)

### (Example 99-2) Synthesis of (S)-2-(cyclohexylmethyl-amino)-3-(4-nitrophenyl)-N-trityloxypropionamide

In 5 ml of DMF was dissolved 308.3 mg of the compound obtained in Example 99-1, to which 188.2 mg (0.862 mmol) of di-t-butyldicarbonate was then added, followed by stirring at 60°C for 17 hours. After the end of reaction, the solvent was distilled off, and the residue was dissolved in 3 ml ofTHF, to which 3 ml of methanol and 3 ml of 1 mol/l sodium hydroxide aqueous solution were then added, followed by stirring at room temperature for 2.5 hours. After the end of reaction, the solvent was distilled off, and the residue was dissolved in distilled water, which was then adjusted to a pH of 5 using a 1 mol/l hydrochloric acid aqueous solution. The resultant solution was extracted with chloroform, and the organic layer was dried over anhydrous sodium sulfate.
In 4.5 ml of chloroform was dissolved 149.1 mg (0.367 mmol) thereof, to which 105.9 mg (0.554 mmol) of WSCI hydrochloride and 106.7 mg (0.387 mmol) of O-tritylhydroxylamine were then added, followed by stirring at room temperature for one day. After the end of reaction, chloroform was added, followed by washing with a 1 mol/l hydrochloric acid aqueous solution, a.1 mol/l sodium hydroxide aqueous solution, and a saturated saline solution before drying the organic layer with anhydrous sodium sulfate. The solvent was distilled off, followed by purifying the residue using silica gel column chromatography (hexane/ethyl acetate) to provide 61.6 mg of a desired compound as a colorless, viscous material.
MS (Fab, Pos.): m/z = 322 [M+H]⁺
¹H-NMR (500 MHz, DMSO-d₆): δ = 0.51-0.70 (2H, m), 0.96-1.12 (4H, m), 1.37-1.49 (2H, m), 1.49-1.60 (3H, m), 1.77-1.83 (1H, m), 1.85-1.91 (1H, m), 2.54 (2H, d, J = 6.8 Hz), 3.06 (1H, dd, J = 7.1, 6.6 Hz), 7.25-7.38 (17H, m), 8.03 (2H, d, J = 8.8 Hz).

### (Example 99-3) Synthesis of (S)-2-(cyclohexylmethylamino)-N-hydroxy-3-(4-nitrophenyl)propionamide

In 1.5 ml of 30% trifluoroacetic acid/chloroform solution was dissolved 52.1 mg (0.092 mmol) of the compound obtained in Example 99-2, followed by stirring at room temperature for 40 minutes. After the end of reaction, the solvent was distilled off, water was added, and the aqueous layer was washed with chloroform. The resultant aqueous layer was concentrated, and dried under reduced pressure after azeotroping with methanol to provide 15.9 mg of the trifluoroacetate of the title compound as a white solid.
MS (Fab, Pos.): m/z = 322 [M+H]+
¹H-NMR (500 MHz, DMSO-d₆): δ = 0.87-1.00 (2H, m), 1.05-1.18 (4H, m), 1.58-1. 77 (4H, m), 1. 79 (1H, brd, J = 12.7 Hz), 2.54-2.64 (1H, m), 2.64-2.75 (1H, m), 3.14 (1H, dd, J = 13.2, 9.8 Hz), 3.33 (1H, dd, J = 13.2, 4.6 Hz),, 3.77-3.85 (1H, m), 7.47 (2H, d, J = 8.8 Hz), 8.21 (2H, d, J = 8.8 Hz), 9.14 (2H, brs), 9.35 (1H, brs), 11.00 (1H, s).

### (Example 100) Synthesis of (S)-2-amino-4-(1-methyl-1H-benzimidazol-2-yl)-N-hydroxybutyramide (compound No. 89)

### (Example 100-1) Synthesis of (S)-2-t-butoxycarbonylamino-4-(1H-benzimidazol-2-yl)-butyric acid benzyl ester

In 30 ml of chloroform was dissolved 3.02 g (8.95 mmol) of commercial N^{α}-Boc-glutamic acid benzyl ester, to which 2.55 g (13.3 mmol) of WSCI hydrochloride and 1.32 g (9.78 mmol) of HOBt were then added, followed by reaction at room temperature for 15 minutes. This solution was added dropwise to 30 ml of a chloroform solution of 1.154 g (10.7 mmol) of 1,2-phenylenediamine at room temperature over a period of one hour. It continued to be stirred at room temperature for further 14 hours and was then washed with a 1 mol/l sodium hydroxide aqueous solution and a saturated saline solution, followed by drying the organic layer with anhydrous sodium sulfate. It was dissolved in 90 ml of acetic acid, which was then stirred at 60°C for 2.5 hours. After the end of reaction, the solvent was distilled off and azeotropically boiled with toluene, followed by dissolving the residue in chloroform before washing with a 1 mol/1 sodium hydroxide aqueous solution and a saturated saline solution. The organic layer was dried over anhydrous sodium sulfate before distilling off the solvent, followed by purifying the residue using silica gel column chromatography (chloroform/ethyl acetate) to provide 2.27 g of the title compound as a white foam.
MS (Fab, Pos.): m/z = 410 [M+H]⁺
¹H-NMR(500 MHz, DMSO-d₆): δ = 1.38 (9H, s), 2.02-2.15 (2H, m), 2.22-2.34 (2H, m), 2.83-2.94 (2H, m), 4.12-4.18 (1H, m), 5.11 (1H, d, J = 12.7 Hz), 5.16 (1H, d, J = 12.7 Hz), 7.07-7.17 (2H, m), 7.30-7.43 (6H, m), 7.52 (2H, d, J = 7.8 Hz), 12.17 (1H, s).

### (Example 100-2) Synthesis of (S)-2-t-butoxycarbonylamino-4-(1-methyl-1H-benzimidazol-2-yl)-butyric acid benzyl ester

In 15 ml of THF was dissolved 501.4 mg (1.22 mmol) of the compound obtained in Example 100-1, to which 51.3 mg of sodium hydride (60%) was then added, followed by stirring at room temperature for 10 minutes. Thereto was then added 0.115 ml (1.83 mmol) of iodomethane, followed by stirring at room temperature for 3 hours. After the end of reaction, the solvent was distilled off before adding chloroform, followed by washing with water and a saturated saline solution. The organic layer was dried over anhydrous sodium sulfate, followed by purifying the residue using silica gel column chromatography (chloroform/ethyl acetate) to provide 239.5 mg of the title compound as a white foam.
MS (Fab, Pos.): m/z = 425 [M+H]⁺
¹H-NMR (500 MHz, DMSO-d₆): δ = 1.38 (9H, s), 2.10-2.20 (2H, m), 2.22-2.32 (2H, m), 2.86-2.98 (2H, m), 3.68 (3H, s), 4.17-4.23 (1H, m), 5.10 (1H, d, J = 12.7 Hz), 5.17 (1H, d, J = 12.7 Hz), 7.14 (1H, td, J = 7.6, 1.2 Hz), 7.19 (1H, dd, J = 7.3, 1.2 Hz), 7.31-7.42 (4H, m), 7.47 (1H, d, J = 8.1 Hz), 7.54 (2H, d, J = 7.6 Hz).

### (Example 100-3) Synthesis of (S)-2-t-butoxycarbonylamino-4-(1-methyl-1H-benzimidazol-2-yl)-butyric acid

In 5 ml of ethanol was dissolved 102.2 mg (0.241 mmol) of the compound obtained in Example 100-2, to which 19.8 mg of 10% palladium carbon was then added, followed by stirring at room temperature for 30 minutes under an atmosphere of hydrogen. After the end of reaction, the catalyst was removed by celite filtration, followed by concentrating the solvent to provide 74.4 mg of the title compound as a white solid.
MS (Fab, Pos.): m/z = 334 [M+H]⁺
¹H-NMR (500 MHz, DMSO-d₆): δ = 1.35 (9H, s), 2.02-2.15 (2H, m), 2.19-2.29 (2H, m), 2.85-3.00 (2H, m), 3.72 (3H, s), 4.00-4.09 (1H, m), 7.16 (1H, td, J = 7.8, 1.2 Hz), 7.20 (1H, dd, J = 7.8, 1.2 Hz), 7.33 (1H, d, J = 8.1 Hz), 7.49 (1H, d, J = 7.6 Hz), 7.55 (1H, d, J = 7.3 Hz).

### (Example 100-4) Synthesis of (S)-2-t-butoxycarbonylamino-4-(1-methyl-1H-benzimidazol-2-yl)-N-trityloxybutyramide

In 2.1 ml of chloroform was dissolved 70.1 mg (0.210 mmol) of the compound obtained in Example 100-3, to which 60.4 mg (0.315 mmol) of WSCI hydrochloride, 34.0 mg (0.252 mmol) of HOBt, and 63.3 mg (0.210 mmol) of O-tritylhydroxylamine were then added, followed by stirring at room temperature for 19 hours. After the end of reaction, the solvent was distilled off before dissolving the residue in chloroform, followed by washing with a 1 mol/l hydrochloric acid aqueous solution, a 1 mol/l sodium hydroxide aqueous solution, and a saturated saline solution. The organic layer was dried over anhydrous sodium sulfate before distilling off the solvent, followed by purifying the residue using silica gel column chromatography (chloroform/ethyl acetate) to provide 105.0 mg of the title compound as a white foam.
¹H-NMR (500 MHz, DMSO-d₆): δ = 1.39 (9H, s), 1.35-1.41 (2H, m), 1.62-1.79 (2H, m), 2.50-2.65 (2H, m), 3.60 (3H, s), 3.91-3.97 (1H, m), 7.01 (1H, d, J = 8.1 Hz), 7.15 (1H, td, J = 7.1, 1.2 Hz), 7.17 (1H, dd, J = 7.1, 1.2 Hz), 7.23-7.45 (15H, m), 7.48 (1H, d, J = 7.8 Hz), 7.49 (1H, d, J = 7.8 Hz), 10.44 (1H, s).

### (Example 100-5) Synthesis of (S)-2-t-amino-4-(1-methyl-1H-benzimidazol-2-yl)-N-hydroxybutyramide

In 1 ml of 20% trifluoroacetic acid/chloroform solution was dissolved 90.7 mg (0.154 mmol) of the compound obtained in Example 100-4, followed by stirring at room temperature for one hour. After the end of reaction, the solvent was distilled off, water was added, and the aqueous layer was washed with chloroform. The resultant aqueous layer was concentrated before redissolving in water, followed by purification using a reverse phase system solid-phase extraction column to provide 52.3 mg of the trifluoroacetate of the title compound as a white solid.
MS (Fab, Pos.): m/z = 249 [M+H]⁺

### (Example 101) Synthesis of (S)-2-propylamino-4-(1-propyl-1H-benzimidazol-2-yl)-N-hydroxybutyramide (compound No. 90)

### (Example 101-1) Synthesis of (S)-2-t-butoxycarbonylamino-4-(1-propyl-1H-benzimidazol-2-yl)-butyric acid benzyl ester

In 51 ml of anhydrous THF was dissolved 1.728 g (4.22 mmol) of the compound obtained in Example 100-1, to which 186 mg (4.64 mmol) of sodium hydride (60%) and 0.823 ml of 1-iodopropane were then added, followed by stirring at room temperature for 1.5 hours. After the end of reaction, a dilute hydrochloric acid aqueous solution was added. The resultant solution was extracted with chloroform, followed by washing the organic layer with a saturated saline solution before drying with anhydrous sodium sulfate. The solvent was distilled off, followed by purifying the residue using silica gel column chromatography (chloroform/ethyl acetate) to provide 199.4 mg of the title compound as a light brown, viscous oil.
MS (Fab, Pos.): m/z = 452 [M+H]⁺
¹H-NMR (500 MHz, DMSO-d₆): δ = 0.85 (3H, t, J = 7.3 Hz), 1.24 and 1.38 (9H, s), 1.68 (2H, sext, J = 7.3 Hz), 2.11-2.20 (1H, m), 2.27-2.35 (1H, m), 2.87-2.99 (2H, m), 4.08 (2H, t, J = 7.3 Hz), 4.18-4.27 (1H, m), 5.10 (1H, d, J = 12.5 Hz), 5.17 (1H, d, J = 12.5 Hz), 7.14 (1H, td, J = 7.6, 1.2 Hz), 7.17 (1H, td, J = 7.6, 1.2 Hz), 7.33-7.42 (5H, m), 7.50 (1H, d, J = 7.6 Hz), 7.55 (2H, d, J = 7.8 Hz).

### (Example 101-2) Synthesis of (S)-2-(N-t-butoxycarbonyl-propylamino)-4-(1-propyl-1H-benzimidazol-2-yl)-butyric acid benzyl ester

In 2 ml of 4 mol/l hydrogen chloride/dioxane was dissolved 195.8 mg (0.434 mmol) of the compound obtained in Example 101-1, followed by stirring at room temperature for 30 minutes. After the end of reaction, the solvent was distilled off, distilled water was added, and the aqueous layer was washed with chloroform. Thereto was added a 1 mol/l sodium hydroxide aqueous solution so as to provide a pH of 14, followed by extraction with chloroform. The organic layer was dried over anhydrous sodium sulfate, concentrated, and dried under reduced pressure. It was dissolved in 4.5 ml of methanol, to which 0.033 ml (0.481 mmol) of propylaldehyde and 0.1 ml of trimethyl ortho-formate were then added, followed by stirring at room temperature for 75 minutes. It was cooled to 0°C, to which 110 mg of sodium triacethoxyborohydride was then added, followed by stirring at room temperature for 45 minutes. After the end of reaction, chloroform was added, followed by washing with a 1 mol/l sodium hydroxide aqueous solution and a saturated saline solution. It was dissolved in 5 ml of DMF, to which 280 mg (1.30 mmol) of di-t-butyldicarbonate was then added, followed by stirring at room temperature for 16.5 hours. After the end of reaction, the solvent was distilled off, followed by purifying the residue using silica gel column chromatography (hexane/ethyl acetate) to provide 123.1 mg of the title compound as a colorless foam.
MS (Fab, Pos.): m/z = 494 [M+H]⁺

### (Example 101-3) Synthesis of (S)-2-(N-t-butoxycarbonyl-propylamino)-4-(1-propyl-1H-benzimidazol-2-yl)-butyric acid

In 6 ml of ethanol was dissolved 123.1 mg (0.249 mmol) of the compound obtained in Example 101-2, to which 37 mg of 10% palladium carbon was then added, followed by stirring at room temperature for 15 minutes under an atmosphere of hydrogen. After the end of reaction, the catalyst was removed by celite filtration, followed by concentrating the solution before azeotroping with toluene to provide 87.4 mg of the title compound as a colorless, viscous material.
MS (Fab, Pos.): m/z = 404 [M+H]⁺
¹H-NMR (500 MHz, DMSO-d₆): δ = 0.78-0.86 (3H, m), 0.87 (3H, t, J = 7.3 Hz), 1.31 and 1.40 (9H, s), 1.40-1.61 (2H, m), 1.67-1.68 (2H, m), 2.12-2.24 (1H, m), 2.44-2.57 (1H, m), 2.82-2.95 (2H, m), 2.94-3.08 (1H, m), 3.13-3.28 (1H, m), 4.31-4.20 (2H, m), 4.20-4.28 and 4.30-4.39 (1H, m), 7.13-7.28 (2H, m), 7.54 (1H, d, J = 7.8 Hz), 7.57 (1H, d, J = 7.6 Hz).

### (Example 101-4) Synthesis of(S)-2-(N-t-butoxycarbonyl-propylamino)-4-(1-propyl-1H-benzimidazol-2-yl)-N-benzyloxybutyramide

In 3 ml of chloroform was dissolved 68.0 mg (0.169 mmol) of the compound obtained in Example 101-3, to which 48.5 mg (0.253 mmol) of WSCI hydrochloride and 25.0 mg (0.204 mmol) of O-benzylhydroxylamine were then added, followed by stirring at room temperature for 4 hours. After the end of reaction, chloroform was added, followed by washing with a 0.5 mol/l hydrochloric acid aqueous solution and a saturated saline solution before drying the organic layer with anhydrous sodium sulfate. The solvent was distilled off, followed by purifying the residue using silica gel column chromatography (chloroform/methanol) to provide 79.5 mg of the title compound as a colorless oil.
MS (Fab, Pos.): m/z = 509 [M+H]⁺
¹H-NMR (500 MHz, DMSO-d₆): δ = 0.80 (3H, t, J = 7.3 Hz), 0.92 (3H, t, J = 7.3 Hz), 1.20-1.58 (11H, m), 1.73-1.82 (2H, m), 2.07-2.20 (1H, m), 2.33-2.52 (1H, m), 2.93-3.20 (4H, m), 4.20-4.34 (1H, m), 4.43-4.42 (1H, m), 4.90 (2H, brs), 7.31-7.49 (7H, m), 7.68-7.75 (1H, m), 7.79-7.91 (1H, m), 11.4 (1H, brs).

### (Example 101-5) Synthesis of (S)-2-(N-t-butoxycarbonyl-propylamino)-4-(1-propyl-1H-benzimidazol-2-yl)-N-hydroxybutyramide

In 2.5 ml of ethanol was dissolved 49.8 mg (0.0979 mmol) of the compound obtained in Example 101-4, to which 15 mg of 10% palladium carbon was then added, followed by stirring at room temperature for 50 minutes under an atmosphere of hydrogen. After the end of reaction, the catalyst was removed by celite filtration, followed by concentrating the solvent before drying under reduced pressure to provide 43.0 mg of the title compound as a light purple solid.
MS (Fab, Pos.): m/z = 419 [M+H]⁺

### (Example 101-6) Synthesis of (S)-2-propylamino-4-(1-propyl-1H-benzimidazol-2-yl)-N-hydroxybutyramide

In 0.5 ml of 20% trifluoroacetic acid/chloroform solution was dissolved 38.0 mg (0.0908 mmol) of the compound obtained in Example 101-5, followed by stirring at room temperature for 30 minutes. After the end of reaction, the solvent was distilled off, and the residue was dissolved in water, followed by purification using a reverse phase system solid-phase extraction column to provide 14.9 mg of the trifluoroacetate of the title compound as a white solid.
MS (Fab, Pos.): m/z = 319 [M+H]⁺
¹H-NMR (500 MHz, DMSO-d₆+D₂O): δ = 0.94 (6H, t, J = 7.6 Hz), 1.67 (2H, sext, J = 7.6 Hz), 1.80 (2H, sext, J = 7.6 Hz), 2.28-2.40 (1H, m), 2.40-2.47 (1H, m), 2.77-2.83 (1H, m), 2.86-2.92 (1H, m), 3.14 (2H, t, J = 7.6 Hz), 3.77-3.82 (1H, m), 4.30 (2H, t, J = 7.3 Hz), 7.46-7.5 5 (2H, m), 7.75 (1H, d, J = 8.0 Hz), 7.87 (1H, d, J = 7.1 Hz).

### (Example 102) Synthesis of (S)-2-(cyclohexylmethyl-amino)-4-(1-methyl-1H-benzimidazol-2-yl)-N-hydroxybutyramide (compound No. 91)

### (Example 102-1) Synthesis of (S)-2-amino-4-(1-methyl-1H-benzimidazol-2-yl)-butyric acid benzyl ester

In 2 ml of 4 mol/l hydrogen chloride/dioxane was dissolved 508.7 mg (1.20 mmol) of the compound obtained in Example 100-2, followed by stirring at room temperature for 30 minutes. After the end of reaction, the solvent was distilled off, distilled water was added, and the aqueous layer was washed with chloroform. Thereto was added a 1 mol/l sodium hydroxide aqueous solution so as to provide a pH of 14, followed by extraction with chloroform. The organic layer was dried over anhydrous sodium sulfate, concentrated, and dried under reduced pressure to provide 400.4 mg of the title compound as a colorless oil.

### (Example 102-2) Synthesis of (S)-2-(N-t-butoxycarbonyl-cyclohexylmethyl-amino)-4-(1-methyl-1H-benzimidazol-2-yl)-butyric acid benzyl ester

In 3 ml of dichloroethane was dissolved 132.9 mg (0.411 mmol) of the compound obtained in Example 102-1, to which 0.0496 ml (0.411 mmol) of cyclohexylcarbaldehyde and 0.1 ml of trimethyl ortho-formate were then added, followed by stirring at room temperature for 50 minutes. Thereto was added 174 mg (0.822 mmol) of sodium triacetoxyborohydride, followed by stirring at room temperature for 20 minutes. After the end of reaction, chloroform was added, followed by washing with a 1 mol/l sodium hydroxide aqueous solution and a saturated saline solution. It was dissolved in 3 ml of DMF, to which 179 mg (0.822 mmol) of di-t-butyldicarbonate was then added, followed by stirring at room temperature for 16 hours. After the end of reaction, the solvent was distilled off, followed by purifying the residue using silica gel column chromatography (hexane/ethyl acetate) to provide 127.7 mg of the title compound as a colorless foam.
MS (Fab, Pos.): m/z = 520 [M+H]⁺

### (Example 102-3) Synthesis of (S)-2-(N-t-butoxycarbonyl-cyclohexylmethylamino)-4-(1-methyl-1H-benzimidazol-2-yl)-butyric acid

In 6 ml of ethanol was dissolved 127.7 mg (0.246 mmol) of the compound obtained in Example 102-2, to which 39 mg of 10% palladium carbon was then added, followed by stirring at room temperature for 30 minutes under an atmosphere of hydrogen. After the end of reaction, the catalyst was removed by celite filtration, and the solution was concentrated, followed by purifying the residue using silica gel column chromatography (chloroform/methanol) to provide 31.4 mg of the title compound as a white solid.
MS (Fab, Pos.): m/z = 430 [M+H]⁺

### (Example 102-4) Synthesis of (S)-2-(N-t-butoxycarbonyl-cyclohexylmethylamino)-4-(1-methyl-1 H-benzimidazol-2-yl)-N-benzyloxybutyramide

In 1 ml of chloroform was dissolved 31.4 mg (0.0731 mmol) of the compound obtained in Example 102-3, to which 16.8 mg (0.088 mmol) of WSCI hydrochloride and 10.8 mg (0.088 mmol) of O-benzylhydroxylamine were then added, followed by stirring at room temperature for 4 hours. After the end of reaction, chloroform was added, followed by washing with a 0.5 mol/l hydrochloric acid aqueous solution and a saturated saline solution before drying the organic layer with anhydrous sodium sulfate. The solvent was distilled off, followed by purifying the residue using silica gel column chromatography (chloroform/ethyl acetate) to provide 28.7 mg of the title compound as a colorless oil.
MS (Fab, Pos.): m/z = 535 [M+H]⁺

### (Example 102-5) Synthesis of (S)-2-(cyclohexylmethylamino)-4-(1-methyl-1H-benzimidazol-2-yl)-N-hydroxybutyramide

In 1.5 ml of ethanol was dissolved 28.7 mg (0.0537 mmol) of the compound obtained in Example 102-4, to which 9 mg of 10% palladium carbon was then added, followed by stirring at room temperature for 30 minutes under an atmosphere of hydrogen. After the end of reaction, the catalyst was removed by celite filtration, and the solvent was concentrated before drying under reduced pressure. It was dissolved in 0.5 ml of 20% trifluoroacetic acid/chloroform solution, followed by stirring at room temperature for 5 hours. After the end of reaction, the solvent was distilled off, and the residue was dissolved in water, followed by purification using a reverse phase system solid-phase extraction column to provide 10.3 mg of the trifluoroacetate of the title compound as a white solid.
MS (Fab, Pos.): m/z = 345 [M+H]⁺
¹H-NMR (500 MHz, DMSO-d₆+D₂O): δ = 0.99-1.01 (2H, m), 1.09-1.29 (3H, m), 1.60-1.84 (6H, m), 2.27-2.43 (2H, m), 2.65 (1H, dd, J = 12.2,7.1 Hz), 2.80 (1H, dd, J = 12.2, 6.6 Hz) 3.10-3.16 (2H, m), 3.72-3.76 (1H, m), 3.87 (3H, s), 7.44-7.53 (2H, m), 7.71 (1H, d, J = 7.1 Hz), 7.81 (1H, d, J = 7.3 Hz).

### (Example 103) Synthesis of (S)-2-propylamino-4-(1-methyl-1H-benzimidazol-2-yl)-N-hydroxybutyramide (compound No. 92)

### (Example 103-1) Synthesis of (S)-2-(N-t-butoxycarbonyl-propylamino)-4-(1-methyl-1H-benzimidazol-2-yl)-butyric acid benzyl ester

In 3 ml of dichloroethane was dissolved 121.2 mg (0.375 mmol) of the compound obtained in Example 102-1, to which 0.026 ml (0.375 mmol) of propylaldehyde and 0.1 ml of trimethyl ortho-formate were then added, followed by stirring at room temperature for 30 minutes. Thereto was added 159 mg (0.750 mmol) of sodium triacetoxyborohydride, followed by stirring at room temperature for 20 minutes. After the end of reaction, chloroform was added, followed by washing with a 1 mol/l sodium hydroxide aqueous solution and a saturated saline solution. It was dissolved in 3 ml of DMF, to which 164 mg (0.750 mmol) of di-t-butyldicarbonate was then added, followed by stirring at room temperature for 15 hours. After the end of reaction, the solvent was distilled off, followed by purifying the residue using silica gel column chromatography (chloroform/ethyl acetate) to provide 81.9 mg of the title compound as a colorless foam.
MS (Fab, Pos.): m/z = 466 [M+H]⁺

### (Example 103-2) Synthesis of (S)-2-(N-t-butoxycarbonyl-propylamino)-4-(1-methyl-1H-benzimidazol-2-yl)-butyric acid

In 4.2 ml of ethanol was dissolved 81.9 mg (0.176 mmol) of the compound obtained in Example 103-1, to which 24.6 mg of 10% palladium carbon was then added, followed by stirring at room temperature for 100 minutes under an atmosphere of hydrogen. After the end of reaction, the catalyst was removed by celite filtration, followed by concentrating the solution before azeotroping with chloroform to provide 55.8 mg of the title compound as a white solid.
MS (Fab, Pos.): m/z = 376 [M+H]⁺

### (Example 103-3) Synthesis of (S)-2-(N-t-butoxycarbonyl-propyl-amino)-4-(1-methyl-1H-benzimidazol-2-yl)-N-benzyloxybutyramide

In 1.5 ml of chloroform was dissolved 55.8 mg (0.141 mmol) of the compound obtained in Example 103-2, to which 34.2 mg (0.169 mmol) of WSCI hydrochloride and 22.0 mg (0.169 mmol) of N-benzylhydroxylamine were then added, followed by stirring at room temperature for 5.5 hours. After the end of reaction, chloroform was added, followed by washing with distilled water and a saturated saline solution before drying the organic layer with anhydrous sodium sulfate. The solvent was distilled off, followed by purifying the residue using silica gel column chromatography (chloroform/ethyl acetate) to provide 40.4 mg of the title compound as a colorless oil.
MS (Fab, Pos.): m/z = 481 [M+H]⁺

### (Example 103-4) Synthesis of (S)-2-propylamino-4-(1-methyl-1H-benzimidazol-2-yl)-N-hydroxybutyramide

In 2 ml of ethanol was dissolved 40.4 mg (0.0841 mmol) of the compound obtained in Example 103-2, to which 12 mg of 10% palladium carbon was then added, followed by stirring at room temperature for 5 hours under an atmosphere of hydrogen. After the end of reaction, the catalyst was removed by celite filtration, and the solvent was concentrated before drying under reduced pressure. It was dissolved in 0.6 ml of 20% trifluoroacetic acid/chloroform solution, followed by stirring at room temperature for 5 hours. After the end of reaction, the solvent was distilled off, and the residue was dissolved in water, followed by purification using a reverse phase system solid-phase extraction column to provide 21.8 mg of the trifluoroacetate of the title compound as a white solid.
MS (Fab, Pos.): m/z = 291 [M+H]⁺
¹H-NMR (500 MHz, DMSO-d₆+D₂O): δ = 0.94 (3H, t, J = 7.6 Hz), 1.65 (2H, sext, J = 7.6 Hz), 2.25-2.42 (2H, m), 2.77-2.82 (1H, m), 2.85-2.90 (1H, m), 3.12 (2H, t, J = 7.6 Hz), 3.73-3.77 (1H, m), 3.87 (3H, s), 7.47-7.56 (2H, m), 7.75 (1H, d, J = 6.6 Hz), 7.82 (1H, d, J = 7.6 Hz).

### (Example 104) Synthesis of (S)-2-octylamino-4-(1-methyl-1H-benzimidazol-2-yl)-N-hydroxybutyramide (compound No. 93)

### (Example 104-1) Synthesis of (S)-2-(N-t-butoxycarbonyl-octylamino)-4-(1-methyl-1H-benzimidazol-2-yl)-butyric acid benzyl ester

In 3 ml of dichloroethane was dissolved 104.5 mg (0.323 mmol) of the compound obtained in Example 102-1, to which 0.0511 ml (0.323 mmol) of n-caprylaldehyde and 0.1 ml of trimethyl ortho-formate were then added, followed by stirring at room temperature for 30 minutes. Thereto was added 137 mg (0.646 mmol) of sodium triacetoxyborohydride, followed by stirring at room temperature for 35 minutes. After the end of reaction, chloroform was added, followed by washing with a 1 mol/l sodium hydroxide aqueous solution and a saturated saline solution. It was dissolved in 3 ml of DMF, to which 141 mg (0.646 mmol) of di-t-butyldicarbonate was then added, followed by stirring at room temperature for 15 hours. After the end of reaction, the solvent was distilled off, followed by purifying the residue using silica gel column chromatography (hexane/ethyl acetate) to provide 77.8 mg of the title compound as a colorless foam.
MS (Fab, Pos.): m/z = 536 [M+H]⁺

### (Example 104-2) Synthesis of (S)-2-(N-t-butoxycarbonyl-octylamino)-4-(1-methyl-1H-benzimidazol-2-yl)-butyric acid

In 3.9 ml of dioxane was dissolved 77.8 mg (0.145 mmol) of the compound obtained in Example 104-1, to which 23 mg of 10% palladium carbon was then added, followed by stirring at room temperature for 4 hours under an atmosphere of hydrogen. After the end of reaction, the catalyst was removed by celite filtration, followed by concentrating the solution before azeotroping with chloroform to provide 48.8 mg of the title compound as a white solid.
MS (Fab, Pos.): m/z = 446 [M+H]⁺

### (Example 104-3) Synthesis of (S)-2-(N-t-butoxycarbonyl-octylamino)-4-(1-methyl-1H-benzimidazol-2-yl)-N-benzyloxybutyramide

In 1.5 ml of chloroform was dissolved 47.3 mg (0.106 mmol) of the compound obtained in Example 104-2, to which 24.7 mg (0.137 mmol) of WSCI hydrochloride and 16.0 mg (0.137 mmol) of O-benzylhydroxylamine were then added, followed by stirring at room temperature for 3 hours. After the end of reaction, chloroform was added, followed by washing with distilled water and a saturated saline solution before drying the organic layer with anhydrous sodium sulfate. The solvent was distilled off, followed by purifying the residue using silica gel column chromatography (hexane/ethyl acetate) to provide 12.4 mg of the title compound as a colorless oil.
MS (Fab, Pos.): m/z = 551 [M+H]⁺

### (Example 104-4) Synthesis of (S)-2-octylamino-4-(1-methyl-1H-benzimidazol-2-yl)-N-hydroxybutyramide

In 2 ml of ethanol was dissolved 12.4 mg (0.0225 mmol) of the compound obtained in Example 104-3, to which 15 mg of 10% palladium carbon was then added, followed by stirring at room temperature for 40 minutes under an atmosphere of hydrogen. After the end of reaction, the catalyst was removed by celite filtration, and the solvent was concentrated before drying under reduced pressure. It was dissolved in 0.3 ml of 20% trifluoroacetic acid/chloroform solution, followed by stirring at room temperature for 6 hours. After the end of reaction, the solvent was distilled off, and the residue was dissolved in water/methanol, followed by purification using a reverse phase system solid-phase extraction column to provide 7.5 mg of the trifluoroacetate of the title compound as a white solid.
MS (Fab, Pos.): m/z = 361 [M+H]⁺
¹H-NMR (500 MHz, DMSO-d₆+D₂O): δ = 0.87 (3H, t, J = 7.3 Hz), 1.20-1.38 (10H, m), 1.63 (2H, quint, J = 7.3 Hz), 2.24-2.42 (2H, m), 2.77-2.82 (1H, m), 2.84-2.92 (1H, m), 3.08-3.15 (3H, m), 3.73-3.77 (1H, m), 3.85 (3H, s), 7.47-7.56 (2H, m), 7.72 (1H, d, J = 7.3 Hz), 7.78 (1H, d, J = 8.1 Hz).

### (Example 105) Synthesis of (S)-2-amino-4-(1-propyl-1H-benzimidazol-2-yl)-N-hydroxybutyramide (compound No. 62)

### (Example 105-1) Synthesis of (S)-2-t-butoxycarbonylamino-4-(2-aminophenylcarbamoyl)-butyric acid benzyl ester

In 50 ml of chloroform was dissolved 5.00 g (14.8 mmol) of commercial N^{α}-Boc-glutamic acid benzyl ester, to which 4.26 g (22.2 mmol) of WSCI hydrochloride and 2.20 g (16.3 mmol) of HOBt were then added, followed by reaction at room temperature for 15 minutes. This solution was added dropwise to 50 ml of a chloroform solution of 1.92 g (17.8 mmol) of 1,2-phenylenediamine at room temperature over a period of 1.2 hours. It continued to be stirred at room temperature for further 15 hours and was then washed with distilled water, a 1 mol/l sodium hydroxide aqueous solution, and a saturated saline solution, followed by drying the organic layer with anhydrous sodium sulfate before distilling off the solvent. The residue was recrystallized from ethyl acetate/hexane to provide 3.73 g of the title compound as a white solid.

### (Example 105-2) Synthesis of (S)-2-t-butoxycarbonylamino)-4-(2-propylaminophenylcarbamoyl)-butyric acid benzyl ester

In 30 ml of dichloroethane was dissolved 1.1095 g (2.38 mmol) of the compound obtained in Example 105-1, to which 0.368 ml (2.62 mmol) of propionaldehyde was then added, followed by stirring at room temperature for 50 minutes. Then, 718 mg (3.39 mmol) of sodium triacetoxyborohydride was added before stirring at room temperature for 5 minutes, followed by adding 4 ml of a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, followed by purifying the residue using silica gel column chromatography (hexane/ethyl acetate) to provide 618.7 mg of the title compound as a white solid.

### (Example 105-3) Synthesis of (S)-2-t-butoxycarbonylamino-4-(1-propyl-1H-benzimidazol-2-yl)-butyric acid benzyl ester

In 15.5 ml of acetic acid was dissolved 793.3 mg (1.69 mmol) of the compound synthesized in Example 105-2, followed by stirring at 60°C for one hour. After the end of reaction, the solvent was distilled off, and dissolved in chloroform after azeotroping with toluene, followed by washing with a 1 mol/l sodium hydroxide aqueous solution and a saturated saline solution. It was dried over anhydrous sodium sulfate before distilling off the solvent, followed by purifying the residue using silica gel column chromatography (hexane/ethyl acetate) to provide 701.3 mg of the title compound as a white solid.

### (Example 105-4) Synthesis of (S)-2-t-butoxycarbonylamino-4-(1-propyl-1H-benzimidazol-2-yl)-N-benzyloxybutyramide

In 40 ml of dioxane was dissolved 898.2 mg (1.99 mmol) of the compound obtained in Example 105-3, to which 5 ml of dioxane in which 270 mg of 10% palladium carbon was suspended was then added, followed by stirring at room temperature for one hour. After the end of reaction, the catalyst was removed by celite filtration, and the solvent was distilled off. The residue was dissolved in 18 ml of chloroform, to which 571.4 mg (2.99 mmol) of WSCI hydrochloride and 269.6 mg (2.19 mmol) of O-benzylhydroxylamine were then added, followed by stirring at room temperature for 20 hours. After the end of reaction, chloroform was added, followed by washing with distilled water and a saturated saline solution before drying with anhydrous sodium sulfate. The solvent was distilled off, followed by recrystallizing the resultant residue from ethyl acetate/hexane to provide 853.1 mg of the title compound as a white crystal.

### (Example 105-5) Synthesis of (S)-2-t-butoxycarbonylamino-4-(1-propyl-1H-benzimidazol-2-yl)-N-hydroxybutyramide

In 50 ml of dioxane was dissolved 837.5 mg (1.80 mmol) of the compound obtained in Example 105-4, to which 250 mg of 10% palladium carbon was then added, followed by stirring at room temperature for 15 hours under an atmosphere of hydrogen. After the end of reaction, the catalyst was removed by celite filtration, followed by concentrating the solution before azeotroping with chloroform. The residue was purified using silica gel column chromatography (chloroform/methanol) to provide 518.4 mg of the title compound as a white solid.

### (Example 105-6) Synthesis of (S)-2-amino-4-(1-propyl-1H-benzimidazol-2-yl)-N-hydroxybutyramide

In 3 ml of 25% trifluoroacetic acid/chloroform was dissolved 283.4 mg (0.753 mmol) of the compound obtained in Example 105-5, followed by stirring at room temperature for 4 hours. After the end of reaction, the solvent was distilled off, followed by purification using a reverse phase system solid-phase extraction column to provide 260.4 mg of the trifluoroacetate of the title compound as a white solid.
MS (Fab, Pos.): m/z = 452 [M+H]⁺
¹H-NMR (500 MHz, DMSO-d₆+D₂O): δ = 0.94 (3H, t, J = 7.3 Hz), 1.81 (2H, sext, J = 7.3 Hz), 2.26-2.34 (2H, m), 3.14-3.19 (2H, m), 3.82 (1H, t, J = 6.6 Hz), 4.31 (2H, t, J = 7.6 Hz), 7.46-7.56 (2H, m), 7.74-7.79 (1H, m), 7.86-7.90 (1H, m).

### (Example 106) Synthesis of (S)-2-amino-4-(1-pentyl-1H-benzimidazol-2-yl)-N-hydroxybutyramide (compound No. 65)

### (Example 106-1) Synthesis of 2-t-butoxycarbonylamino-4-(2-pentylaminophenylcarbamoyl)-butyric acid benzyl ester

In 15 ml of dichloroethane was dissolved 504.4 mg (1.14 mmol) of the compound obtained in Example 105-1, to which 0.150 ml (1.37 mmol) of n-valeraldehyde and 0.25 ml of trimethyl ortho-formate were then added, followed by stirring at room temperature for 50 minutes. Then, 359 mg (1.71 mmol) of sodium triacetoxyborohydride was added before stirring at room temperature for 90 minutes, followed by adding a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, followed by purifying the residue using silica gel column chromatography (hexane/ethyl acetate) to provide 324.5 mg of the title compound as a white solid.
¹H-NMR (500 MHz, DMSO-d₆): δ = 0.85-0.89 (3H, m), 1.16-1.44 (4H, m), 1.38 (9H, s), 1.51-1.59 (2H, m), 1.80-1.89 (1H, m), 1.99-2.13 (1H, m), 2.43 (2H, t, J = 7.3 Hz), 2.99-3.04 (2H, m), 4.04-4.12 (1H, m), 4.80 (1H, t, J = 5.4 Hz), 5.11 (1H, d, J = 12.5 Hz), 5.16(1H,d,J=12.SHz),6.54(1H,t,J=7.6Hz),6.60(1H,d,J=7.6Hz),7.00(1H,t,J= 7.6 Hz), 7.09 (1H, d, J = 7.6 Hz), 7.30-7.43 (5H, m), 9.11 (1H, s).

### (Example 106-2) Synthesis of (S)-2-t-butoxycarbonylamino-4-(1-pentyl-1H-benzimidazol-2-yl)-butyric acid benzyl ester

In 5 ml of acetic acid was dissolved 597.6 mg (1.20 mmol) of the compound synthesized in Example 106-1, followed by stirring at room temperature for 24 hours. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the resultant residue using silica gel column chromatography (hexane/ethyl acetate) to provide 536.5 mg of the title compound as a white solid.
¹H-NMR (500 MHz, DMSO-d₆): δ = 0.84 (3H, t, J = 6.8 Hz), 1.20-1.45 (4H, m), 1.38 (9H, s), 1.62-1.70 (2H, m), 2.12-2.20 (1H, m), 2.28-2.38 (1H, m), 2.87-2.95 (2H, m), 4.11 (2H, t, J = 7.3 Hz), 4.19-4.25 (1H, m), 5.11 (1H, d, J = 12.7 Hz), 5.16 (1H, d, J = 12.7 Hz), 7.13 (1H, td, J = 7.6 Hz), 7.18 (1H, t, J = 7.3 Hz), 7.30-7.43 (5H, m), 7.48 (1H, d, J = 7.6 Hz), 7.54 (2H, d, J = 7.6 Hz).

### (Example 106-3) Synthesis of (S)-2-t-butoxycarbonylamino-4-(1-pentyl-1H-benzimidazol-2-yl)-N-benzyloxybutyramide

In 25 ml of dioxane was dissolved 500.2 mg (1.04 mmol) of the compound obtained in Example 106-2, to which 150 mg of 10% palladium carbon was then added, followed by stirring at room temperature for one hour. After the end of reaction, the catalyst was removed by celite filtration, and the solvent was distilled off. The residue was dissolved in 10 ml of chloroform, to which 300.0 mg (1.56 mmol) of WSCI hydrochloride and 141 mg (1.14 mmol) of O-benzylhydroxylamine were then added, followed by stirring at room temperature for 20 hours. After the end of reaction, chloroform was added, followed by washing with distilled water and a saturated saline solution before drying with anhydrous sodium sulfate. The solvent was distilled off, followed by recrystallizing the resultant residue from ethyl acetate/hexane to provide 425.8 mg of the title compound as a white crystal.

### (Example 106-4) Synthesis of (S)-2-t-butoxycarbonylamino-4-(1-pentyl-1H-benzimidazol-2-yl)-N-hydroxybutyramide

In 28 ml of dioxane was dissolved 408.2 mg (0.825 mmol) of the compound obtained in Example 106-3, to which 160 mg of 10% palladium carbon was then added, followed by stirring at room temperature for 14 hours under an atmosphere of hydrogen. After the end of reaction, the catalyst was removed by celite filtration, followed by concentrating the solution before azeotroping with chloroform. The residue was purified using silica gel column chromatography (chloroform/methanol) to provide 296.9 mg of the title compound as a white solid.

### (Example 106-5) Synthesis of(S)-2-amino-4-(1-pentyl-1H-benzimidazol-2-yl)-N-hydroxybutyramide

In 2 ml of 25% trifluoroacetic acid/chloroform was dissolved 199.1 mg (0.492 mmol) of the compound obtained in Example 106-4, followed by stirring at room temperature for one hour. After the end of reaction, the solvent was distilled off, followed by purification using a reverse phase system solid-phase extraction column to provide 165.9 mg of the trifluoroacetate of the title compound as a white solid.
MS (Fab, Pos.): m/z = 305 [M+H]⁺
¹H-NMR (500 MHz, DMSO-d₆+D₂O): δ = 0.87 (3H, t, J = 6.8 Hz), 1.28-1.40 (4H, m), 1.75-1.84 (2H, m), 2.27-2.33 (2H, m), 3.14-3.21 (2H, m), 3.82 (1H, t, J = 6.6 Hz), 4.33 (2H, t, J = 7.6 Hz), 7.48-7.55 (2H, m), 7.75-7.79 (1H, m), 7.85-7.89 (1H, m).

### (Example 107) Synthesis of (S)-2-amino-4-(1-octyl-1H-benzimidazol-2-yl)-N-hydroxybutyramide (compound No. 69)

### (Example 107-1) Synthesis of (S)-2-t-butoxycarbonylamino-4-(1-octyl-1H-benzimidazol-2-yl)-butyric acid benzyl ester

In 30 ml of dichloroethane was dissolved 1.0049 g (2.35 mmol) of the compound obtained in Example 105-1, to which 0.444 ml (2.82 mmol) of n-caprylaldehyde was then added, followed by stirring at room temperature for 80 minutes. Then, 718 mg (3.39 mmol) of sodium triacetoxyborohydride was added before stirring at room temperature for 90 minutes, followed by adding a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, and the residue was dissolved in 10 ml of acetic acid, followed by stirring at 60°C for 50 minutes. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the resultant residue using silica gel column chromatography (hexane/ethyl acetate) to provide 808.8 mg of the title compound as a pale yellow solid.
¹H-NMR (500 MHz, DMSO-d₆): δ = 0.84 (3H, t, J = 6.8 Hz), 1.18-1.45 (10H, m), 1.38 (9H, s), 1.62-1.70 (2H, m), 2.11-2.20 (1H, m), 2.26-2.37 (1H, m), 2.87-2.97 (2H, m), 4.04 (2H, t, J = 7.3 Hz), 4.19-4.25 (1H, m), 5.11 (1H, d, J = 12.7 Hz), 5.16 (1H, d, J = 12.7 Hz), 7.13 (1H, td, J = 7.6 Hz), 7.18 (1H, t, J = 7.3 Hz), 7.30-7.43 (5H, m), 7.48 (1H, d, J = 7.6 Hz), 7.54 (2H, d, J = 7.6 Hz).

### (Example 107-2) Synthesis of (S)-2-t-butoxycarbonylamino-4-(1-octyl-1H-benzimidazol-2-yl)-N-benzyloxybutyramide

In 30 ml of dioxane was dissolved 698.9 mg (1.34 mmol) of the compound obtained in Example 107-1, to which 5 ml of dioxane in which 210 mg of 10% palladium carbon was suspended was then added, followed by stirring at room temperature for one hour. After the end of reaction, the catalyst was removed by celite filtration, and the solvent was distilled off. The residue was dissolved in 15 ml of chloroform, to which 384.9 mg (2.01 mmol) of WSCI hydrochloride and 181.5 mg (1.47 mmol) of O-benzylhydroxylamine were then added, followed by stirring at room temperature for 3 days. After the end of reaction, chloroform was added, followed by washing with distilled water and a saturated saline solution before drying with anhydrous sodium sulfate. The solvent was distilled off, followed by purifying the resultant residue using silica gel column chromatography (ethyl acetate/ hexane) to provide 598.1 mg of the title compound as a white crystal.

### (Example 107-3) Synthesis of (S)-2-t-butoxycarbonylamino-4-(1-octyl-1H-benzimidazol-2-yl)-N-hydroxybutyramide

In 25 ml of dioxane was dissolved 584.2 mg (1.09 mmol) of the compound obtained in Example 107-2, to which 5 ml of dioxane in which 425 mg of 10% palladium carbon was suspended was then added, followed by stirring at room temperature for 27 hours under an atmosphere of hydrogen. After the end of reaction, the catalyst was removed by celite filtration, followed by concentrating the solution before azeotroping with chloroform to provide 360.3 mg of the title compound as a white solid.

### (Example 107-4) Synthesis of (S)-2-amino-4-(1-octyl-1H-benzimidazol-2-yl)-N-hydroxybutyramide

In 3.5 ml of 25% trifluoroacetic acid/chloroform was dissolved 352.3 mg (0.789 mmol) of the compound obtained in Example 107-3, followed by stirring at room temperature for 19 hours. After the end of reaction, the solvent was distilled off, followed by purification using a reverse phase system solid-phase extraction column and silica gel column chromatography (chloroform/methanol) to provide 163.5 mg of the trifluoroacetate of the title compound as a white solid.
MS (Fab, Pos.): m/z = 348 [M+H]⁺
¹H-NMR (500 MHz, DMSO-d₆+D₂O): δ = 0.85 (3H, t, J = 6.8 Hz), 1.18-1.39 (10H, m), 1.72-1.82 (2H, m), 2.28-2.35 (2H, m), 3.09-3.17 (2H, m), 3.81 (1H, t, J = 6.6 Hz), 4.30 (2H, t, J = 7.3 Hz), 7.41-7.52 (2H, m), 7.73 (1H, d, J = 7.0 Hz), 7.81 (2H, d, J = 7.3 Hz).

### (Example 108) Synthesis of (S)-2-amino-4-(1-cyclohexylmethyl-1H-benzimidazol-2-yl)-N-hydroxybutyramide (compound No. 70)

### (Example 108-1) Synthesis of (S)-2-t-butoxycarbonylamino-4-(2-cyclohexylmethylaminophenylcarbamoyl)-butyric acid benzyl ester

In 30 ml of dichloroethane was dissolved 1.000 g (2.26 mmol) of the compound obtained in Example 105-1, to which 0.301 ml (2.49 mmol) of cyclohexylcarbaldehyde was then added, followed by stirring at room temperature for 120 minutes. Then, 718 mg (3.39 mmol) of sodium triacetoxyborohydride was added before stirring at room temperature for 2 hours, followed by adding a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, followed by purifying the resultant residue using silica gel column chromatography (hexane/ethyl acetate) to provide 968.1 mg of the title compound as a white solid.
MS (Fab, Pos.): m/z = 524 [M+H]⁺
¹H-NMR (500 MHz, DMSO-d₆): δ = 0.86-0.97 (2H, m), 1.10-1.23 (3H, m), 1.38 (9H, s), 1.49-1.70 (4H, m), 1.77-1.86 (2H, m), 1.80-1.91 (1H, m), 2.02 2.13 (1H, m), 2.43 (2H, t, J = 7.3 Hz), 2.87 (2H, t, J = 6.1 Hz), 4.06-4.17 (1H, m), 4.85 (1H, t, J = 5.8 Hz), 5.11 (1H, d, J = 12.7 Hz), 5.16 (1H, d, J = 12.7 Hz), 6.52 (1H, t, J = 7.4 Hz), 6.58 (1H, d, J = 8.1 Hz), 6.98-7.02 (1H, m), 7.06 (1H, dd, J =1.3, 7.6 Hz), 7.32-7.46 (6H, m), 9.10 (1H, s).

### (Example 108-2) Synthesis of (S)-2-t-butoxycarbonylamino-4-(1-pentyl-1H-benzimidazol-2-yl)-butyric acid

In 20 ml of acetic acid was dissolved 968 mg (1.85 mmol) of the compound obtained in Example 108-1, followed by stirring at room temperature for 24 hours. After the end of reaction, the solvent was distilled off before azeotroping with toluene, followed by dissolving the residue in chloroform before washing with a 1 mol/l sodium hydroxide aqueous solution and a saturated saline solution. It was dried over anhydrous sodium sulfate before distilling off the solvent, and the residue was dissolved in 10 ml of THF and 10 ml of methanol, to which a 1 mol/l sodium hydroxide aqueous solution was then added, followed by stirring at room temperature for 2 hours. After the end of reaction, the solvent was distilled off, followed by adding 5 ml of distilled water and 1 mol/l of hydrochloric acid before extraction with chloroform. The organic layer was washed with a saturated saline solution and dried over anhydrous sodium sulfate, from which the solvent was then distilled off, followed by purifying the resultant residue using silica gel column chromatography (chloroform/methanol) to provide 713.2 mg of the title compound as a white solid.
MS (Fab, Pos.): m/z = 416 [M+H]⁺

### (Example 108-3) Synthesis of (S)-2-t-butoxycarbonylamino-4-(1-cyclohexylmethyl-1H-benzimidazol-2-yl)-N-benzyloxybutyramide

In 20 ml of chloroform was dissolved 685 mg (1.65 mmol) of the compound synthesized in Example 108-2, to which 474 mg (2.48 mmol) of WSCI hydrochloride and 213 mg (1.73 mmol) of O-benzylhydroxylamine were then added, followed by stirring at room temperature for 2 days. After the end of reaction, chloroform was added, followed by washing with distilled water and a saturated saline solution before drying with anhydrous sodium sulfate. The solvent was distilled off, followed by purifying the residue using silica gel column chromatography (hexane/ethyl acetate) to provide 906 mg of the title compound as a white solid.
MS (Fab, Pos.): m/z = 521 [M+H]⁺

### (Example 108-4) Synthesis of (S)-2-t-butoxycarbonylamino-4-(1-cyclohexylmethyl-1H-benzimidazol-2-yl)-N-hydroxybutyramide

In 25 ml of dioxane was dissolved 466 mg (0.896 mmol) of the compound synthesized in Example 108-3, to which 140 mg of 10% palladium carbon was then added, followed by stirring at room temperature for 15 hours under an atmosphere of hydrogen. After the end of reaction, the catalyst was removed by celite filtration, followed by concentrating the solution before azeotroping with chloroform. The residue was purified using silica gel column chromatography (chloroform/methanol) to provide 329 mg of the title compound as a dark violet solid.
MS (Fab, Pos.): m/z = 431 [M+H]⁺

### (Example 108-5) Synthesis of (S)-2-amino-4-(1-cyclohexylmethyl-1H-benzimidazol-2-yl)-N-hydroxybutyramide

In 4 ml of 25% trifluoroacetic acid/chloroform was dissolved 329 mg (0.763 mmol) of the compound synthesized in Example 108-4, followed by stirring at room temperature for 4 hours. After the end of reaction, the solvent was distilled off, followed by purification using a reverse phase system solid-phase extraction column to provide 289 mg of the trifluoroacetate of the title compound as a white solid.
MS (Fab, Pos.): m/z = 331 [M+H]⁺
¹H-NMR (500 MHz, DMSO-d₆+D₂O): δ = 1.04-1.21 (5H, m), 1.53-1.62 (2H, m), 1.60-1.72 (3H, m), 1.81-1.92 (1H, m), 2.27-2.35 (2H, m), 3.17-3.21 (2H, m), 3.83 (1H, t, J = 6.6 Hz), 4.21 (2H, d, J = 7.6 Hz), 7.52-7.57 (2H, m), 7.76-7.80 (1H, m), 7.91-7.95 (1H, m).

### (Example 109) Synthesis of (S)-2-amino-4-[1-(4-t-butylbenzyl)-1H-benzimidazol-2-yl]-N-hydroxybutyramide (compound No. 74)

### (Example 109-1) Synthesis of (S)-2-t-butoxycarbonylamino-4-[2-(4-t-butylbenzyl)aminophenylcarbamoyl]-butyric acid benzyl ester

In 15 ml of dichloroethane was dissolved 1.00 g (2.29 mmol) of the compound obtained in Example 105-1, to which 0.416 ml (2.52 mmol) of 4-t-butylbenzaldehyde was then added, followed by stirring at room temperature for 75 minutes. Then, 718 mg (3.39 mmol) of sodium triacetoxyborohydride was added before stirring at room temperature for 23 hours, followed by adding a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, followed by purifying the resultant residue using silica gel column chromatography (hexane/ethyl acetate) to provide 1.02 g of the title compound as a white solid.
MS (Fab, Pos.): m/z = 574 [M+H]⁺
¹H-NMR (500 MHz, DMSO-d₆): δ=1.25 (9H, s), 1.38 (9H, s), 1.80-1.92 (1H, m), 2.02-2.16 (1H, m), 2.46 (2H, t, J = 7.3 Hz), 4.07-4.15 (1H, m), 4.27 (2H, d, J = 6.0 Hz), 5.10 (1H, d, J = 12.7 Hz), 5.16 (1H, d, J = 12.7 Hz), 5.55 (1H, t, J = 6.0 Hz), 6.49-6.54 (2H, m), 6.91 (1H, t, J = 7.5 Hz), 7.11 (1H, dd, J = 1.3, 7.7 Hz), 7.31-7.42 (10H, m), 9.15 (1H, s).

### (Example 109-2) Synthesis of (S)-2-t-butoxycarbonylamino-4-[1-(4-t-butyl-benzyl)-1H-benzimidazol-2-yl]-butyric acid benzyl ester

In 5 ml of acetic acid was dissolved 1.02 g (1.77 mmol) of the compound obtained in Example 109-1, followed by stirring at 60°C for 1.5 hours. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate, followed by distilling off the solvent to provide 976 mg of the title compound as a pale yellow foam.
MS (Fab, Pos.): m/z = 556 [M+H]⁺
¹H-NMR (500 MHz, DMSO-d₆): δ = 1.22 (9H, s), 1.37 (9H, s), 2.09-2.20 (1H, m), 2.23-2.32 (1H, m), 2.86-2.99 (2H, m), 4.20-4.27 (1H, m), 5.09 (1H, d, J = 12.7 Hz), 5.13 (1H, d, J = 12.7 Hz), 5.37 (1h, d, J = 16.8 Hz), 5.42 (1H, d, J = 16.7 Hz), 7.00 (2H, d, J = 8.2 Hz), 7.17 (2H, d, J = 8.2 Hz), 7.32-7.39 (7H, m), 7.40-7.44 (1H, m), 7.57-7.61 (1H, m).

### (Example 109-3) Synthesis of (S)-2-t-butoxycarbonylamino-4-[1-(4-t-butyl-benzyl)-1H-benzimidazol-2-yl]-butyric acid

In 10 ml of THF and 10 ml of methanol was dissolved 976 mg (1.76 mmol) of the compound obtained in Example 109-2, to which 10 ml of a 1 mol/l sodium hydroxide aqueous solution was then added, followed by stirring at room temperature for 90 minutes. After the end of reaction, the solvent was distilled off, and the residue was dissolved in distilled water, to which 10 ml of 1 mol/l hydrochloric acid was then added. The aqueous layer was extracted with chloroform before washing the extract with a saturated saline solution, followed by drying with anhydrous sodium sulfate before distilling off the solvent. The resultant residue was purified using silica gel column chromatography (chloroform/methanol) to provide 753 mg of the title compound as a white solid.
MS (Fab, Pos.): m/z = 466 [M+H]⁺

### (Example 109-4) Synthesis of (S)-2-t-butoxycarbonylamino-4-[1-(4-t-butyl-benzyl)-1H-benzimidazol-2-yl]-N-trityloxybutyramide

In 22 ml of chloroform was dissolved 748 mg (1.61 mmol) of the compound obtained in Example 109-3, to which 462 mg (2.42 mmol) of WSCI hydrochloride, 218 mg (1.61 mmol) of HOBt, and 488 mg (1.77 mmol) of O-tritylhydroxylamine were then added, followed by stirring at room temperature for 15 hours. After the end of reaction, chloroform was added, followed by washing with 1 mol/l hydrochloric acid, a 1 mol/l sodium hydroxide aqueous solution, and a saturated saline solution before drying with anhydrous sodium sulfate. The solvent was distilled off, followed by purifying the resultant residue using silica gel column chromatography (ethyl acetate/ hexane) to provide 321 mg of the title compound as a white crystal.
¹H-NMR (500 MHz, DMSO-d₆): δ = 1.22 (9H, s), 1.33 (9H, s), 1.63-1.82 (2H, m), 2.56-2.61 (2H, m), 3.92-3.98 (1H, m), 5.31 (2H, s), 6.97 (2H, d, J = 8.4 Hz), 7.02 (1H, d, J = 8.0 Hz), 7.14 (2H, d, J = 8.2 Hz), 7.21-7.37 (17H, m), 7.39-7.43 (1H, m), 7.53-7.58 (1H, m), 10.44 (1H, s).

### (Example 109-5) Synthesis of(S)-2-amino-4-[1-(4-t-butyl-benzyl)-1H-benzimidazol-2-yl]-N-hydroxybutyramide

In 3 ml of 25% trifluoroacetic acid/chloroform was dissolved 313 mg (0.437 mmol) of the compound obtained in Example 109-4, followed by stirring at room temperature for 7 hours. After the end of reaction, the solvent was distilled off before azeotroping with chloroform. The residue was suspended in distilled water, and washed with chloroform. The aqueous layer was concentrated to dryness and azeotroped with methanol to provide 235 mg of the trifluoroacetate of the title compound as a white solid.
MS (Fab, Pos.): m/z = 381 [M+H]⁺
¹H-NMR (500 MHz, DMSO-d₆+D₂O): δ = 1.24 (9H, s), 2.29-2.35 (2H, m), 3.19-3.26 (2H, m), 3.83 (1H, t, J = 6.6 Hz), 5.60 (2H, d, J = 10.4 Hz), 7.20 (2H, d, J = 8.5 Hz), 7.39 (2H, d, J = 8.5 Hz), 7.41-7.51 (2H, m), 7.70 (1H, d, J = 8.1 Hz), 7.79(1H, d, J = 8.1 Hz).

### (Example 110) Synthesis of (S)-2-amino-4-(5,6-dimethyl-1-propyl-1H-benzimidazol-2-yl)-N-hydroxybutyramide (compound No. 94)

### (Example 110-1) Synthesis of (S)-2-t-butoxycarbonylamino-4-(2-amino-4,5-dimethyl-phenylcarbamoyl)butyric acid benzyl ester

In 50 ml of chloroform was dissolved 5.00 g (14.8 mmol) of commercial N^{α}-Boc-glutamic acid benzyl ester, to which 4.26 g (22.2 mmol) of WSCI hydrochloride and 2.20 g (16.3 mmol) of HOBt were then added, followed by reaction at room temperature for 15 minutes. This solution was added dropwise to 50 ml of a chloroform solution of 2.42 g (17.8 mmol) of 4,5-dimethyl-1,2-phenylenediamine at room temperature over a period of 2.5 hours. It continued to be stirred at room temperature for further 2 days and was then washed with a 1 mol/l sodium hydroxide aqueous solution and a saturated saline solution, followed by drying the organic layer with anhydrous sodium sulfate. The residue was recrystallized from ethyl acetate/hexane to provide 3.03 g of the title compound as a white crystal.
MS (Fab, Pos.): m/z = 456 [M+H]⁺

### (Example 110-2) Synthesis of (S)-2-t-butoxycarbonylamino-4-(5,6-dimethyl-1-propyl-1H-benzimidazol-2-yl)-butyric acid benzyl ester

In 15 ml of dichloroethane was dissolved 519 mg (1.19 mmol) of the compound obtained in Example 110-1, to which 0.083 ml (1.25 mmol) of propionaldehyde was then added, followed by stirring at room temperature for 15 minutes. Then, 350 mg (1.65 mmol) of sodium triacetoxyborohydride was added before stirring at room temperature for 5 minutes, followed by adding a 1 mol/l sodium hydroxide aqueous solution for separation. The aqueous layer was extracted with chloroform, and the extract was combined with the above organic layer, washed with a saturated saline solution, and dried over anhydrous sodium sulfate. The solvent was distilled off, and the residue was dissolved in 5 ml of acetic acid, followed by stirring at 60°C for 1.5 hours. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the resultant residue using silica gel column chromatography (hexane/ethyl acetate) to provide 281 mg of the title compound as a colorless foam.
MS (Fab, Pos.): m/z = 480 [M+H]⁺
¹H-NMR (500 MHz, DMSO-d₆): δ = 0.83 (1H, t, J = 7.3 Hz), 1.38 (9H, s), 1.66 (2H, sext, J = 7.3 Hz), 2.04-2.17 (1H, m), 2.23-2.35 (1H, m), 2.27 (3H, s), 2.31 (3H, s), 2.79-2.92 (2H, m), 4.01 (2H, t, J = 7.3 Hz), 4.15-4.22 (1H, m), 5.09 (1H, d, J = 12.5 Hz), 5.17 (1H, d, J = 12.5 Hz), 7.26 (1H, s), 7.30 (1H, s), 7.31-7.40 (5H, m), 7.54 (1H, d, J = 7.8 Hz).

### (Example 110-3) Synthesis of (S)-2-t-butoxycarbonylamino-4-(5,6-dimethyl-1-propyl-1H-benzimidazol-2-yl)-N-trityloxybutyramide

In 15 ml of dioxane was dissolved 280 mg (0.584 mmol) of the compound obtained in Example 110-2, to which 84 mg of 10% palladium carbon was then added, followed by stirring at room temperature for 3 hours under an atmosphere of hydrogen. After the end of reaction, the catalyst was removed by celite filtration, and the residue was dissolved in 7 ml of chloroform, to which 167 mg (0.872 mmol) of WSCI hydrochloride, 94.2 mg (0.697 mmol) of HOBt, and 161 mg (0.585 mmol) of O-tritylhydroxylamine were then added, followed by stirring at room temperature for 2 days. After the end of reaction, chloroform was added, followed by washing with distilled water and a saturated saline solution before drying with anhydrous sodium sulfate. The solvent was distilled off, followed by purifying the resultant residue using silica gel column chromatography (hexane /ethyl acetate) to provide 276 mg of the title compound as a white solid.
¹H-NMR (500 MHz, DMSO-d₆): δ = 0.83 (1H, t, J = 7.3 Hz), 1.34 (9H, s), 1.63 (2H, sext, J = 7.3 Hz), 1.60-1.71 (1H, m), 1.72-1.82 (1H, m), 2.28 (3H, s), 2.31 (3H, s), 2.43-2.60 (2H, m), 3.90-3.97 (3H, m), 7.02 (1H, d, J = 8.2 Hz), 7.21-7.39 (17H, m).

### (Example 110-4) Synthesis of (S)-2-amino-4-(5,6-dimethyl-1-propyl-1H-benzimidazol-2-yl)-N-hydroxybutyramide

To 0°C was cooled 276 mg of the compound obtained in Example 110-3, to which 2.8 ml of a 25% trifluoroacetic acid/chloroform solution was then added, followed by stirring at room temperature for 10 hours. After the end of reaction, the solvent was distilled off, and water was added to the residue, followed by washing with chloroform. The aqueous layer was concentrated, followed by purification using a reverse phase system solid-phase extraction column before purification by silica gel column chromatography (chloroform/methanol/water) to provide 57 mg of the trifluoroacetate of the title compound as a white solid.
MS (Fab, Pos.): m/z = 305 [M+H]⁺
¹H-NMR (500 MHz, DMSO-d₆+D₂O): δ = 0.94 (1H, t, J = 7.3 Hz), 1.83 (2H, sext, J = 7.3 Hz), 2.23-2.30 (2H, m), 2.39 (3H, s), 2.41 (3H, s), 3.13-3.22 (2H, m), 3.80 (1H, t, J = 6.6 Hz), 4.28-4.36 (2H, m), 7.55 (1H, s), 7.75 (1H, s).

### (Example 111) Synthesis of (S)-2-amino-4-[1-(4-t-butylbenzyl)-5,6-dimethyl-1H-benzimidazol-2-yl]-N-hydroxybutyramide (compound No. 85)

### (Example 111-1) Synthesis of 2-t-butoxycarbonylamino-4-[1-(4-t-butyl-benzyl)-5,6-dimethyl-1H-benzimidazol-2-yl]-butyric acid benzyl ester

In 15 ml of dichloroethane was dissolved 500 mg (1.10 mmol) of the compound obtained in Example 110-1, to which 0.200 ml (1.32 mmol) of 4-t-butylbenzaldehyde was then added, followed by stirring at room temperature for 60 minutes. Then, 346 mg (1.63 mmol) of sodium triacetoxyborohydride was added before stirring at room temperature for 16 hours. After the end of reaction, a 1 mol/l sodium hydroxide aqueous solution was added for separation. The aqueous layer was extracted with chloroform, and the extract was combined with the above organic layer, washed with a saturated saline solution, and dried over anhydrous sodium sulfate. The solvent was distilled off, and the residue was dissolved in 7 ml of acetic acid, followed by stirring at 60°C for 2.5 hours. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the resultant residue using silica gel column chromatography (hexane/ethyl acetate) to provide 483 mg of the title compound as a colorless foam.

### (Example 111-2) Synthesis of (S)-2-t-butoxycarbonylamino-4-[1-(4-t-butyl-benzyl)-5,6-dimethyl-1H-benzimidazol-2-yl]-butyric acid

In 5 ml of THF and 5 ml of methanol was dissolved 483 mg (0.827 mmol) of the compound obtained in Example 111-1, to which 5 ml of a 1 mol/l sodium hydroxide aqueous solution was then added, followed by stirring at room temperature for one hour. After the end of reaction, the solvent was distilled off, followed by adding 1 mol/l hydrochloric acid to the residue to adjust the pH to about 5. It was extracted with chloroform, and the extract was washed with a saturated saline solution before drying with anhydrous sodium sulfate. The solvent was distilled off, followed by reprecipitating the residue from ethyl acetate/hexane to provide 355 mg of the title compound as a white solid.

### (Example 111-3) Synthesis of (S)-2-t-butoxycarbonylamino-4-[1-(4-t-butyl-benzyl)-5,6-dimethyl-1H-benzimidazol-2-yl]-N-trityloxybutyramide

In 10 ml of chloroform was dissolved 352 mg (0.712 mmol) of the compound obtained in Example 111-2, to which 204 mg (1.07 mmol) of WSCI hydrochloride and 215 mg (0.781 mmol) of O-tritylhydroxylamine were then added, followed by stirring at room temperature for 2 days. After the end of reaction, chloroform was added, followed by washing with distilled water and a saturated saline solution before drying with anhydrous sodium sulfate. The solvent was distilled off, followed by purifying the residue using silica gel column chromatography (hexane /ethyl acetate) to provide 441 mg of the title compound as a white solid.

### (Example 111-4) Synthesis of (S)-2-amino-4-[1-(4-t-butyl-benzyl)-5,6-dimethyl-1H-benzimidazol-2-yl]-N-hydroxybutyramide

To 0°C was cooled 276 mg of the compound obtained in Example 111-3, to which 2.2 ml of a 4 mol/l hydrogen chloride/dioxane solution was then added, followed by stirring at room temperature for 2 hours. After the end of reaction, the solvent was distilled off, and water was added to the residue, followed by washing with chloroform. The aqueous layer was concentrated, followed by purification using a reverse phase system solid-phase extraction column before purification by silica gel column chromatography (chloroform/methanol) to provide 66.7 mg of the hydrochloride of the title compound as a white solid.
MS (Fab, Pos.): m/z = 409 [M+H]⁺
¹H-NMR (500 MHz, DMSO-d₆+D₂O): δ = 1.25 (9H, s), 2.30-2.37 (2H, m), 2.34 (3H, s), 2.38 (3H, s), 3.25-3.35 (2H, m), 3.86 (1H, t, J = 6.6 Hz), 5.63 (2H, s), 7.21 (2H, d, J = 8.5 Hz), 7.39 (2H, d, J = 8.5 Hz), 7.58 (2H, s).

### (Example 112) Synthesis of (S)-2-amino-4-(5,6-dichloro-1-propyl-1H-benzimidazol-2-yl)-N-hydroxybutyramide (compound No. 95)

### (Example 112-1) Synthesis of (S)-2-t-butoxycarbonylamino-4-(2-amino-4,5-dichloro-phenylcarbamoyl)-butyric acid benzyl ester

In 20 ml of chlorofprm was dissolved 2.00 g (0.592 mmol) of commercial N^{α}-Boc-glutamic acid benzyl ester, to which 1.70 g (0.888 mmol) of WSCI hydrochloride and 0.881 g (0.651 mmol) of HOBt were added, followed by reaction at room temperature for 15 minutes. This solution was added dropwise to 20 ml of a DMF solution of 1.26 g (0.710 mmol) of 4,5-dichloro-1,2-phenylenediamine at room temperature over a period of 2.5 hours. It continued to be stirred at room temperature for further 22 hours and the solvent was then distilled off. The residue was redissolved in chloroform, and then washed with a 1 mol/l sodium hydroxide aqueous solution and a saturated saline solution, followed by drying the organic layer with anhydrous sodium sulfate. The residue was recrystallized from ethyl acetate/hexane to provide 1.95 g of the title compound as a white crystal.

### (Example 112-2) Synthesis of (S)-2-t-butoxycarbonylamino-4-(2-propylamino-4,5-dichloro-phenylcarbamoyl)-butyric acid benzyl ester

In 15 ml of dichloroethane was dissolved 500 mg (1.01 mmol) of the compound obtained in Example 112-1, to which 0.116 ml (1.62 mmol) of propionaldehyde was then added, followed by stirring at room temperature for 15 minutes. Then, 360 mg (1.70 mmol) of sodium triacetoxyborohydride was added before stirring at room temperature for 22 hours. After the end of reaction, a 1 mol/l sodium hydroxide aqueous solution was added for separation. The aqueous layer was extracted with chloroform, and the extract was combined with the above organic layer, washed with a saturated saline solution, and dried over anhydrous sodium sulfate. The solvent was distilled off, followed by purifying the residue using silica gel column chromatography (chloroform/ethyl acetate) to provide 373 mg of the title compound as a colorless foam.

### (Example 112-3) Synthesis of(S)-2-t-butoxycarbonylamino-4-(5,6-dichloro-1-propyl-1H-benzimidazol-2-yl)-butyric acid benzyl ester

In 3.5 ml of acetic acid was dissolved 373 mg of the compound obtained in Example 112-2, followed by stirring at 60°C for 2 days. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the resultant residue using silica gel column chromatography (chloroform/ethyl acetate) to provide 255 mg of the title compound as a colorless foam.

### (Example 112-4) Synthesis of (S)-2-t-butoxycarbonylamino-4-(5,6-dichloro-1-propyl-1H-benzimidazol-2-yl)-butyric acid

In 2.5 ml of THF and 2.5 ml of methanol was dissolved 259 mg (0.490 mmol) of the compound obtained in Example 112-3, to which 2.5 ml of a 1 mol/l sodium hydroxide aqueous solution was then added, followed by stirring at room temperature for 2 hours. After the end of reaction, the solvent was distilled off, followed by adding distilled water and 1 mol/l hydrochloric acid to the residue to adjust the pH to about 6 before extraction with chloroform. The extract was washed with a saturated saline solution and then dried over anhydrous sodium sulfate, followed by distilling off the solvent. The residue was purified using silica gel column chromatography (chloroform/methanol) to provide 197 mg of the title compound as a pale yellow solid.

### (Example 112-5) Synthesis of (S)-2-t-butoxyamino-4-(5,6-dichloro-1-propyl-1H-benzimidazol-2-yl)-N-trityloxybutyramide

In 6 ml of chloroform was dissolved 197 mg (0.458 mmol) of the compound obtained in Example 112-4, to which 132 mg (0.689 mmol) of WSCI hydrochloride, 68.0 mg (0.503 mmol) of HOBt, and 139 mg (0.505 mmol) of O-tritylhydroxylamine were then added, followed by stirring at room temperature for 22 hours. After the end of reaction, chloroform was added, followed by washing with distilled water and a saturated saline solution before drying with anhydrous sodium sulfate. The solvent was distilled off, followed by purifying the residue using silica gel column chromatography (hexane /ethyl acetate) to provide 153 mg of the title compound as a white solid.
¹H-NMR (500 MHz, DMSO-d₆): δ = 0.83 (1H, t, J = 7.3 Hz), 1.34 (9H, s), 1.63 (2H, sext, J = 7.3 Hz), 1.60-1.71 (1H, m), 1.72-1.82 (1H, m), 2.28 (3H, s), 2.31 (3H, s), 2.43-2.60 (2H, m), 3.90-3.97 (3H, m), 7.02 (1H, d, J = 8.2 Hz), 7.21-7.39 (17H, m).

### (Example 112-6) Synthesis of (S)-2-amino-4-(5,6-dichloro-1-propyl-1H-benzimidazol-2-yl)-N-hydroxybutyramide

To 0°C was cooled 153 mg (0.223 mmol) of the compound obtained in Example 112-5, to which 2.0 ml of a 25% trifluoroacetic acid/chloroform solution was then added, followed by stirring at room temperature for 1.5 hours. After the end of reaction, the solvent was distilled off, and water was added to the residue, followed by washing with chloroform. The aqueous layer was concentrated, followed by purification using a reverse phase system solid-phase extraction column to provide 62.7 mg of the trifluoroacetate of the title compound as a white solid.
MS (Fab, Pos.): m/z = 345, 347, 349 [M+H]⁺
¹H-NMR (500 MHz, DMSO-d₆+D₂O): δ = 0.88 (3H, t, J = 7.3 Hz), 1.72 (2H, sext, J = 7.3 Hz), 2.23-2.35 (2H, m), 2.87-3.02 (2H, m), 3.80 (2H, t, J = 6.6 Hz), 4.14 (2H, t, J = 7.3 Hz), 7.86 (1H, s), 8.01 (1H, s).

### (Example 113) Synthesis of (S)-2-amino-4-(5,6-dichloro-1-pentyl-1H-benzimidazol-2-yl)-N-hydroxybutyramide (compound No. 96)

### (Example 113-1) Synthesis of 2-t-butoxycarbonylamino-4-(2-pentylamino-4,5-dichloro-phenylcarbamoyl)-butyric acid benzyl ester

In 15 ml of dichloroethane was dissolved 503 mg (1.02 mmol) of the compound obtained in Example 112-1, to which 0.172 ml (1.62 mmol) of propionaldehyde was then added, followed by stirring at room temperature for 60 minutes. Then, 360 mg (1.70 mmol) of sodium triacetoxyborohydride was added before stirring at room temperature for 18 hours. After the end of reaction, a 1 mol/l sodium hydroxide aqueous solution was added for separation. The aqueous layer was extracted with chloroform, and the extract was combined with the above organic layer, washed with a saturated saline solution, and dried over anhydrous sodium sulfate. The solvent was distilled off, followed by purifying the residue using silica gel column chromatography (chloroform/ethyl acetate) to provide 457 mg of the title compound as a colorless foam.

### (Example 113-2) Synthesis of (S)-2-t-butoxycarbonylamino-4-(5,6-dichloro-1-pentyl-1H-benzimidazol-2-yl)-butyric acid benzyl ester

In 4 ml of acetic acid was dissolved 457 mg of the compound obtained in Example 113-1, followed by stirring at 60°C for 2 days. After the end of reaction, the solvent was distilled off before azeotroping with toluene, and the residue was dissolved in chloroform, followed by washing with a 1 mol/l sodium hydroxide aqueous solution and a saturated saline solution. It was dried over anhydrous sodium sulfate before distilling off the solvent, followed by purifying the residue using silica gel column chromatography (chloroform/ethyl acetate) to provide 249 mg of the title compound as a colorless foam.

### (Example 113-3) Synthesis of (S)-2-t-butoxycarbonylamino-4-(5,6-dichloro-1-pentyl-1H-benzimidazol-2-yl)-butyric acid

In 2.5 ml ofTHF and 2.5 ml of methanol was dissolved 249 mg (0.453 mmol) of the compound obtained in Example 113-2, to which 2.5 ml of a 1 mol/l sodium hydroxide aqueous solution was then added, followed by stirring at room temperature for 2 hours. After the end of reaction, the solvent was distilled off, followed by adding distilled water and 1 mol/l hydrochloric acid to the residue to adjust the pH to about 6 before extraction with chloroform. The extract was washed with a saturated saline solution and then dried over anhydrous sodium sulfate, followed by distilling off the solvent. The residue was purified using silica gel column chromatography (chloroform/methanol) to provide 112 mg of the title compound as a pale yellow solid.

### (Example 113-4) Synthesis of (S)-2-t-butoxycarbonylamino-4-(5,6-dichloro-1-pentyl-1H-benzimidazol-2-yl)-N-trityloxybutyramide

In 4 ml of chloroform was dissolved 112 mg (0.245 mmol) of the compound obtained in Example 113-3, to which 70.3 mg (0.367 mmol) of WSCI hydrochloride, 36.4 mg (0.269 mmol) of HOBt, and 70.8 mg (0.257 mmol) of O-tritylhydroxylamine were then added, followed by stirring at room temperature for 4 days. After the end of reaction, chloroform was added, followed by washing with distilled water and a saturated saline solution before drying with anhydrous sodium sulfate. The solvent was distilled off, followed by purifying the residue using silica gel column chromatography (hexane /ethyl acetate) to provide 112 mg of the title compound as a white solid.

### (Example 113-5) Synthesis of (S)-2-amino-4-(5,6-dichloro-1-pentyl-1H-benzimidazol-2-yl)-N-hydroxybutyramide

To 0°C was cooled 112 mg (0.223 mmol) of the compound obtained in Example 113-4, to which 1.2 ml of a 25% trifluoroacetic acid/chloroform solution was then added, followed by stirring at room temperature for 2 hours. After the end of reaction, the solvent was distilled off, and water was added to the residue, followed by washing with chloroform. The aqueous layer was concentrated, followed by purification using a reverse phase system solid-phase extraction column to provide 41.0 mg of the trifluoroacetate of the title compound as a white solid.
MS (Fab, Pos.): m/z = 373, 375, 377 [M+H]⁺
¹H-NMR (500 MHz, DMSO-d₆+D₂O): δ = 0.86 (3H, t, J = 7.3 Hz), 1.20-1.37 (4H, m), 1.69 (2H, quint, J = 7.3 Hz), 2.23-2.35 (2H, m), 2.87-3.01 (2H, m), 3.80 (2H, t, J = 6.6 Hz), 4.17 (2H, t, J = 7.3 Hz), 7.87 (1H, s), 8.00 (1H, s).

### (Example 114) Synthesis of (S)-2-(cyclohexylmethyl-amino)-4-(1-propyl-1H-benzimidazol-2-yl)-N-hydroxybutyramide (compound No. 97)

### (Example 114-1) Synthesis of (S)-2-(N-t-butoxycarbonyl-cyclohexylmethyl-amino)-4-(1-propyl-1H-benzimidazol-2-yl)-butyric acid benzyl ester

In 8 ml of 4 mol/l hydrogen chloride/dioxane was dissolved 798 mg (1.65 mmol) of the compound obtained in Example 105-3, followed by stirring at room temperature for one hour. After the end of reaction, the solvent was distilled off, and the residue was dissolved in distilled water. The aqueous layer was washed with chloroform, and pH adjusted to neutral by adding a 1 mol/l sodium hydroxide aqueous solution, followed by extraction with chloroform. The extract was washed with anhydrous sodium sulfate, from which the solvent was then distilled off, and the residue was dissolved in 20 ml of dichloroethane, to which 0.220 ml (1.82 mmol) of cyclohexylcarbaldehyde was then added, followed by stirring at room temperature for 60 minutes. Then, 420 mg (1.98 mmol) of sodium triacetoxyborohydride was added before stirring at room temperature for 30 minutes. After the end of reaction, a 1 mol/l sodium hydroxide aqueous solution was added for separation. The aqueous layer was extracted with chloroform, and the extract was combined with the above organic layer, washed with a saturated saline solution, and dried over anhydrous sodium sulfate. The solvent was distilled off, and the residue was dissolved in 12 ml of DMF, to which 396 mg (1.82 mmol) of di-t-butoxydicarbonate was then added, followed by stirring at 60°C for 2 days. After the end of reaction, the solvent was distilled off, followed by purifying the residue using silica gel column chromatography (hexane/ethyl acetate) to provide 652 mg of the title compound as a colorless, viscous liquid.

### (Example 114-2) Synthesis of (S)-2-(N-t-butoxycarbonyl-cyclohexylmethyl-amino)-4-(1-propyl-1H-benzimidazol-2-yl)-butyric acid

In 30 ml of dioxane was dissolved 652 mg (1.19 mmol) of the compound obtained in Example 114-1, to which 196 mg of 10% palladium carbon was then added, followed by stirring at room temperature for 2 days under an atmosphere of hydrogen. After the end of reaction, the catalyst was removed by celite filtration, and the solvent was distilled off, followed by drying the residue to provide 507 mg of the title compound as a light purple, viscous liquid.

### (Example 114-3) Synthesis of (S)-2-(N-t-butoxycarbonylcyclohexylmethyl-amino)-4-(1-propyl-1H-benzimidazol-2-yl)-N-benzyloxybutyramide

In 10 ml of chloroform was dissolved 347 mg (0.757 mmol) of the compound obtained in Example 114-2, to which 182 mg (0.950 mmol) of WSCI hydrochloride and 94 mg (0.757 mmol) of O-benzylhydroxylamine were then added, followed by stirring at room temperature for 22 hours. After the end of reaction, it was washed with 0.1 mol/l hydrochloric acid and a saturated saline solution before drying with anhydrous sodium sulfate. The solvent was distilled off, followed by purifying the residue using silica gel column chromatography (chloroform/methanol) to provide 295 mg of the title compound as a white foam.

### (Example 114-4) Synthesis of (S)-2-(cyclohexylmethyl-amino)-4-(1-propyl-1H-benzimidazol-2-yl)-N-hydroxybutyramide

In 10 ml of dioxane and 5 ml of ethanol was dissolved 292 mg (0.519 mmol) of the compound obtained in Example 114-3, to which 90 mg of 10% palladium carbon was then added, followed by stirring at room temperature for 15 hours. After the end of reaction, the catalyst was removed by celite filtration, and the solvent was distilled off. In 1.0 ml of 4 mol/l hydrogen chloride/dioxane was dissolved 86.4 mg (equivalent to 0.183 mmol) out of 260 mg of the resultant residue, followed by stirring at room temperature for 45 minutes. After the end of reaction, the solvent was distilled off, followed by purifying the resultant residue using silica gel column chromatography (chloroform/methanol) to provide 66.6 mg of the hydrochloride of the title compound as a white solid.
MS (Fab, Pos.): m/z = 373 [M+H]⁺
¹H-NMR (500 MHz, DMSO-d₆+D₂O): δ = 0.94 (3H, t, J = 7.3 Hz), 0.95-1.02 (2H, m), 1.12-1.27 (3H, m), 1.60-1.88 (8H, m), 2.32-2.40 (1H, m), 2.40-2.49 (1H, m), 2.65 (1H, dd, J = 12.2, 7.1 Hz), 2.82 (1H, dd, J = 12.2, 6.6 Hz), 3.18-3.22 (2H, m), 3.79-3.83 (1H, m), 4.33 (2H, J, t = 7.3 Hz), 7.51-7.55 (2H, m), 7.76 (2H, m), 7.90 (2H, m).

### (Example 115) Synthesis of (S)-2-amino-4-[1-(2,3,5,6-tetrafluoro-4-methoxy-benzyl)-1H-benzimidazol-2-yl]-N-hydroxybutyramide (compound No. 98)

### (Example 115-1) Synthesis of (S)-2-t-butoxycarbonylamino-4-(1-pentafluorophenylmethyl-1H-benzimidazol-2-yl)-butyric acid benzyl ester

In 10 ml of DMF was dissolved 502 mg (1.18 mmol) of the compound obtained in Example 105-1, to which 174 mg (1.26 mmol) of potassium carbonate and 0.230 ml (1.53 mmol) of pentafluorobenzyl bromide were then added, followed by stirring at room temperature for 6 hours. After the end of reaction, the solvent was distilled off, and the residue was dissolved in chloroform and washed with 0.1 mol/l hydrochloric acid and a saturated saline solution. It was dried over anhydrous sodium sulfate, followed by distilling off the solvent. To the resultant residue was added 10 ml of acetic acid, followed by stirring at 60°C for 22 hours. After the end of reaction, the solvent was distilled off before adding chloroform to the residue, followed by washing with a sodium hydroxide aqueous solution and a saturated saline solution. After drying with anhydrous sodium sulfate, the residue was purified using column chromatography (hexane/ethyl acetate) to provide 578 mg of the title compound as a colorless foam.

### (Example 115-2) Synthesis of (S)-2-amino-4-[1-(2,3,5,6-tetrafluoro-4-methoxy-benzyl)-1H-benzimidazol-2-yl]-N-trityloxybutyramide

In 6 ml of THF and 6 ml of methanol was dissolved 578 mg (0.980 mmol) of the compound obtained in Example 108-1, to which 6 ml of a 1 mol/l sodium hydroxide aqueous solution was then added, followed by stirring at room temperature for 4.5 hours. After the end of reaction, the solvent was distilled off, followed by adding 1 mol/l hydrochloric acid to the residue to adjust the pH to 5 to 6 before extraction with chloroform. The extract was dried over anhydrous sodium sulfate before distilling off the solvent, followed by drying under reduced pressure to provide 517 mg of a carboxylic acid as an intermediate. In 6 ml of chloroform was dissolved 309 mg of the carboxylic acid, to which 187 mg (0.977 mmol) of WSCI hydrochloride and 178 mg (0.646 mmol) of O-tritylhydroxylamine were then added, followed by stirring at room temperature for 15 hours. After the end of reaction, chloroform was added thereto before washing with distilled water and a saturated saline solution, followed by drying with anhydrous sodium sulfate. The solvent was distilled off, and the residue was purified using silica gel column chromatography (hexane/ethyl acetate) to provide 124 mg of the title compound as a white solid.

### (Example 115-3) Synthesis of (S)-2-amino-4-[1-(2,3,5,6-tetrafluoro-4-methoxy-benzyl)-1H-benzimidazol-2-yl]-N-hydroxybutyramide

To 0°C was cooled 124 mg (0.291 mmol) of the compound obtained in Example 115-2, to which 1 ml of a 4 mol/l hydrogen chloride/dioxane solution was then added, followed by stirring at 0°C for 2 hours. After the end of reaction, the solvent was distilled off, and methanol was added to the residue, followed by washing with hexane. The methanol layer was concentrated, followed by purifying the residue using silica gel column chromatography (chloroform/methanol/water) to provide 33.9 mg of the hydrochloride of the title compound as a white solid.
MS (Fab, Pos.): m/z = 427 [M+H]⁺
¹H-NMR (500 MHz, DMSO-d₆+D₂O): δ = 2.33 (2H, m), 3.20 (2H, t, J = 7.5 Hz), 3.83 (1H, t, J = 6.8 Hz), 4.06 (3H, s), 5.75 (2H, s), 7.46-7.54 (2H, m), 7.73 (1H, d, J = 7.6 Hz), 7.77 (1H, d, J = 7.6 Hz).

### (Example 116) Synthesis of (S)-2-amino-3-(4-propoxyphenyl)-N-hydroxypropionamide (compound No. 49)

### (Example 116-1) Synthesis of (S)-2-t-butoxycarbonylamino-3-(4-propoxyphenyl)propionic acid methyl ester

In 15 ml of THF was dissolved 509 mg (1.71 mmol) of commercial N^{α}-Boc-tyrosine methyl ester, to which 74 mg (1.85 mmol) of sodium hydride (60%) was then added, followed by stirring at room temperature for 5 minutes. Thereto was then added 0.700 ml (5.23 mmol) of 1-iodopropane, followed by stirring at 60°C for 4 hours. After the end of reaction, the solvent was distilled off, and the residue was suspended in chloroform, followed by washing with 0.5 mol/l hydrochloric acid and a saturated saline solution. The solvent was dried over anhydrous sodium sulfate before distilling off the solvent, followed by purifying the residue using silica gel column chromatography (hexane/ethyl acetate) to provide 279 mg of the title compound as a white solid.

### (Example 116-2) Synthesis of (S)-2-t-butoxycarbonylamino-3-(4-propoxyphenyl)-N-trityloxypropionamide

In 2.8 ml of THF and 2.8 ml of methanol was dissolved 279 mg (0.828 mmol) of the compound obtained in Example 116-1, to which 2.8 ml of a 1 mol/l sodium hydroxide aqueous solution was then added, followed by stirring at room temperature for 27 hours. After the end of reaction, the solvent was distilled off, followed by adding 1 mol/l hydrochloric acid to the residue to adjust the pH to 2 to 3. The solution was extracted with chloroform, and the extract was washed with a saturated saline solution before drying with anhydrous sodium sulfate. The solvent was distilled off, and the residue was dissolved in 8 ml of chloroform, to which 234 mg (1.22 mmol) of WSCI hydrochloride, 132 mg (0.977 mmol) of HOBt, and 236 mg (0.857 mmol) of O-tritylhydroxylamine were then added, followed by stirring at room temperature for 3 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 285 mg of the title compound as a white solid.

### (Example 116-3) Synthesis of (S)-2-amino-3-(4-propoxyphenyl)-N-hydroxypropionamide

To 0°C was cooled 137 mg (0.236 mmol) of the compound obtained in Example 116-2, to which 1.4 ml of a 25% trifluoroacetic acid/chloroform solution was then added, followed by stirring at room temperature for 2 hours. After the end of reaction, the solvent was distilled off, and water was added to the residue, followed by washing with chloroform. The aqueous layer was concentrated and dried to provide 78 mg of the trifluoroacetate of the title compound as a white solid.
MS (Fab, Pos.): m/z = 239 [M+H]⁺
¹H-NMR (500 MHz, DMSO-d₆+D₂O): δ = 0.97 (3H, t, J = 7.3 Hz), 1.71 (2H, sext, J = 7.3 Hz), 2.89 (1H, dd, J = 13.7, 6.9 Hz), 2.95 (1H, dd, J = 13.7, 7.5 Hz), 3.65-3.70 (1H, m), 3.90 (2H, t, J = 6.6 Hz), 6.89 (2H, d, J = 8.7 Hz), 7.11 (2H, d, J = 8.7 Hz).

### (Example 117) Synthesis of (S)-2-amino-3-[4-(4-t-butylbenzyloxy)phenyl]-N-hydroxypropionamide (compound No. 99)

### (Example 117-1) Synthesis of 2-t-butoxycarbonylamino-3-[4-(4-t-butylbenzyloxy)phenyl]propionic acid methyl ester

In 15 ml of THF was dissolved 513 mg (1.72 mmol) of commercial N^{α}-Boc-tyrosine methyl ester, to which 74 mg (1.85 mmol) of sodium hydride (60%) was then added, followed by stirring at room temperature for 5 minutes. Thereto was then added 0.370 ml (2.06 mmol) of 4-t-butylbenzyl bromide, followed by stirring at 60°C for 2 days. After the end of reaction, the solvent was distilled off, and the residue was suspended in chloroform, followed by washing with 0.1 mol/l hydrochloric acid and a saturated saline solution. The solvent was dried over anhydrous sodium sulfate before distilling off the solvent, followed by purifying the residue using silica gel column chromatography (hexane/ethyl acetate) to provide 659 mg of the title compound as a white solid.

### (Example 117-2) Synthesis of (S)-2-t-butoxycarbonylamino-3-[4-(4-t-butylbenzyloxy)phenyl]-N-trityloxypropionamide

In 6.5 ml of THF and 6.5 ml of methanol was dissolved 615 mg (1.49 mmol) of the compound obtained in Example 117-1, to which 6.5 ml of a 1 mol/l sodium hydroxide aqueous solution was then added, followed by stirring at room temperature for 1.5 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The solvent was distilled off, and the residue was dissolved in 10 ml of chloroform, to which 428 mg (2.23 mmol) of WSCI hydrochloride and 410 mg (1.49 mmol) of O-tritylhydroxylamine were then added, followed by stirring at room temperature for 3 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 410 mg of the title compound as a white solid.

### (Example 117-3) Synthesis of (S)-2-amino-3-[4-(4-t-butylbenzyloxy)phenyl]-N-hydroxypropionamide

To 0°C was cooled 193 mg (0.282 mmol) of the compound obtained in Example 117-2, to which 2 ml of a 4 mol/l hydrogen chloride/dioxane solution and 1 ml of chloroform were then added, followed by stirring at room temperature for 3 hours. After the end of reaction, the solvent was distilled off, and methanol was added to the residue, followed by washing with hexane. The methanol layer was concentrated, dried, and then washed with chloroform/hexane to provide 83.9 mg of the hydrochloride of the title compound as a white solid.
MS (Fab, Pos.): m/z = 343 [M+H]⁺
¹H-NMR (500 MHz, DMSO-d₆+D₂O): δ = 1.28 (9H, s), 2.90 (1H, dd, J = 6.7, 13.9 Hz), 2.96 (1H, dd, J = 7.3, 13.9 Hz), 3.70 (1H, dd, J = 6.7, 7.3 Hz), 6.97 (1H, d, J = 8.7 Hz), 7.13 (1H, d, J = 8.7 Hz), 7.37 (1H, d, J = 8.5 Hz), 7.41 (1H, d, J = 8.5 Hz).

### (Example 118) Synthesis of (S)-2-amino-3-[4-(2-phenylbenzyloxy)phenyl]-N-hydroxypropionamide (compound No. 100)

### (Example 118-1) Synthesis of (S)-2-t-butoxycarbonylamino-3-[4-(2-phenylbenzyloxy)phenyl]propionic acid methyl ester

In 15 ml ofTHF was dissolved 503 mg (1.69 mmol) of commercial N^{α}-Boc-tyrosine methyl ester, to which 74 mg (1.85 mmol) of sodium hydride (60%) was then added, followed by stirring at room temperature for 5 minutes. Thereto was then added 0.368 ml (1.41 mmol) of 2-phenylbenzyl bromide, followed by stirring at 60°C for 18 hours. After the end of reaction, the solvent was distilled off, and the residue was suspended in chloroform, followed by washing with 0.1 mol/l hydrochloric acid and a saturated saline solution. The solvent was dried over anhydrous sodium sulfate before distilling off the solvent, followed by purifying the residue using silica gel column chromatography (hexane/ethyl acetate) to provide 648 mg of the title compound as a colorless, viscous liquid.

### (Example 118-2) Synthesis of (S)-2-t-butoxycarbonylamino-3-[4-(2-phenylbenzyloxy)phenyl]-N-trityloxypropionamide

In 6.5 ml ofTHF and 6.5 ml of methanol was dissolved 648 mg (1.47 mmol) of the compound obtained in Example 118-1, to which 6.5 ml of a 1 mol/l sodium hydroxide aqueous solution was then added, followed by stirring at room temperature for 2 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The solvent was distilled off, and the residue was dissolved in 12 ml of chloroform, to which 437 mg (2.28 mmol) of WSCI hydrochloride and 422 mg (1.53 mmol) of O-tritylhydroxylamine were then added, followed by stirring at room temperature for 3 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 821 mg of the title compound as a white solid.

### (Example 118-3) Synthesis of (S)-2-amino-3-[4-(2-phenylbenzyloxy)phenyl]-N-hydroxypropionamide

In 0.5 ml of chloroform was dissolved 154 mg (0.218 mmol) of the compound obtained in Example 118-2, which was then cooled to 0°C before adding 2 ml of a 4 mol/l hydrogen chloride/dioxane solution, followed by stirring at room temperature for 3 hours. After the end of reaction, the solvent was distilled off, and the residue was purified using silica gel column chromatography (chloroform/ethyl acetate) to provide 77.2 mg of the hydrochloride of the title compound as a white solid.
MS (Fab, Pos.): m/z = 363 [M+H]⁺
¹H-NMR (500 MHz, DMSO-d₆+D₂O): δ = 2.90 (1 H, dd, J = 6.6, 13.7 Hz), 2.96 (1H, dd, J = 7.6, 13.9 Hz), 3.68-3.73 (1H, m), 6.83 (1H, d, J = 8.7 Hz), 7.10 (1H, d, J = 8.7 Hz), 7.34 (1H, d, J = 7.5 Hz), 7.37-7.48 (5H, m), 7.59 (1H, d, J = 7.5 Hz).

### (Example 119) Synthesis of (S)-2-amino-3-[4-(4-nitrobenzyloxy)phenyl]-N-hydroxypropionamide (compound No. 101)

### (Example 119-1) Synthesis of (2-t-butoxycarbonylamino-3-[4-(4-nitrobenzyloxy)phenyl]propionic acid methyl ester

In 7.5 ml of THF was dissolved 257 mg (0.865 mmol) of commercial N^{α}-Boc-tyrosine methyl ester, to which 37 mg (0.925 mmol) of sodium hydride (60%) was then added, followed by stirring at room temperature for 20 minutes. Thereto was then added 217 ml (1.04 mmol) of 4-nitrobenzyl bromide, followed by stirring at room temperature for 11 hours. After the end of reaction, the solvent was distilled off, and the residue was suspended in chloroform, followed by washing with 0.1 mol/l hydrochloric acid and a saturated saline solution. The solvent was dried over anhydrous sodium sulfate before distilling off the solvent, followed by purifying the residue using silica gel column chromatography (hexane/ethyl acetate) to provide 288 mg of the title compound as a white solid.

### (Example 119-2) Synthesis of (S)-2-t-butoxycarbonylamino-3-[4-(4-nitrobenzyloxy)phenyl]-N-trityloxypropionamide

In 3.5 ml of THF and 3.5 ml of methanol was dissolved 288 mg (0.669 mmol) of the compound obtained in Example 119-1, to which 3.5 ml of a 1 mol/l sodium hydroxide aqueous solution was then added, followed by stirring at room temperature for 1.5 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The residue was purified using silica gel column chromatography (chloroform/methanol) to provide a carboxylic acid. It was dissolved in 7.5 ml of chloroform, to which 172 mg (0.898 mmol) of WSCI hydrochloride and 181 mg (0.657 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 344 mg of the title compound as a white solid.

### (Example 119-3) Synthesis of (S)-2-amino-3-[4-(4-nitrobenzyloxy)phenyl]-N-hydroxypropionamide

To 0°C was cooled 337 mg (0.500 mmol) of the compound obtained in Example 119-2, to which 3.3 ml of a 4 mol/l hydrogen chloride/dioxane solution was then added, followed by stirring at room temperature for 2.5 hours. After the end of reaction, the solvent was distilled off, and methanol was added to the residue, followed by washing with hexane. The methanol layer was concentrated, dried, and then washed with chloroform/hexane to provide 129 mg of the hydrochloride of the title compound as a white solid.
MS (Fab, Pos.): m/z = 332 [M+H]⁺
¹H-NMR (500 MHz, DMSO-d₆+D₂O): δ = 2.91 (1H, dd, J = 13.7, 6.6 Hz), 2.97 (1H, dd, J = 13.7, 7.6 Hz), 3.71 (1H, t, J = 7.1 Hz), 5.26 (2H, s), 7.00 (2H, d, J = 8.7 Hz), 7.15 (2H, d, J = 8.7 Hz), 7.72 (2H, d, J = 8.8 Hz), 8.26 (2H, d, J = 8.8 Hz).

### (Example 120) Synthesis of (S)-2-amino-3-[4-(2,4-difluorobenzyloxy)phenyl]-N-hydroxypropionamide (compound No. 102)

### (Example 120-1) Synthesis of (S)-2-t-butoxycarbonylamino-3-[4-(2,4-difluorobenzyloxy)phenyl]propionic acid methyl ester

In 10 ml of THF was dissolved 500 mg (1.68 mmol) of commercial N"-Boc-tyrosine methyl ester, to which 74 mg (1.85 mmol) of sodium hydride (60%) was then added, followed by stirring at room temperature for 60 minutes. Thereto was then added 0.256 ml (2.02 mmol) of 2,4-difluorobenzyl bromide, followed by stirring at room temperature for 25 hours. After the end of reaction, the solvent was distilled off, and the residue was suspended in chloroform, followed by washing with 0.1 mol/l hydrochloric acid and a saturated saline solution. The solvent was dried over anhydrous sodium sulfate before distilling off the solvent, followed by purifying the residue using silica gel column chromatography (hexane/ethyl acetate) to provide 632 mg of the title compound as a white foam.

### (Example 120-2) Synthesis of (S)-2-t-butoxycarbonylamino-3-[4-(2,4-difluorobenzyloxy)phenyl]-N-trityloxypropionamide

In 6 ml of THF and 6 ml of methanol was dissolved 632 mg (1.50 mmol) of the compound obtained in Example 120-1, to which 6 ml of a 1 mol/l sodium hydroxide aqueous solution was then added, followed by stirring at room temperature for 7 hours. After the end of reaction, the solvent was distilled off, followed by adding 1 mol/1 hydrochloric acid to the residue to adjust the pH to 3 to 4. The solution was extracted with chloroform, and the extract was washed with a saturated saline solution before drying with anhydrous sodium sulfate. The solvent was distilled off, and the residue was dissolved in 10 ml of chloroform, to which 430 mg (2.25 mmol) of WSCI hydrochloride and 453 mg (1.65 mmol) of O-tritylhydroxylamine were then added, followed by stirring at room temperature for 3 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 626 mg of the title compound as a white solid.

### (Example 120-3) Synthesis of (S)-2-amino-3-[4-(2,4-difluorobenzyloxy)phenyl]-N-hydroxypropionamide

To 0°C was cooled 306 mg (0.416 mmol) of the compound obtained in Example 120-2, to which 3 ml of a 4 mol/l hydrogen chloride/dioxane solution was then added, followed by stirring at room temperature for 3 hours. After the end of reaction, the solvent was distilled off, and methanol was added to the residue, followed by washing with hexane. The methanol layer was concentrated, dried, and then washed with chloroform/hexane to provide 125 mg of the hydrochloride of the title compound as a white solid.
MS (Fab, Pos.): m/z = 323 [M+H]⁺
¹H-NMR (500 MHz, DMSO-d₆+D₂O): δ=2.91 (1H, dd, J = 13.7, 6.7 Hz), 2.98 (1H, dd, J = 13.7, 7.5 Hz), 3.72 (1H, t, J = 7.2 Hz), 5.07 (2H, s), 7.00 (2H, d, J = 8.7 Hz), 7.11-7.20 (1H, m), 7.16 (2H, d, J = 8.7 Hz), 7.27-7.32 (1H, m), 7.59-7.64 (1H, m).

### (Example 121) Synthesis of (S)-2-amino-3-[4-(4-cyanobenzyloxy)phenyl]-N-hydroxypropionamide (compound No. 103)

### (Example 121-1) Synthesis of(S)-2-t-butoxycarbonylamino-3-[4-(4-cyanobenzyloxy)phenyl]propionic acid methyl ester

In 5 ml of THF was dissolved 255 mg (0.858 mmol) of commercial N^{α}-Boc-tyrosine methyl ester, to which 37 mg (0.925 mmol) of sodium hydride (60%) was then added, followed by stirring at room temperature for 20 minutes. Thereto was then added 197 mg (1.03 mmol) of 4-cyanobenzyl bromide, followed by stirring at room temperature for 5 hours. After the end of reaction, the solvent was distilled off, and the residue was suspended in chloroform, followed by washing with 0.1 mol/l hydrochloric acid and a saturated saline solution. The solvent was dried over anhydrous sodium sulfate before distilling off the solvent, followed by purifying the residue using silica gel column chromatography (hexane/ethyl acetate) to provide 329 mg of the title compound as a white solid.

### (Example 121-2) Synthesis of (S)-2-t-butoxycarbonylamino-3-[4-(4-cyanobenzyloxy)phenyl]-N-trityloxypropionamide

In 3 ml of THF and 3 ml of methanol was dissolved 329 mg (0.802 mmol) of the compound obtained in Example 121-1, to which 3 ml of a 1 mol/l sodium hydroxide aqueous solution was then added, followed by stirring at room temperature for 4 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The residue was purified using silica gel column chromatography (chloroform/methanol) to provide a carboxylic acid. It was dissolved in 6 ml of chloroform, to which 222 mg (1.16 mmol) of WSCI hydrochloride and 234 mg (0.850 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (chloroform/ethyl acetate) to provide 246 mg of the title compound as a white solid.

### (Example 121-3) Synthesis of (S)-2-amino-3-[4-(4-cyanobenzyloxy)phenyl]-N-hydroxypropionamide

To 0°C was cooled 151 mg (0.231 mmol) of the compound obtained in Example 121-2, to which 1.5 ml of a 4 mol/l hydrogen chloride/dioxane solution was then added, followed by stirring at room temperature for 2 hours. After the end of reaction, the solvent was distilled off, and methanol was added to the residue, followed by washing with hexane. The methanol layer was concentrated, dried, and then washed with chloroform/hexane to provide 69.8 mg of the hydrochloride of the title compound as a white solid.
MS (Fab, Pos.): m/z = 312 [M+H]⁺
¹H-NMR (500 MHz, DMSO-d₆+D₂O): δ = 2.91 (1H, dd, J = 13.9,6.7 Hz), 2.97 (1H, dd, J = 13.9,7.5 Hz), 3.71 (1H, t, J = 7.0 Hz), 5.20 (2H, s), 6.98 (2H, d, J = 8.7 Hz), 7.15 (2H, d, J = 8.7 Hz), 7.64 (2H, d, J = 8.5 Hz), 7.88 (2H, d, J = 8.5 Hz).

### (Example 122) Synthesis of (S)-2-amino-3-(1-pentafluorophenylmethyl-1H-indol-3-yl)-N-hydroxypropionamide (compound No. 104)

### (Example 122-1) Synthesis of 2-t-butoxycarbonylamino-3-(1-pentafluorophenylmethyl-1H-indol-3-yl)propionic acid benzyl ester

In 15 ml of THF was dissolved 504 mg (1.28 mmol) of commercial N^{α}-Boc-tryptophan benzyl ester, to which 56 mg (1.4 mmol) of sodium hydride (60%) was then added, followed by stirring at 60°C for 10 minutes. Thereto was then added 0.230 ml (1.54 mmol) of pentafluorobenzyl bromide, followed by stirring at room temperature for 2.5 hours. After the end of reaction, the solution was concentrated, to which chloroform was added, followed by washing with 0.1 mol/l hydrochloric acid and a saturated saline solution. It was dried over anhydrous sodium sulfate before distilling off the solvent, followed by purifying the residue using silica gel column chromatography (hexane/ethyl acetate) to provide 615 mg of the title compound as a colorless, viscous liquid.

### (Example 122-2) Synthesis of (S)-2-t-butoxycarbonylamino-3-(1-pentafluorophenylmethyl-1H-indol-3-yl)-N-trityloxypropionamide

In 6.2 ml of THF and 6.2 ml of methanol was dissolved 615 mg (1.07 mmol) of the compound obtained in Example 122-1, to which 6.2 ml of a 1 mol/l sodium hydroxide aqueous solution was then added, followed by stirring at room temperature for one hour. After the end of reaction, the solvent was distilled off, followed by adding 1 mol/l hydrochloric acid to the residue to adjust the pH to 3 to 4 before collecting the precipitated solid by filtration. It was dissolved in 10 ml of chloroform, to which 289 mg (1.51 mmol) of WSCI hydrochloride and 306 mg (1.11 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate) to provide 602 mg of the title compound as a white solid.

### (Example 122-3) Synthesis of (S)-2-amino-3-(1-pentafluorophenylmethyl-1H-indol-3-yl)-N-hydroxypropionamide

To 0°C was cooled 196 mg (0.264 mmol) of the compound obtained in Example 122-2, to which 2 ml of a 4 mol/l hydrogen chloride/dioxane solution was then added, followed by stirring at 0°C for 3.5 hours. After the end of reaction, the solvent was distilled off, and the residue was purified using silica gel column chromatography (chloroform/methanol/water) to provide 98.8 mg of the hydrochloride of the title compound as a pale yellow solid.
MS (Fab, Pos.): m/z = 400 [M+H]⁺
¹H-NMR (500 MHz, DMSO-d₆+D₂0): δ = 3.06 (1H, dd, J = 7.6, 14.5 Hz), 3.15 (1H, dd, J = 6.7, 14.5 Hz), 3.75 (1H, dd, J = 6.7, 7.6 Hz), 5.49 (2H, s), 7.09-7.13 (1H, m), 7.23-7.29 (1H, m), 7.54 (1H, d, J = 8.4 Hz), 7.65 (1H, d, J = 7.9 Hz).

### (Example 123) Synthesis of (RS)-2-amino-3-(1-pentyl-1H-indol-3-yl)-N-hydroxypropionamide (compound No. 105)

### (Example 123-1) Synthesis of2-t-butoxycarbonylamino-3-(1-pentyl-1H-indol-3-yl)propionic acid benzyl ester

In 20 ml of THF was dissolved 495 mg (1.28 mmol) of commercial N"-Boc-tryptophan benzyl ester, to which 1 ml of 1-iodopentane and then 48 mg (1.2 mmol) of sodium hydride (60%) were subsequently added, followed by stirring at room temperature for 3 hours. After the end of reaction, the solution was concentrated, to which chloroform was then added, followed by washing with 0.1 mol/l hydrochloric acid and a saturated saline solution. It was dried over anhydrous sodium sulfate before distilling off the solvent, followed by purifying the residue using silica gel column chromatography (hexane/ethyl acetate) to provide 243 mg of the title compound as a colorless, viscous liquid.

### (Example 123-2) Synthesis of (RS)-2-t-butoxycarbonylamino-3-(1-pentyl-1H-indol-3-yl)-N-trityloxypropionamide

In 2.5 ml of THF and 2.5 ml of methanol was dissolved 243 mg (0.523 mmol) of the compound obtained in Example 123-1, to which 2.5 ml of a 1 mol/l sodium hydroxide aqueous solution was then added, followed by stirring at room temperature for 4 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The residue was dissolved in 4 ml of chloroform, to which 159 mg (0.830 mmol) of WSCI hydrochloride and 166 mg (0.602 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate) to provide 166 mg of the title compound as a white solid.

### (Example 123-3) Synthesis of (RS)-2-amino-3-(1-pentyl-1H-indol-3-yl)-N-hydroxypropionamide

To 0°C was cooled 148 mg (0.234 mmol) of the compound obtained in Example 123-2, to which 1.5 ml of a 4 mol/l hydrogen chloride/dioxane solution was then added, followed by stirring at 0°C for 3.5 hours. After the end of reaction, the solvent was distilled off, and methanol was added to the residue, followed by washing with hexane. The methanol layer was concentrated, dried, and then washed with chloroform/hexane to provide 77.0 mg of the hydrochloride of the title compound as a pale yellow solid.
MS (Fab, Pos.): m/z = 290 [M+H]⁺
¹H-NMR (500 MHz, DMSO-d₆+D₂O): δ = 0.85 (3H, td, J = 7.2, 3.8 Hz), 1.19-1.43 (4H, m), 1.73 (2H, quint, J = 7.3 Hz), 3.07 (1H, dd, J = 14.5, 7.2 Hz), 3.18 (1H, dd, J = 14.5, 7.2 Hz), 3.76 (1H, t, J = 7.2 Hz), 7.06 (1H, t, J = 7.9 Hz), 7.17 (1H, t, J = 7.9 Hz), 7.20 (1H, s), 7.44 (1 H, d, J = 8.0 Hz), 7.63 (1H, d, J = 8.0 Hz).

### (Example 124) Synthesis of (RS)-2-amino-3-[1-(4-t-butylbenzyl)-1H-indol-3-yl]-N-hydroxypropionamide (compound No. 106)

### (Example 124-1) Synthesis of (RS)-2-t-butoxycarbonylamino-3-[1-(4-t-butylbenzyl)-1H-indol-3-yl]propionic acid benzyl ester

In 5 ml ofTHF was dissolved 251 mg (0.636 mmol) of commercial N"-Boc-tryptophan benzyl ester, to which 0.140 ml of 4-t-butylbenzyl bromide and then 24 mg (0.60 mmol) of sodium hydride (60%) were subsequently added, followed by stirring at room temperature for 18 hours. After the end of reaction, the solution was concentrated, to which chloroform was then added, followed by washing with 0.1 mol/l hydrochloric acid and a saturated saline solution. It was dried over anhydrous sodium sulfate before distilling off the solvent, followed by purifying the residue using silica gel column chromatography (hexane/ethyl acetate) to provide 218 mg of the title compound as a colorless, viscous liquid.

### (Example 124-2) Synthesis of (RS)-2-t-butoxycarbonylamino-3-[1-(4-t-butylbenzyl)-1H-indol-3-yl]-N-trityloxypropionamide

In 2 ml of THF and 2 ml of methanol was dissolved 205 mg (0.379 mmol) of the compound obtained in Example 124-1, to which 2 ml of a 1 mol/l sodium hydroxide aqueous solution was then added, followed by stirring at room temperature for 4 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The residue was dissolved in 4 ml of chloroform, to which 118 mg (0.616 mmol) of WSCI hydrochloride and 125 mg (0.453 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate) to provide 153 mg of the title compound as a white solid.

### (Example 124-3) Synthesis of(RS)-2-amino-3-[1-(4-t-butylbenzyl)-1H-indol-3-yl]-N-hydroxypropionamide

To 0°C was cooled 153 mg (0.216 mmol) of the compound obtained in Example 124-2, to which 1.5 ml of a 4 mol/l hydrogen chloride/dioxane solution was then added, followed by stirring at 0°C for 2 hours. After the end of reaction, the solvent was distilled off, and methanol was added to the residue, followed by washing with hexane. The methanol layer was concentrated, dried, and then washed with chloroform/hexane to provide 65.8 mg of the hydrochloride of the title compound as a pale yellow solid.
MS (Fab, Pos.): m/z = 366 [M+H]⁺
¹H-NMR (500 MHz, DMSO-d₆+D₂O): δ = 1.23 (9H, s), 3.10 (1H, dd, J = 14.5, 7.2 Hz), 3.18 (1H, dd, J = 14.5, 7.0 Hz), 3.79 (1H, t, J = 7.2 Hz), 5.36 (2H, s), 7.06 (1H, t, J = 7.9 Hz), 7.11-7.20 (3H, m), 7.29-7.35 (3H, m), 7.42 (1H, d, J = 8.2 Hz), 7.65 (1H, d, J = 7.8 Hz).

### (Example 125) Synthesis of (RS)-2-amino-3-[1-(4-cyanobenzyl)-1H-indol-3-yl]-N-hydroxypropionamide (compound No. 107)

### (Example 125-1) Synthesis of (RS)-2-t-butoxycarbonylamino-3-[1-(4-cyanobenzyl)-1H-indol-3-yl]propionic acid benzyl ester

In 5 ml of THF was dissolved 507 mg (1.29 mmol) of commercial N^{α}-Boc-tryptophan benzyl ester, to which 298 mg (1.52 mmol) of 4-cyanobenzyl bromide and then 50 mg (1.25 mmol) of sodium hydride (60%) were subsequently added, followed by stirring at room temperature for 18 hours. After the end of reaction, the solution was concentrated, to which chloroform was added, followed by washing with 0.1 mol/l hydrochloric acid and a saturated saline solution. It was dried over anhydrous sodium sulfate before distilling off the solvent, followed by purifying the residue using silica gel column chromatography (hexane/ethyl acetate) to provide 517 mg of the title compound as a colorless, viscous liquid.

### (Example 125-2) Synthesis of (RS)-2-t-butoxyamino-3-[1-(4-cyanobenzyl)-1H-indol-3-yl]-N-trityloxypropionamide

In 5 ml ofTHF and 5 ml of methanol was dissolved 517 mg (1.02 mmol) of the compound obtained in Example 125-1, to which 5 ml of a 1 mol/l sodium hydroxide aqueous solution was then added, followed by stirring at room temperature for 4.5 hours. After the end of reaction, the solvent was distilled off, followed by adding 1 mol/l hydrochloric acid to the residue to adjust the pH to 3 to 4. The solution was extracted with chloroform, and the extract was washed with a saturated saline solution before drying with anhydrous sodium sulfate. The residue was dissolved in 7 ml of chloroform, to which 251 mg (1.31 mmol) of WSCI hydrochloride and 264 mg (0.959 mmol) of O-tritylhydroxylamine were then added, followed by stirring at room temperature for 2 days. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate) to provide 416 mg of the title compound as a white solid.

### (Example 125-3) Synthesis of (RS)-2-amino-3-[1-(4-cyanobenzyl)-1H-indol-3-yl]-N-hydroxypropionamide

To 0°C was cooled 209 mg (0.309 mmol) of the compound obtained in Example 125-2, to which 2 ml of a 4 mol/l hydrogen chloride/dioxane solution was then added, followed by stirring at 0°C for 2 hours. After the end of reaction, the solvent was distilled off, and methanol was added to the residue, followed by washing with hexane. The methanol layer was concentrated, dried, and then washed with chloroform/hexane to provide 97.8 mg of the hydrochloride of the title compound as a pale yellow solid.
MS (Fab, Pos.): m/z = 336 [M+H]⁺
¹H-NMR (500 MHz, DMSO-d₆+D₂O): δ = 3.12 (1H, dd, J = 14.7, 7.2 Hz), 3.19 (1H, dd, J = 14.7, 7.3 Hz), 3.80 (1H, t, J = 7.2 Hz), 5.50 (2H, s), 7.09 (1H, t, J = 7.6 Hz), 7.14 (1H, t, J = 7.6 Hz), 7.30-7.39 (4H, m), 7.68 (1H, d, J = 7.8 Hz), 7.76 (2H, d, J = 8.6 Hz).

### (Example 126) Synthesis of (RS)-2-amino-3-[1-(3,4-difluorobenzyl)-1H-indol-3-yl]-N-hydroxypropionamide (compound No. 108)

### (Example 126-1) Synthesis of (RS)-2-t-butoxycarbonylamino-3-[1-(3,4-difluorobenzyl)-1H-indol-3-yl]propionic acid benzyl ester

In 10 ml of THF was dissolved 502 mg (1.27 mmol) of commercial N^{α}-Boc-tryptophan benzyl ester, to which 0.196 mg (1.52 mmol) of 3,4-difluorobenzyl bromide and then 50 ml (1.25 mmol) of sodium hydride (60%) were subsequently added, followed by stirring at room temperature for 20 hours. After the end of reaction, the solution was concentrated, to which chloroform was then added, followed by washing with 0.3 mol/l hydrochloric acid and a saturated saline solution. It was dried over anhydrous sodium sulfate before distilling off the solvent, followed by purifying the residue using silica gel column chromatography (hexane/ethyl acetate) to provide 416 mg of the title compound as a colorless, viscous liquid.

### (Example 126-2) Synthesis of (RS)-2-t-butoxycarbonylamino-3-[1-(3,4-difluorobenzyl)-1H-indol-3-yl]-N-trityloxypropionamide

In 4 ml of THF and 4 ml of methanol was dissolved 416 mg (0.800 mmol) of the compound obtained in Example 126-1, to which 4 ml of a 1 mol/l sodium hydroxide aqueous solution was then added, followed by stirring at room temperature for 4.5 hours. After the end of reaction, the solvent was distilled off, followed by adding 1 mol/l hydrochloric acid to the residue to adjust the pH to 2 to 3. The solution was extracted with chloroform, and the extract was washed with a saturated saline solution before drying with anhydrous sodium sulfate. The residue was dissolved in 8 ml of chloroform, to which 264 mg (1.38 mmol) of WSCI hydrochloride and 306 mg (1.11 mmol) of O-tritylhydroxylamine were then added, followed by stirring at room temperature for 11 hours. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate) to provide 350 mg of the title compound as a white solid.

### (Example 126-3) Synthesis of (RS)-2-amino-3-[1-(3,4-difluorobenzyl)-1H-indol-3-yl]-N-hydroxypropionamide

To 0°C was cooled 200 mg (0.291 mmol) of the compound obtained in Example 126-2, to which 2 ml of a 4 mol/l hydrogen chloride/dioxane solution was then added, followed by stirring at 0°C for 2 hours. After the end of reaction, the solvent was distilled off, and methanol was added to the residue, followed by washing with hexane. The methanol layer was concentrated, dried, and then washed with chloroform/hexane to provide 99.1 mg of the hydrochloride of the title compound as a pale yellow solid.
MS (Fab, Pos.): m/z = 345 [M+H]⁺
¹H-NMR (500 MHz, DMSO-d₆+D₂O): δ = 3.10 (1H, dd, J = 14.5,7.2 Hz), 3.20 (1H, dd, J = 14.5, 7.2 Hz), 3.79 (1H, t, J = 7.2 Hz), 5.38 (2H, s), 7.05-7.12 (2H, m), 7.15 (1H, td, J = 8.1,1.1 Hz), 7.25-7.31 (1H, m), 7.33-7.41 (3H, m), 7.67 (1H, d, J = 7.6 Hz).

### (Example 127) Synthesis of (S)-2-amino-3-(1-pentyl-1H-indol-3-yl)-N-hydroxypropionamide (compound No. 109)

### (Example 127-1) Synthesis of (S)-2-t-butoxycarbonylamino-3-(1-pentyl-1H-indol-3-yl)propionic acid benzyl ester

In 82 ml of anhydrous THF was dissolved 4.10 g (10.4 mmol) of commercial N^{α-}Boc-tryptophan benzyl ester, to which 8.2 ml of iodopentane was then added. Thereto was added 2.03 g (6.24 mmol) of cesium carbonate, followed by stirring at room temperature for 24 hours. After the end of reaction, the solvent was distilled off, and toluene was added to the residue, followed by washing with a hydrochloric acid aqueous solution adjusted to a pH of about 3 and a saturated saline solution before drying the organic layer with anhydrous sodium sulfate. The solvent was distilled off, and the residue was purified using silica gel column chromatography (hexane/ethyl acetate) to provide 752.6 mg of the desired compound as a colorless, viscous oil.

### (Example 127-2) Synthesis of (S)-2-t-butoxycarbonylamino-3-(1-pentyl-1H-indol-3-yl)propionic acid

In 90 ml of dioxane was dissolved 1.84 g (3.96 mmol) of the compound obtained in Example 127-1, to which 368 mg of 10% palladium carbon was then added, followed by stirring for 2 hours under an atmosphere of hydrogen. After the end of reaction, the catalyst was removed by celite filtration, and the solvent was distilled off, followed by drying the residue under reduced pressure to provide 1.56 g of the desired compound as a colorless, viscous liquid.

### (Example 127-3) Synthesis of (S)-2-t-butoxycarbonylamino-3-(1-pentyl-1H-indol-3-yl)-N-benzyloxypropionamide

In 9 ml of chloroform was suspended 410 mg (2.46 mmol) of O-benzylhydroxylamine hydrochloride, to which 3 ml of a 1 mol/l sodium hydroxide aqueous solution was then added, followed by vigorous stirring. The solution was separated, and the organic layer was washed with a saturated saline solution before drying with anhydrous sodium sulfate.

The above prepared O-benzylhydroxylamine/chloroform solution was added to a solution consisting of 770 mg (2.05 mmol) of the compound obtained in Example 127-2 dissolved in 8 ml of chloroform, to which 615 mg (3.21 mmol) of WSCI hydrochloride and 304 mg (2.25 mmol) of HOBt had been added, followed by stirring at room temperature for 6 hours. After the end of reaction, chloroform was added thereto, followed by washing with a 0.1 mol/l hydrochloric acid aqueous solution and a saturated saline solution. The organic layer was dried over anhydrous sodium sulfate, and the solvent was then distilled off. The residue was recrystallized from ethyl acetate/hexane to provide 678 mg of the desired compound as a colorless solid.

### (Example 127-4) Synthesis of (S)-2-amino-3-(1-pentyl-1H-indol-3-yl)-N-hydroxypropionamide

In 5.5 ml of dioxane was dissolved 114 mg (0.239 mmol) of the compound obtained in Example 127-3, to which 55 mg of 10% palladium carbon was then added, followed by stirring at room temperature for 3 hours under an atmosphere of hydrogen. After the end of reaction, the catalyst was removed by celite filtration, and the solvent was distilled off, followed by drying the residue under reduced pressure to provide 95.3 mg of the desired compound as a colorless, viscous liquid. To this compound was added 1 ml of a 4 mol/l hydrogen chloride/dioxane solution under cooling with ice, followed by stirring under cooling with ice for 3 hours. After the end of reaction, the solvent and hydrochloric acid were distilled off. The residue was purified using silica gel column chromatography (chloroform/methanol) and then suspended in 0.5 ml of a 0.5 mol/l hydrochloric acid aqueous solution, followed by concentration and drying under reduced pressure to provide 62.3 mg of the hydrochloride of the desired compound as a white solid.
MS (Fab, Pos.): m/z = 290 [N4+H]⁺
¹H-NMR (500 MHz, DMSO-d₆+D₂O): δ = 0.85 (3H, td, J = 7.2, 3.8 Hz), 1.19-1.43 (4H, m), 1.73 (2H, quint, J = 7.3 Hz), 3.07 (1H, dd, J = 14.5, 7.2 Hz), 3.18 (1H, dd, J = 14.5, 7.2 Hz), 3.76 (1H, t, J = 7.2 Hz), 7.06 (1H, t, J = 7.9 Hz), 7.17 (1H, t, J = 7.9 Hz), 7.20 (1H, s), 7.44 (1H, d, J = 8.0 Hz), 7.63 (1H, d, J = 8.0 Hz).

### (Example 128) Synthesis of (R)-2-amino-3-(1-pentyl-1H-indol-3-yl)-N-hydroxypropionamide (compound No. 110)

### (Example 128-1) Synthesis of (R)-2-t-butoxycarbonylamino-3-(1-pentyl-1H-indol-3-yl)propionic acid benzyl ester

In 60 ml of DMF was dissolved 3.00 g (9.86 mmol) of commercial N^{α}-Boc-D-tryptophan, to which 1.77 g (5.42 mmol) of cesium carbonate and 1.17 ml (9.86 mmol) of benzyl bromide were then added, followed by stirring at room temperature for 2 hours. After the end of reaction, DMF was distilled off, followed by suspending the residue in toluene/ethyl acetate before washing with distilled water and a saturated saline solution. The organic layer was dried over anhydrous sodium sulfate, and vacuum concentrated. The residue was recrystallized from ethyl acetate/hexane to provide 3.50 g of the desired compound as a white solid.

### (Example 128-2) Synthesis of (R)-2-t-butoxycarbonylamino-3-(1-pentyl-1H-indol-3-yl)propionic acid benzyl ester

In 50 ml of acetone was dissolved 2.50 g (6.34 mmol) of the compound obtained in Example 128-1, to which 10 ml of iodopentane was then added. Thereto was added 1.14 g (3.49 mmol) of cesium carbonate, followed by stirring at 60°C for 2 days. After the end of reaction, the solvent was distilled off, and toluene was added to the residue before washing with distilled water and a saturated saline solution, followed by drying the organic layer with anhydrous sodium sulfate. The solvent was distilled off, followed by purifying the residue using silica gel column chromatography (hexane/ethyl acetate) to provide 1.11 g of the desired compound as a colorless, viscous oil.

### (Example 128-3) Synthesis of (R)-2-t-butoxycarbonylamino-3-(1-pentyl-1H-indol-3-yl)propionic acid

In 56 ml of dioxane was dissolved 1.11 g (2.38 mmol) of the compound obtained in Example 128-2, to which 222 mg of 10% palladium carbon was then added, followed by stirring for 2 hours under an atmosphere of hydrogen. After the end of reaction, the catalyst was removed by celite filtration, and the solvent was distilled off, followed by drying the residue under reduced pressure to provide 1.56 g of the desired compound as a light purple solid.

### (Example 128-4) Synthesis of (R)-2-t-butoxycarbonylamino-3-(1-pentyl-1H-indol-3-yl)-N-benzyloxypropionamide

To a solution consisting of 870 mg (2.25 mmol) of the compound obtained in Example 128-3 dissolved in 17.4 ml of chloroform, to which 647 mg (3.38 mmol) of WSCI hydrochloride and 334 mg (2.48 mmol) of HOBt had been added, was added 291 mg (2.36 mmol) of O-benzylhydroxylamine, followed by stirring at room temperature for 4 hours. After the end of reaction, chloroform was added thereto, followed by washing with a 0.1 mol/l hydrochloric acid aqueous solution and a saturated saline solution. The organic layer was dried over anhydrous sodium sulfate, and the solvent was then distilled off. The residue was purified using silica gel column chromatography (hexane/ethyl acetate) to provide 702 mg of the desired compound as a colorless foam.

### (Example 128-5) Synthesis of (R)-2-amino-N-hydroxy-3-(1-pentyl-1H-indol-3-yl)propionamide

In 35 ml of dioxane was dissolved 680 mg (1.42 mmol) of the compound obtained in Example 128-4, to which 350 mg of 10% palladium carbon was then added, followed by stirring at room temperature for 19 hours under an atmosphere of hydrogen. After the end of reaction, the catalyst was removed by celite filtration, and the solvent was distilled off, followed by drying the residue under reduced pressure. Thereto was added 5.5 ml of a 4 mol/l hydrogen chloride/dioxane solution under cooling with ice, followed by stirring under cooling with ice for 4 hours. After the end of reaction, the solvent and hydrochloric acid were distilled off. The residue was purified using silica gel column chromatography (chloroform/methanol) and then suspended in 0.5 ml of a 0.5 mol/l hydrochloric acid aqueous solution, followed by concentration and vacuum drying to provide 349 mg of the hydrochloride of the desired compound as a light purple solid.
MS (Fab, Pos.): m/z = 290 [M+H]⁺
¹H-NMR (500 MHz, DMSO-d₆+D₂O): δ = 0.85 (3H, td, J = 7.2, 3.8 Hz), 1.19-1.43 (4H, m), 1.73 (2H, quint, J = 7.3 Hz), 3.07 (1H, dd, J = 14.5, 7.2 Hz), 3.18 (1H, dd, J = 14.5, 7.2 Hz), 3.76 (1H, t, J = 7.2 Hz), 7.06 (1H, t, J = 7.9 Hz), 7.17 (1H, t, J = 7.9 Hz), 7.20 (1H, s), 7.44 (1H, d, J = 8.0 Hz), 7.63 (1H, d, J = 8.0 Hz).

### (Example 129) Maillard reaction-inhibiting effects

This Example examined Maillard reaction-inhibiting effects using, as test compounds, the compounds produced in the above-described Examples and the commercially available substances arginine hydroxamate (compound No. 111), tyrosine hydroxamate (compound No. 112), and tryptophan hydroxamate (compound No. 113).
More specifically, 10 mg/mL lysozyme, 200 mM fructose, and each of the above-described test compounds dissolved in a 0.4 mM sodium phosphate buffer (pH 7.4) so as to provide a concentrations of 0.1, 0.3, 1, or 3 mM were incubated at 37°C for 4 days. After the incubation, the samples were subjected to SDS-PAGE, stained with Coomassie Brilliant Blue R-250, and quantitated for the formed dimmers using a densitometer. The Maillard reaction-inhibiting activity was defined as the dimmer formation inhibition rate of a test compound calculated on the basis that the amount of dimmer is 100% in the absence of the test compound, using, as blank, the amount of dimer in the absence of fructose and the test compound. The results are shown in Tables 1 and 2. As indicated in these Tables, each of the test compounds had an excellent Maillard reaction-inhibiting effect.

[Table 1]
Table 1

**[Table 2]**

| Test Compound | Maillard reaction-inhibiting activity (%) | | | |
|---|---|---|---|---|
| | Test compound concentration | | | |
| | 3 mM | 1 mM | 0. 3mM | 0. 1mM |
| 2 | 100 | 100 | 100 | 86 |
| 5 | 97 | 35 | 5 | -3 |
| 6 | 100 | 103 | 97 | 49 |
| 7 | 72 | 1 3 | -4 | -3 |
| 15 | 98 | 43 | -2 | -12 |
| 16 | 98 | 71 | 11 | -7 |
| 19 | 101 | 95 | 54 | 10 |
| 25 | 100 | 40 | 6 | 7 |
| 26 | 100 | 68 | 17 | 11 |
| 27 | 100 | 32 | 7 | 1 |
| 28 | 100 | 100 | 100 | 100 |
| 30 | 92 | 80 | 74 | 56 |
| 31 | 100 | 100 | 72 | 29 |
| 32 | 102 | 89 | 50 | 23 |
| 33 | 102 | 98 | 93 | 57 |
| 34 | 102 | 102 | 98 | 37 |
| 36 | 102 | 69 | 33 | 15 |
| 37 | 94 | 35 | 16 | 1 |
| 38 | 100 | 100 | 100 | 100 |
| 40 | 100 | 96 | 49 | 37 |
| 71 | 99 | 98 | 97 | 71 |
| 72 | 101 | 100 | 94 | 53 |
| 73 | 100 | 98 | 98 | 94 |

**Table 2**

| Test compound | Maillard reaction-inhibiting activity (%) | | | |
|---|---|---|---|---|
| | Test compound concentration | | | |
| | 3 mM | 1mM | 0. 3mM | 0. 1mM |
| 74 | 100 | 101 | 99 | 98 |
| 75 | 100 | 100 | 100 | 82 |
| 76 | 98 | 95 | 65 | 13 |
| 85 | 98 | 98 | 100 | 99 |
| 94 | 102 | 100 | 83 | 12 |
| 95 | 108 | 107 | 106 | 102 |
| 96 | 104 | 104 | 103 | 99 |
| 97 | 98 | 98 | 98 | 96 |
| 99 | 101 | 99 | 95 | 95 |
| 100 | 97 | 96 | 96 | 93 |
| 101 | 98 | 102 | 102 | 102 |
| 102 | 95 | 94 | 88 | 78 |
| 103 | 97 | 100 | 95 | 89 |
| 104 | 100 | 94 | 97 | 95 |
| 105 | 98 | 98 | 97 | 95 |
| 106 | 95 | 97 | 95 | 94 |
| 107 | 101 | 101 | 100 | 101 |
| 108 | 104 | 104 | 103 | 102 |
| 109 | 103 | 104 | 105 | 104 |
| 110 | 101 | 101 | 103 | 96 |
| 111 | 98 | 75 | 17 | 10 |
| 112 | 101 | 99 | 61 | 24 |
| 113 | 102 | 99 | 76 | 42 |
| Aminoguanidine | 2 6 | 7 | ―5 | ―3 |

### (Example 130) Methyl glyoxal-trapping activity test

In 0.2 mL of PBS were incubated 0.02 mM of methyl glyoxal and 0.2 mM of test compound at 37°C for 30 minutes. Subsequently, a volume of 5 mM DMB (1,2-diamino-4,5-methylenedioxy benzene) solution equivalent to 10 µL of the incubation sample was added, followed by further incubation at 4°C for 7 hours to react the remaining methyl glyoxal with DMB. After the end of reaction, a 0.4 M phosphate buffer solution (pH 7.2) was added for neutralization, followed by quantitating the modified product of methyl glyoxal-DMB using HPLC.
HPLC having a YMC-Pack Pro C 18 column (φ4.6 mm × 150 mm) and employing 5 mM sodium phosphate buffer (pH 7.4)-25% acetonitrile as a mobile phase was used at a flow rate of 0.7 mL/min to determine fluorescence strength at Ex352 nm and Em385 nm.
The proportion of methyl glyoxal trapped by the test compound was calculated as % trapping activity, based on the quantitative value of the total modified product of methyl glyoxal-DMB in the absence of the test compound.
The results are shown in Table 3. As indicated in Table 3, each of the test compounds had an excellent methyl glyoxal-trapping activity.

[Table 3]

**Table3**

| Test compound | Methyl-glyoxal-trapping activity (% trapping) |
|---|---|
| 95 | 84. 2±0. 6 |
| 104 | 81. 9±2. 1 |
| 105 | 93. 3±0. 8 |
| 107 | 87. 3±0. 5 |
| 111 | 77. 6±0. 5 |
| 1 1 2 | 74. 4±2. 7 |
| 113 | 96. 2±0. 7 |
| Aminoguanidine | 5 2. 8 ± 1. 5 |

### (Example 131) Glyoxal-trapping activity test

In 0.2 mL of PBS were incubated 0.02 mM of glyoxal and 0.2 mM of test compound at 37°C for 30 minutes. Subsequently, a volume of 5 mM DMB (1,2-diamino-4,5-methylenedioxy benzene) solution equivalent to 10 µL of the incubation sample was added, followed by further incubation at 4°C for 7 hours to react the remaining methyl glyoxal with DMB. After the end of reaction, a 0.4 M phosphate buffer solution (pH 7.2) was added for neutralization, followed by quantitating the modified product of glyoxal-DMB using HPLC.
HPLC having a YMC-Pack Pro C18 column (φ4.6 mm × 150 mm) and employing 5 mM sodium phosphate buffer (pH 7.4)-25% acetonitrile as a mobile phase was used at a flow rate of 0.7 mL/min to determine fluorescence strength at Ex352 nm and Em385 nm.
The proportion of glyoxal trapped by the test compound was calculated as % trapping activity, based on the quantitative value of the total modified product of glyoxal-DMB in the absence of the test compound. The results are shown in Table 4. As indicated in Table 4, each of the test compounds had an excellent glyoxal-trapping activity.

[Table 4]

**Table4**

| Test compound | Glyoxal-trapping activity (% trapping) |
|---|---|
| 95 | 65. 7±2. 2 |
| 1 04 | 66. 5±2. 4 |
| 1 05 | 69. 7±1. 1 |
| 1 07 | 69. 8±1. 2 |
| 1 1 1 | 75. 2±1. 3 |
| 1 1 2 | 67. 8±1. 2 |
| 113 | 75. 9±0. 9 |
| Aminoguanidine | 3 2. 9±3. 8 |

### (Example 132) Synthesis of (R)-2-amino-3-[1-(3,4-difluorobenzyl)-1H-indol-3-yl]-N-hydroxypropionamide (compound No. 114)

### (Example 132-1) Synthesis of (R)-2-t-butoxycarbonylamino-3-[1-(3,4-difluorobenzyl)-1H-indol-3-yl]propionic acid benzyl ester

In 6 ml of acetone was dissolved 300 mg (0.761 mmol) of the compound obtained in Example 128-1, to which 0.0967 ml (0.913 mmol) of 3,4-difluorobenzyl bromide and 136 mg (0.419 mmol) of cesium carbonate were then added, followed by stirring at 55°C for 24 hours. After the end of reaction, the solvent was distilled off, and chloroform was added to the residue before washing with distilled water and a saturated saline solution. It was dried over anhydrous sodium sulfate before distilling off the solvent, followed by purifying the residue using silica gel column chromatography (hexane/ethyl acetate) to provide 209 mg of the title compound as a colorless oil.

### (Example 132-2) Synthesis of (R)-2-t-butoxyamino-3-[1-(3,4-difluorobenzyl)-1H-indol-3-yl]-N-trityloxypropionamide

In 10 ml of dioxane was dissolved 203 mg (0.390 mmol) of the compound obtained in Example 132-1, to which 20 mg of 10% palladium carbon was then added, followed by stirring at room temperature for 4 hours under an atmosphere of hydrogen. After the end of reaction, the catalyst was removed by celite filtration, and the filtrate was concentrated and vacuum dried. It was dissolved in 6 ml of chloroform, to which 112 mg (0.585 mmol) of WSCI hydrochloride, 55.3 mg (0.409 mmol) of HOBt, and 113 mg (0.410 mmol) of O-tritylhydroxylamine were then added, followed by stirring at room temperature for 20 hours. After the end of reaction, chloroform was added thereto, followed by washing with hydrochloric acid, a sodium hydroxide aqueous solution, and a saturated saline solution before drying with anhydrous sodium sulfate. The solvent was distilled off, followed by purifying the resultant residue using silica gel column chromatography (hexane/ethyl acetate) to provide 160 mg of the title compound as a pale yellow foam.

### (Example 132-3) Synthesis of (R)-2-amino-3-[1-(3,4-difluorobenzyl)-1H-indol-3-yl]-N-hydroxypropionamide (compound No. 114)

To 0°C was cooled 160 mg (0.233 mmol) of the compound obtained in Example 132-2, to which 1.6 ml of a 4 mol/l hydrogen chloride/dioxane was then added, followed by stirring at room temperature for 4 hours. After the end of reaction, the solvent was distilled off, and the solid was dissolved in methanol. The methanol layer was washed with hexane, followed by distilling off the methanol. The resultant solid was further washed with hexane and vacuum dried to provide 77.8 mg of the hydrochloride of the title compound as a white solid.
MS (Fab, Pos.): m/z = 346 [M+H]⁺
¹H-NMR (500 MHz, DMSO-d₆+D₂O) δ = 3.11 (1H, dd, J = 7.3, 14.7 Hz), 3.20 (1H, dd, J = 7.3, 14.7 Hz), 3.79 (1H, t, J = 7.3 Hz), 5.37 (2H, s), 7.06-7.14 (2H, m), 7.15 (1H, dt, J = 1.2, 7.1 Hz), 7.27 (1H, ddd, J = 2.2, 7.8, 11.4 Hz), 7.32-7.41 (3H, m), 7.67 (1 H, d, J = 7.8 Hz).

### (Example 133) Synthesis of (R)-2-amino-3-(1-methyl-1H-indol-3-yl)-N-hydroxypropionamide (compound No. 115)

### (Example 133-1) Synthesis of (R)-2-t-butoxycarbonylamino-3-(1-methyl-1H-indol-3-yl)propionic acid benzyl ester

In 40 ml of acetone was dissolved 2.00 g (5.07 mmol) of the compound obtained in Example 128-1, to which 1.89 ml of iodomethane and 909 mg (2.78 mmol) of cesium carbonate were then added, followed by stirring at 50°C for 48 hours. After the end of reaction, the precipitate was removed by filtration, and the solvent was distilled off, followed by adding toluene/ethyl acetate before washing with distilled water. It was dried over anhydrous sodium sulfate before distilling off the solvent, followed by purifying the residue using silica gel column chromatography (hexane/ethyl acetate) to provide 460 mg of the title compound as a colorless oil.

### (Example 133-2) Synthesis of (R)-2-t-butoxycarbonylamino-3-(1-methyl-1H-indol-3-yl)-N-benzyloxypropionamide

In 25 ml of DME was dissolved 460 mg (1.13 mmol) of the compound obtained in Example 133-1, to which 90 mg of 10% palladium carbon was then added, followed by stirring at room temperature for 4 hours under an atmosphere of hydrogen. After the end of reaction, the catalyst was removed by celite filtration, and the filtrate was concentrated and vacuum dried. It was dissolved in 10 ml of chloroform, to which 324 mg (1.69 mmol) of WSCI hydrochloride and 143 mg (1.19 mmol) of O-benzylhydroxylamine were then added, followed by stirring at room temperature for 16 hours. After the end of reaction, chloroform was added thereto, followed by washing with hydrochloric acid and a saturated saline solution before drying with anhydrous sodium sulfate. The solvent was distilled off, followed by purifying the resultant residue using silica gel column chromatography (hexane/ethyl acetate) to provide 257 mg of the title compound as a white solid.

### (Example 133-3) Synthesis of (R)-2-amino-3-(1-methyl-1H-indol-3-yl)-N-hydroxypropionamide (compound No. 115)

In 13 ml of DME was dissolved 257 mg (0.607 mmol) of the compound obtained in Example 133-2, to which 120 mg of 10% palladium carbon was then added, followed by stirring at room temperature for 24 hours under an atmosphere of hydrogen. After the end of reaction, the catalyst was removed by celite filtration, and the solvent was distilled off. The residue was vacuum dried and cooled to 0°C, to which 2.3 ml of 4 mol/l hydrogen chloride/dioxane was then added, followed by stirring at room temperature for 2 hours. After the end of reaction, the solvent was distilled off before azeotroping with methanol, followed by vacuum drying to provide 156 mg of the hydrochloride of the title compound as a white solid.
MS (Fab, Pos.): m/z = 234 [M+H]⁺
¹H-NMR (500 MHz, DMSO-d₆+D₂O) δ = 3.08 (1H, dd, J = 7.5, 14.5 Hz), 3.17 (1H, dd, J = 6.6, 14.5 Hz), 3.74 (3H, s), 3.72-3.77 (1H, m), 7.08 (1H, dd, J = 7.2, 7.8 Hz), 7.17 (1H, s), 7.19 (1H, dd, J = 7.2, 8.2 Hz), 7.42 (1 H, d, J = 8.2 Hz), 7.65 (1H, d, J = 7.8 Hz).

### (Example 134) Synthesis of (R)-2=amino-4-(1-cyclohexylmethyl-1H-benzimidazol-2-yl)-N-hydroxybutyramide (compound No. 116)

### (Example 134-1) Synthesis of (R)-4-(2-aminophenylcarbamoyl)-2-t-butoxycarbonylaminobutyric acid benzyl ester

In 25 ml of chloroform was dissolved 2.50 g (7.41 mmol) of commercial Boc-D-glutamic acid benzyl ester, to which 2.13 g (11.1 mmol) of WSCI hydrochloride and 1.10 g (8.14 mmol) of HOBt were then added, and the solution was allowed to stand at room temperature for 5 minutes. It was added dropwise into 25 ml of chloroform solution of 0.962 g (8.89 mmol) of 1,2-phenylenediamine over a period of 2 hours, followed by stirring at room temperature for 18 hours. After the end of reaction, chloroform was supplemented thereto, followed by washing with hydrochloric acid, a sodium hydroxide aqueous solution, and a saturated saline solution. It was dried over anhydrous sodium sulfate before distilling off the solvent, followed by recrystallizing the residue from hexane/ethyl acetate to provide 1.96 g of the title compound as a white crystal.

### (Example 134-2) Synthesis of (R)-2-t-butoxycarbonylamino-4-(1-cyclohexylmethyl-1H-benzimidazol-2-yl)butyric acid benzyl ester

In 9 ml of dichloroethane was dissolved 298 mg (0.697 mmol) of the compound obtained in Example 134-1, to which 0.094 ml (0.772 mmol) of cyclohexanecarbaldehyde was then added, followed by stirring at room temperature for 50 minutes. Thereto was added 223 mg (1.05 mmol) of sodium triacetoxyborohydride, followed by stirring at room temperature for 5 hours. After the end of reaction, chloroform was added thereto, followed by washing with a sodium hydroxide aqueous solution and a saturated saline solution before drying with anhydrous sodium sulfate. The solvent was distilled off, and the residue was dissolved in 4 ml of acetic acid before stirring at 60°C for 2 hours. After the end of reaction, the solvent was distilled off, and chloroform was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 247 mg of the title compound as a white solid.

### (Example 134-3) Synthesis of (R)-2-t-butoxycarbonylamino-4-(1-cyclohexylmethyl-1H-benzimidazol-2-yl)-N-trityloxybutyramide

In 10 ml of dioxane was dissolved 228 mg (0.450 mmol) of the compound obtained in Example 134-2, to which 46 mg of 10% palladium carbon was then added, followed by stirring at room temperature for 4 hours under an atmosphere of hydrogen. After the end of reaction, the catalyst was removed by celite filtration, and the filtrate was concentrated and vacuum dried. It was dissolved in 6 ml of chloroform, to which 129 mg (0.674 mmol) of WSCI hydrochloride, 60.8 mg (0.450 mmol) of HOBt, and 130 mg (0.472 mmol) of O-tritylhydroxylamine were then added, followed by stirring at room temperature for 20 hours. After the end of reaction, chloroform was added thereto, followed by washing with hydrochloric acid, a sodium hydroxide aqueous solution, and a saturated saline solution before drying with anhydrous sodium sulfate. The solvent was distilled off, followed by purifying the resultant residue using silica gel column chromatography (chloroform/ethyl acetate) to provide 169 mg of the title compound as a pale yellow foam.

### (Example 134-4) Synthesis of (R)-2-amino-4-(1-cyclohexylmethyl-1H-benzimidazol-2-yl)-N-hydroxybutyramide (compound No. 116)

To 0°C was cooled 169 mg (0.251 mmol) of the compound obtained in Example 134-3, to which 1.7 ml of 4 mol/l hydrogen chloride/dioxane was then added, followed by stirring at room temperature for 5 hours. After the end of reaction, the solvent was distilled off, and the solid was dissolved in methanol. The methanol layer was washed with hexane, followed by distilling off the methanol. The resultant solid was further washed with hexane and vacuum dried to provide 81.2 mg of the hydrochloride of the title compound as a white solid.
MS (Fab, Pos.): m/z = 331 [M+H]⁺
¹H-NMR (500 MHz, DMSO-d₆+D₂O) δ = 1.08-1.22 (5H, m), 1.53-1.72 (5H, m), 1.85-1.95 (1H, m), 2.32-2.38 (2H, m), 3.23 (1H, dd, J = 8.3, 16.4 Hz), 3.31 (1H, dd, J = 8.3, 16.4 Hz), 3.26-3.48 (2H, m), 3.85-3.89 (1H, m), 4.26 (2 H, d, J = 7.6 Hz), 7.55-7.60 (2H, m), 7.78-7.83 (1H, m), 7.95-7.98 (1H, m).

### (Example 135) Synthesis of (R)-2-amino-4-(1-cyclohexylmethyl-5,6-dimethyl-1H-benzimidazol-2-yl)-N-hydroxybutyramide (compound No. 117)

### (Example 135-1) Synthesis of (R)-4-(2-amino-4,5-dimethyl-phenylcarbamoyl)-2-t-butoxycarbonylaminobutyric acid benzyl ester

In 25 ml of chloroform was dissolved 2.50 g (7.41 mmol) of commercial Boc-D-glutamic acid benzyl ester, to which 2.13 g (12.1 mmol) of WSCI hydrochloride and 1.10 g (8.14 mmol) of HOBt were then added, and the solution was allowed to stand at room temperature for 5 minutes. It was added dropwise into 25 ml of chloroform solution of 1.21 g (8.89 mmol) of 4,5-dimethyl-1,2-phenylenediamine over a period of 2 hours, followed by stirring at room temperature for 2 days. After the end of reaction, chloroform was supplemented, followed by washing with hydrochloric acid, a sodium hydroxide aqueous solution, and a saturated saline solution. It was dried over anhydrous sodium sulfate before distilling off the solvent, followed by purifying the residue using silica gel column chromatography (chloroform/ethyl acetate) to provide 1.96 g of the title compound as a white solid.

### (Example 135-2) Synthesis of (R)-2-t-butoxycarbonylamino-4-(1-cyclohexylmethyl-5,6-dimethyl-1H-benzimidazol-2-yl)butyric acid benzyl ester

In 3 ml of acetonitrile was dissolved 100 mg (0.220 mmol) of the compound obtained in Example 135-1, to which 0.029 ml (0.242 mmol) of cyclohexanecarbaldehyde was then added, followed by stirring at room temperature for 60 minutes. Thereto was added 69.9 mg (0.330 mmol) of sodium triacetoxyborohydride, followed by stirring at room temperature for 3 hours. After the end of reaction, chloroform was added thereto, followed by washing with a sodium hydroxide aqueous solution and a saturated saline solution before drying with anhydrous sodium sulfate. The solvent was distilled off, and the residue was dissolved in 1 ml of acetic acid before stirring at 55°C for 2.5 hours. After the end of reaction, the solvent was distilled off, and chloroform was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 51.8 mg of the title compound as a white solid.

### (Example 135-3) Synthesis of (R)-2-t-butoxycarbonylamino-4-(1-cyclohexylmethyl-5,6-dimethyl-1H-benzimidazol-2-yl)-N-trityloxybutyramide

In 10 ml of dioxane was dissolved 227 mg (0.425 mmol) of the compound obtained in Example 135-2, to which 46 mg of 10% palladium carbon was then added, followed by stirring at room temperature for 4 hours under an atmosphere of hydrogen. After the end of reaction, the catalyst was removed by celite filtration, and the filtrate was concentrated and vacuum dried. It was dissolved in 6 ml of chloroform, to which 122 mg (0.637 mmol) of WSCI hydrochloride, 57.4 mg (0.425 mmol) of HOBt, and 122 mg (0.443 mmol) of O-tritylhydroxylamine were then added, followed by stirring at room temperature for 20 hours. After the end of reaction, chloroform was added thereto, followed by washing with hydrochloric acid, a sodium hydroxide aqueous solution, and a saturated saline solution before drying with anhydrous sodium sulfate. The solvent was distilled off, followed by purifying the resultant residue using silica gel column chromatography (chloroform/ethyl acetate) to provide 257 mg of the title compound as a pale yellow foam.

### (Example 135-4) Synthesis of (R)-2-amino-4-(1-cyclohexylmethyl-5,6-dimethyl-1H-benzimidazol-2-yl)-N-hydroxybutyramide (compound No. 117)

To 0°C was cooled 147 mg (0.209 mmol) of the compound obtained in Example 135-3, to which 1.5 ml of 4 mol/1 hydrogen chloride/dioxane was then added, followed by stirring at room temperature for 4 hours. After the end of reaction, the solvent was distilled off, and the solid was dissolved in methanol. The methanol layer was washed with hexane, followed by distilling off the methanol. The resultant solid was further washed with chloroform/hexane and vacuum dried to provide 60.5 mg of the hydrochloride of the title compound as a white solid.
MS (Fab, Pos.): m/z = 359 [M+H]⁺
¹H-NMR (500 MHz, DMSO-d₆+D₂O) δ = 1.08-1.22 (5H, m), 1.53-1.60 (2H, m), 1.60-1.72 (3H, m), 1.83-1.92 (1H, m), 2.30-2.37 (2H, m), 2.39 (3H, s), 2.41 (3H, s), 3.20-3.35 (2H, m), 3.87 (1H, t, J = 6.6 Hz), 4.21 (2H, d, J = 7.6 Hz), 7.57 (1H, s), 7.77 (1H, s).

(Example 136) Synthesis of (S)-2-amino-4-[1-(3,4-difluorobenzyl)-5,6-dimethyl-1H-benzimidazol-2-yl]-N-hydroxybutyramide (compound No. 118)

### (Example 136-1) Synthesis of (S)-2-t-butoxycarbonylamino-4-(1-(3,4-difluorobenzyl)-5,6-dimethyl-1H-benzimidazol-2-yl)butyric acid benzyl ester

In 10 ml of dichloroethane was dissolved 500 mg (1.10 mmol) of the compound obtained in Example 110-1, to which 0.131 ml (1.21 mmol) of 3,4-difluorobenzaldehyde was then added, followed by stirring at room temperature for 60 minutes. Thereto was added 354 mg (1.65 mmol) of sodium triacetoxyborohydride, followed by stirring at room temperature for 24 hours. After the end of reaction, chloroform was added thereto, followed by washing with a sodium hydroxide aqueous solution and a saturated saline solution before drying with anhydrous sodium sulfate. The solvent was distilled off, and the residue was purified using silica gel column chromatography (hexane/ethyl acetate). This purified fraction was concentrated, and the residue was dissolved in 5 ml of acetic acid before stirring at 60°C for 5 hours. After the end of reaction, the solvent was distilled off, and chloroform was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 378 mg of the title compound as a white solid.

### (Example 136-2) Synthesis of(S)-2-t-butoxycarbonylamino-4-[1-(3,4-difluorobenzyl)-5,6-dimethyl- 1H-benzimidazol-2-yl]-N-trityloxybutyramide

In 12.8 ml of DME was dissolved 255 mg (0.453 mmol) of the compound obtained in Example 136-1, to which 76 mg of 10% palladium carbon was then added, followed by stirring at room temperature for one hour under an atmosphere of hydrogen. After the end of reaction, the catalyst was removed by celite filtration, and the filtrate was concentrated and vacuum dried. In 6 ml of chloroform was dissolved 184 mg (0.388 mmol) thereof, to which 112 mg (0.585 mmol) of WSCI hydrochloride, 52.4 mg (0.388 mmol) of HOBt, and 112 mg (0.407 mmol) of O-tritylhydroxylamine were then added, which was then adjusted to pH 6 using triethylamine, followed by stirring at room temperature for 20 hours. After the end of reaction, chloroform was added thereto, followed by washing with distilled water, a sodium hydroxide aqueous solution, and a saturated saline solution before drying with anhydrous sodium sulfate. The solvent was distilled off, followed by purifying the resultant residue using silica gel column chromatography (hexane/ethyl acetate) to provide 202 mg of the title compound as a colorless foam.

### (Example 136-3) Synthesis of (S)-2-amino-4-[1-(3,4-difluorobenzyl)-5,6-dimethyl-1H-benzimidazol-2-yl]-N-hydroxybutyramide (compound No. 118)

To 0°C was cooled 85.3 mg (0.117 mmol) of the compound obtained in Example 136-2, to which 1 ml of 4 mol/l hydrogen chloride/dioxane was then added, followed by stirring at room temperature for 1.5 hours. After the end of reaction, the solvent was distilled off, and the solid was dissolved in methanol. The methanol layer was washed with hexane, followed by distilling off the methanol. The resultant solid was further washed with chloroform/hexane and vacuum dried to provide 40.5 mg of the hydrochloride of the title compound as a white solid.
MS (Fab, Pos.): m/z = 389 [M+H]⁺
¹H-NMR (500 MHz, DMSO-d₆+D₂O) δ = 2.28 (1H, dd, J = 8.5, 15.4 Hz), 2.34 (3H, s), 2.38 (3H, s), 3.22-3.28 (2H, m), 3.85 (1H, t, J = 6.6 Hz), 5.64 (2H, s), 7.4 (1H, br), 7.39-7.51 (2H, m), 7.53 (1H, s) 7.60 (1H, s).

### (Example 137) Synthesis of (S)-2-amino-4-(1-phenyl-1H-benzimidazol-2-yl)-N-hydroxybutyramide (compound No. 119)

### (Example 137-1) Synthesis of (S)-4-(2-phenylamino-phenylcarbamoyl)-2-t-butoxycarbonylaminobutyric acid benzyl ester

In 10 ml of chloroform was dissolved 1.00 g (2.96 mmol) of commercial Boc-D-glutamic acid benzyl ester, to which 681 mg (3.56 mmol) of WSCI hydrochloride and 440 mg (3.26 mmol) of HOBt were then added, and the solution was allowed to stand at room temperature for 5 minutes. It was added dropwise into 10 ml of chloroform solution of 615 mg (3.55 mmol) of N-phenyl-1,2-phenylenediamine over a period of 2 hours, followed by stirring at room temperature for one day. After the end of reaction, chloroform was supplemented, followed by washing with hydrochloric acid, a sodium hydroxide aqueous solution, and a saturated saline solution. It was dried over anhydrous sodium sulfate before distilling off the solvent, followed by recrystallizing the residue from hexane/ethyl acetate to provide 1.49 g of the title compound as a light orange solid.

### (Example 137-2) Synthesis of (S)-2-t-butoxycarbonylamino-4-(1-phenyl-1H-benzimidazol-2-yl)butyric acid benzyl ester

In 28 ml of acetic acid was dissolved 1.42 g (2.82 mmol) of the compound obtained in Example 137-1, followed by stirring at 60°C for 15 hours. After the end of reaction, the solvent was distilled off, and chloroform was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 1.08 g of the title compound as a white solid.

### (Example 137-3) Synthesis of (S)-2-t-butoxycarbonylamino-4-(1-phenyl-1H-benzimidazol-2-yl)-N-trityloxybutyramide

In 50 ml of DME was dissolved 1.08 g (2.22 mmol) of the compound obtained in Example 137-2, to which 220 mg of 10% palladium carbon was then added, followed by stirring at room temperature for 4 hours under an atmosphere of hydrogen. After the end of reaction, the catalyst was removed by celite filtration, and the filtrate was concentrated and vacuum dried. In 8 ml of chloroform was dissolved 425.1 mg (1.07 mmol) thereof, to which 309 mg (1.61 mmol) of WSCI hydrochloride, 152 mg (1.12 mmol) of HOBt, and 324 mg (1.18 mmol) of O-tritylhydroxylamine were then added, followed by stirring at room temperature for 24 hours. After the end of reaction, chloroform was added thereto, followed by washing with hydrochloric acid, a sodium hydroxide aqueous solution, and a saturated saline solution before drying with anhydrous sodium sulfate. The solvent was distilled off, followed by purifying the resultant residue using silica gel column chromatography (hexane/ethyl acetate) to provide 536 mg of the title compound as a white foam.

### (Example 137-4) Synthesis of (S)-2-amino-4-(1-phenyl-1H-benzimidazol-2-yl)-N-hydroxybutyramide (compound No. 119)

To 0°C was cooled 507 mg (0.777 mmol) of the compound obtained in Example 137-3, to which 5 ml of 4 mol/l hydrogen chloride/dioxane was then added, followed by stirring at room temperature for 4 hours. After the end of reaction, the solvent was distilled off, and the solid was washed with chloroform/hexane and vacuum dried to provide 218 mg of the hydrochloride of the title compound as a white solid.
MS (Fab, Pos.): m/z = 311 [M+H]⁺
¹H-NMR (500 MHz, DMSO-d₆+D₂O) δ = 2.26-2.34 (2H, m), 2.99-3.09 (2H, m), 7.29((1H,d,J=8.1Hz),7.51(1H,dt,J=1.1,8.2Hz),7.58(1H,dt,J=1.1,8.2Hz), 7.59-7.67 (5H, m), 7.88 (1H, d, J = 8.2 Hz).

### (Example 138) Synthesis of (R)-2-amino-4-(1-phenyl-5,6-dimethyl-1H-benzimidazol-2-yl)-N-hydroxybutyramide (compound No. 120)

### (Example 138-1) Synthesis of (R)-2-t-butoxycarbonylamino-4-(5,6-dimethyl-1H-benzimidazol-2-yl)butyloc acid benzyl ester

In 8.5 ml of acetic acid was dissolved 834 mg (1.83 mmol) of the compound obtained in Example 135-1, followed by stirring at 60°C for 3 hours. After the end of reaction, the solvent was distilled off, and toluene/ethyl acetate was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 731 mg of the title compound as a white solid.

### (Example 138-2) Synthesis of (R)-2-t-butoxycarbonylamino-4-(1-phenyl-5,6-dimethyl-1H-benzimidazol-2-yl)butyric acid benzyl ester

In 10.8 ml of THF was dissolved 362 mg (0.826 mmol) of the compound obtained in Example 138-1, to which 201 mg (1.65 mmol) ofphenylboronic acid, 225 mg (1.24 mmol) of copper (II) acetate, 0.127 ml (1.65 mmol) of pyridine, and 500 mg of molecular sieves were then added, followed by stirring at room temperature for 48 hours under an atmosphere of oxygen. After the end of reaction, the catalyst was removed by celite filtration, followed by concentrating the solution before adding distilled water thereto. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 173 mg of the title compound as a yellow viscous substance.

### (Example 138-3) Synthesis of (R)-2-t-butoxycarbonylamino-4-(1-phenyl-5,6-dimethyl-1H-benzimidazol-2-yl)-N-trityloxybutyramide

In 8.5 ml of DME was dissolved 173 mg (0.318 mmol) of the compound obtained in Example 138-2, to which 51 mg of 10% palladium carbon was then added, followed by stirring at room temperature for 1.5 hours under an atmosphere of hydrogen. After the end of reaction, the catalyst was removed by celite filtration, and the filtrate was concentrated and vacuum dried. It was dissolved in 4 ml of chloroform, to which 91 mg (0.475 mmol) of WSCI hydrochloride, 43 mg (0.318 mmol) of HOBt, and 92 mg (0.334 mmol) of O-tritylhydroxylamine were then added before stirring at room temperature for 18 hours, followed by adding 46 mg of O-tritylhydroxylamine and 5 drops of triethylamine thereto before further stirring at room temperature for 2 days. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 178 mg of the title compound as a white foam.

### (Example 138-4) Synthesis of (R)-2-amino-4-(1-phenyl-5,6-dimethyl-1H-benzimidazol-2-yl)-N-hydroxybutyramide (compound No. 120)

To 0°C was cooled 178 mg (0.261 mmol) of the compound obtained in Example 138-3, to which 2 ml of 4 mol/l hydrogen chloride/dioxane was then added, followed by stirring at room temperature for 4 hours. After the end of reaction, the solvent was distilled off, and the solid was washed with chloroform/hexane, followed by further repeating the washing with hexane before vacuum drying to provide 95.9 mg of the hydrochloride of the title compound as a white solid.
MS (Fab, Pos.): m/z = 339 [M+H]⁺
¹H-NMR (500 MHz, DMSO-d₆+D₂O) δ = 2.26 (1H, dd, J = 8.2, 15.4 Hz), 2.29 (1H, dd, J = 8.2, 15.4 Hz), 2.32 (3H, s), 2.40 (3H, s), 2.97-3.02 (2H, m), 3.65-3.75 (1H, m), 7.07 (1H, s), 7.61-7.70 (3H, m), 7.71-7.80 (3H, m).

### (Example 139) Synthesis of (R)-2-amino-4-[1-(4-methylphenyl)-5,6-dimethyl-1H-benzimidazol-2-yl]-N-hydroxybutyramide (compound No. 121)

### (Example 139-1) Synthesis of (R)-2-t-butoxycarbonylamino-4-[1-(4-methylphenyl)-5,6-dimethyl-1H-benzimidazol-2-yl]butyric acid benzyl ester

In 12 ml of THF was dissolved 373 mg (0.852 mmol) of the compound obtained in Example 138-1, to which 231 mg (1.70 mmol) of 4-methylphenylboronic acid, 232 mg (1.28 mmol) of copper (II) acetate, 0.131 ml (1.70 mmol) of pyridine, and 500 mg of molecular sieves were then added, followed by stirring at room temperature for 48 hours under an atmosphere of oxygen. After the end of reaction, the catalyst was removed bv celite filtration, followed by concentrating the solution before adding distilled water thereto. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 246 mg of the title compound as a yellow viscous substance.

### (Example 139-2) Synthesis of (R)-2-t-butoxycarbonylamino-4-[1-(4-methylphenyl)-5,6-dimethyl-1H-benzimidazol-2-yl]-N-trityloxybutyramide

In 12 ml of DME was dissolved 246 mg (0.478 mmol) of the compound obtained in Example 139-1, to which 72 mg of 10% palladium carbon was then added, followed by stirring at room temperature for 1.5 hours under an atmosphere of hydrogen. After the end of reaction, the catalyst was removed by celite filtration, and the filtrate was concentrated and vacuum dried. It was dissolved in 5.5 ml of chloroform, to which 122 mg (0.637 mmol) of WSCI hydrochloride, 57 mg (0.422 mmol) of HOBt, and 121 mg (0.439 mmol) of O-tritylhydroxylamine were then added before stirring at room temperature for 18 hours, followed by adding 60 mg (0.218 mmol) of O-tritylhydroxylamine and 5 drops of triethylamine before further stirring at room temperature for 2 days. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 172 mg of the title compound as a white foam.

### (Example 139-3) Synthesis of(R)-2-amino-4-[1-(4-methylphenyl)-5,6-dimethyl-1H-benzimidazol-2-yl]-N-hydroxybutyramide (compound No. 121)

To 0°C was cooled 172 mg (0.248 mmol) of the compound obtained in Example 139-2, to which 2 ml of 4 mol/1 hydrogen chloride/dioxane was then added, followed by stirring at room temperature for 4 hours. After the end of reaction, the solvent was distilled off, and the solid was washed with chloroform/hexane, followed by further repeating the washing with hexane before vacuum drying to provide 96.7 mg of the hydrochloride of the title compound as a white solid.
MS (Fab, Pos.): m/z = 353 [M+H]⁺
¹H-NMR (500 MHz, DMSO-d₆+D₂O) δ = 2.25 (1H, dd, J = 8.2, 15.2 Hz), 2.28 (1H, dd, J = 8.2, 15.2 Hz), 2.32 (3H, s), 2.40 (3H, s), 2.47 (3H, s), 2.96-3.01 (2H, m), 3.65-3.75 (1H, m), 7.07 (1H, s), 7.50-7.56 (4H, m), 7.64 (1H, s).

### (Example 140) Synthesis of (S)-2-amino-4-(1-phenyl-5,6-dimethyl-1H-benzimidazol-2-yl)-N-hydroxybutyramide (compound No. 122)

### (Example 140-1) Synthesis of (S)-2-t-butoxycarbonylamino-4-(5,6-dimethyl-1H-benzimidazol-2-yl)butyric acid benzyl ester

In 32 ml of acetic acid was dissolved 3.17 g (6.96 mmol) of the compound obtained in Example 110-1, followed by stirring at 60°C for 2.5 hours. After the end of reaction, the solvent was distilled off, and toluene/ethyl acetate was added to the residue, followed by washing with a sodium hydroxide aqueous solution, distilled water, and a saturated saline solution before drying with anhydrous sodium sulfate. The solvent was distilled off, followed by drying the residue under reduced pressure to provide 3.05 g of the title compound as a light orange solid.

### (Example 140-2) Synthesis of (S)-2-t-butoxycarbonylamino-4-(1-phenyl-5,6-dimethyl-1H-benzimidazol-2-yl)butyric acid benzyl ester

In 20 ml of THF was dissolved 505 mg (1.15 mmol) of the compound obtained in Example 140-1, to which 278 mg (2.28 mmol) of phenylboronic acid, 311 mg(1.71 mmol) of copper (II) acetate, 0.175 ml (2.28 mmol) of pyridine, and 670 mg of molecular sieves were then added, followed by stirring at room temperature for 48 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 206 mg of the title compound as a pale yellow foam.

### (Example 140-3) Synthesis of (S)-2-t-butoxycarbonylamino-4-(1-phenyl-5,6-dimethyl-1H-benzimidazol-2-yl)-N-trityloxybutyramide

In 10 ml of DME was dissolved 204 mg (0.396 mmol) of the compound obtained in Example 140-2, to which 61 mg of 10% palladium carbon was then added, followed by stirring at room temperature for 3 hours under an atmosphere of hydrogen. After the end of reaction, the catalyst was removed by celite filtration, and the filtrate was concentrated and vacuum dried. It was dissolved in 6 ml of chloroform, to which 114 mg (0.595 mmol) of WSCI hydrochloride and 115 mg (0.417 mmol) of O-tritylhydroxylamine were then added, followed by stirring at room temperature for 23 hours. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 72.0 mg of the title compound as a white foam.

### (Example 140-4) Synthesis of (S)-2-amino-4-(1-phenyl-5,6-dimethyl-1H-benzimidazol-2-yl)-N-hydroxybutyramide (compound No. 122)

To 0°C was cooled 72.0 mg (0.106 mmol) of the compound obtained in Example 140-3, to which 0.72 ml of 4 mol/1 hydrogen chloride/dioxane was then added, followed by stirring at room temperature for 2 hours. After the end of reaction, the solvent was distilled off, and the solid was washed with chloroform/hexane. It was purified using silica gel column chromatography (chloroform/methanol) and treated with hydrochloric acid to provide 20.2 mg of the hydrochloride of the title compound as a white solid.
MS (Fab, Pos.): m/z = 339 [M+H]⁺
¹H-NMR (500 MHz, DMSO-d₆+D₂O) δ = 2.24-2.31 (2H, m), 2.32 (3H, s), 2.41 (3H, s), 3.00-3.07 (2H, m), 3.65-3.75 (1H, m), 7.09 (1H, s), 7.65-7.68 (3H, m), 7.72-7.77 (3H, m).

### (Example 141) Synthesis of (S)-2-amino-4-[1-(4-methylphenyl)-5,6-dimethyl-1H-benzimidazol-2-yl]-N-hydroxybutyramide (compound No. 123)

### (Example 141-1) Synthesis of (S)-2-t-butoxycarbonylamino-4-(1-(4-methylphenyl)-5,6-dimethyl-1H-benzimidazol-2-yl)butyric acid benzyl ester

In 20 ml of THF was dissolved 507 mg (1.16 mmol) of the compound obtained in Example 140-1, to which 310 mg (2.28 mmol) of 4-methylphenylboronic acid, 311 mg (1.71 mmol) of copper (II) acetate, 0.175 ml (2.28 mmol) of pyridine, and 670 mg of molecular sieves were then added, followed by stirring at room temperature for 48 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 246 mg of the title compound as a pale yellow foam.

### (Example 141-2) Synthesis of (S)-2-t-butoxycarbonylamino-4-[(1-(4-methylphenyl)-5,6-dimethyl-1H-benzimidazol-2-yl]-N-trityloxybutyramide

In 12 ml of DME was dissolved 243 mg (0.461 mmol) of the compound obtained in Example 141-1, to which 72 mg of 10% palladium carbon was then added, followed by stirring at room temperature for 3 hours under an atmosphere of hydrogen. After the end of reaction, the catalyst was removed by celite filtration, and the filtrate was concentrated and vacuum dried. It was dissolved in 6.3 ml of chloroform, to which 133 mg (0.695 mmol) of WSCI hydrochloride and 133 mg (0.483 mmol) of O-tritylhydroxylamine were then added, followed by stirring at room temperature for 20 hours. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 269 mg of the title compound as a white foam.

### (Example 141-3) Synthesis of (S)-2-amino-4-[1-(4-methylphenyl)-5,6-dimethyl-1H-benzimidazol-2-yl]-N-hydroxybutyramide (compound No. 123)

To 0°C was cooled 269 mg (0.386 mmol) of the compound obtained in Example 141-2, to which 2.7 ml of 4 mol/l hydrogen chloride/dioxane was then added, followed by stirring at room temperature for 5 hours. After the end of reaction, the solvent was distilled off, and the solid was washed with chloroform/hexane. It was purified using silica gel column chromatography (chloroform/methanol) and treated with hydrochloric acid to provide 110 mg of the hydrochloride of the title compound as a white solid.
MS (Fab, Pos.): m/z = 353 [M+H]⁺
¹H-NMR (500 MHz, DMSO-d₆+D₂O) δ = 2.24-2.30 (2H, m), 2.32 (3H, s), 2.40 (3H, s), 2.48 (3H, s), 3.00-3.04 (2H, m), 3.65-3.75 (1H, m), 7.09 (1H, s), 7.54-7.55 (4H, m), 7.66 (1H, s).

### (Example 142) Synthesis of (S)-2-amino-4-[1-(4-methoxyphenyl)-5,6-dimethyl-1H-benzimidazol-2-yl]-N-hydroxybutyramide (compound No. 124)

### (Example 142-1) Synthesis of (S)-2-t-butoxycarbonylamino-4-[(1-(4-methoxyphenyl)-5,6-dimethyl-1H-benzimidazol-2-yl]butyric acid benzyl ester

In 20 ml of THF was dissolved 508 mg (1.18 mmol) of the compound obtained in Example 140-1, to which 347 mg (2.28 mmol) of 4-methoxyphenylboronic acid, 311 mg (1.71 mmol) of copper (II) acetate, 0.175 ml (2.28 mmol) of pyridine, and 670 mg of molecular sieves were then added, followed by stirring at room temperature for 48 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 253 mg of the title compound as a pale yellow foam.

### (Example 142-2) Synthesis of (S)-2-t-butoxycarbonylamino-4-[(1-(4-methoxyphenyl)-5,6-dimethyl-1H-benzimidazol-2-yl]-N-trityloxybutyramide

In 12.5 ml of DME was dissolved 250 mg (0.460 mmol) of the compound obtained in Example 142-1, to which 75 mg of 10% palladium carbon was then added, followed by stirring at room temperature for 3 hours under an atmosphere of hydrogen. After the end of reaction, the catalyst was removed by celite filtration, and the filtrate was concentrated and vacuum dried. It was dissolved in 6.6 ml of chloroform, to which 132 mg (0.689 mmol) of WSCI hydrochloride and 133 mg (0.483 mmol) of O-tritylhydroxylamine were then added, followed by stirring at room temperature for 20 hours. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 256 mg of the title compound as a white foam.

### (Example 142-3) Synthesis of (S)-2-amino-4-[1-(4-methoxyphenyl)-5,6-dimethyl-1H-benzimidazol-2-yl]-N-hydroxybutyramide (compound No. 124)

To 0°C was cooled 256 mg (0.360 mmol) of the compound obtained in Example 142-2, to which 2.6 ml of 4 mol/l hydrogen chloride/dioxane was then added, followed by stirring at room temperature for 5 hours. After the end of reaction, the solvent was distilled off, and the solid was washed with chloroform/hexane. It was purified using silica gel column chromatography (chloroform/methanol) and treated with hydrochloric acid to provide 125 mg of the hydrochloride of the title compound as a white solid.
MS (Fab, Pos.): m/z = 369 [M+H]⁺
¹H-NMR (500 MHz, DMSO-d₆+D₂O) δ = 2.23-2.28 (2H, m), 2.32 (3H, s), 2.40 (3H, s), 2.98-3.02 (2H, m), 3.65-3.75 (1H, m), 3.89 (3H, s), 7.08 (1H, s), 7.25 (1H, d, J = 9.0 Hz), 7.59 (2H, d, J = 9.0 Hz), 7.65 (1H, s).

### (Example 143) Synthesis of (S)-2-amino-4-[1-(4-fluorophenyl)-5,6-dimethyl-1H-benzimidazol-2-yl]-N-hydroxybutyramide (compound No. 125)

### (Example 143-1) Synthesis of (S)-2-t-butoxycarbonylamino-4-(1-(4-fluorophenyl)-5,6-dimethyl-1H-benzimidazol-2-yl)butyric acid benzyl ester

In 20 ml of THF was dissolved 505 mg (1.15 mmol) of the compound obtained in Example 140-1, to which 319 mg (2.28 mmol) of 4-fluorophenylboronic acid, 311 mg (1.71 mmol) of copper (II) acetate, 0.175 ml (2.28 mmol) of pyridine, and 670 mg of molecular sieves were then added, followed by stirring at room temperature for 48 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 276 mg of the title compound as a pale yellow foam.

### (Example 143-2) Synthesis of (S)-2-t-butoxycarbonylamino-4-[1-(4-fluorophenyl)-5,6-dimethyl-1H-benzimidazol-2-yl]-N-trityloxybutyramide

In 13.5 ml of DME was dissolved 272 mg (0.513 mmol) of the compound obtained in Example 143-1, to which 81 mg of 10% palladium carbon was then added, followed by stirring at room temperature for 3 hours under an atmosphere of hydrogen. After the end of reaction, the catalyst was removed by celite filtration, and the filtrate was concentrated and vacuum dried. It was dissolved in 6.6 ml of chloroform, to which 148 mg (0.773 mmol) of WSCI hydrochloride and 141 mg (0.512 mmol) of O-tritylhydroxylamine were then added, followed by stirring at room temperature for 22 hours. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 176 mg of the title compound as a white foam.

### (Example 143-3) Synthesis of (S)-2-amino-4-1-(4-fluorophenyl)-5,6-dimethyl-1H-benzimidazol-2-yl)-N-hydroxybutyramide (compound No. 125)

To 0°C was cooled 176 mg (0.252 mmol) of the compound obtained in Example 143-2, to which 1.8 ml of 4 mol/l hydrogen chloride/dioxane was then added, followed by stirring at room temperature for 5 hours. After the end of reaction, the solvent was distilled off, and the solid was washed with chloroform/hexane. It was purified using silica gel column chromatography (chloroform/methanol) and treated with hydrochloric acid to provide 68.7 mg of the hydrochloride of the title compound as a white solid.
MS (Fab, Pos.): m/z = 357 [M+H]⁺
¹H-NMR (500 MHz, DMSO-d₆+D₂O) δ = 2.22-2.29 (1H, m), 2.33 (3H, s), 2.41 (3H, s), 2.99-3.06 (2H, m), 3.65-3.75 (1H, m), 7.13 (1H, s), 7.58 (2H, t, J = 8.8 Hz), 7.67 (1H, s), 7.75 (2H, dd, J = 4.9, 8.8 Hz).

### (Example 144) Synthesis of (R)-2-amino-4-[1-(4-methylphenyl)-1H-benzimidazol-2-yl]-N-hydroxybutyramide (compound No. 126)

### (Example 144-1) Synthesis of (R)-2-t-butoxycarbonylamino-4-(1H-benzimidazol-2-yl)-butyric acid benzyl ester

In 5 ml of acetic acid was dissolved 501 mg (1.17 mmol) of the compound obtained in Example 134-1, followed by stirring at 60°C for 4 hours. After the end of reaction, the solvent was distilled off, and toluene/ethyl acetate was added to the residue, followed by washing with distilled water and a saturated saline solution before drying with anhydrous sodium sulfate. The solvent was distilled off, followed by purifying the residue using silica gel column chromatography (chloroform/ethyl acetate) to provide 357 mg of the title compound as a white foam.

### (Example 144-2) Synthesis of (R)-2-t-butoxycarbonylamino-4-[(1-(4-methylphenyl)-1H-benzimidazol-2-yl]butyric acid benzyl ester

In 10 ml of THF was dissolved 308 mg (0.852 mmol) of the compound obtained in Example 144-1, to which 204 mg (1.70 mmol) of 4-methylphenylboronic acid, 206 mg (1.28 mmol) of copper (II) acetate, 0.116 ml (1.70 mmol) of pyridine, and 450 mg of molecular sieves were then added, followed by stirring at room temperature for 30 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 222 mg of the title compound as a pale yellow foam.

### (Example 144-3) Synthesis of (R)-2-t-butoxycarbonylamino-4-[1-(4-methylphenyl)-1H-benzimidazol-2-yl]-N-trityloxybutyramide

In 12 ml of DME was dissolved 222 mg (0.443 mmol) of the compound obtained in Example 144-2, to which 66 mg of 10% palladium carbon was then added, followed by stirring at room temperature for 2.5 hours under an atmosphere of hydrogen. After the end of reaction, the catalyst was removed by celite filtration, and the filtrate was concentrated and vacuum dried. It was dissolved in 5 ml of chloroform, to which 72.9 mg (0.381 mmol) of WSCI hydrochloride, 51.5 mg (0.381 mmol) of HOBt, and 110 mg (0.399 mmol) of O-tritylhydroxylamine were then added, which was then adjusted to pH 6 to 7 by adding triethylamine, followed by stirring at room temperature for 3 days. After the end of reaction, chloroform was added thereto, followed by washing with distilled water before drying with anhydrous sodium sulfate. The solvent was distilled off, followed by purifying the resultant residue using silica gel column chromatography (hexane/ethyl acetate) to provide 158 mg of the title compound as a white foam.

### (Example 144-4) Synthesis of (R)-2-amino-4-[1-(4-methylphenyl)-1H-benzimidazol-2-yl]-N-hydroxybutyramide (compound No. 126)

To 0°C was cooled 153 mg (0.229 mmol) of the compound obtained in Example 144-3, to which 2 ml of 4 mol/l hydrogen chloride/dioxane was then added, followed by stirring at room temperature for 3 hours. After the end of reaction, the solvent was distilled off, and the solid was washed with chloroform/hexane. It was purified using silica gel column chromatography (chloroform/methanol) and treated with hydrochloric acid to provide 70.1 mg of the hydrochloride of the title compound as a white solid.
MS (Fab, Pos.): m/z = 325 [M+H]⁺
¹H-NMR (500 MHz, DMSO-d₆+D₂O) δ = 2.26-2.31 (1H, m), 2.32 (3H, s), 2.45 (3H, s), 2.99-3.04 (2H, m), 3.65-3.75 (1H, m), 7.29 (1H, d, J = 8.1 Hz), 7.48-7.59 (5H, m), 7.87 (1H, d, J=8.1 Hz).

### (Example 145) Synthesis of (S)-2-amino-4-[1-(3-fluorobenzyl)-5,6-dimethyl-1H-benzimidazol-2-yl]-N-hydroxybutyramide (compound No. 127)

### (Example 145-1) Synthesis of (S)-2-t-butoxycarbonylamino-4-[1-(3-fluorobenzyl)-5,6-dimethyl-1H-benzimidazol-2-yl]butyric acid benzyl ester

In 6 ml of DMF was dissolved 298 mg (0.682 mmol) of the compound obtained in Example 140-1, to which 0.094 ml (0.755 mmol) of 3-fluorobenzyl bromide and 223 mg (0.686 mmol) of cesium carbonate were then added, followed by stirring at room temperature for 2 hours. After the end of reaction, the solvent was distilled off, and ethyl acetate and toluene were added to the residue, followed by washing with distilled water before drying with anhydrous sodium sulfate. The solvent was distilled off, followed by purifying the residue using silica gel column chromatography (chloroform/ethyl acetate) to provide 96 mg of the title compound as a white foam.

### (Example 145-2) Synthesis of (S)-2-t-butoxycarbonylamino-4-[1-(3-fluorobenzyl)-5,6-dimethyl-1H-benzimidazol-2-yl]-N-trityloxybutyramide

In 5 ml of DME was dissolved 96 mg (0.175 mmol) of the compound obtained in Example 145-1, to which 20 mg of 10% palladium carbon was then added, followed by stirring at room temperature for 3 hours under an atmosphere of hydrogen. After the end of reaction, the catalyst was removed by celite filtration, and the filtrate was concentrated and vacuum dried. It was dissolved in 2.2 ml of chloroform, to which 48.3 mg (0.252 mmol) of WSCI hydrochloride, 22.7 mg (0.168 mmol) of HOBt, and 48.6 mg (0.177 mmol) of O-tritylhydroxylamine were then added, followed by stirring at room temperature for 16 hours. After the end of reaction, chloroform was added thereto, followed by washing with distilled water, a potassium carbonate aqueous solution, and a saturated saline solution before drying with anhydrous sodium sulfate. The solvent was distilled off, followed by purifying the resultant residue using silica gel column chromatography (hexane/ethyl acetate) to provide 72.1 mg of the title compound as a colorless foam.

### (Example 145-3) Synthesis of (S)-2-amino-4-(1-(3-fluorobenzyl)-5,6-dimethyl-1H-benzimidazol-2-yl)-N-hydroxybutyramide (compound No. 127)

To 0°C was cooled 72.1 mg (0.101 mmol) of the compound obtained in Example 145-2, to which 1.4 ml of 4 mol/l hydrogen chloride/dioxane was then added, followed by stirring at room temperature for 4 hours. After the end of reaction, the solvent was distilled off, and the solid was washed with hexane. It was purified using silica gel column chromatography (chloroform/methanol) to provide 31.9 mg of the hydrochloride of the title compound as a white solid.
MS (Fab, Pos.): m/z = 371 [M+H]⁺
¹H-NMR (500 MHz, DMSO-d₆+D₂O) δ = 2.23-2.40 (2H, m), 2.31 (3H, s), 2.35 (3H, s), 3.14-3.23 (2H, m), 3.84 (1H, t, J = 6.6 Hz), 5.61 (2H, s), 7.03 (1H, d, J = 7.5 Hz), 7.08 (1H, d, J = 9.5 Hz) 7.18 (1H, dt, J = 2.2, 8.5 Hz), 7.39-7.45 (2H, m), 7.54 (1H, s).

### (Example 146) Synthesis of (S)-2-amino-4-[1-(3-nitrophenyl)-5,6-dimethyl-1H-benzimidazol-2-yl]-N-hydroxybutyramide (compound No. 128)

### (Example 146-1) Synthesis of (S)-2-t-butoxycarbonylamino-4-[1-(3-nitrophenyl)-5,6-dimethyl-1H-benzimidazol-2-yl]butyric acid benzyl ester

In 9 ml of THF was dissolved 301 mg (0.688 mmol) of the compound obtained in Example 140-1, to which 229 mg (1.37 mmol) of 3-nitrophenylboronic acid, 187 mg (1.03 mmol) of copper (II) acetate, 0.106 ml (1.37 mmol) of pyridine, and 450 mg of molecular sieves were then added, followed by stirring at room temperature for 23 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 75.3 mg of the title compound as a yellow, viscous substance.

### (Example 146-2) Synthesis of (S)-2-t-butoxycarbonylamino-4-[1-(3-nitrophenyl)-5,6-dimethyl-1H-benzimidazol-2-yl]-N-trityloxybutyramide

In 10 ml of THF and 10 ml of methanol was dissolved 75.3 mg (0.135 mmol) of the compound obtained in Example 146-1, to which 10 ml of a 2% potassium carbonate aqueous solution was then added, followed by stirring at room temperature for 5 hours. After the end of reaction, the organic solvent content was distilled off, followed by adding a 1 mol/l hydrochloric acid aqueous solution to the residue to adjust the pH to 6. The precipitated solid was collected by filtration, washed with distilled water, and vacuum dried. It was dissolved in 1.5 ml of chloroform, to which 28.0 mg (0.146 mmol) of WSCI hydrochloride and 28.3 mg (0.103 mmol) of O-tritylhydroxylamine were then added, followed by stirring at room temperature for 22 hours. After the end of reaction, chloroform was added thereto, followed by washing with distilled water, a potassium carbonate aqueous solution, and a saturated saline solution before drying with anhydrous sodium sulfate. The solvent was distilled off, and the residue was purified using silica gel column chromatography (hexane/ethyl acetate) to provide 34.7 mg of the title compound as a colorless foam.

### (Example 146-3) Synthesis of (S)-2-amino-4-[1-(3-fluorobenzyl)-5,6-dimethyl-1H-benzimidazol-2-yl]-N-hydroxybutyramide (compound No. 128)

To 0°C was cooled 34.7 mg (0.0478 mmol) of the compound obtained in Example 146-2, to which 0.7 ml of 4 mol/l hydrogen chloride/dioxane was then added, followed by stirring at room temperature for 4 hours. After the end of reaction, the solvent was distilled off, and the solid was washed with hexane. It was purified using silica gel column chromatography (chloroform/methanol) to provide 17.3 mg of the hydrochloride of the title compound as a light orange solid.
MS (Fab, Pos.): m/z = 384 [M+H]⁺
¹H-NMR (500 MHz, DMSO-d₆+D₂O) δ = 2.19-2.30 (2H, m), 2.31 (3H, s), 2.38 (3H, s), 2.89-3.01 (2H, m), 3.69-3.77 (1H, m), 7.14 (1H, s), 7.61 (1H, s), 8.01 (1H, t, J = 7.8 Hz), 8.08 (1H, d, J = 7.8 Hz), 8.52-8.55 (2H, m).

### (Example 147) Synthesis of (S)-2-amino-4-[1-(4-trifluoromethylbenzyl)-5,6-dimethyl-1H-benzimidazol-2-yl]-N-hydroxybutyramide (compound No. 129)

### (Example 147-1) Synthesis of (S)-2-t-butoxycarbonylamino-4-[1-(4-trifluoromethylbenzyl)-5,6-dimethyl-1H-benzimidazol-2-yl]butyric acid benzyl ester

In 10 ml of DMF was dissolved 448 mg (1.03 mmol) of the compound obtained in Example 140-1, to which 0.269 ml (1.12 mmol) of 4-trifluoromethylbenzyl bromide, 0.165 ml (1.02 mmol) of diisopropylethylamine, and 163 mg (0.57 mmol) of cesium carbonate were then added, followed by stirring at room temperature for 2 hours. After the end of reaction, the solvent was distilled off, and chloroform was added to the residue, followed by washing with distilled water before drying with anhydrous sodium sulfate. The solvent was distilled off, followed by purifying the residue using silica gel column chromatography (chloroform/ethyl acetate) to provide 213 mg of the title compound as a white foam.

### (Example 147-2) Synthesis of (S)-2-t-butoxycarbonylamino-4-[1-(4-trifluoromethylbenzyl)-5,6-dimethyl-1H-benzimidazol-2-yl]-N-trityloxybutyramide

In 7.5 ml of DME was dissolved 158 mg (0.265 mmol) of the compound obtained in Example 147-1, to which 30 mg of 10% palladium carbon was then added, followed by stirring at room temperature for 1.5 hours under an atmosphere of hydrogen. After the end of reaction, the catalyst was removed by celite filtration, and the filtrate was concentrated and vacuum dried. It was dissolved in 3.6 ml of chloroform, to which 72.1 mg (0.377 mmol) of WSCI hydrochloride, 33.9 mg (0.251 mmol) of HOBt, and 72.6 mg (0.263 mmol) of O-tritylhydroxylamine were then added, followed by stirring at room temperature for 20 hours. After the end of reaction, chloroform was added thereto, followed by washing with distilled water, a potassium carbonate aqueous solution, and a saturated saline solution before drying with anhydrous sodium sulfate. The solvent was distilled off, followed by purifying the resultant residue using silica gel column chromatography (hexane/ethyl acetate) to provide 166 mg of the title compound as a colorless foam.

### (Example 147-3) Synthesis of (S)-2-amino-4-[1-(4-trifluoromethylbenzyl)-5,6-dimethyl-1H-benzimidazol-2-yl]-N-hydroxybutyramide (compound No. 129)

To 0°C was cooled 166 mg (0.218 mmol) of the compound obtained in Example 147-2, to which 3.3 ml of 4 mol/l hydrogen chloride/dioxane was then added, followed by stirring at room temperature for 4 hours. After the end of reaction, the solvent was distilled off, and the solid was washed with hexane. It was purified using silica gel column chromatography (chloroform/methanol) to provide 78.1 mg of the hydrochloride of the title compound as a white solid.
MS (Fab, Pos.): m/z = 421 [M+H]⁺
¹H-NMR (500 MHz, DMSO-d₆+D₂O) δ = 2.25-2.34 (2H, m), 2.33 (3H, s), 2.37 (3H, s), 3.20-3.28 (2H, m), 3.84 (1H, t, J = 6.6 Hz), 5.77 (2H, s), 7.45 (2H, d, J = 8,2 Hz), 7.51 (1H, s), 7.60 (1H, s), 7.75 (2H, d, J = 8.2 Hz).

### (Example 148) Synthesis of (S)-2-amino-4-(1-phenyl-5,6-dichloro-1H-benzimidazol-2-yl)-N-hydroxybutyramide (compound No. 130)

### (Example 148-1) Synthesis of (S)-2-t-butoxycarbonylamino-4-(5,6-dichloro-1H-benzimidazol-2-yl)butyric acid benzyl ester

In 28 ml of acetic acid was dissolved 2.84 g (5.72 mmol) of the compound obtained in Example 112-1, followed by stirring at 60°C for 15 hours. After the end of reaction, the solvent was distilled off, followed by azeotroping thrice with toluene before drying under reduced pressure to provide 2.99 g of the acetate of the title compound as a light brown foam.

### (Example 148-2) Synthesis of (S)-2-t-butoxycarbonylamino-4-(1-phenyl-5,6-dichloro-1H-benzimidazol-2-yl)butyric acid benzyl ester

In 15 ml of THF was dissolved 504 mg (0.965 mmol) of the compound obtained in Example 148-1, to which 233 mg (2.08 mmol) of phenylboronic acid, 260 mg (1.56 mmol) of copper (II) acetate, 0.156 ml (2.08 mmol) of pyridine, and 670 mg of molecular sieves were then added, followed by stirring at room temperature for 72 hours under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 162 mg of the title compound as a pale yellow foam.

### (Example 148-3) Synthesis of (S)-2-t-butoxycarbonylamino-4-(1-phenyl-5,6-dichloro-1H-benzimidazol-2-yl)-N-trityloxybutyramide

In 8 ml of THF and 16 ml of methanol was dissolved 162 mg (0.288 mmol) of the compound obtained in Example 148-2, to which 8 ml of a 2.5% potassium carbonate aqueous solution was then added, followed by stirring at room temperature for 2 hours. After the end of reaction, the organic solvent content was distilled off, followed by adding a 1 mol/l hydrochloric acid aqueous solution to the residue to adjust the pH to 6. The solution was extracted with chloroform, followed by drying the extract with anhydrous sodium sulfate. The solvent was distilled off, and the residue was washed with hexane and vacuum dried. It was dissolved in 3.6 ml of chloroform, to which 77.0 mg (0.402 mmol) of WSCI hydrochloride and 77.5 mg (0.281 mmol) of O-tritylhydroxylamine were then added, followed by stirring at room temperature for 19 hours. After the end of reaction, chloroform was added thereto, followed by washing with distilled water, a potassium carbonate aqueous solution, and a saturated saline solution before drying with anhydrous sodium sulfate. The solvent was distilled off, and the residue was purified using silica gel column chromatography (hexane/ethyl acetate) to provide 120 mg of the title compound as a pale yellow foam.

### (Example 148-4) Synthesis of (S)-2-amino-4-(1-phenyl-5,6-dichloro-1H-benzimidazol-2-yl)-N-hydroxybutyramide (compound No. 130)

To 0°C was cooled 103 mg (0.143 mmol) of the compound obtained in Example 148-3, to which 2 ml of 4 mol/l hydrogen chloride/dioxane was then added, followed by stirring at room temperature for 4 hours. After the end of reaction, the solvent was distilled off, and the solid was washed with hexane. It was purified using silica gel column chromatography (chloroform/methanol) to provide 48.4 mg of the hydrochloride of the title compound as a light orange solid.
MS (Fab, Pos.): m/z = 379, 381, 383 [M+H]⁺
¹H-NMR (500 MHz, DMSO-d₆+D₂O) δ = 2.21-2.34 (2H, m), 2.75-2.90 (2H, m), 3.68-3.75 (1H, m), 5.61 (2H, s), 7.34 (1H, s), 7.75 (2H, dd, J = 1.7, 7.1 Hz), 7.62-7.74 (3H, m), 8.00 (1H, s).

### (Example 149) Synthesis of (S)-2-amino-4-(1-benzyl-5,6-dichloro-1H-benzimidazol-2-yl)-N-hydroxybutyramide (compound No. 131)

### (Example 149-1) Synthesis of (S)-2-t-butoxycarbonylamino-4-(1-benzyl-5,6-dichloro-1H-benzimidazol-2-yl)butyric acid benzyl ester

In 10 ml of DMF was dissolved 500 mg (0.972 mmol) of the compound obtained in Example 148-1, to which 0.125 ml (1.05 mmol) of benzyl bromide and 311 mg (0.954 mmol) of cesium carbonate were then added, followed by stirring at room temperature for 2 hours. After the end of reaction, the solvent was distilled off, and ethyl acetate was added to the residue, followed by washing with distilled water before drying with anhydrous sodium sulfate. The solvent was distilled off, followed by purifying the residue using silica gel column chromatography (chloroform/ethyl acetate) to provide 396 mg of the title compound as a white solid.

### (Example 149-2) Synthesis of (S)-2-t-butoxycarbonylamino-4-(1-benzyl-5,6-dichloro-1H-benzimidazol-2-yl)-N-trityloxybutyramide

In 64 ml of THF and 24 ml of methanol was dissolved 396 mg (0.696 mmol) of the compound obtained in Example 149-1, to which 36 ml of a 2.5% potassium carbonate aqueous solution was then added, followed by stirring at room temperature for 6 hours. After the end of reaction, the organic solvent content was distilled off, followed by adding a 1 mol/l hydrochloric acid aqueous solution to the residue to adjust the pH to 7. The solution was extracted with chloroform, followed by drying with anhydrous sodium sulfate. The solvent was distilled off, and the residue was washed with hexane and vacuum dried. In 6 ml of chloroform was dissolved 208 mg (0.435 mmol) thereof, to which 125 mg (0.652 mmol) of WSCI hydrochloride and 126 mg (0.458 mmol) of O-tritylhydroxylamine were then added, followed by stirring at room temperature for 20 hours. After the end of reaction, chloroform was added thereto, followed by washing with distilled water, a potassium carbonate aqueous solution, and a saturated saline solution before drying with anhydrous sodium sulfate. The solvent was distilled off, and the residue was purified using silica gel column chromatography (hexane/ethyl acetate) to provide 237 mg of the title compound as a pale yellow foam.

### (Example 149-3) Synthesis of (S)-2-amino-4-(1-(3-fluorobenzyl)-5,6-dimethyl-1H-benzimidazol-2-yl)-N-hydroxybutyramide (compound No. 131)

To 0°C was cooled 204 mg (0.283 mmol) of the compound obtained in Example 149-2, to which 4 ml of 4 mol/l hydrogen chloride/dioxane was then added, followed by stirring at room temperature for 4 hours. After the end of reaction, the solvent was distilled off, and the solid was washed with hexane. It was purified using silica gel column chromatography (chloroform/methanol) to provide 104 mg of the hydrochloride of the title compound as a white solid.
MS (Fab, Pos.): m/z = 393, 395, 397 [M+H]⁺
¹H-NMR (500 MHz, DMSO-d₆+D₂O) δ = 2.23-2.38 (2H, m), 3.03-3.08 (2H, m), 3.83 (1H, t, J = 6.6 Hz), 5.57 (2H, s), 7.18 (2H, d, J = 8.6 Hz), 7.31-7.40 (3H, m), 7.94 (1H, s), 7.97 (1H, s).

### (Example 150) Synthesis of (S)-2-amino-3-(1-pentyl-1H-benzimidazol-2-yl)-N-hydroxypropionamide (compound No. 132)

### (Example 150-1) Synthesis of (S)-3-(2-amino-phenylcarbamoyl)-2-t-butoxycarbonylaminopropionic acid benzyl ester

In 35 ml of chloroform was dissolved 5.00 g (15.5 mmol) of commercial Boc-aspartic acid benzyl ester, to which 4.46 g (23.2 mmol) of WSCI hydrochloride and 2.30 g (17.0 mmol) of HOBt were then added, and the solution was allowed to stand at room temperature for 5 minutes. It was added dropwise into 100 ml of chloroform solution of 2.51 g (23.3 mmol) of 1,2-phenylenediamine over a period of 2 hours, followed by stirring at room temperature for 16 hours. After the end of reaction, chloroform was supplemented, followed by washing with hydrochloric acid, a potassium bicarbonate aqueous solution, and a saturated saline solution. It was dried over anhydrous sodium sulfate before distilling off the solvent, followed by recrystallizing the residue from hexane/ethyl acetate to provide 3.38 g of the title compound as a white solid.

### (Example 150-2) Synthesis of (S)-3-(2-pentylamino-phenylcarbamoyl)-2-t-butoxycarbonylaminopropionic acid benzyl ester

In 15 ml of acetonitrile was dissolved 501 mg (1.27 mmol) of the compound obtained in Example 150-1, to which 0.140 ml (1.40 mmol) of valeraldehyde was then added, followed by stirring at room temperature for 120 minutes. Thereto was added 340 mg (1.91 mmol) of sodium triacetoxyborohydride, followed by stirring at room temperature for 21 hours. After the end of reaction, chloroform was added thereto, followed by washing with a sodium hydroxide aqueous solution and a saturated saline solution before drying with anhydrous sodium sulfate. The solvent was distilled off, and the residue was purified using silica gel column chromatography (hexane/ethyl acetate) to provide 213 mg of the title compound as a white solid.

### (Example 150-3) Synthesis of (S)-2-t-butoxycarbonylamino-3-(1-pentyl-1H-benzimidazol-2-yl)propionic acid benzyl ester

In 2.2 ml of acetic acid was dissolved 206 mg (0.426 mmol) of the compound obtained in Example 150-2, followed by stirring at 60°C for 75 minutes. After the end of reaction, the solvent was distilled off, and chloroform was added to the residue, followed by washing with a sodium hydroxide aqueous solution, distilled water, and a saturated saline solution before drying with anhydrous sodium sulfate. The solvent was distilled off, followed by purifying the residue using silica gel column chromatography (hexane/ethyl acetate) to provide 164 mg of the title compound as a white foam.

### (Example 150-4) Synthesis of (S)-2-t-butoxycarbonylamino-3-(1-pentyl-1H-benzimidazol-2-yl)-N-trityloxypropionamide

In 8 ml of DME was dissolved 164 mg (0.352 mmol) of the compound obtained in Example 150-3, to which 48 mg of 10% palladium carbon was then added, followed by stirring at room temperature for 30 minutes under an atmosphere of hydrogen. After the end of reaction, the catalyst was removed by celite filtration, and the filtrate was concentrated and vacuum dried. It was dissolved in 6 ml of chloroform, to which 88.7 mg (0.463 mmol) of WSCI hydrochloride and 89.3 mg (0.324 mmol) of O-tritylhydroxylamine were then added, followed by stirring at room temperature for 20 hours. After the end of reaction, chloroform was added thereto, followed by washing with hydrochloric acid, a potassium carbonate aqueous solution, and a saturated saline solution before drying with anhydrous sodium sulfate. The solvent was distilled off, followed by purifying the resultant residue using silica gel column chromatography (hexane/ethyl acetate) to provide 144 mg of the title compound as a white foam.

### (Example 150-5) Synthesis of (S)-2-amino-3-(1-pentyl-1H-benzimidazol-2-yl)-N-hydroxypropionamide (compound No. 132)

To 0°C was cooled 144 mg (0.228 mmol) of the compound obtained in Example 150-4, to which 2.8 ml of 4 mol/l hydrogen chloride/dioxane was then added, followed by stirring at room temperature for 2 hours. After the end of reaction, the solvent was distilled off, the solid was washed with hexane, and methanol was distilled off. The resultant solid was further washed with chloroform/hexane, and purified using silica gel column chromatography (chloroform/methanol) to provide 75.3 mg of the hydrochloride of the title compound as a pale yellow solid.
MS (Fab, Pos.): m/z = 291 [M+H]⁺
¹H-NMR (500 MHz, DMSO-d₆+D₂O) δ = 0.87 (3H, t, J = 7.3 Hz), 1.29-1.39 (4H, m), 1.72-1.81 (2H, m), 3.61 (1H, dd, J = 8.3, 15.7 Hz), 3.64 (1H, dd, J = 7.1, 15.7 Hz), 4.27 (1H, t, J = 7.3 Hz), 4.19-4.22 (2H, m), 7.48-7.5 (2H, m), 7.78 (1H, d, J = 7.1 Hz), 7.87 (1H, d, J=7.1 Hz).

### (Example 151) Synthesis of (S)-2-amino-3-[1-(4-methylphenyl)-1H-benzimidazol-2-yl]-N-hydroxypropionamide (compound No. 133)

### (Example 151-1) Synthesis of (S)-2-t-butoxycarbonylamino-3-(1H-benzimidazol-2-yl)propionic acid benzyl ester

In 10 ml of acetic acid was dissolved 511 mg (1.24 mmol) of the compound obtained in Example 150-1, followed by stirring at 60°C for 120 minutes. After the end of reaction, the solvent was distilled off, and chloroform was added to the residue, followed by washing with a potassium carbonate aqueous solution, distilled water, and a saturated saline solution before drying with anhydrous sodium sulfate. The solvent was distilled off, followed by purifying the residue using silica gel column chromatography (hexane/ethyl acetate) to provide 234 mg of the title compound as a white foam.

### (Example 151-2) Synthesis of (S)-2-t-butoxycarbonylamino-3-[1-(4-methylphenyl)-1H-benzimidazol-2-yl]propionic acid benzyl ester

In 7 ml of THF was dissolved 224 mg (0.566 mmol) of the compound obtained in Example 151-1, to which 154 mg (1.13 mmol) of 4-methylphenylboronic acid, 206 mg (0.849 mmol) of copper (II) acetate, 0.095 ml (1.13 mmol) of pyridine, and 350 mg of molecular sieves were then added, followed by stirring at room temperature for 2 days under an atmosphere of oxygen. After the end of reaction, the catalyst was removed by celite filtration, followed by concentrating the solution before adding distilled water. It was extracted with ethyl acetate, and the extract was washed with a saturated saline solution. After drying the resultant extract with anhydrous sodium sulfate, the solvent was distilled off, followed by purifying the residue using silica gel column chromatography (hexane/ethyl acetate) to provide 87.8 mg of the title compound as a pale yellow foam.

### (Example 151-3) Synthesis of (S)-2-t-butoxycarbonylamino-3-[1-(4-methylphenyl)-1H-benzimidazol-2-yl]-N-trityloxypropionamide

In 4.5 ml of DME was dissolved 87.7 mg of the compound obtained in Example 151-2, to which 27 mg of 10% palladium carbon was then added, followed by stirring at room temperature for 30 minutes under an atmosphere of hydrogen. After the end of reaction, the catalyst was removed by celite filtration, and the filtrate was concentrated and vacuum dried. It was dissolved in 2.5 ml of chloroform, to which 51.1 mg of WSCI hydrochloride and 51.5 mg of O-tritylhydroxylamine were then added, followed by stirring at room temperature for 15 hours. After the end of reaction, chloroform was added thereto, followed by washing with hydrochloric acid, a potassium carbonate aqueous solution, and a saturated saline solution before drying with anhydrous sodium sulfate. The solvent was distilled off, followed by purifying the resultant residue using silica gel column chromatography (hexane/ethyl acetate) to provide 76.8 mg of the title compound as a white foam.

### (Example 151-4) Synthesis of (S)-2-amino-3-(1-(4-methylphenyl)-1H-benzimidazol-2-yl)-N-hydroxypropionamide (compound No. 133)

To 0°C was cooled 76.8 mg (0.118 mmol) of the compound obtained in Example 151-3, to which 1.5 ml of 4 mol/l hydrogen chloride/dioxane was then added, followed by stirring at room temperature for 2 hours. After the end of reaction, the solvent was distilled off, the solid was washed with hexane, and methanol was distilled off. The resultant solid was further washed with chloroform/hexane, and purified using silica gel column chromatography (chloroform/methanol) to provide 28.2 mg of the hydrochloride of the title compound as a white solid.
MS (Fab, Pos.): m/z = 311 [M+H]⁺
¹H-NMR (500 MHz, DMSO-d₆+D₂O) δ = 2.46 (3H, s), 3.26 (1H, dd, J = 7.3, 13.2 Hz), 3.29 (1H, dd, J = 6.8, 13.2 Hz), 4.29 (1H, t, J = 6.8 Hz), 7.19 (1H, d, J = 8.1 Hz), 7.34-7.48 (2H, m), 7.40 (2H, d, J = 8.3 Hz), 7.50 (2H, d, J = 8.3 Hz), 7.78 (1H, d, J = 7.3 Hz).

### (Example 152) Synthesis of (S)-2-amino-3-[1-(4-t-butylbenzyl)-1H-benzimidazol-2-yl]-N-hydroxypropionamide (compound No. 134)

### (Example 152-1) Synthesis of (S)-2-t-butoxycarbonylamino-3-[1-(4-t-butylbenzyl)-1H-benzimidazol-2-yl]propionic acid benzyl ester

In 3 ml of DMF was dissolved 156 mg (0.394 mmol) of the compound obtained in Example 151-1, to which 0.109 ml (0.591 mmol) of 4-t-butylbenzyl bromide and 128 mg (0.393 mmol) of cesium carbonate were then added, followed by stirring at room temperature for 80 minutes. After the end of reaction, the solvent was distilled off, and ethyl acetate was added to the residue, followed by washing with distilled water before drying with anhydrous sodium sulfate. The solvent was distilled off, followed by purifying the residue using silica gel column chromatography (hexane/ethyl acetate) to provide 192 mg of the title compound as a white foam.

### (Example 152-2) Synthesis of (S)-2-t-butoxycarbonylamino-3-[1-(4-t-butylbenzyl)-1H-benzimidazol-2-yl]-N-trityloxypropionamide

In 9.8 ml of DME was dissolved 192 mg (0.354 mmol) of the compound obtained in Example 152-1, to which 57 mg of 10% palladium carbon was then added, followed by stirring at room temperature for 30 minutes under an atmosphere of hydrogen. After the end of reaction, the catalyst was removed by celite filtration, and the filtrate was concentrated and vacuum dried. It was dissolved in 4.8 ml of chloroform, to which 101 mg (0.527 mmol) of WSCI hydrochloride and 102 mg (0.370 mmol) of O-tritylhydroxylamine were then added, followed by stirring at room temperature for 16 hours. After the end of reaction, chloroform was added thereto, followed by washing with hydrochloric acid, a potassium carbonate aqueous solution, and a saturated saline solution before drying with anhydrous sodium sulfate. The solvent was distilled off, followed by purifying the resultant residue using silica gel column chromatography (hexane/ethyl acetate) to provide 196 mg of the title compound as a white foam.

### (Example 152-3) Synthesis of (S)-2-amino-3-[1-(4-t-butylbenzyl)-1H-benzimidazol-2-yl]-N-hydroxypropionamide (compound No. 134)

To 0°C was cooled 196 mg (0.127 mmol) of the compound obtained in Example 152-2, to which 4 ml of 4 mol/1 hydrogen chloride/dioxane was then added, followed by stirring at room temperature for 150 minutes. After the end of reaction, the solvent was distilled off, the solid was washed with hexane, and methanol was distilled off. The resultant solid was further washed with chloroform/hexane, and purified using silica gel column chromatography (chloroform/methanol) to provide 118 mg of the hydrochloride of the title compound as a pale yellow solid.
MS (Fab, Pos.): m/z = 367 [M+H]⁺
¹H-NMR (500 MHz, DMSO-d₆+D₂O) δ =1.24 (9H, s), 3.55-3.64 (2H, m), 4.33 (1H, t, J = 7.1 Hz), 7.17 (2H, d, J = 8.1 Hz), 7.37 (2H, d, J = 8.3 Hz), 7.40-7.48 (2H, m), 7.68 (1H, d, J = 7.0 Hz), 7.79 (2H, d, J = 7.0 Hz).

### (Example 153) Synthesis of (R)-2-amino-3-(1-ethyl-1H-indol-3-yl)-N-hydroxypropionamide (compound No. 135)

### (Example 153-1) Synthesis of (R)-2-t-butoxycarbonylamino-3-(1-ethyl-1H-indol-3-yl)propionic acid benzyl ester

In 20 ml of acetone was dissolved 1.00 g (2.54 mmol) of the compound obtained in Example 128-1, to which 1.22 ml of iodoethane was then added. Thereto was added 456 mg (1.40 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 608 mg of the title compound as a colorless, viscous oil.

### (Example 153-2) Synthesis of (R)-2-t-butoxycarbonylamino-3-(1-ethyl-1H-indol-3-yl)N-benzyloxypropionamide

In 30 ml of DME was dissolved 596 mg (1.41 mmol) of the compound obtained in Example 153-1, to which 120 mg of 10% palladium carbon was then added, followed by stirring for 4 hours under an atmosphere of hydrogen. After the end of reaction, the catalyst was removed by celite filtration, and the solvent was distilled off, followed by drying the residue under reduced pressure. In 6 ml of chloroform was dissolved 200 mg (0.603 mmol) thereof, to which 173 mg (0.903 mmol) of WSCI hydrochloride and 85.6 mg (0.633 mmol) of HOBt, and then 89.1 mg (0.724 mmol) of O-benzylhydroxylamine were subsequently added, followed by stirring at room temperature for 18 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 191 mg of the desired compound as a colorless foam.

### (Example 153-3) Synthesis of (R)-2-amino-N-hydroxy-3-(1-ethyl-1H-indol-3-yl)propionamide (compound No. 135)

In 8 ml of DME was dissolved 169 mg (0.386 mmol) of the compound obtained in Example 153-2, to which 80 mg of 10% palladium carbon was then added, followed by stirring at room temperature for 19 hours under an atmosphere of hydrogen. After the end of reaction, the catalyst was removed by celite filtration, and the solvent was distilled off, followed by drying the residue under reduced pressure. Thereto was added 3 ml of a 4 mol/l hydrogen chloride/dioxane solution under cooling with ice, followed by stirring under cooling with ice for 3 hours. After the end of reaction, the solvent was distilled off, and the solid was dissolved in methanol. This methanol layer was washed with hexane, and the methanol was distilled off. The resultant solid was further washed with chloroform/hexane and vacuum dried to provide 113 mg of the hydrochloride of the title compound as a light purple solid.
MS (Fab, Pos.): m/z = 248 [M+H]⁺
¹H-NMR (500 MHz, DMSO-d₆+D₂0): δ = 1.35 (3H, t, J = 7.3 Hz), 3.08 (1H, dd, J = 7.6, 14.4 Hz), 3.17 (1H, dd, J = 7.1, 14.4 Hz), 3.77 (1H, t, J = 7.3 Hz), 4.16 (2H, q, J = 7.3 Hz), 7.04-7.09 (1H, m), 7.15-7.20 (1H, m), 7.22 (1H, s), 7.45 (1H, d, J = 8.3 Hz), 7.64 (1H, d, J = 8.1 Hz).

### (Example 154) Synthesis of (R)-2-amino-3-(1-propyl-1H-indol-3-yl)-N-hydroxypropionamide (compound No. 136)

### (Example 154-1) Synthesis of (R)-2-t-butoxycarbonylamino-3-(1-propyl-1H-indol-3-yl)propionic acid benzyl ester

In 20 ml of acetone was dissolved 1.00 g (2.54 mmol) of the compound obtained in Example 128-1, to which 1.48 ml of iodopropane was then added. Thereto was added 456 mg (1.40 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 520 mg of the title compound as a colorless, viscous oil.

### (Example 154-2) Synthesis of (R)-2-t-butoxycarbonylamino-3-(1-propyl-1H-indol-3-yl)N-benzyloxypropionamide

In 26 ml of DME was dissolved 516 mg (1.18 mmol) of the compound obtained in Example 154-1, to which 103 mg of 10% palladium carbon was then added, followed by stirring for 4 hours under an atmosphere of hydrogen. After the end of reaction, the catalyst was removed by celite filtration, and the solvent was distilled off, followed by drying the residue under reduced pressure. In 6 ml of chloroform was dissolved 218 mg (0.628 mmol) thereof, to which 180 mg (0.940 mmol) of WSCI hydrochloride and 89.1 mg (0.659 mmol) of HOBt, and then 92.8 mg (0.754 mmol) of O-benzylhydroxylamine were subsequently added, followed by stirring at room temperature for 18 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 213 mg of the desired compound as a colorless foam.

### (Example 154-3) Synthesis of (R)-2-amino-N-hydroxy-3-(1-propyl-1H-indol-3-yl)propionamide (compound No. 136)

In 8 ml of DME was dissolved 186 mg (0.412 mmol) of the compound obtained in Example 154-2, to which 90 mg of 10% palladium carbon was then added, followed by stirring at room temperature for 16 hours under an atmosphere of hydrogen. After the end of reaction, the catalyst was removed by celite filtration, and the solvent was distilled off, followed by drying the residue under reduced pressure. Thereto was added 3.2 ml of a 4 mol/l hydrogen chloride/dioxane solution under cooling with ice, followed by stirring under cooling with ice for 3.5 hours. After the end of reaction, the solvent was distilled off, followed by azeotroping with toluene. The resultant solid was purified using silica gel column chromatography (chloroform/methanol) to provide 90.8 mg of the hydrochloride of the title compound as a light brown solid.
MS (Fab, Pos.): m/z = 262 [M+H]⁺
¹H-NMR (500 MHz, DMSO-d₆+D₂O): δ = 0.85 (3H, t, J = 7.6 Hz), 1.75 (2H, sext, J = 7.6 Hz), 3.09 (1H, dd, J = 7.1, 14.4 Hz), 3.16 (1H, dd, J = 6.8, 14.4 Hz), 3.77 (1H, t, J = 7.1 Hz), 4.01-4.12 (2H, m), 7.04-7.08 (1H, m), 7.14-7.19 (1H, m), 7.21 (1H, s), 7.45 (1H, d, J = 8.3 Hz), 7.63 (1H, d, J = 8.1 Hz).

### (Example 155) Synthesis of (R)-2-amino-3-(1-butyl-1H-indol-3-yl)-N-hydroxypropionamide (compound No. 137)

### (Example 155-1) Synthesis of (R)-2-t-butoxycarbonylamino-3-(1-butyl-1H-indol-3-yl)propionic acid benzyl ester

In 20 ml of acetone was dissolved 1.00 g (2.54 mmol) of the compound obtained in Example 128-1, to which 1.75 ml of iodopropane was then added. Thereto was added 456 mg (1.40 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 675 mg of the title compound as a colorless, viscous oil.

### (Example 155-2) Synthesis of (R)-2-t-butoxycarbonylamino-3-(1-butyl-1H-indol-3-yl)N-benzyloxypropionamide

In 33 ml of DME was dissolved 667 mg (1.48 mmol) of the compound obtained in Example 155-1, to which 133 mg of 10% palladium carbon was then added, followed by stirring for 4 hours under an atmosphere of hydrogen. After the end of reaction, the catalyst was removed by celite filtration, and the solvent was distilled off, followed by drying the residue under reduced pressure. In 6 ml of chloroform was dissolved 210 mg (0.582 mmol) thereof, to which 167 mg (0.872 mmol) of WSCI hydrochloride and 82.6 mg (0.611 mmol) of HOBt, and then 86.0 mg (0.698 mmol) of O-benzylhydroxylamine were subsequently added, followed by stirring at room temperature for 21 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 206 mg of the desired compound as a colorless foam.

### (Example 155-3) Synthesis of (R)-2-amino-N-hydroxy-3-(1-butyl-1H-indol-3-yl)propionamide (compound No. 137)

In 8 ml of DME was dissolved 182 mg (0.391 mmol) of the compound obtained in Example 155-2, to which 90 mg of 10% palladium carbon was then added, followed by stirring at room temperature for 16 hours under an atmosphere of hydrogen. After the end of reaction, the catalyst was removed by celite filtration, and the solvent was distilled off, followed by drying the residue under reduced pressure. Thereto was added 3 ml of a 4 mol/l hydrogen chloride/dioxane solution under cooling with ice, followed by stirring under cooling with ice for 4 hours. After the end of reaction, the solvent was distilled off, followed by azeotroping with toluene. The resultant solid was purified using silica gel column chromatography (chloroform/methanol) to provide 64.4 mg of the hydrochloride of the title compound as a light brown solid.
MS (Fab, Pos.): m/z = 276 [M+H]⁺
¹H-NMR (500 MHz, DMSO-d₆+D₂O): δ = 0.89 (3H, t, J = 7.3 Hz), 1.26 (2H, sext, J = 7.3 Hz), 1.72 (2H, qui, J = 7.6 Hz), 3.05 (1H, dd, J= 7.3, 14.4 Hz), 3.15 (1H, dd, J = 7.1, 14.4 Hz), 3.72 (1H, t, J = 7.3 Hz), 4.09-4.13 (2H, m), 7.03-7.07 (1H, m), 7.14-7.18 (1H, m), 7.19 (1H, s), 7.44 (1H, d, J = 8.3 Hz), 7.63 (1H, d, J = 7.8 Hz).

### (Example 156) Synthesis of (S)-2-amino-3-(1-ethyl-1H-indol-3-yl)-N-hydroxypropionamide (compound No. 138)

### (Example 156-1) Synthesis of (S)-2-t-butoxycarbonylamino-3-(1-ethyl-1H-indol-3-yl)propionic acid benzyl ester

In 10 ml of acetone was dissolved 500 mg (1.27 mmol) of commercial N^{α}-Boc-tryptophan benzyl ester, to which 0.61 ml of iodoethane was then added. Thereto was added 278 mg (0.853 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the resultant residue using silica gel column chromatography (hexane/ethyl acetate) to provide 366 mg of the title compound as a colorless, viscous oil.

### (Example 156-2) Synthesis of (S)-2-t-butoxycarbonylamino-3-(1-ethyl-1H-indol-3-yl)N-trityloxypropionamide

In 18 ml of DME was dissolved 366 mg (0.866 mmol) of the compound obtained in Example 156-1, to which 73.2 mg of 10% palladium carbon was then added, followed by stirring for 3 hours under an atmosphere of hydrogen. After the end of reaction, the catalyst was removed by celite filtration, and the solvent was distilled off, followed by drying the residue under reduced pressure. In 6 ml of chloroform was dissolved 209 mg (0.628 mmol) thereof, to which 180 mg (0.940 mmol) of WSCI hydrochloride and 84.8 mg (0.628 mmol) of HOBt, and then 182 mg (0.661 mmol) of O-tritylhydroxylamine were subsequently added, followed by stirring at room temperature for 18 hours. After the end of reaction, chloroform was added, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 275 mg of the desired compound as a colorless foam.

### (Example 156-3) Synthesis of (S)-2-amino-N-hydroxy-3-(1-ethyl-1H-indol-3-yl)propionamide (compound No. 138)

To 6 ml of a 4 mol/l hydrogen chloride/dioxane solution was added 234 mg (0.396 mmol) of the compound obtained in Example 156-2 under cooling with ice, followed by stirring under cooling with ice for 3 hours. After the end of reaction, the solvent was distilled off, and the solid was washed with hexane. It was purified using silica gel column chromatography (chloroform/methanol) to provide 94.3 mg of the hydrochloride of the title compound as a white solid.
MS (Fab, Pos.): m/z = 248 [M+H]⁺
¹H-NMR (500 MHz, DMSO-d₆+D₂0): δ =1.35 (3H, t, J = 7.3 Hz), 3.08 (1H, dd, J = 7.6, 14.4 Hz), 3.17 (1H, dd, J = 7.1, 14.4 Hz), 3.77 (1H, t, J = 7.3 Hz), 4.16 (2H, q, J = 7.3 Hz), 7.04-7.09 (1H, m), 7.15-7.20 (1H, m), 7.22 (1H, s), 7.45 (1H, d, J = 8.1 Hz), 7.64 (1H, d, J = 8.1 Hz).

### (Example 157) Synthesis of (S)-2-amino-3-(1-propyl-1H-indol-3-yl)-N-hydroxypropionamide (compound No. 139)

### (Example 157-1) Synthesis of (S)-2-t-butoxycarbonylamino-3-(1-propyl-1H-indol-3-yl)propionic acid benzyl ester

In 10 ml of acetone was dissolved 500 mg (1.27 mmol) of commercial N^{α}-Boc-tryptophan benzyl ester, to which 0.74 ml of iodoethane was then added. Thereto was added 278 mg (0.853 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 331 mg of the title compound as a colorless, viscous oil.

### (Example 157-2) Synthesis of (S)-2-t-butoxycarbonylamino-3-(1-propyl-1H-indol-3-yl)N-trityloxypropionamide

In 16.5 ml of DME was dissolved 331 mg (0.759 mmol) of the compound obtained in Example 157-1, to which 66.2 mg of 10% palladium carbon was then added, followed by stirring for 4 hours under an atmosphere of hydrogen. After the end of reaction, the catalyst was removed by celite filtration, and the solvent was distilled off, followed by drying the residue under reduced pressure. In 6 ml of chloroform was dissolved 206 mg (0.593 mmol) thereof, to which 170 mg (0.888 mmol) of WSCI hydrochloride and 80.1 mg (0.593 mmol) of HOBt, and then 171 mg (0.622 mmol) of O-tritylhydroxylamine were subsequently added, followed by stirring at room temperature for 18 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 226 mg of the desired compound as a colorless foam.

### (Example 157-3) Synthesis of (S)-2-amino-N-hydroxy-3-(1-propyl-1H-indol-3-yl)propionamide (compound No. 139)

To 4.4 ml of a 4 mol/l hydrogen chloride/dioxane solution was added 224 mg (0.370 mmol) of the compound obtained in Example 157-3 under cooling with ice, followed by stirring under cooling with ice for 3 hours. After the end of reaction, the solvent was distilled off, and the resultant solid was washed with hexane. It was purified using silica gel column chromatography (chloroform/methanol) to provide 92.8 mg of the hydrochloride of the title compound as a light orange solid.
MS (Fab, Pos.): m/z = 262 [M+H]⁺
¹H-NMR (500 MHz, DMSO-d₆+D₂O): δ = 0.85 (3H, t, J = 7.6 Hz), 1.75 (2H, sext, J = 7.6 Hz), 3.09 (1H, dd, J = 7.1, 14.4 Hz), 3.16 (1H, dd, J = 7.1, 14.4 Hz), 3.77 (1H, t, J = 7.1 Hz), 4.01-4.12 (2H, m), 7.04-7.08 (1H, m), 7.14-7.19 (1H, m), 7.21 (1H, s), 7.45 (1H, d, J = 8.3 Hz), 7.63 (1H, d, J = 7.8 Hz).

### (Example 158) Synthesis of (S)-2-amino-3-(1-butyl-1H-indol-3-yl)-N-hydroxypropionamide (compound No. 140)

### (Example 158-1) Synthesis of (S)-2-t-butoxycarbonylamino-3-(1-butyl-1H-indol-3-yl)propionic acid benzyl ester

In 10 ml of acetone was dissolved 500 mg (1.27 mmol) of commercial N^{α}-Boc-tryptophan benzyl ester, to which 0.88 ml of iodopropane was then added. Thereto was added 278 mg (0.853 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the resultant residue using silica gel column chromatography (hexane/ethyl acetate) to provide 234 mg of the title compound as a colorless, viscous oil.

### (Example 158-2) Synthesis of (S)-2-t-butoxycarbonylamino-3-(1-butyl-1H-indol-3-yl)N-benzyloxypropionamide

In 12 ml of DME was dissolved 234 mg (0.519 mmol) of the compound obtained in Example 158-1, to which 47 mg of 10% palladium carbon was then added, followed by stirring for 3 hours under an atmosphere of hydrogen. After the end of reaction, the catalyst was removed by celite filtration, and the solvent was distilled off, followed by drying the residue under reduced pressure. It was dissolved in 5.5 ml of chloroform, to which 147 mg (0.768 mmol) of WSCI hydrochloride and 69.1 mg (0.519 mmol) of HOBt, and then 148 mg (0.537 mmol) of O-tritylhydroxylamine were subsequently added, followed by stirring at room temperature for 19 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 193 mg of the desired compound as a colorless foam.

### (Example 158-3) Synthesis of (S)-2-amino-N-hydroxy-3-(1-butyl-1H-indol-3-yl)propionamide (compound No. 140)

To 3.8 ml of a 4 mol/l hydrogen chloride/dioxane solution was added 190 mg (0.308 mmol) of the compound obtained in Example 158-2 under cooling with ice, followed by stirring under cooling with ice for 3 hours. After the end of reaction, the solvent was distilled off, and the solid was washed with hexane. It was purified using silica gel column chromatography (chloroform/methanol) to provide 82.9 mg of the hydrochloride of the title compound as a light brown solid.
MS (Fab, Pos.): m/z = 276 [M+H]⁺
¹H-NMR (500 MHz, DMSO-d₆+D₂O): δ = 0.89 (3H, t, J = 7.3 Hz), 1.26 (2H, sext, J = 7.3Hz), 1.72 (2H, qui, J = 7.6Hz), 3.08 (1H, dd, J = 7.3, 14.4Hz), 3.16(1H, dd, J = 7.1, 14.4 Hz), 3.76 (1H, t, J = 7.3 Hz), 4.09-4.13 (2H, m), 7.04-7.08 (1H, m), 7.14-7.19 (1 H, m), 7.20 (1H, s), 7.45 (1 H, d, J = 8.3 Hz), 7.64 (1H, d, J = 7.8 Hz).

### (Example 159) Synthesis of (S)-2-amino-4-(1-pentyl-5,6-dimethyl-1H-benzimidazol-2-yl)-N-hydroxybutyramide (compound No. 141)

### (Example 159-1) Synthesis of (S)-2-t-butoxycarbonylamino-4-(2-pentylamino-4,5-dimethyl-phenylcarbamoyl)-butyric acid benzyl ester

In 10 ml of acetonitrile was dissolved 507 mg (1.11 mmol) of the compound obtained in Example 110-1, to which 0.128 ml (1.22 mmol) of valeraldehyde was then added, followed by stirring at room temperature for 3 hours. Thereto was then added 353 mg (1.67 mmol) of sodium triacetoxyborohydride and 15 ml of acetonitrile, followed by stirring at room temperature for 20 hours. After the end of reaction, the solvent was distilled off, and ethyl acetate was added to the residue, followed by washing with distilled water and a saturated saline solution. The organic layer was dried over anhydrous sodium sulfate. The solvent was distilled off, and the residue was purified using silica gel column chromatography (hexane/ethyl acetate) to provide 173 mg of the title compound as a pale yellow solid.

### (Example 159-2) Synthesis of 2-t-butoxycarbonylamino-4-(1-pentyl-5,6-dimethyl-1H-benzimidazol-2-yl)-butyric acid benzyl ester

In 5 ml of acetic acid was dissolved 173 mg of the compound obtained in Example 159-1, followed by stirring at 60°C for 2 hours. After the end of reaction, the solvent was distilled off before azeotroping with toluene, followed by redissolving the residue in toluene before washing a potassium carbonate aqueous solution and a saturated saline solution. It was dried over anhydrous sodium sulfate, and the solvent was distilled off, followed by purifying the resultant residue using silica gel column chromatography (hexane/ethyl acetate) to provide 144 mg of the title compound as a colorless foam.

### (Example 159-3) Synthesis of (S)-2-t-butoxycarbonylamino-4-(1-pentyl-5,6-dimethyl-1H-benzimidazol-2-yl)-N-trityloxybutyramide

In 7 ml of DME was dissolved 141 mg (0.278 mmol) of the compound obtained in Example 159-2, to which 28 ml of 10% palladium carbon was then added, followed by stirring at room temperature for 2.5 hours under an atmosphere of hydrogen. After the end of reaction, the catalyst was removed by celite filtration, and the solvent was distilled off. The residue was dissolved in 3 ml of chloroform, to which 71.8 mg (0.375 mmol) of WSCI hydrochloride, 33.8 mg (0.250 mmol) of HOBt, and 72.8 mg (0.263 mmol) of O-tritylhydroxylamine were then added, followed by stirring at room temperature for 16 hours. After the end of reaction, chloroform was added thereto, followed by washing with distilled water and a saturated saline solution before drying with anhydrous sodium sulfate. The solvent was distilled off, followed by purifying the resultant residue using silica gel column chromatography (hexane/ethyl acetate) to provide 123 mg of the title compound as a white foam.

### (Example 159-4) Synthesis of (S)-2-amino-4-(1-pentyl-5,6-dimethyl-1H-benzimidazol-2-yl-N-hydroxybutyramide (compound No. 141)

To 0°C was cooled 123 mg of the compound obtained in Example 159-3, to which 2.4 ml of a 4 mol/l hydrogen chloride/dioxane solution was then added, followed by stirring at room temperature for 4 hours. After the end of reaction, the solvent was distilled off, the solid was washed with hexane. It was purified using silica gel column chromatography (chloroform/methanol) to provide 59.6 mg of the hydrochloride of the title compound as a white solid.
MS (Fab, Pos.): m/z = 333 [M+H]⁺
¹H-NMR (500 MHz, DMSO-d₆+D₂O): δ = 0.87 (3H, t, J = 7.0 Hz), 1.28-1.38 (4H, m), 1.71-1.81 (2H, m), 2.25-2.35 (2H, m), 2.37 (3H, s), 2.40 (3H, s), 3.13-3.28 (2H, m), 3.86 (1H, t, J = 6.6 Hz), 4.31 (2H, t, J = 7.0 Hz), 7.53 (1H, s), 7.67 (1H, s).

### (Example 160) Synthesis of (S)-2-amino-4-[1-(2,2-dimethylpropyl)-5,6-dimethyl-1H-benzimidazol-2-yl)]-N-hydroxybutyramide (compound No. 142)

### (Example 160-1) Synthesis of (S)-2-t-butoxycarbonylamino-4-[2-(2,2-dimethylpropyl)amino-4,5-dimethyl-phenylcarbamoyl]-butyric acid benzyl ester

In 10 ml of acetonitrile was dissolved 454 mg (0.996 mmol) of the compound obtained in Example 110-1, to which 0.121 ml (1.10 mmol) of pivalaldehyde was then added, followed by stirring at room temperature for 3 hours. Thereto was then added 317 mg (1.45 mmol) of sodium triacetoxyborohydride and 15 ml of acetonitrile, followed by stirring at room temperature for 44 hours. After the end of reaction, the solvent was distilled off, and ethyl acetate was added to the residue, followed by washing with distilled water and a saturated saline solution. The organic layer was dried over anhydrous sodium sulfate. The solvent was distilled off, and the residue was purified using silica gel column chromatography (hexane/ethyl acetate) to provide 102 mg of the title compound as a colorless oil.

### (Example 160-2) Synthesis of 2-t-butoxycarbonylamino-4-[1-(2,2-dimethylpropyl)-5,6-dimethyl-1H-benzimidazol-2-yl]-butyric acid benzyl ester

In 3 ml of acetic acid was dissolved 102 mg of the compound obtained in Example 160-1, followed by stirring at 60°C for 2 hours. After the end of reaction, the solvent was distilled off before azeotroping with toluene, followed by redissolving the residue in toluene before washing a potassium carbonate aqueous solution and a saturated saline solution. It was dried over anhydrous sodium sulfate, and the solvent was distilled off, followed by purifying the resultant residue using silica gel column chromatography (hexane/ethyl acetate) to provide 74.4 mg of the title compound as a colorless oil.

### (Example 160-3) Synthesis of (S)-2-amino-4-[1-(2,2-dimethylpropyl)-5,6-dimethyl-1H-benzimidazol-2-yl]-N-hydroxybutyramide (compound No. 142)

In 3.6 ml of DME was dissolved 72.4 mg (0.143 mmol) of the compound obtained in Example 160-2, to which 14 ml of 10% palladium carbon was then added, followed by stirring at room temperature for 2.5 hours under an atmosphere of hydrogen. After the end of reaction, the catalyst was removed by celite filtration, and the solvent was distilled off. The residue was dissolved in 1.8 ml of chloroform, to which 39.1 mg (0.204 mmol) of WSCI hydrochloride, 18.4 mg (0.136 mmol) of HOBt, and 39.3 mg (0.142 mmol) of O-tritylhydroxylamine were then added, followed by stirring at room temperature for 24 hours. After the end of reaction, chloroform was added thereto, followed by washing with distilled water and a saturated saline solution before drying with anhydrous sodium sulfate. The solvent was distilled off, and the residue was purified using silica gel column chromatography (hexane/ethyl acetate). It was cooled to 0°C, to which 1.2 ml of a 4 mol/l hydrogen chloride/dioxane solution was then added, followed by stirring at room temperature for 2 hours. After the end of reaction, the solvent was distilled off, and the solid was washed with hexane. It was purified using silica gel column chromatography (chloroform/methanol) to provide 13.7 mg of the hydrochloride of the title compound as a white solid.
MS (Fab): m/z = 333 [M+H]⁺
¹H-NMR (500 MHz, DMSO-d₆+D₂O): δ = 1.02 (9H, s), 2.29-2.36 (2H, m), 2.38 (3H, s), 2.40 (3H, s) 3.17-3.34 (2H, m), 3.83 (1H, t, J = 6.8 Hz), 4.24 (2H, s), 7.55 (1H, s), 7.78 (1H, s).

### (Example 161) Synthesis of (S)-2-amino-3-(1-hexyl-1H-indol-3-yl)-N-hydroxypropionamide (compound No. 143)

### (Example 161-1) Synthesis of (S)-2-t-butoxycarbonylamino-3-(1-hexyl-1H-indol-3-yl)propionic acid benzyl ester

In 10 ml of acetone was dissolved 510 mg (1.29 mmol) of commercial N^{α}-Boc-tryptophan benzyl ester, to which 1.12 ml of iodohexane was then added. Thereto was added 278 mg (0.853 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 176 mg of the title compound as a colorless, viscous oil.

### (Example 161-2) Synthesis of (S)-2-t-butoxycarbonylamino-3-(1-hexyl-1H-indol-3-yl)N-benzyloxypropionamide

In 8 ml of DME was dissolved 176 mg (0.367 mmol) of the compound obtained in Example 161-1, to which 35 mg of 10% palladium carbon was then added, followed by stirring for 4 hours under an atmosphere of hydrogen. After the end of reaction, the catalyst was removed by celite filtration, and the solvent was distilled off, followed by drying under reduced pressure. It was dissolved in 3.8 ml of chloroform, to which 105 mg (0.550 mmol) of WSCI hydrochloride and 106 mg (0.385 mmol) of O-tritylhydroxylamine were then added, followed by stirring at room temperature for 18 hours. After the end of reaction, chloroform was added thereto, followed by washing with a 0.1 mol/l hydrochloric acid aqueous solution, a potassium carbonate aqueous solution, and a saturated saline solution. The organic layer was dried over anhydrous sodium sulfate, and the solvent was distilled off. The resultant residue was purified using silica gel column chromatography (hexane/ethyl acetate) to provide 147 mg of the desired compound as a colorless foam.

### (Example 161-3) Synthesis of (S)-2-amino-N-hydroxy-3-(1-hexyl-1H-indol-3-yl)propionamide (compound No. 143)

To 2.8 ml of a 4 mol/l hydrogen chloride/dioxane solution was added 147 mg (0.278 mmol) of the compound obtained in Example 161-2 under cooling with ice, followed by stirring under cooling with ice for 3 hours. After the end of reaction, the solvent was distilled off, and the solid was washed with hexane. It was purified using silica gel column chromatography (chloroform/methanol) to provide 67.2 mg of the hydrochloride of the title compound as a light brown solid.
MS (Fab): m/z = 304 [M+H]⁺
¹H-NMR (500 MHz, DMSO-d₆+D₂O): δ = 0.84 (3H, t, J = 6.8 Hz), 1.17-1.34 (6H, m), 1.67-1.78 (2H, m), 3.00 (1H, dd, J = 7.1, 14.4 Hz), 3.13 (1H, dd, J = 6.8, 14.4 Hz), 3.62-3.68 (1H, m), 4.10 (1H, t, J = 6.8 Hz), 7.05 (1H, t, J = 7.1 Hz), 7.15 (1H, t, J = 7.1 Hz), 7.19 (1H, s), 7.43 (1H, d, J = 8.3 Hz), 7.62 (1H, d, J = 7.8 Hz).

### (Example 162) Synthesis of (S)-2-amino-3-[1-(3-methylbutyl)-1H-indol-3-yl]-N-hydroxypropionamide (compound No. 144)

### (Example 162-1) Synthesis of (S)-2-t-butoxycarbonylamino-3-[1-(3-methylbutyl)-1H-indol-3-yl]propionic acid benzyl ester

In 10 ml of acetone was dissolved 530 mg (1.35 mmol) of commercial N^{α}-Boc-tryptophan benzyl ester, to which 1.00 ml of 3-methylbutylbromide was then added. Thereto was added 278 mg (0.853 mmol) of cesium carbonate, followed by stirring at 60°C for 2 days. After the end of reaction, the solvent was distilled off, followed by adding hexane and ethyl acetate to the residue before removing the insoluble matter by filtration. The solvent was distilled off, followed by purifying the residue using silica gel column chromatography (hexane/ethyl acetate) to provide 126 mg of the title compound as a colorless, viscous oil.

### (Example 162-2) Synthesis of (S)-2-t-butoxycarbonylamino-3-[1-(3-methylbutyl)-1H-indol-3-yl]N-benzyloxypropionamide

In 6 ml ofDME was dissolved 126 mg (0.367 mmol) of the compound obtained in Example 162-1, to which 25 mg of 10% palladium carbon was then added, followed by stirring for 4 hours under an atmosphere of hydrogen. After the end of reaction, the catalyst was removed by celite filtration, and the solvent was distilled off, followed by drying under reduced pressure. It was dissolved in 3 ml of chloroform, to which 78.1 mg (0.408 mmol) of WSCI hydrochloride and 78.6 mg (0.285 mmol) of O-tritylhydroxylamine were then added, followed by stirring at room temperature for 18 hours. After the end of reaction, chloroform was added thereto, followed by washing with a 0.1 mol/l hydrochloric acid aqueous solution, a potassium carbonate aqueous solution, and a saturated saline solution. The organic laver was dried over anhydrous sodium sulfate, and the solvent was distilled off. The resultant residue was purified using silica gel column chromatography (hexane/ethyl acetate) to provide 104 mg of the desired compound as a colorless foam.

### (Example 162-3) Synthesis of (S)-2-amino-N-hydroxy-3-[1-(3-methylbutyl)-1H-indol-3-yl] propionamide (compound No. 144)

To 2 ml of a 4 mol/l hydrogen chloride/dioxane solution was added 104 mg (0.165 mmol) of the compound obtained in Example 162-2 under cooling with ice, followed by stirring under cooling with ice for 3 hours. After the end of reaction, the solvent was distilled off, and the solid was washed with hexane. It was purified using silica gel column chromatography (chloroform/methanol) to provide 44.8 mg of the hydrochloride of the title compound as a light brown solid.

### MS (Fab): m/z = 290 [M+H]⁺

¹H-NMR (500 MHz, DMSO-d₆+D₂O): δ = 0.92 (6H, d, J = 6.6 Hz), 1.48-1.60 (1H, m), 1.63 (2H, q, J = 7.1 Hz), 3.03 (1H, dd, J = 7.3, 14.4 Hz), 3.15 (1H, dd, J = 6.8, 14.4 Hz), 3.63-3.71 (1H, m), 4.12 (1H, t, J = 6.6 Hz), 7.05 (1H, t, J = 7.1 Hz), 7.17 (1H, t, J = 7.1 Hz), 7.20 (1H, s), 7.43 (1H, d, J = 8.3 Hz), 7.62 (1H, d, J = 7.8 Hz).

## Claims

1. A compound of the following formula (I) or a pharmaceutically acceptable salt thereof 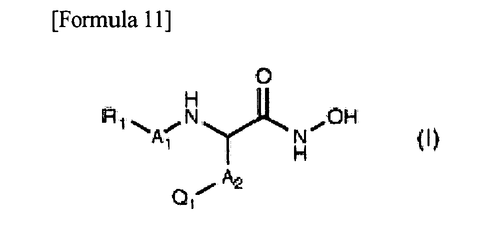 wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
Q₁ represents -Y₁-A₃-R₂, an aromatic ring compound group Q₂, a heteroaromatic ring compound group Q₃, or a saturated cyclic compound group Q₄;
Y₁ represents -O-, -S-, -NR₃-, -CONR₃-, -NR₃CO-, -NR₃COO-, -NR₃CONR₄-, - NR₃SO₂-, or -NR₃SO₂NR₄-;
A₃ represents a single bond or an alkylene group having 1 to 6 carbon atoms;
R₂ represents an alkyl group having 1 to 10 carbon atoms, an alkenyl group having 2 to 10 carbon atoms, an aryl group, a heteroaryl group, or a saturated heterocyclic group;
R₃ and R₄ each independently represent a hydrogen atom, an alkyl group, an alkenyl group, an aryl group, a heteroaryl group, a saturated heterocyclic group, or a group selected from the group consisting of an aryl or heteroaryl group and an alkylene group having 1 to 3 carbon atoms; R₂ and R₃ optionally bond together to form a ring;
Q₂ is a group of the following formula (II-a): 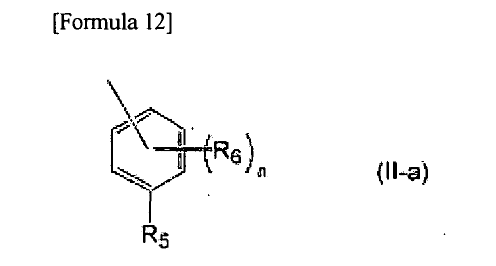 wherein R₅ represents a nitro group, a cyano group, or -Y₂-A₃-R₂;
n represents an integer of 0 to 4;
Y₂ represents a single bond, -O-, -S-, -NR₃-, -CONR₃-, -NR₃CO-, -NR₃COO-, - NR₃CONR₄-, -NR₃SO₂-, or -NR₃SO₂NR₄-;
R₆s are each independently optionally substituted at any carbon atom on the ring, each independently represent a halogen atom, an alkyl group, a nitro group, a cyano group, -OR₇, -COOR₇, or -CONR₇R₈, and optionally form a ring; and
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;
Q₃ represents a group selected from groups of the following formula (II-b): 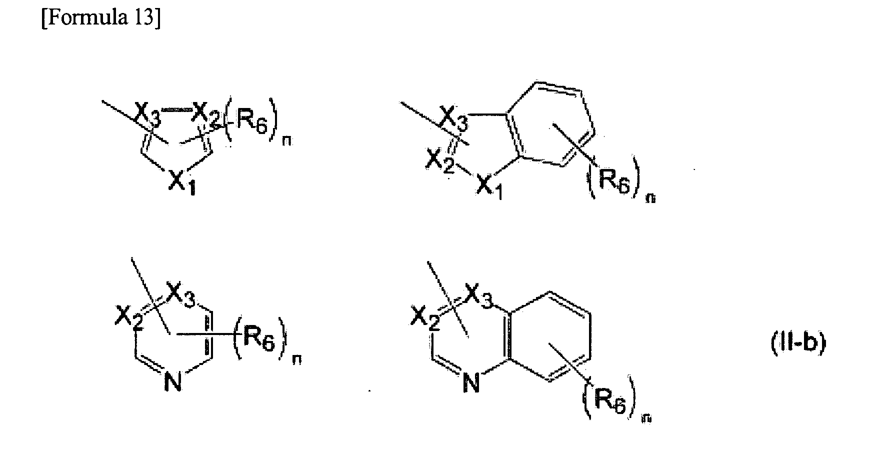 wherein X₁ represents -O- or -N(-Y₃-A₃-R₂)-; X₂ and X₃ each represent N or CH; Y₃ represents a single bond -CO- or -SO₂-; and
Q₄ represents a 3- to 10-membered hydrocarbon optionally substituted in any position or a cyclic compound containing 1 to 3 nitrogen, oxygen, or sulfur atoms;
provided that the following cases are excluded in which: R₁ is a hydrogen atom, A₁ is a single bond, A₂ is methylene or ethylene, Q₁ is Q₂, R₅ is Y₂-A₃-R₂, Y₂ is an oxygen atom, A₃ is methylene, and R₂ is phenyl;
R₁ is a hydrogen atom, A₁ is a single bond, A₂ is methylene, Q₁ is Y₁-A₃-R₂, Y₁ is S, and A₃ is ethylene;
R₁ is a hydrogen atom, A₁ is a single bond, A₂ is ethylene, Q₁ is Y₁-A₃-R₂, Y₁ is S, and A₃ is a single bond, and R₂ is ethyl; and
A₂ is methylene, Q₁ is -Y₁-A₃-R₂, Y₁ is NR₃CO, A₃ is ethylene, and R₂ is phenyl.

2. A compound of the following formula (III) or a pharmaceutically acceptable salt thereof: 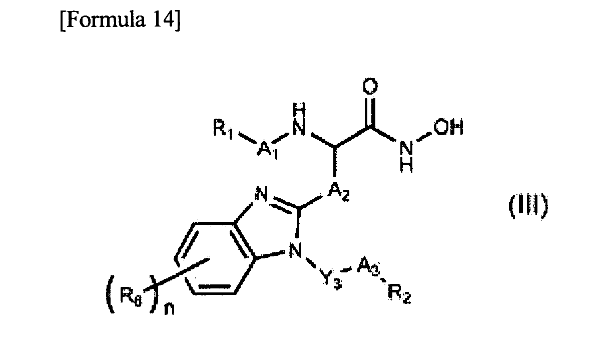 wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₆s are each independently optionally substituted at any carbon atom on the ring, each independently represent a halogen atom, an alkyl group, a nitro group, a cyano group, -OR₇, -COOR₇, or -CONR₇R₈, and optionally form a ring;
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;
n represents an integer of 0 to 4;
Y₃ represents a single bond, -CO- or -SO₂-;
A₃ represents a single bond or an alkylene group having 1 to 6 carbon atoms; and
R₂ represents an alkyl group having 1 to 10 carbon atoms, an alkenyl group having 2 to 10 carbon atoms, an aryl group, a heteroaryl group, or a saturated heterocyclic group.

3. The compound of the formula (III) or a pharmaceutically acceptable salt thereof according to claim 2, wherein A₂ represents methylene or ethylene; R₁ represents a hydrogen atom, an alkyl group, or an aryl group; A₁ represents a single bond, methylene, or ethylene; Y₃ represents a single bond; and R₆ represents a hydrogen atom, a halogen atom, or an alkyl group.

4. The compound of the formula (III) or a pharmaceutically acceptable salt thereof according to claim 3, wherein A₂ represents ethylene; A₁ represents a single bond; and R₁ represents a hydrogen atom.

5. The compound of the formula (III) or a pharmaceutically acceptable salt thereof according to claim 3, wherein R₁ is a hydrogen atom, A₁ is a single bond, A₂ is ethylene, n is 0, Y₃ and A₃ are each a single bond, and R₂ is a propyl group;
R₁ is a hydrogen atom, A₁ is a single bond, A₂ is ethylene, n is 0, Y₃ is a single bond, A₃ is ethylene, and R₂ is a phenyl group;
R₁ is a hydrogen atom, A₁ is a single bond, A₂ is ethylene, n is 0, Y₃ is a single bond, A₃ is methylene, and R₂ is a 4-nitrophenyl group;
R₁ is a hydrogen atom, A₁ is a single bond, A₂ is ethylene, n is 0, Y₃ and A₃ are each a single bond, and R₂ is a pentyl group;
R₁ is a hydrogen atom, A₁ is a single bond, A₂ is ethylene, n is 0, Y₃ is a single bond, A₃ is methylene, and R₂ is a 2-methoxyphenyl group;
R₁ is a hydrogen atom, A₁ is a single bond, A₂ is ethylene, n is 0, Y₃ is a single bond, A₃ is methylene, and R₂ is a pyridin-2-yl group;
R₁ is a hydrogen atom, A₁ is a single bond, A₂ is ethylene, n is 0, Y₃ and A₃ are each a single bond, and R₂ is a butyl group;
R₁ is a hydrogen atom, A₁ is a single bond, A₂ is ethylene, n is 0, Y₃ and A₃ are each a single bond, and R₂ is an octyl group;
R₁ is a hydrogen atom, A₁ is a single bond, A₂ is ethylene, n is 0, Y₃ is a single bond, A₃ is methylene, and R₂ is a cyclohexyl group;
R₁ is a hydrogen atom, A₁ is a single bond, A₂ is ethylene, n is 0, Y₃ and A₃ are each a single bond, and R₂ is a 2,2-dimethylpropyl group;
R₁ is a hydrogen atom, A₁ is a single bond, A₂ is ethylene, n is 0, Y₃ and A₃ are each a single bond, and R₂ is an isobutyl group;
R₁ is a hydrogen atom, A₁ is a single bond, A₂ is ethylene, n is 0, Y₃ is a single bond, A₃ is methylene, and R₂ is a 4-fluorophenyl group;
R₁ is a hydrogen atom, A₁ is a single bond, A₂ is ethylene, n is 0, Y₃ and A₃ are each a single bond, and R₂ is a t-butyl group;
R₁ is a hydrogen atom, A₁ is a single bond, A₂ is ethylene, n is 0, Y₃ and A₃ are each a single bond, and R₂ is a cyclohexyl group;
R₁ is a hydrogen atom, A₁ is a single bond, A₂ is ethylene, n is 0, Y₃ and A₃ are each a single bond, and R₂ is a tetrahydropyran-4-yl group;
R₁ is a hydrogen atom, A₁ is a single bond, A₂ is ethylene, n is 0, Y₃ and A₃ are each a single bond, and R₂ is a 1-methylpiperidin-4-yl group;
R₁ is a hydrogen atom, A₁ is a single bond, A₂ is ethylene, n is 0, Y₃ and A₃ are each a single bond, and R₂ is a 2-methylbutyl group;
R₁ is a hydrogen atom, A₁ is a single bond, A₂ is ethylene, n is 0, Y₃ and A₃ are each a single bond, and R₂ is a hexyl group;
R₁ is a hydrogen atom, A₁ is a single bond, A₂ is ethylene, n is 0, Y₃ and A₃ are each a single bond, and R₂ is a heptyl group;
R₁ is a hydrogen atom, A₁ is a single bond, A₂ is ethylene, n is 0, Y₃ is a single bond, A₃ is 2-methylpropylen-2-yl, and R₂ is a 4-t-butylphenyl group;
R₁ is a hydrogen atom, A₁ is a single bond, A₂ is ethylene, n is 0, Y₃ is a single bond, A₃ is methylene, and R₂ is a naphthalen-1-yl group;
R₁ is a hydrogen atom, A₁ is a single bond, A₂ is ethylene, n is 0, Y₃ is a single bond, A₃ is methylene, and R₂ is a 5-chlorothiophen-2-yl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a cyclohexyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-t-butylphenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-fluorophenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-methoxybenzyl group;
R₁ is a hydrogen atom, A₁ is a single bond, A₂ is ethylene, n is 0, Y₃ and A₃ are each a single bond, and R₂ is a methyl group;
R₁ is a propyl group, A₁ is a single bond, A₂ is ethylene, n is 0, Y₃ and A₃ are each a single bond, and R₂ is a propyl group;
R₁ is a cyclohexyl group, A₁ is methylene, A₂ is ethylene, n is 0, Y₃ and A₃ are each a single bond, and R₂ is a methyl group;
R₁ is a propyl group, A₁ is methylene, A₂ is ethylene, n is 0, Y₃ and A₃ are each a single bond, and R₂ is a methyl group;
R₁ is an octyl group, A₁ is methylene, A₂ is ethylene, n is 0, Y₃ and A₃ are each a single bond, and R₂ is a methyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a propyl group;
R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a propyl group;
R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a pentyl group;
R₁ is a cyclohexyl group, A₁ is methylene, A₂ is ethylene, n is 0, Y₃ and A₃ are each a single bond, and R₂ is a propyl group;
R₁ is a hydrogen atom, A₁ is a single bond, A₂ is ethylene, n is 0, Y₃ is a single bond, A₃ is methylene, and R₂ is a 2,3,5,6-tetrafluoro-4-methoxyphenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a cyclohexyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 3,4-difluorophenyl group;
R₁ is a hydrogen atom, A₁ is a single bond, A₂ is ethylene, n is 0, Y₃ and A₃ are each a single bond, and R₂ is a phenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a phenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 4-methylphenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 4-methoxyphenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 4-fluorophenyl group;
R₁ is a hydrogen atom, A₁ is a single bond, A₂ is ethylene, n is 0, Y₃ and A₃ are each a single bond, and R₂ is a 4-methylphenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 3-fluorophenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a 3-nitrophenyl group;
R₁ is a hydrogen atom, A₁ is a single bond, A₂ is ethylene, n is 2, R₆s are methyl groups present at the 5-and 6-positions, Y₃ is a single bond, A₃ is methylene, and R₂ is a 4-trifluoromethylphenyl group;
R₁ is a hydrogen atom, A₁ is a single bond, A₂ is ethylene, n is 2, R₆s are chlorine atoms present at the 5-and 6-positions, Y₃ and A₃ are each a single bond, and R₂ is a phenyl group;
R₁ is a hydrogen atom, A₁ is a single bond, A₂ is ethylene, n is 2, R₆s are chlorine atoms present at the 5-and 6-positions, Y₃ is a single bond, A₃ is methylene, and R₂ is a phenyl group;
R₁ is a hydrogen atom, A₁ is a single bond, A₂ is methylene, n is 0, Y₃ and A₃ are each a single bond, and R₂ is a pentyl group;
R₁ is a hydrogen atom, A₁ is a single bond, A₂ is methylene, n is 0, Y₃ and A₃ are each a single bond, and R₂ is a 4-methylphenyl group;
R₁ is a hydrogen atom, A₁ is a single bond, A₂ is methylene, n is 0, Y₃ is a single bond, A₃ is methylene, and R₂ is a 4-t-butylphenyl group;
R₁ is a hydrogen atom, A₁ is a single bond, A₂ is ethylene, n is 2, R₆s are methyl groups present at the 5-and 6-positions, Y₃ and A₃ are each a single bond, and R₂ is a pentyl group; or
R₁ is a hydrogen atom, A₁ is a single bond, A₂ is ethylene, n is 2, R₆s are methyl groups present at the 5-and 6-positions, Y₃ and A₃ are each a single bond, and R₂ is a 2,2-dimethylpropyl group.

6. A compound of the following formula (IV) or a pharmaceutically acceptable salt thereof: 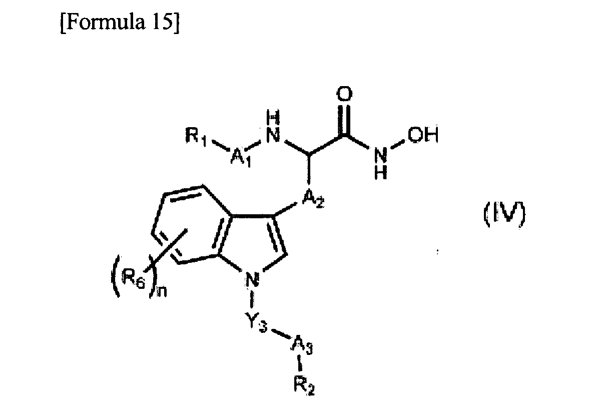 wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₆s are each independently optionally substituted at any carbon atom on the ring, each independently represent a halogen atom, an alkyl group, a nitro group, a cyano group, -OR₇, -COOR₇, or -CONR₇R₈, and optionally form a ring;
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms;
n represents an integer of 0 to 4; Y₃ represents a single bond -CO- or -SO₂-; and
A₃ represents a single bond or an alkylene group having 1 to 6 carbon atoms; and R₂ represents an alkyl group having 1 to 10 carbon atoms, an alkenyl group having 2 to 10 carbon atoms, an aryl group, a heteroaryl group, or a saturated heterocyclic group.

7. The compound of the formula (IV) or a pharmaceutically acceptable salt thereof according to claim 6, wherein R₆ represents a hydrogen atom.

8. The compound of the formula (IV) or a pharmaceutically acceptable salt thereof according to claim 7, wherein R₁ represents a hydrogen atom and A₁ represents a single bond.

9. The compound of the formula (IV) or a pharmaceutically acceptable salt thereof according to claim 8, wherein Y₃ represents a single bond.

10. The compound of the formula (IV) or a pharmaceutically acceptable salt thereof according to claim 6, wherein R₁ is a hydrogen atom, A₁ is a single bond, A₂ is methylene, n is 0, Y₃ is a single bond, A₃ is methylene, and R₂ is a 2,4-difluorophenyl group;
R₁ is a hydrogen atom, A₁ is a single bond, A₂ is methylene, n is 0, Y₃ is a single bond, A₃ is methylene, and R₂ is a 4-trifluorophenyl group;
R₁ is a hydrogen atom, A₁ is a single bond, A₂ is methylene, n is 0, Y₃ is a single bond, A₃ is methylene, and R₂ is a 4-nitrophenyl group;
R₁ is a hydrogen atom, A₁ is a single bond, A₂ is methylene, n is 0, Y₃ is a single bond, A₃ is ethylene, and R₂ is a phenyl group;
R₁ is a hydrogen atom, A₁ is a single bond, A₂ is methylene, n is 0, Y₃ is a single bond, A₃ is methylene, and R₂ is a 2,3,4,5,6-pentafluorophenyl group;
R₁ is a hydrogen atom, A₁ is a single bond, A₂ is methylene, n is 0, Y₃ and A₃ are each a single bond, and R₂ is a pentyl group;
R₁ is a hydrogen atom, A₁ is a single bond, A₂ is methylene, n is 0, Y₃ is a single bond, A₃ is methylene, and R₂ is a 4-t-butylphenyl group;
R₁ is a hydrogen atom, A₁ is a single bond, A₂ is methylene, n is 0, Y₃ is a single bond, A₃ is methylene, and R₂ is a 4-cyanophenyl group;
R₁ is a hydrogen atom, A₁ is a single bond, A₂ is methylene, n is 0, Y₃ is a single bond, A₃ is methylene, and R₂ is a 3,4-difluorophenyl group;
R₁ is a hydrogen atom, A₁ is a single bond, A₂ is methylene, n is 0, Y₃ and A₃ are each a single bond, and R₂ is a methyl group;
R₁ is a hydrogen atom, A₁ is a single bond, A₂ is methylene, n is 0, Y₃ and A₃ are each a single bond, and R₂ is an ethyl group;
R₁ is a hydrogen atom, A₁ is a single bond, A₂ is methylene, n is 0, Y₃ and A₃ are each a single bond, and R₂ is a propyl group;
R₁ is a hydrogen atom, A₁ is a single bond, A₂ is methylene, n is 0, Y₃ and A₃ are each a single bond, and R₂ is a butyl group;
R₁ is a hydrogen atom, A₁ is a single bond, A₂ is methylene, n is 0, Y₃ and A₃ are each a single bond, and R₂ is a hexyl group; or
R₁ is a hydrogen atom, A₁ is a single bond, A₂ is methylene, n is 0, Y₃ and A₃ are each a single bond, and R₂ is a 3-methylbutyl group.

11. A compound of the following formula (V) or a pharmaceutically acceptable salt thereof: 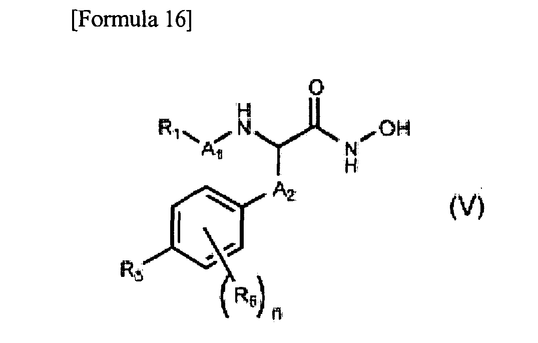 wherein R₁ represents a hydrogen atom, an alkyl group having 1 to 10 carbon atoms, an alkenyl group having 2 to 10 carbon atoms, an aryl group, a heteroaryl group, or a saturated heterocyclic group; A₁ and A₂ each independently represent a single bond or an alkylene group having 1 to 6 carbon atoms; R₅ represents a nitro group, a cyano group, or - Y₂-A₃-R₂; n represents an integer of 0 to 4; Y₂ represents a single bond, -O-, -S-, -NR₃-, - CONR₃-, -NR₃CO-, -NR₃COO-, -NR₃CONR₄-, -NR₃SO₂-, or -NR₃SO₂NR₄-;
R₆s are each independently optionally substituted at any carbon atom on the ring, each independently represent a halogen atom, an alkyl group, a nitro group, a cyano group, -OR₇, -COOR₇, or -CONR₇R₈, and optionally form a ring; and
R₇ and R₈ each independently represent an alkyl group, an alkenyl group, an aryl group, a heteroaryl group, a saturated heterocyclic group, or a group selected from the group consisting of an aryl or heteroaryl group and an alkylene group having 1 to 3 carbon atoms.

12. The compound of the formula (V) or a pharmaceutically acceptable salt thereof according to claim 11, wherein A₂ represents methylene and R₆ represents a hydrogen atom.

13. The compound of the formula (V) or a pharmaceutically acceptable salt thereof according to claim 12, wherein R₁ represents a hydrogen atom, A₁ represents a single bond, R₅ represents a nitro group, -NR₉R₁₀, or -OR₉, R₉ and R₁₀ each represent -Y₄-R₁₁, Y₄ represents a single bond, -CO-, -COO-, -CONR₁₂-, or SO₂- and R₁₁ and R₁₂ each independently represent an alkyl group having 1 to 10 carbon atoms, an aryl group, a heteroaryl group, a saturated heterocyclic group, or a group selected from the group consisting of an aryl or heteroaryl group and an alkylene group having 1 to 3 carbon atoms, provided that the following cases are excluded in which: R₅ represents -OR₉, R₉ represents -Y₄R₁₁, and Y₄ represents 0; or R₁₁ represents a benzyl group.

14. The compound of the formula (V) or a pharmaceutically acceptable salt thereof according to claim 12, wherein R₅ is a nitro group.

15. The compound of the formula (V) or a pharmaceutically acceptable salt thereof according to claim 11, wherein R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a methyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a 4-methylphenyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a butyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is a single bond, and R₂ is a t-butyl group;
R₁ is a hydrogen atom, A₁ is a single bond, A₂ is methylene, n is 0, R₅ is -Y₂-A₃-R₂, Y₂ is -NR₃CO-, R₃ is a hydrogen atom, A₃ is methylene, and R₂ is a 4-fluorophenyl group;
R₁ is a hydrogen atom, A₁ is a single bond, A₂ is methylene, n is 0, R₅ is -Y₂-A₃-R₂, Y₂ is -NR₃SO₂-, R₃ is a hydrogen atom, A₃ is a single bond, and R₂ is a 4-methylphenyl group;
R₁ is a hydrogen atom, A₁ is a single bond, A₂ is methylene, n is 0, R₅ is -Y₂-A₃-R₂, Y₂ is -NR₃CONR₄-, R₃ and R₄ are each a hydrogen atom, A₃ is a single bond, and R₂ is a 4-trifluoromethylphenyl group;
R₁ is a hydrogen atom, A₁ is a single bond, A₂ is methylene, n is 0, R₅ is -Y₂-A₃-R₂, Y₂ is -NR₃COO-, R₃ is a hydrogen atom, A₃ is a single bond, and R₂ is a methyl group;
R₁ is a hydrogen atom, A₁ is a single bond, A₂ is methylene, n is 0, R₅ is -Y₂-A₃-R₂, Y₂ is -NR₃CONR₄-, R₃ is a hydrogen atom, A₃ is a single bond, and R₂ and R₄ are each a methyl group;
R₁ is a hydrogen atom, A₁ is a single bond, A₂ is methylene, n is 0, R₅ is -Y₂-A₃-R₂, Y₂ is -NR₃-, A₃ is a single bond, and R₂ and R₃ are each a propyl group;
R₁ is a hydrogen atom, A₁ is a single bond, A₂ is methylene, n is 0, R₅ is -Y₂-A₃-R₂, Y₂ is -NR₃-, A₃ is methylene, R₂ is a hydrogen atom, and R₃ is a 4-methylphenyl group;
R₁ is a hydrogen atom, A₁ is a single bond, A₂ is methylene, n is 0, and R₅ is a nitro group;
R₁ is a phenyl group, A₁ is methylene, A₂ is methylene, n is 0, and R₅ is a nitro group;
R₁ is a pyridin-2-yl group, A₁ is methylene, A₂ is methylene, n is 0, and R₅ is a nitro group;
R₁ is a propyl group, A₁ is a single bond, A₂ is methylene, n is 0, and R₅ is a nitro group;
R₁ is a cyclohexyl group, A₁ is methylene, A₂ is methylene, n is 0, and R₅ is a nitro group;
R₁ is a 2-propyl group, A₁ is a single bond, A₂ is methylene, n is 0, and R₅ is a nitro group;
R₁ is a cyclohexyl group, A₁ is a single bond, A₂ is methylene, n is 0, and R₅ is a nitro group;
R₁ is a 1-methylpiperazin-4-yl group, A₁ is a single bond, A₂ is methylene, n is 0, and R₅ is a nitro group;
R₁ is a hydrogen atom, A₁ is a single bond, A₂ is methylene, n is 0, and R₅ is -Y₂₋A₃-R₂, Y₂ is -O-, A₃ is ethylene, and R₂ is a 4-phenyl group;
R₁ is a hydrogen atom, A₁ is a single bond, A₂ is methylene, n is 0, and R₅ is -Y₂₋A₃-R₂, Y₂ is -O-, A₃ is a single bond, and R₂ is a propyl group;
R₁ is a hydrogen atom, A₁ is a single bond, A₂ is methylene, n is 0, and R₅ is -Y₂₋A₃-R₂, Y₂ is -O-, A₃ is ethylene, and R₅ is a morpholin-4-yl group;
R₁ is a hydrogen atom, A₁ is a single bond, A₂ is methylene, n is 0, and R₅ is -Y₂₋A₃-R₂, Y₂ is -O-, A₃ is a single bond, and R₂ is a butyl group;
R₁ is a hydrogen atom, A₁ is a single bond, A₂ is methylene, n is 0, and R₅ is -Y₂₋A₃-R₂, Y₂ is -O-, A₃ is a single bond, and R₂ is a pentyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-fluorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a naphthalen-2-yl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2-chlorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-trifluoromethylphenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2,3,4,5,6-pentafluorophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-t-butylphenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2-biphenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 4-nitrophenyl group;
R₁ is a hydrogen atom, A₁ is a single bond, A₂ is methylene, n is 0, and R₅ is -Y₂₋A₃-R₂, Y₂ is -O-, A₃ is methylene, and R₂ is a 2,4-difluorophenyl group; or
R₁ is a hydrogen atom, A₁ is a single bond, A₂ is methylene, n is 0, and R₅ is -Y₂₋A₃-R₂, Y₂ is -O-, A₃ is methylene, and R₂ is a 4-cyanophenyl group.

16. A compound of the following formula (VI) or a pharmaceutically acceptable salt thereof: 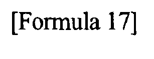 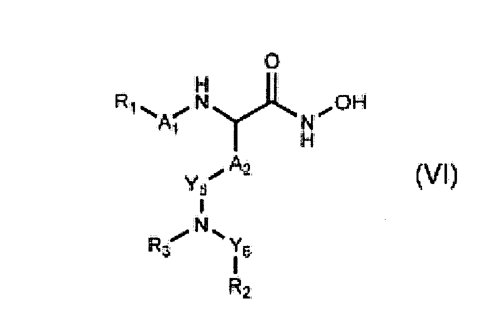 wherein R₁ represents a hydrogen atom, an alkyl group having 1 to 10 carbon atoms, an alkenyl group having 2 to 10 carbon atoms, an aryl group, a heteroaryl group, or a saturated heterocyclic group;
A₁ and A₂ each independently represent a single bond or an alkylene group having 1 to 6 carbon atoms;
R₂ represents an alkyl group having 1 to 10 carbon atoms, an alkenyl group having 2 to 10 carbon atoms, an aryl group, a heteroaryl group, or a saturated heterocyclic group;
Y₅ represents a single bond or a carbonyl group; Y₆ represents a single bond, -CO-, -COO-, -CONR₄-, or -SO₂-; and
R₃ and R₄ each represent a hydrogen atom, an alkyl group, an alkenyl group, an aryl group, a heteroaryl group, a saturated heterocyclic group, or a group selected from the group consisting of an aryl or heteroaryl group and an alkylene group having 1 to 3 carbon atoms.

17. The compound of the formula (VI) or a pharmaceutically acceptable salt thereof according to claim 16, wherein A₂ represents ethylene or propylene.

18. The compound of the formula (VI) or a pharmaceutically acceptable salt thereof according to claim 17, wherein R₁ represents a hydrogen atom and A₁ represents a single bond.

19. The compound of the formula (VI) or a pharmaceutically acceptable salt thereof according to claim 16, wherein R₁ is a hydrogen atom, A₁ is a single bond, A₂ is ethylene, Y₅ is a carbonyl group, Y₆ is a single bond, R₃ is a hydrogen atom, and R₂ is a naphthalen-1-ylmethyl group;
R₁ is a hydrogen atom, A₁ is a single bond, A₂ is ethylene, Y₅ is a carbonyl group, Y₆ is a single bond, R₃ is a hydrogen atom, and R₂ is a 4-t-butylphenyl group;
R₁ is a hydrogen atom, A₁ is a single bond, A₂ is ethylene, Y₅ is a carbonyl group, Y₆ is a single bond, R₃ is a hydrogen atom, and R₂ is a 4-fluorobenzyl group;
R₁ is a hydrogen atom, A₁ is a single bond, A₂ is ethylene, Y₅ is a carbonyl group, Y₆ is a single bond, R₃ is a hydrogen atom, and R₂ is a 4-t-butylbenzyl group;
R₁ is a hydrogen atom, A₁ is a single bond, A₂ is ethylene, Y₅ is a carbonyl group, Y₆ is a single bond, R₃ is a hydrogen atom, and R₂ is a cyclohexyl group;
R₁ is a hydrogen atom, A₁ is a single bond, A₂ is ethylene, Y₅ is a carbonyl group, Y₆ is a single bond, R₃ is a hydrogen atom, and R₂ is a 2,2-diphenylethyl group;
R₁ is a hydrogen atom, A₁ is a single bond, A₂ is ethylene, Y₅ is a carbonyl group, Y₆ is a single bond, R₃ is a hydrogen atom, and R₂ is a pyridin-2-ylmethyl group;
R₁ is a hydrogen atom, A₁ is a single bond, A₂ is ethylene, Y₅ is a carbonyl group, Y₆ is a single bond, R₃ is a methyl group, and R₂ is a benzyl group;
R₁ is a hydrogen atom, A₁ is a single bond, A₂ is ethylene, Y₅ is a carbonyl group, Y₆ is a single bond, R₃ is an ethyl group, and R₂ is an ethyl group;
R₁ is a hydrogen atom, A₁ is a single bond, A₂ is ethylene, Y₅ is a carbonyl group, Y₆ is a single bond, and R₂ and R₃ are each a pentylene group and form a ring;
R₁ is a hydrogen atom, A₁ is a single bond, A₂ is ethylene, Y₅ is a carbonyl group, Y₆ is a single bond, R₃ is a hydrogen atom, and R₂ is an adamantan-1-yl group;
R₁ is a hydrogen atom, A₁ is a single bond, A₂ is ethylene, Y₅ is a carbonyl group, Y₆ is a single bond, R₃ is a hydrogen atom, and R₂ is a benzyl group;
R₁ is a hydrogen atom, A₁ is a single bond, A₂ is propylene, Y₅ is a single bond, Y₆ is -CO-, R₃ is a hydrogen atom, and R₂ is a methyl group;
R₁ is a hydrogen atom, A₁ is a single bond, A₂ is propylene, Y₅ is a single bond, Y₆ is -CO-, R₃ is a hydrogen atom, and R₂ is a phenyl group;
R₁ is a hydrogen atom, A₁ is a single bond, A₂ is propylene, Y₅ is a single bond, Y₆ is -CO-, R₃ is a hydrogen atom, and R₂ is a 4-nitrophenyl group;
R₁ is a hydrogen atom, A₁ is a single bond, A₂ is propylene, Y₅ is a single bond, Y₆ is -CO-, R₃ is a hydrogen atom, and R₂ is a 4-nitrobenzyl group;
R₁ is a hydrogen atom, A₁ is a single bond, A₂ is propylene, Y₅ is a single bond, Y₆ is -CO-, R₃ is a hydrogen atom, and R₂ is a naphthalen-1-yl group;
R₁ is a hydrogen atom, A₁ is a single bond, A₂ is propylene, Y₅ is a single bond, Y₆ is -CO-, R₃ is a hydrogen atom, and R₂ is a naphthalen-2-yl group;
R₁ is a hydrogen atom, A₁ is a single bond, A₂ is propylene, Y₅ is a single bond, Y₆ is -CO-, R₃ is a hydrogen atom, and R₂ is a cyclohexylmethyl group;
R₁ is a hydrogen atom, A₁ is a single bond, A₂ is propylene, Y₅ is a single bond, Y₆ is -CO-, R₃ is a hydrogen atom, and R₂ is a 4-chlorophenyl group;
R₁ is a hydrogen atom, A₁ is a single bond, A₂ is propylene, Y₅ is a single bond, Y₆ is -CO-, R₃ is a hydrogen atom, and R₂ is a benzhydryl group;
R₁ is a hydrogen atom, A₁ is a single bond, A₂ is propylene, Y₅ is a single bond, Y₆ is -CO-, R₃ is a hydrogen atom, and R₂ is a 2-phenylbenzyl group;
R₁ is a hydrogen atom, A₁ is a single bond, A₂ is propylene, Y₅ is a single bond, Y₆ is -CO-, R₃ is a hydrogen atom, and R₂ is a 3,5-di-t-butylbenzyl group;
R₁ is a hydrogen atom, A₁ is a single bond, A₂ is propylene, Y₅ is a single bond, Y₆ is -CO-, R₃ is a hydrogen atom, and R₂ is a t-butyl group;
R₁ is a hydrogen atom, A₁ is a single bond, A₂ is propylene, Y₅ is a single bond, Y₆ is a single bond, R₃ is a propyl group, and R₂ is a propyl group;
R₁ is a hydrogen atom, A₁ is a single bond, A₂ is propylene, Y₅ is a single bond, Y₆ is a single bond, R₃ is a pentyl group, and R₂ is a pentyl group;
R₁ is a hydrogen atom, A₁ is a single bond, A₂ is propylene, Y₅ is a single bond, Y₆ is a single bond, R₃ is a hydrogen atom, and R₂ is a cyclohexyl group; or
R₁ is a hydrogen atom, A₁ is a single bond, A₂ is propylene, Y₅ is a single bond, Y₆ is a single bond, R₃ is a 4-methylbenzyl group, and R₂ is a 4-methylbenzyl group.

20. An AGE generation inhibitor comprising a compound of the following formula (VII) or a pharmaceutically acceptable salt thereof: 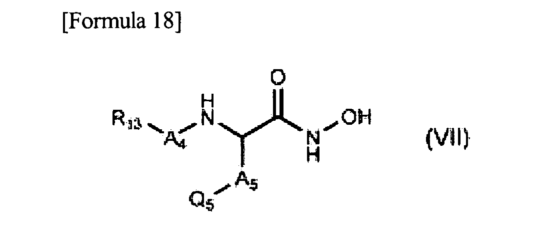 wherein R₁₃ represents an alkyl group having 1 to 10 carbon atoms, an aryl group, a heteroaryl group, or a saturated heterocyclic group;
A₄ and A₅ each independently represent a single bond or an alkylene group having 1 to 6 carbon atoms;
Q₅ represents -Y₇-A₆-R₁₄, an aromatic ring compound group Q₆, a heteroaromatic ring compound group Q₇, or a saturated cyclic compound group Q₈;
Y₇ represents a single bond, -O-, -S-, -NR₁₅-, -CONR₁₅-, -NR₁₅CO-, -NR₁₅COO-, - NR₁₅CONR₁₆-, -NR₁₅SO₂-, or -NR₁₅SO₂NR₁₆-;
A₆ represents a single bond or an alkylene group having 1 to 6 carbon atoms;
R₁₄ represents a hydrogen atom, an alkyl group having 1 to 10 carbon atoms, an aryl group, a heteroaryl group, or a saturated heterocyclic group;
R₁₅ and R₁₆ each represent an alkyl group having 1 to 6 carbon atoms , an aryl group, a heteroaryl group, a saturated heterocyclic group, or a group selected from the group consisting of an aryl or heteroaryl group and an alkylene group having 1 to 3 carbon atoms; R₁₄ and R₁₅ optionally bond together to form a ring;
Q₆ is a group of the following formula (VIII-a): 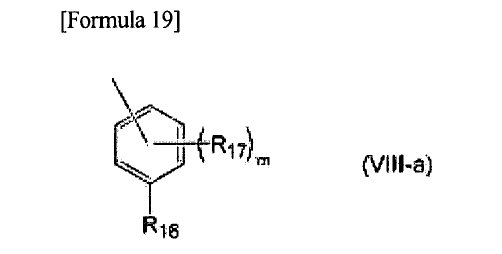 wherein R₁₆ represents a hydrogen atom, a halogen atom, a nitro group, a cyano group, or - Y₇-A₆-R₁₄;
m represents an integer of 0 to 4;
R₁₇ may be each independently substituted at any atom on the ring, each independently represents a halogen atom, an alkyl group, a nitro group, a cyano group, - OR₁₈, -COOR₁₈, or -CONR₁₈R₁₉, and optionally form a ring; and
R₁₈ and R₁₉ each represent an alkyl group having 1 to 6 carbon atoms, an aryl group, a heteroaryl group, a saturated heterocyclic group;
Q₇ is any of groups of the following formula (VIII-b): 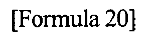 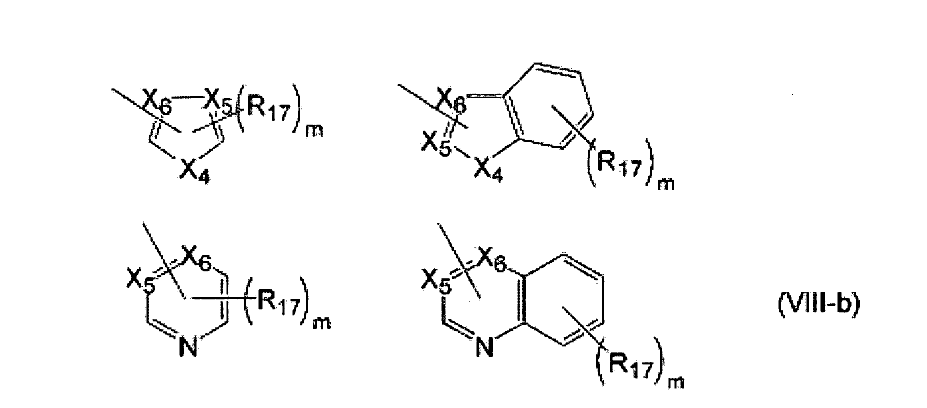 wherein X₄ represents -O-, -S-, or -N(-Y₇-A₆-R)₁₄)-; X₅ and X₆ each represent N or CH; and
Q₈ represents a 3- to 10-membered hydrocarbon optionally substituted in any position or a cyclic compound group which can contain 1 to 3 nitrogen, oxygen, and/or sulfur atoms.

21. A medicinal composition comprising the AGE generation inhibitor according to claim 20.

22. A food additive composition comprising the AGE generation inhibitor according to claim 20.

23. A cosmetic additive composition comprising the AGE generation inhibitor according to claim 20.

24. A cosmetic comprising the AGE generation inhibitor according to claim 20.
